# EUROPEAN PATENT SPECIFICATION

(11) **EP 4 061 815 B1**
(45) Date of publication and mention of the grant of the patent: **20.03.2024**
(21) Application number: 20803868.7
(22) Date of filing: 16.11.2020
(51) Int. Cl.: C07D 487/04, A01N 43/54, A01N 43/90

(54) **PYRIMIDONE DERIVATIVES CONTAINING TWO FUSED BICYCLIC RINGS**
PYRIMIDON-DERIVATE MIT ZWEI KONDENSIERTEN BICYCLISCHEN RINGEN
DÉRIVÉS DE PYRIMIDINONE CONTENANT DEUX SUBSTITUANTS BICYCLIQUES FUSIONNÉS

(30) Priority: 22.11.2019 EP 19210836
(43) Date of publication of application: 28.09.2022
(73) Proprietor: BASF SE, 67056 Ludwigshafen am Rhein (DE)
(72) Inventor: VON DEYN, Wolfgang, 67056 Ludwigshafen (DE); SHAIKH, Rizwan Shabbir, Mumbai 400705 (IN); ADISECHAN, Ashokkumar, Mumbai 400705 (IN)
(74) Representative: BASF IP Association
(86) International application number: PCT/EP2020/082186
(87) International publication number: WO 2021/099240

(56) References cited:
- EP-A1- 3 257 853
- WO-A1-2017/167832
- WO-A1-2018/206479

## Description

The invention relates to compounds of formula (I) or an agrochemically or verterinarily acceptable salt, stereoisomer, tautomer, or N-oxide thereof 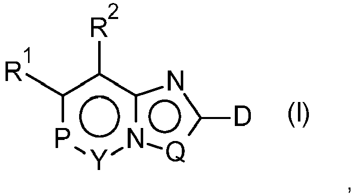 wherein the variables are as defined below. The invention also relates to the use of compounds of formula (I) as an agrochemical pesticide; to pesticidal mixtures comprising a compound of formula (I) and another pesticidal ingredient; to a non-therapeutic method for combating or controlling invertebrate pests, which method comprises contacting said pest or its food supply, habitat or breeding grounds with a pesticidally effective amount of at least one compound of the formula (I) the pesticidal mixture; to a method for protecting growing plants from attack or infestation by invertebrate pests, which method comprises contacting a plant, or soil or water in which the plant is growing, with a pesticidally effective amount of at least one compound of the formula (I) or the pesticidal mixture; and to seeds comprising a compound of the formula (I) or the pesticidal composition in an amount of from 0.1 g to 10 kg per 100 kg of seeds; to a use of a compound of the formula (I) or of the pesticidal compositions, for protecting growing plants from attack or infestation by invertebrate pests; and to a non-therapeutic method for treating or protecting an animal from infestation or infection by invertebrate pests which comprises bringing the animal in contact with a pesticidally effective amount of a compound of the formula (I).

Invertebrate pests and in particular insects, arachnids and nematodes destroy growing and harvested crops and attack wooden dwelling and commercial structures, thereby causing large economic loss to the food supply and to property. Accordingly, there is an ongoing need for new agents for combating invertebrate pests.

EP3257853A1 discloses condensed heterocyclic compounds and their use as pesticides, which compounds have two fused bicyclic moieties. WO2017/167832A1 discloses bicyclic pyrimidone compounds and their pesticidal activity. WO2018/206479 describes bicyclic imidazopyrimidone compounds substituted with a phenyl or 5-or 6-membered heteroaryl and their pesticidal activity.

Due to the ability of target pests to develop resistance to pesticidally-active agents, there is an ongoing need to identify further compounds, which are suitable for combating invertebrate pests such as insects, arachnids and nematodes. Furthermore, there is a need for new compounds having a high pesticidal activity and showing a broad activity spectrum against a large number of different invertebrate pests, especially against difficult to control insects, arachnids and nematodes.

It is therefore an object of the present invention to identify and provide compounds, which exhibit a high pesticidal activity and have a broad activity spectrum against invertebrate pests.

It has been found that these objects can be achieved by substituted bicyclic compounds of formula (I), as depicted and defined below, including their stereoisomers, their salts, in particular their agriculturally or veterinarily acceptable salts, their tautomers and their N-oxides.

In a first aspect, the present invention relates to a compound of formula (I), 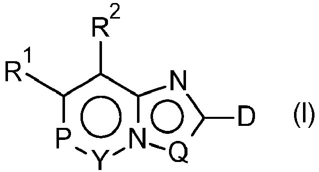 wherein
the circle in ring represents that ring is fully unsaturated;
- Y: is C=X, wherein X is O or S;
- P: is N(R³) or C(R⁴);
- Q: is N(R⁵) or C(R⁶);
- R¹: is H, halogen, C₁-C₆-alkyl, C₂-C₆-alkenyl, C₂-C₆-alkynyl, C₁-C₆-alkoxy, C₁-C₆-alkoxy-C₁-C₆-alkyl, C₃-C₆-cycloalkyl, C₃-C₆-cycloalkoxy, C₁-C₆-sulfenyl, C₁-C₆-sulfinyl, or C₁-C₆-sulfonyl, which groups are unsubstituted or halogenated;
- R³, R⁵: are independently C₁-C₆-alkyl, C₁-C₆-alkoxy, C₂-C₆-alkenyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkyl-C₁-C₄-alkyl, or C₃-C₆-cycloalkoxy-C₁-C₄-alkyl, which are unsubstituted or halogenated; C(=O)OR^{A}, NR^{B}R^{C}, C₁-C₆-alkylen-NR^{B}R^{C}, O-C₁-C₆-alkylen-NR^{B}R^{C}, C₁₋C₆-alkylen-CN, NH-C₁-C₆-alkylen-NR^{B}R^{C}, C(=O)NR^{B}R^{C}, C(=O)R^{D}, C(=S)R^{D}, SO₂NR^{B}R^{C}, S(=O)ₙR^{E}; phenyl or benzyl, wherein the phenyl ring is unsubstituted or substituted with one or more, same or different substituents R^{F};
- R², R⁴, R⁶: are independently H, halogen, N₃, CN, NO₂, SCN, SF₅; C₁-C₆-alkyl, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxyx-C₁-C₄-alkyl, which groups are unsubstituted or substituted with halogen, C(=O)OR^{A}, NR^{B}R^{C}, NOR^{A}, ONR^{B}R^{C}, C₁-C₆-alkylen-NR^{B}R^{C}, O-C₁-C₆-alkylen-NR^{B}R^{C}, C₁-C₆-alkylen-CN, NH-C₁-C₆-alkylen-NR^{B}R^{C}, C(=O)NR^{B}R^{C}, C(=O)R^{D}, C(=S)R^{D}, SO₂NR^{B}R^{C}, S(=O)ₙR^{E}; phenyl or benzyl, wherein the phenyl ring is unsubstituted or substituted with one or more, same or different substituents R^{F};
- D: is a fused bicyclic ring of the following formula 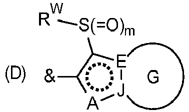
wherein the "&"-symbol signifies the connection to the remainder of formula (I), wherein the dotted circle in the 5-membered ring means that the 5-membered ring may be saturated, partially unsaturated, or fully unsaturated; and wherein
- A: is N, S, O, CR⁷, or NR⁸;
- E, J: are independently C or N, wherein at least one of the variables selected from E and J is C;
- G: is a 5- or 6-membered saturated, partially unsaturated, or fully unsaturated carbo- or heterocycle, which carbo- or heterocycle includes the atoms E and J as ring members and is unsubstituted, or substituted with one or more, same or different substituents R⁹, and wherein said heterocycle comprises no, one or more, same or different heteroatoms O, N, or S in addition to those that may be present as ring members E and J;
R⁷ is H, halogen, OH, CN, NC, NO₂, N₃, SCN, NCS, NCO, SF₅; C₁-C₆-alkyl, C₃-C₆-cycloalkyl, C₂-C₆-alkenyl, C₃-C₆-cycloalkenyl, C₂-C₆-alkynyl, which groups are unsubstituted, or substituted with one or more, same or different substituents R^{G};
a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents R^{H}, and wherein said N- and S-atoms are independently oxidized, or non-oxidized; phenyl, which is unsubstituted, or substituted with one or more, same or different substituents R^{J};
OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T};

R⁸ is H, halogen, CN;
C₁-C₆-alkyl, C₃-C₆-cycloalkyl, C₂-C₆-alkenyl, C₃-C₆-cycloalkenyl, C₂-C₆-alkynyl, which groups are unsubstituted, or substituted with one or more, same or different substituents R^{G};
a 3- to 12-membered saturated, partially unsaturated, or fully unsaturated heterocyclic ring or ring system, wherein said heterocyclic ring or ring system comprises one or more, same or different heteroatoms O, N, or S, and is
unsubstituted, or substituted with one or more, same or different substituents R^{H}, and wherein said N- and S-atoms are independently oxidized, or non-oxidized; phenyl, which is unsubstituted, or substituted with one or more, same or different substituents R^{J};
OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T};
each R⁹ is independently H, halogen, OH, CN, NC, NO₂, N₃, SCN, NCS, NCO, SF₅; C₁-C₆-alkyl, C₃-C₆-cycloalkyl, C₂-C₆-alkenyl, C₃-C₆-cycloalkenyl, C₂-C₆-alkynyl, C₃-C₆-cycloalkyl-C₁-C₃-alkyl, which groups are unsubstituted, or substituted with one or more, same or different substituents R^{G};
a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents R^{H}, and wherein said N- and S-atoms are independently oxidized, or non-oxidized; phenyl, which is unsubstituted, or substituted with one or more, same or different substituents R^{J};
OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T};
or two substituents R⁹ form, together with the ring members of ring G to which they are bound, a 5- or 6- membered saturated, partially unsaturated, or fully unsaturated carbo- or heterocycle, which carbo- or heterocycle is unsubstituted, or substituted with one or more, same or different substituents R^{J}, and wherein said heterocycle comprises one or more, same or different heteroatoms O, N, or S;

each R^{A} is independently H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; C₁-C₆-alkylen-NR^{b}R^{c}, C₁-C₆-alkylen-CN; or phenyl or benzyl, wherein the phenyl ring is unsubstituted or substituted with one or more, same or different substitutents R^{F};
each R^{B} is independently H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; C₁-C₆-alkylen-CN;
phenyl and benzyl, which groups are unsubstituted or substituted with one or more, same or different substituents R^{F};

each R^{C} is independently H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; C₁-C₆-alkylen-CN; or phenyl or benzyl, wherein the phenyl ring is unsubstituted or substituted with one or more, same or different substituents R^{F};
each moiety NR^{B}R^{C} may also form an N-bound, saturated 5- to 8-membered heterocycle, which in addition to the nitrogen atom may have 1 or 2 further heteroatoms or heteroatom moieties selected from O, S(=O)ₘ and N-R', wherein R' is H or C₁-C₆-alkyl and wherein the N-bound heterocycle is unsubstituted or substituted with one or more, same or different substituents selected from halogen, C₁-C₄-alkyl, C₁-C₄-haloalkyl, C₁-C₄-alkoxy and C₁-C₄-haloalkoxy;

each R^{B} is independently H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; phenyl or benzyl, wherein the phenyl ring is unsubstituted or substituted with one or more, same or different substituents R^{F};
each R^{E} is indepentently C₁-C₆-alkyl, C₃-C₆-cycloalkyl, C₃-C₆-cycloalkyl-C₁-C₄-alkyl, which are unsubstituted or substituted with halogen; or phenyl or benzyl, wherein the phenyl ring is unsubstituted or substituted with one or more, same or different substituents R^{F};
each R^{F} is independently halogen, N₃, OH, CN, NO₂, SCN, SF₅; C₁-C₆ alkyl, C₁-C₆ alkoxy, C₂-C₆ alkenyl, C₂-C₆-alkynyl, C₁-C₆-alkoxy-C₁-C₄-alkyl, C₁-C₆ alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxy-C₁-C₄-alkyl, which groups are unsubstituted or substituted with halogen;
each R^{G} is independently halogen, OH, CN, NC, NO₂; C₁-C₆-alkyl, C₃-C₆-cycloalkyl, C₃-C₆-cycloalkenyl, which groups are unsubstituted, or substituted with one or more, same or different substituents selected from halogen, OH, CN, C₁-C₃-alkoxy, C₁-C₃-haloalkoxy, and C₁-C₃-alkyl-carbonyl;
a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents selected from halogen, OH, CN, C₁-C₃-alkoxy, C₁-C₃-haloalkoxy, and C₁-C₃-alkyl-carbonyl, and wherein said N- and S-atoms are independently oxidized, or non-oxidized;
phenyl, which is unsubstituted, or substituted with one or more, same or different substituents selected from halogen, OH, CN, NO₂, C₁-C₃-alkyl, C₁-C₃-haloalkyl, C₁-C₃-alkoxy, C₁-C₃-haloalkoxy, and C₁-C₃-alkyl-carbonyl; OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T};

each R^{H} is independently halogen, CN, NC, NO₂, SCN, NCS, NCO; C₁-C₆-alkyl, C₃-C₆-cycloalkyl, C₃-C₆-cycloalkenyl, which groups are unsubstituted, or substituted with one or more, same or different substituents selected from halogen, OH, CN, C₁-C₁₀-alkoxy, C₁-C₃-haloalkoxy, and C₁-C₃-alkyl-carbonyl; phenyl, which is unsubstituted, or substituted with one or more, same or different substituents selected from halogen, OH, CN, NO₂, C₁-C₃-alkyl, C₁-C₃-haloalkyl, OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T}; or two geminal substituents R^{H} form together with the atom to which they are bound a group =O, =S, or =NR^{L}.
each R^{J} is independently halogen, CN, NC, NO₂, SCN, NCS, NCO; C₁-C₆-alkyl, C₃-C₆-cycloalkyl, C₃-C₆-cycloalkenyl, which groups are unsubstituted, or substituted with one or more, same or different substituents selected from halogen, OH, CN, C₁-C₁₀-alkoxy, C₁-C₃-haloalkoxy, and C₁-C₃-alkyl-carbonyl; phenyl, which is unsubstituted, or substituted with one or more, same or different substituents selected from halogen, OH, CN, NO₂, C₁-C₃-alkyl, C₁-C₃-haloalkyl, OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T};
each R^{K} is independently H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with one or more, same or different substituents selected from halogen, CN, NR^{M}R^{N}; C(=O)NR^{M}R^{N}, C(=O)R^{T}; or phenyl or benzyl, wherein the phenyl ring is unsubstituted or substituted with one or more, same or different substitutents R^{X};
each R^{L} is independently H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; C1-C₆-alkylen-CN; phenyl and benzyl, which groups are unsubstituted or substituted with one or more, same or different substituents R^{X};
each R^{M}, R^{R} is independently H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; C1-C₆-alkylen-CN; or phenyl or benzyl, wherein the phenyl ring is unsubstituted or substituted with one or more, same or different substituents R^{X};
each moiety NR^{M}R^{R}, NR^{L}R^{M} may also form an N-bound, saturated 5- to 8-membered heterocycle, which in addition to the nitrogen atom may have 1 or 2 further heteroatoms or heteroatom moieties selected from O, S(=O)ₘ and N-R', wherein R' is H or C₁-C₆-alkyl and wherein the N-bound heterocycle is unsubstituted or substituted with one or more, same or different substituents selected from halogen, C₁-C₄-alkyl, C₁-C₄-haloalkyl, C₁-C₄-alkoxy and C₁-C₄-haloalkoxy;
each R^{N} is independently H, halogen, CN, NO₂, SCN;
C₁-C₁₀-alkyl, C₃-C₈-cycloalkyl, C₂-C₆-alkenyl, C₃-C₆-cycloalkenyl, C₂-C₆-alkynyl, which groups are unsubstituted, or substituted with one or more, same or different substituents selected from halogen, C₁-C₆-alkyl, C₁-C₆-alkoxy, C₁-C₆-haloalkyl, and C₁-C₆-haloalkoxy;
a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents selected from halogen, C₁-C₃-alkyl, C₁-C₃-alkoxy, C₁-C₃-haloalkyl, and C₁-C₃-haloalkoxy, and wherein said N- and S-atoms are independently oxidized, or non-oxidized;
phenyl, which is unsubstituted, or substituted with one or more, same or different substituents selected from halogen, C₁-C₃-alkyl, C₁-C₃-alkoxy, C₁-C₃-haloalkyl, and C₁-C₃-haloalkoxy;

each R^{O} is independently H, C₁-C₄-alkyl, C₁-C₆-cycloalkyl, C₁-C₂-alkoxy-C₁-C₂-alkyl, phenyl, or benzyl;
each R^{P} is independently H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; phenyl or benzyl, wherein the phenyl ring is unsubstituted or substituted with one or more, same or different substituents R^{X};
each R^{S}, R^{T} is independently H, C₁-C₆-alkyl, C₁-C₆-haloalkyl, C₁-C₆-alkoxy, C₁-C₄-alkoxy-C₁-C₄-alkyl, C₃-C₆-cycloalkyl, C₃-C₆-halocycloalkyl, C₁-C₄-haloalkoxy-C₁-C₄-alkyl, or phenyl;
each R^{V} is indepentently C₁-C₆-alkyl, C₃-C₆-cycloalkyl, C₃-C₆-cycloalkyl-C₁-C₄-alkyl, which are unsubstituted or substituted with halogen; or phenyl or benzyl, wherein the phenyl ring is unsubstituted or substituted with R^{X};
each R^{W} is C₁-C₆-alkyl, C₃-C₆-cycloalkyl, C₃-C₆-cycloalkyl-C₁-C₄-alkyl, which are unsubstituted or substituted with halogen; or phenyl or benzyl, wherein the phenyl ring is unsubstituted or substituted with one or more, same or different substituents R^{X};
each R^{X} is independently halogen, N₃, OH, CN, NO₂, SCN, SF₅;
C₁-C₆ alkyl, C₁-C₆ alkoxy, C₂-C₆ alkenyl, C₂-C₆ alkynyl, C₁-C₆ alkoxy-C₁-C₄ alkyl, C₁-C₆ alkoxy-C₁-C₄ alkoxy, C₃-C₆ cycloalkyl, C₃-C₆ cycloalkoxy, C₃-C₆ cycloalkyl-C₁-C₄ alkyl, C₃-C₆ cycloalkoxy-C₁-C₄ alkyl, which groups are unsubstituted or substituted with halogen;
- m: is 0, 1 or 2;
- n: is 0, 1, 2, or 3 ;
and the N-oxides, stereoisomers, tautomers and agriculturally or veterinarily acceptable salts thereof.

The compounds of the formula (I), and their agriculturally acceptable salts are highly active against animal pest, i.e. harmful arthropodes and nematodes, especially against insects and acaridae which are difficult to control by other means.

Moreover, the present invention relates to and includes the following embodiments:
- compositions comprising at least one compound of formula (I) as defined above and a liquid or solid carrier;
- agricultural and veterinary compositions comprising an amount of at least one compound of formula (I) or an enantiomer, diasteromer or salt thereof as defined above;
- non-therapeutic methods for combating invertebrate pests, infestation, or infection by invertebrate pests, which method comprises contacting said pest or its food supply, habitat or breeding grounds with a pesticidally effective amount of at least one compound of formula (I) as defined above or a composition thereof;
- non-therapeutic methods for controlling invertebrate pests, infestation, or infection by invertebrate pests, which method comprises contacting said pest or its food supply, habitat or breeding grounds with a pesticidally effective amount of at least one compound of formula (I) as defined above or a composition comprising at least one compound of formula (I);
- non-therapeutic methods for preventing or protecting against invertebrate pests comprising contacting the invertebrate pests, or their food supply, habitat or breeding grounds with substituted imidazolium compounds of the general formula (I) as defined above or a composition comprising at least one compound of formula (I) as defined above or a composition comprising at least one compound of formula (I);
- methods for protecting crops, plants, plant propagation material and/or growing plants from attack or infestation by invertebrate pests comprising contacting or treating the crops, plants, plant propagation material and growing plants, or soil, material, surface, space, area or water in which the crops, plants, plant propagation material is stored or the plant is growing, with a pesticidally effective amount of at least one compound of formula (I) as defined above or a composition comprising at least one compound of formula (I);
- non-therapeutic methods for treating animals infested or infected by parasites or preventing animals of getting infected or infested by parasites or protecting animals against infestation or infection by parasites which comprises orally, topically or parenterally administering or applying to the animals a parasiticidally effective amount of a compound of formula (I) as defined above or a composition comprising at least one compound of formula (I);
- non-therapeutic methods for treating, controlling, preventing or protecting animals against infestation or infection by parasites by administering or applying orally, topically or parenterally to the animals a compound of the general formula (I) as defined above or a composition comprising at least one compound of formula (I);
- seed comprising a compound of formula (I) as defined above, in an amount of from 0.1 g to 10 kg per 100 kg of seed;
- the use of the compounds of formula (I) as defined above for protecting growing plants or plant propagation material from attack or infestation by invertebrate pests;
- the non-therapeutic use of compounds of formula (I) or the enantiomers, diastereomers or veterinary acceptable salts thereof for combating parasites in and on animals;
- a process for the preparation of a veterinary composition for treating, controlling, preventing or protecting animals against infestation or infection by parasites which comprises adding a parasiticidally effective amount of a compound of formula (I) or the enantiomers, diastereomers and/or veterinary acceptable salt thereof to a carrier composition suitable for veterinary use;
- the use of a compound of formula (I) or the enantiomers, diastereomers and/or veterinary acceptable salt thereof for the preparation of a medicament for treating, controlling, preventing or protecting animals against infestation or infection by parasites.

All the compounds of formula (I) and, if applicable, their stereoisomers, their tautomers, their salts or their N-oxides as well as compositions thereof are particularly useful for controlling invertebrate pests, in particular for controlling arthropods and nematodes and especially insects. Therefore, the invention relates to the use of a compound of formula (I) as an agrochemical pesticide, preferably for combating or controlling invertebrate pests, in particular invertebrate pests of the group of insects, arachnids or nematodes.

The term "compound(s) according to the invention" or "compound(s) of formula (I)" as used in the present invention refers to and comprises the compound(s) as defined herein and/or stereoisomer(s), salt(s), tautomer(s) or N-oxide(s) thereof. The term "compound(s) of the present invention" is to be understood as equivalent to the term "compound(s) according to the invention", therefore also comprising stereoisomer(s), salt(s), tautomer(s) or N-oxide(s) of compounds of formula (I).

The term "composition(s) according to the invention" or "composition(s) of the present invention" encompasses composition(s) comprising at least one compound of formula (I) according to the invention as defined above, therefore also including a stereoisomer, an agriculturally or veterinary acceptable salt, tautomer or an N-oxide of the compounds of formula (I).

The compounds of the present invention may be amorphous or may exist in one or more different crystalline states (polymorphs) or modifications which may have a different macroscopic properties such as stability or show different biological properties such as activities. The present invention includes both amorphous and crystalline compounds of the formula (I), mixtures of different crystalline states or modifications of the respective compound I, as well as amorphous or crystalline salts thereof.

The compounds of the formula (I) may have one or, depending on the substitution pattern, more centers of chirality, in which case they are present as mixtures of enantiomers or diastereomers. The invention provides both the single pure enantiomers or pure diastereomers of the compounds of formula (I), and their mixtures and the use according to the invention of the pure enantiomers or pure diastereomers of the compound of formula (I) or its mixtures. Suitable compounds of the formula (I) also include all possible geometrical stereoisomers (cis/trans isomers) and mixtures thereof. Cis/trans isomers may be present with respect to an alkene, carbon-nitrogen double-bond or amide group. The term "stereoisomer(s)" encompasses both optical isomers, such as enantiomers or diastereomers, the latter existing due to more than one center of chirality in the molecule, as well as geometrical isomers (cis/trans isomers). The present invention relates to every possible stereoisomer of the compounds of formula (I), i.e. to single enantiomers or diastereomers, as well as to mixtures thereof.

Depending on the substitution pattern, the compounds of the formula (I) may be present in the form of their tautomers. Hence the invention also relates to the tautomers of the formula (I) and the stereoisomers, salts, tautomers and N-oxides of said tautomers.

Salts of the compounds of the formula (I) are preferably agriculturally and/or veterinary acceptable salts. They can be formed in a customary method, e.g. by reacting the compound with an acid of the anion in question if the compound of formula (I) has a basic functionality or by reacting an acidic compound of formula (I) with a suitable base.

Suitable agriculturally or veterinary useful salts are especially the salts of those cations or the acid addition salts of those acids whose cations and anions, respectively, do not have any adverse effect on the action of the compounds according to the present invention. Suitable cations are in particular the ions of the alkali metals, preferably lithium, sodium and potassium, of the alkaline earth metals, preferably calcium, magnesium and barium, and of the transition metals, preferably manganese, copper, zinc and iron, and also ammonium (NH₄⁺) and substituted ammonium in which one to four of the hydrogen atoms are replaced by C₁-C₄-alkyl, C₁-C₄-hydroxyalkyl, C₁-C₄-alkoxy, C₁-C₄-alkoxy-C₁-C₄-alkyl, hydroxy-C₁-C₄-alkoxy-C₁-C₄-alkyl, phenyl or benzyl. Examples of substituted ammonium ions comprise methylammonium, isopropylammonium, dimethylammonium, diisopropylammonium, trimethylammonium, tetramethylammonium, tetraethylammonium, tetrabutylammonium, 2-hydroxyethylammonium, 2-(2-hydroxyethoxy)ethyl-ammonium, bis(2-hydroxyethyl)ammonium, benzyltrimethylammonium and benzyltriethylammonium, furthermore phosphonium ions, sulfonium ions, preferably tri(C₁-C₄-alkyl)sulfonium, and sulfoxonium ions, preferably tri(C₁-C₄-alkyl)sulfoxonium.

Anions of useful acid addition salts are primarily chloride, bromide, fluoride, hydrogen sulfate, sulfate, dihydrogen phosphate, hydrogen phosphate, phosphate, nitrate, hydrogen carbonate, carbonate, hexafluorosilicate, hexafluorophosphate, benzoate, and the anions of C₁-C₄-alkanoic acids, preferably formate, acetate, propionate and butyrate. They can be formed by reacting the compounds of the formulae I with an acid of the corresponding anion, preferably of hydrochloric acid, hydrobromic acid, sulfuric acid, phosphoric acid or nitric acid.

The term "N-oxide" includes any compound of the present invention which has at least one tertiary nitrogen atom that is oxidized to an N-oxide moiety.

The organic moieties groups mentioned in the above definitions of the variables are - like the term halogen - collective terms for individual listings of the individual group members. The prefix Cₙ-Cₘ indicates in each case the possible number of carbon atoms in the group. "Halogen" will be taken to mean F, Cl, Br, and I, preferably F.

The term "substituted with", e.g. as used in "partially, or fully substituted with" means that one or more, e.g. 1, 2, 3, 4 or 5 or all of the hydrogen atoms of a given radical have been replaced by one or more, same or different substituents, such as a halogen, in particular F. Accordingly, for substituted cyclic moieties, e.g. 1-cyanocyclopropyl, one or more of the hydrogen atoms of the cyclic moiety may be replaced by one or more, same or different substituents.

The term "Cₙ-Cₘ-alkyl" as used herein (and also in Cₙ-Cₘ-alkylamino, di-Cₙ-Cₘ-alkylamino, Cₙ-Cₘ-alkylaminocarbonyl, di-(Cₙ-Cₘ-alkylamino)carbonyl, Cₙ-Cₘ-alkylthio, Cₙ-Cₘ-alkylsulfinyl and Cₙ-Cₘ-alkylsulfonyl) refers to a branched or unbranched saturated hydrocarbon group having n to m, e.g. 1 to 10 carbon atoms, preferably 1 to 6 carbon atoms, for example methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, heptyl, octyl, 2-ethylhexyl, nonyl and decyl and their isomers. C₁-C₄-alkyl means for example methyl, ethyl, propyl, 1-methylethyl, butyl, 1-methylpropyl, 2-methylpropyl or 1,1-dimethylethyl.

The term "Cₙ-Cₘ-haloalkyl" as used herein (and also in Cₙ-Cₘ-haloalkylsulfinyl and Cₙ-Cₘ-haloalkylsulfonyl) refers to a straight-chain or branched alkyl group having n to m carbon atoms, e.g. 1 to 10 in particular 1 to 6 carbon atoms (as mentioned above), where some or all of the hydrogen atoms in these groups may be replaced by halogen atoms as mentioned above, for example C₁-C₄-haloalkyl, such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl and the like. The term C₁-C₁₀-haloalkyl in particular comprises C₁-C₂-fluoroalkyl, which is synonym with methyl or ethyl, wherein 1, 2, 3, 4 or 5 hydrogen atoms are substituted with fluorine atoms, such as fluoromethyl, difluoromethyl, trifluoromethyl, 1-fluoroethyl, 2-fluoroethyl, 2,2-difluoroethyl, 2,2,2-trifluoroethyl and pentafluoromethyl.

Similarly, "Cₙ-Cₘ-alkoxy" and "Cₙ-Cₘ-alkylthio" (or Cₙ-Cₘ-alkylsulfenyl, respectively) refer to straight-chain or branched alkyl groups having n to m carbon atoms, e.g. 1 to 10, in particular 1 to 6 or 1 to 4 carbon atoms (as mentioned above) bonded through oxygen (or sulfur linkages, respectively) at any bond in the alkyl group. Examples include C₁-C₄-alkoxy such as methoxy, ethoxy, propoxy, isopropoxy, butoxy, sec-butoxy, isobutoxy and tert-butoxy, further C₁-C₄-alkylthio such as methylthio, ethylthio, propylthio, isopropylthio, and n-butylthio.

Accordingly, the terms "Cₙ-Cₘ-haloalkoxy" and "Cₙ-Cₘ-haloalkylthio" (or Cₙ-Cₘ-haloalkylsulfenyl, respectively) refer to straight-chain or branched alkyl groups having n to m carbon atoms, e.g. 1 to 10, in particular 1 to 6 or 1 to 4 carbon atoms (as mentioned above) bonded through oxygen or sulfur linkages, respectively, at any bond in the alkyl group, where some or all of the hydrogen atoms in these groups may be replaced by halogen atoms as mentioned above, for example C₁-C₂-haloalkoxy, such as chloromethoxy, bromomethoxy, dichloromethoxy, trichloromethoxy, fluoromethoxy, difluoromethoxy, trifluoromethoxy, chlorofluoromethoxy, dichlorofluoromethoxy, chlorodifluoromethoxy, 1-chloroethoxy, 1-bromoethoxy, 1-fluoroethoxy, 2-fluoroethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy and pentafluoroethoxy, further C₁-C₂-haloalkylthio, such as chloromethylthio, bromomethylthio, dichloromethylthio, trichloromethylthio, fluoromethylthio, difluoromethylthio, trifluoromethylthio, chlorofluoromethylthio, dichlorofluoromethylthio, chlorodifluoromethylthio, 1-chloroethylthio, 1-bromoethylthio, 1-fluoroethylthio, 2-fluoroethylthio, 2,2-difluoroethylthio, 2,2,2-trifluoroethylthio, 2-chloro-2-fluoroethylthio, 2-chloro-2,2-difluoroethylthio, 2,2-dichloro-2-fluoroethylthio, 2,2,2-trichloroethylthio and pentafluoroethylthio and the like. Similarly, the terms C₁-C₂-fluoroalkoxy and C₁-C₂-fluoroalkylthio refer to C₁-C₂-fluoroalkyl which is bound to the remainder of the molecule via an oxygen atom or a sulfur atom, respectively.

The term "C₂-Cₘ-alkenyl" as used herein intends a branched or unbranched unsaturated hydrocarbon group having 2 to m, e.g. 2 to 10 or 2 to 6 carbon atoms and a double bond in any position, such as ethenyl, 1-propenyl, 2-propenyl, 1-methyl-ethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl.

The term "C₂-Cₘ-alkynyl" as used herein refers to a branched or unbranched unsaturated hydrocarbon group having 2 to m, e.g. 2 to 10 or 2 to 6 carbon atoms and containing at least one triple bond, such as ethynyl, propynyl, 1-butynyl, 2-butynyl, and the like.

The term "Cₙ-Cₘ-alkoxy-Cₙ-Cₘ-alkyl" as used herein refers to alkyl having n to m carbon atoms, e.g. like specific examples mentioned above, wherein one hydrogen atom of the alkyl radical is replaced by an Cₙ-Cₘ-alkoxy group; wherein the value of n and m of the alkoxy group are independently chosen from that of the alkyl group.

The suffix "-carbonyl" in a group or "C(=O)" denotes in each case that the group is bound to the remainder of the molecule via a carbonyl C=O group. This is the case e.g. in alkylcarbonyl, haloalkylcarbonyl, aminocarbonyl, alkylaminocarbonyl, dialkylaminocarbonyl, alkoxycarbonyl, haloalkoxycarbonyl.

The term "aryl" as used herein refers to a mono-, bi- or tricyclic aromatic hydrocarbon radical such as phenyl or naphthyl, in particular phenyl (also referred as to C₆H₅ as subsitituent).

The term "C₃-Cₘ-cycloalkyl" as used herein refers to a monocyclic ring of 3- to m-membered saturated cycloaliphatic radicals, e.g. cyclopropyl, cyclobutyl, cyclopentyl, cyclohexyl, cycloheptyl, cyclooctyl and cyclodecyl.

The term "alkylcycloalkyl" denotes as well as the term "alkyl which may be substituted with cycloalkyl" an alkyl group which is substituted with a cycloalkyl ring, wherein alkyl and cycloakyl are as herein defined.

The term "cycloalkylalkyl" denotes as well as the term "cycloalkyl which may be substituted with alkyl" a cycloalkyl ring which is substituted with an alkyl group, wherein alkyl and cycloakyl are as herein defined.

The term "alkylcycloalkylalkyl" denotes as well as the term "alkylcycloalkyl which may be substituted with alkyl" an alkylcycloalkyl group which is substituted with an alkyl, wherein alkyl and alkylcycloakyl are as herein defined.

The term " C₃-Cₘ-cycloalkenyl" as used herein refers to a monocyclic ring of 3- to m-membered partially unsaturated cycloaliphatic radicals.

The term "cycloalkylcycloalkyl" denotes as well as the term "cycloalkyl which may be substituted with cycloalkyl" a cycloalkyl substitution on another cycloalkyl ring, wherein each cycloalkyl ring independently has from 3 to 7 carbon atom ring members and the cycloalkyls are linked through one single bond or have one common carbon atom. Examples of cycloalkylcycloalkyl include cyclopropylcyclopropyl (e.g. 1,1'-bicyclopropyl-2-yl), cyclohexylcyclohexyl wherein the two rings are linked through one single common carbon atom (e.g. 1,1'-bicyclohexyl-2-yl), cyclohexylcyclopentyl wherein the two rings are linked through one single bond (e.g. 4-cyclopentylcyclohexyl) and their different stereoisomers such as (1R,2S)-1, 1'-bicyclopropyl-2-yl and (1R,2R)-1,1'-bicyclopropyl-2-yl.The term "carbocycle" or "carbocyclyl" includes, unless otherwise indicated, in general a 3- to 12-membered, preferably a 3- to 8-membered or a 5- to 8-membered, more preferably a 5- or 6-membered mono-cyclic, ring comprising 3 to 12, preferably 3 to 8 or 5 to 8, more preferably 5 or 6 carbon atoms.

The carbocyclic radicals may be saturated, partially unsaturated, or fully unsaturated. Preferably, the term "carbocycle" covers cycloalkyl and cycloalkenyl groups as defined above, for example cyclopropane, cyclobutane, cyclopentane and cyclohexane rings. When it is referred to "fully unsaturated" carbocycles, this term also includes "aromatic" carbocycles. In certain preferred embodiments, a fully unsaturated carbocycle is an aromatic carbocycle as defined below, preferably a 6-membered aromatic carbocycle.

The term "hetaryl" or "aromatic heterocycle" or "aromatic heterocyclic ring" includes monocyclic 5- or 6-membered heteroaromatic radicals comprising as ring members 1, 2, 3 or 4 heteroatoms selected from N, O and S. Examples of 5- or 6-membered heteroaromatic radicals include pyridyl, i.e. 2-, 3-, or 4-pyridyl, pyrimidinyl, i.e. 2-, 4- or 5-pyrimidinyl, pyrazinyl, pyridazinyl, i.e. 3- or 4-pyridazinyl, thienyl, i.e. 2- or 3-thienyl, furyl, i.e. 2-or 3-furyl, pyrrolyl, i.e. 2- or 3-pyrrolyl, oxazolyl, i.e. 2-, 3- or 5-oxazolyl, isoxazolyl, i.e. 3-, 4- or 5-isoxazolyl, thiazolyl, i.e. 2-, 3- or 5-thiazolyl, isothiazolyl, i.e. 3-, 4- or 5-isothiazolyl, pyrazolyl, i.e. 1-, 3-, 4- or 5-pyrazolyl, i.e. 1-, 2-, 4- or 5-imidazolyl, oxadiazolyl, e.g. 2- or 5-[1,3,4]oxadiazolyl, 4- or 5-(1,2,3-oxadiazol)yl, 3- or 5-(1,2,4-oxadiazol)yl, 2- or 5-(1,3,4-thiadiazol)yl, thiadiazolyl, e.g. 2- or 5-(1,3,4-thiadiazol)yl, 4- or 5-(1,2,3-thiadiazol)yl, 3- or 5-(1,2,4-thiadiazol)yl, triazolyl, e.g. 1H-, 2H- or 3H-1,2,3-triazol-4-yl, 2H-triazol-3-yl, 1H-, 2H-, or 4H-1,2,4-triazolyl and tetrazolyl, i.e. 1H- or 2H-tetrazolyl. The term "hetaryl" also includes bicyclic 8 to 10-membered heteroaromatic radicals comprising as ring members 1, 2 or 3 heteroatoms selected from N, O and S, wherein a 5- or 6-membered heteroaromatic ring is fused to a phenyl ring or to a 5- or 6-membered heteroaromatic radical. Examples of a 5- or 6-membered heteroaromatic ring fused to a phenyl ring or to a 5- or 6-membered heteroaromatic radical include benzofuranyl, benzothienyl, indolyl, indazolyl, benzimidazolyl, benzoxathiazolyl, benzoxadiazolyl, benzothiadiazolyl, benzoxazinyl, chinolinyl, isochinolinyl, purinyl, 1,8-naphthyridyl, pteridyl, pyrido[3,2-d]pyrimidyl or pyridoimidazolyl and the like. These fused hetaryl radicals may be bonded to the remainder of the molecule via any ring atom of 5- or 6-membered heteroaromatic ring or via a carbon atom of the fused phenyl moiety.

The terms "heterocycle", "heterocyclyl" or "heterocyclic ring" includes, unless otherwise indicated, in general 3- to 12-membered, preferably 3- to 8-membered, 3- to 7-membered, or 5- to 8-membered, more preferably 5- or 6-membered, in particular 6-membered monocyclic heterocyclic radicals. The heterocyclic radicals may be saturated, partially unsaturated, or fully unsaturated. As used in this context, the term "fully unsaturated" also includes "aromatic". In a preferred embodiment, a fully unsaturated heterocycle is thus an aromatic heterocycle, preferably a 5- or 6-membered aromatic heterocycle comprising one or more, e.g. 1, 2, 3, or 4, preferably 1, 2, or 3 heteroatoms selected from N, O and S as ring members. Examples of aromatic heterocycles are provided above in connection with the definition of "hetaryl". Unless otherwise indicated, "hetaryls" are thus covered by the term "heterocycles". The heterocyclic non-aromatic radicals usually comprise 1, 2, 3, 4 or 5, preferably 1, 2 or 3 heteroatoms selected from N, O and S as ring members, where S-atoms as ring members may be present as S, SO or SO₂. Examples of 5- or 6-membered heterocyclic radicals comprise saturated or unsaturated, non-aromatic heterocyclic rings, such as oxiranyl, oxetanyl, thietanyl, thietanyl-S-oxid (S-oxothietanyl), thietanyl-S-dioxid (S-dioxothiethanyl), pyrrolidinyl, pyrrolinyl, pyrazolinyl, tetrahydrofuranyl, dihydrofuranyl, 1,3-dioxolanyl, thiolanyl, S-oxothiolanyl, S-dioxothiolanyl, dihydrothienyl, S-oxodihydrothienyl, S-dioxodihydrothienyl, oxazolidinyl, oxazolinyl, thiazolinyl, oxathiolanyl, piperidinyl, piperazinyl, pyranyl, dihydropyranyl, tetrahydropyranyl, 1,3- and 1,4-dioxanyl, thiopyranyl, S.oxothiopyranyl, S-dioxothiopyranyl, dihydrothiopyranyl, S-oxodihydrothiopyranyl, S-dioxodihydrothiopyranyl, tetrahydrothiopyranyl, S-oxotetrahydrothiopyranyl, S-dioxotetrahydrothiopyranyl, morpholinyl, thiomorpholinyl, S-oxothiomorpholinyl, S-dioxothiomorpholinyl, thiazinyl and the like. Examples for heterocyclic ring also comprising 1 or 2 carbonyl groups as ring members comprise pyrrolidin-2-onyl, pyrrolidin-2,5-dionyl, imidazolidin-2-onyl, oxazolidin-2-onyl, thiazolidin-2-onyl and the like. The erms "alkylene", "alkenylene", and "alkynylene" refer to alkyl, alkenyl, and alkynyl as defined above, respectively, which are bonded to the remainder of the molecule, via two atoms, preferably via two carbon atoms, of the respective group, so that they represent a linker between two moieties of the molecule. In particular, the term "alkylene" may refer to alkyl chains such as CH₂CH₂, - CH(CH₃)-, CH₂CH₂CH₂, CH(CH₃)CH₂, CH₂CH(CH₃), CH₂CH₂CH₂CH₂, CH₂CH₂CH₂CH₂CH₂, CH₂CH₂CH₂CH₂CH₂CH₂, and CH₂CH₂CH₂CH₂CH₂CH₂CH₂ Similarly, "alkenylene" and "alkynylene" may refer to alkenyl and alkynyl chains, respectively.

The term "5- to 6-membered carbocyclic ring" as used herein refers to cyclopentane and cyclohexane rings.

Examples of 5- or 6-membered saturated heterocyclic rings include: 2-tetrahydrofuranyl, 3-tetrahydrofuranyl, 2-tetrahydrothienyl, 3-tetrahydrothienyl, 2-pyrrolidinyl, 3-pyrrolidinyl, 3-pyrazolidinyl, 4-pyrazolidinyl, 5-pyrazolidinyl, 2-imidazolidinyl, 4-imidazolidinyl, 2-oxazolidinyl, 4-oxazolidinyl, 5-oxazolidinyl, 3-isoxazolidinyl, 4-isoxazolidinyl, 5-isoxazolidinyl, 2-thiazolidinyl, 4-thiazolidinyl, 5-thiazolidinyl, 3-isothiazolidinyl, 4-isothiazolidinyl, 5-isothiazolidinyl, 1,2,4-oxadiazolidin-3-yl, 1,2,4-oxadiazolidin 5 yl, 1,2,4-thiadiazolidin-3-yl, 1,2,4-thiadiazolidin-5-yl, 1,2,4-triazolidin-3-yl,-1,3,4-oxadiazolidin-2-yl, 1,3,4-thiadiazolidin-2-yl, 1,3,4-triazolidin-2-yl, 2-tetrahydropyranyl, 4-tetrahydropyranyl, 1,3-dioxan-5-yl, 1,4-dioxan-2-yl, 2-piperidinyl, 3-piperidinyl, 4-piperidinyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, 1,3,5-hexahydrotriazin-2-yl and 1,2,4-hexahydrotriazin-3-yl, 2-morpholinyl, 3-morpholinyl, 2-thiomorpholinyl, 3-thiomorpholinyl, 1-oxothiomorpholin-2-yl, 1-oxothiomorpholin-3-yl, 1,1-dioxothiomorpholin-2-yl, 1,1-dioxothiomorpholin-3-yl.

Examples of 5- or 6-membered partially unsaturated heterocyclyl or heterocyclic rings include: 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin 3 yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3 dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-, 3-, 4-, 5- or 6-di- or tetrahydropyridinyl, 3-di- or tetrahydropyridazinyl, 4-di- or tetrahydropyridazinyl, 2-di- or tetrahydropyrimidinyl, 4-di- or tetrahydropyrimidinyl, 5-di- or tetrahydropyrimidinyl, di- or tetrahydropyrazinyl, 1,3,5-di- or tetrahydrotriazin-2-yl.

Examples of 5- or 6-membered fully unsaturated heterocyclic (hetaryl) or heteroaromatic rings are: 2-furyl, 3-furyl, 2-thienyl, 3-thienyl, 2-pyrrolyl, 3-pyrrolyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 2-imidazolyl, 4-imidazolyl, 1,3,4-triazol-2-yl, 2-pyridinyl, 3-pyridinyl, 4-pyridinyl, 3-pyridazinyl, 4-pyridazinyl, 2-pyrimidinyl, 4-pyrimidinyl, 5-pyrimidinyl and 2-pyrazinyl.

A "C₂-Cₘ-alkylene" is divalent branched or preferably unbranched saturated aliphatic chain having 2 to m, e.g. 2 to 7 carbon atoms, for example CH₂CH₂, -CH(CH₃)-, CH₂CH₂CH₂, CH(CH₃)CH₂, CH₂CH(CH₃), CH₂CH₂CH₂CH₂, CH₂CH₂CH₂CH₂CH₂, CH₂CH₂CH₂CH₂CH₂CH₂, and CH₂CH₂CH₂CH₂CH₂CH₂CH₂.

The term "alkylamino" as used herein refers to a straight-chain or branched saturated alkyl group having 1 to 10 carbon atoms, preferably 1 to 4 carbon atoms, more preferably 1 to 3 carbon atoms, which is bonded via a nitrogen atom, e.g. an -NH- group.

The term "dialkylamino" as used herein refers to a straight-chain or branched saturated alkyl group having 1 to 10 carbon atoms, preferably 1 to 4 carbon atoms, more preferably 1 to 3 carbon atoms, which is bonded via a nitrogen atom, which is substituted by another straight-chain or branched saturated alkyl group having 1 to 10 carbon atoms, preferably 1 to 4 carbon atoms, more preferably 1 to 3 carbon atoms, e.g. a methylamino or ethylamino group.

The term "alkylthio "( alkylsulfanyl: alkyl-S-)" as used herein refers to a straight-chain or branched saturated alkyl group having 1 to 10 carbon atoms, preferably 1 to 4 carbon atoms (= C₁-C₄-alkylthio), more preferably 1 to 3 carbon atoms, which is attached via a sulfur atom. Examples include methylthio, ethylthio, propylthio, isopropylthio, and n-butylthio.

The term "haloalkylthio" as used herein refers to an alkylthio group as mentioned above wherein the hydrogen atoms are partially or fully substituted by fluorine, chlorine, bromine and/or iodine. Examples include chloromethylthio, bromomethylthio, dichloromethylthio, trichloromethylthio, fluoromethylthio, difluoromethylthio, trifluoromethylthio, chlorofluoromethylthio, dichlorofluoromethylthio, chlorodifluoromethylthio, 1-chloroethylthio, 1-bromoethylthio, 1-fluoroethylthio, 2-fluoroethylthio, 2,2-difluoroethylthio, 2,2,2-trifluoroethylthio, 2-chloro-2-fluoroethylthio, 2-chloro-2,2-difluoroethylthio, 2,2-dichloro-2-fluoroethylthio, 2,2,2-trichloroethylthio and pentafluoroethylthio and the like.

The term "alkylsulfinyl" (alkylsulfoxyl: C₁-C₆-alkyl-S(=O)-), as used herein refers to a straight-chain or branched saturated alkyl group (as mentioned above) having 1 to 10 carbon atoms, preferably 1 to 4 carbon atoms (= C₁-C₄-alkylsulfinyl), more preferably 1 to 3 carbon atoms bonded through the sulfur atom of the sulfinyl group at any position in the alkyl group.

The term "alkylsulfonyl" (alkyl-S(=O)₂-) as used herein refers to a straight-chain or branched saturated alkyl group having 1 to 10 carbon atoms, preferably 1 to 4 carbon atoms (= C₁-C₄-alkylsulfonyl), preferably 1 to 3 carbon atoms, which is bonded via the sulfur atom of the sulfonyl group at any position in the alkyl group.

The term "alkylcarbonyl" (C₁-C₆-C(=O)-) refers to a straight-chain or branched alkyl group as defined above, which is bonded via the carbon atom of a carbonyl group (C=O) to the remainder of the molecule.

The term "alkoxycarbonyl" refers to an alkoxygroup group as defined above, which is bonded via the carbon atom of a carbonyl group (C=O) to the remainder of the molecule.

The term "alkylaminocarbonyl" (C₁-C₆-NH-C(=O)-) refers to a straight-chain or branched alkylamino group as defined above, which is bonded via the carbon atom of a carbonyl group (C=O) to the remainder of the molecule. Similarly, the term "dialkylaminocarbonyl" refers to a straight-chain or branched saturated alkyl group as defined above, which is bonded to a nitrogen atom, which is substituted with another straight-chain or branched saturated alkyl group as defined above, which nitrogen atom in turn is bonded via a carbonyl group (C=O) to the remainder of the molecule.

### Preparation Methods

The compounds of formula (I) can be prepared by standard methods of organic chemistry. If certain derivatives cannot be prepared by the processes outlined below, they can be obtained by derivatization of other compounds of formula (I) that are accessible by these methods.

Preparation methods that are generally useful for the preparation of compounds of formula (I) have been disclosed in EP3257853A1, especially on p.33-57 and in the experimental section, as well as in WO2017/167832A1, especially p.4-6 and in the experimental section.

Compounds of formula (II), corresponding to compounds of formula (I) wherein Q is C(R⁶), may be prepared by reaction of compounds of formula (1) with compounds of formula (2) as displayed under Process 1.

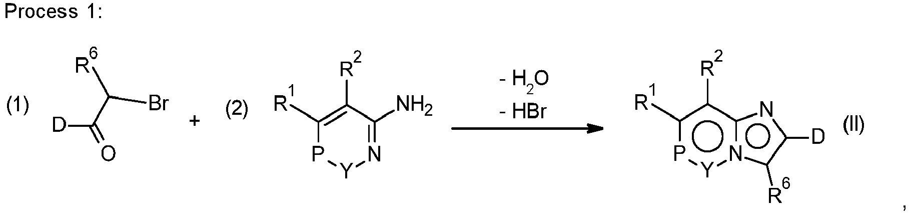

All variables in formulae (1), (2) and (II) have a meaning as defined for formula (I). Compounds of formula (1) and compounds of formula (2) are typically reacted with one another in equimolar amounts. In terms of yield, it may be advantageous to employ an excess of compounds of formula (2). Reactions of this type have been described in EP3257853A1, p.33-34. The reaction is typically carried out under elevated temperatures of from 50-160 °C in an inert solvent. Suitable solvents are aliphatic hydrocarbons, such as pentane, hexane, cyclohexane, or petrol ether; aromatic hydrocarbons, such as benzene, toluene, o-, m-, and p-xylene; halogenated hydrocarbons, or halogenated aromatic C₆-C₁₀-hydrocarbons, such as CH₂Cl₂, CHCl₃, CCl₄, CH₂ClCH₂Cl, CCl₃CH₃, CHCl₂CH₂Cl, CCl₂CCl₂, or chlorobenzene; ethers, such as CH₃CH₂OCH₂CH₃, (CH₃)₂CHOCH(CH₃)₂, CH₃OC(CH₃)₃ (MTBE), CH₃OCH₃ (DME), CH₃OCH₂CH₂OCH₃, CH₃OC(CH₃)₂CH₂CH₃, dioxane, anisole, 2-methyltetrahydrofuran, tetrahydrofurane (THF), and diethylene glycol; nitriles, such as CH₃CN, and CH₃CH₂CN; alcohols, such as CH₃OH, CH₃CH₂OH, CH₃CH₂CH₂OH, CH₃CH(OH)CH₃, CH₃(CH₂)₃OH, and C(CH₃)₃OH, CH₂(OH)CH₂(OH), CH₃CH(OH)CH₂OH; amides and urea derivatives, such as dimethyl formamide (DMF), N-methyl-2-pyrrolidone (NMP), dimethyl acetamide (DMA), 1,3-dimethyl-2-imidazolidinone (DMI), 1,3-dimethyl-3,4,5,6-tetrahydro-2(1H)-pyrimidinone (DMPU), hexamethylphosphamide (HMPA); moreover dimethyl sulfoxide (DMSO), sulfolane, and water. Mixtures of the above solvents are also possible. The reacton may be carried out in the presence of a catalyst, such as an acid or a base, preferably a base. Suitable bases are, in general, inorganic bases, such as LiOH, NaOH, KOH, and Ca(OH)₂; alkali metal and alkaline earth metal oxides, such as Li₂O, Na₂O, CaO, and MgO; alkali metal and alkaline earth metal hydrides, such as LiH, NaH, KH and CaH₂; alkali metal and alkaline earth metal carbonates, such as Li₂CO₃, K₂CO₃ and CaCO₃; alkali metal bicarbonates, such as NaHCO₃; organic bases, such as pyrrolidine; tertiary amines, such as diisopropylethylamine, trimethylamine, triethylamine, triisopropylamine and N-methylpiperidine, imidazol, pyridine; substituted pyridines, such as collidine, lutidine and 4-dimethylaminopyridine, and polycyclic amides and amidines, such as 1,8-diazabicycloundec-7-ene (DBU), 1,4-Diazabicyclo[2.2.2]octane (DABCO); alkali metal salts of secondary amines, such as alkali diisopropylamide, alkali bis(trimethylsilyl)amide, alkali tetramethylpiperidene; alcoholates, such as alkali methanolate, alkali ethanolate, alkali isopropanolate, alkali tert-butanolate; alkali metal - alkyl, and alkali metal - aryl salts, such as n-butyl lithium, tert-butyl lithium, phenyl lithium. Mixtures of the aforementioned bases are also possible. The bases are generally employed in catalytic amounts; however, they can also be used in equimolar amounts, in excess or, if appropriate, as solvent.

Compounds of formula (2) can be prepared as described in WO2017/167832A1, e.g. Example C-1.

Compounds of formula (1) are commercially available or can be prepared as described in EP3257853A1, e.g. p.51, Reaction Scheme 1.

Accordingly, compounds of formula (1a), wherein all variables have a definition as in formula (I), and falling under the definition of formula (1), may for example be prepared as follows.

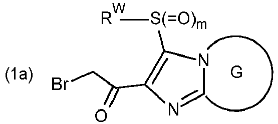

In a first step depicted under Process 2, the compound 1-bromobutane-2,3-dione is reacted with a compound of formula (1b) under elevated temperatures to yield imidazole derifatives of formula (1c) 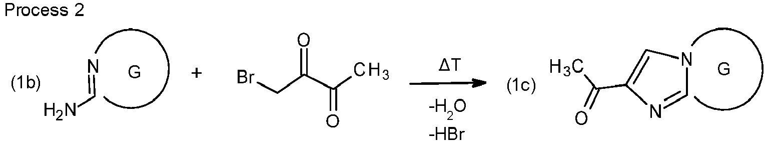 wherein all variables have a meaning as defined for formula (I), and wherein ΔT means elevated temperature. This transformation is usually carried out at temperatures of from 40 °C to 100 °C, preferably from 45 °C to 60 °C, in an inert solvent. Suitable inert solvents are aliphatic hydrocarbons, preferably an aliphatic C₅-C₁₆-hydrocarbon, more preferably a C₅-C₁₆-alkane, or C₅-C₁₆-cycloalkane, such as pentane, hexane, cyclohexane, or petrol ether; aromatic hydrocarbons, preferably an aromatic C₆-C₁₀-hydrocarbons, such as benzene, toluene, o-, m-, and p-xylene; halogenated hydrocarbons, preferably halogenated aliphatic C₁-C₆-alkanes, or halogenated aromatic C₆-C₁₀-hydrocarbons, such as CH₂Cl₂, CHCl₃, CCl₄, CH₂ClCH₂Cl, CCl₃CH₃, CHCl₂CH₂Cl, CCl₂CCl₂, or chlorobenzene; ethers, preferably C₁-C₆-cycloalkyl ethers, C₁-C₆-alkyl-C₁-C₆-alkyl ethers and C₁-C₆-alkyl-C₆-C₁₀-aryl ethers, such as CH₃CH₂OCH₂CH₃, (CH₃)₂CHOCH(CH₃)₂, CH₃OC(CH₃)₃ (MTBE), CH₃OCH₃ (DME), CH₃OCH₂CH₂OCH₃, dioxane, anisole, and tetrahydrofurane (THF); esters, preferably esters of aliphatic C₁-C₆-alcohols with aliphatic C₁-C₆-carboxylic acids, esters of aromatic C₆-C₁₀-alcohols with aromatic C₆-C₁₀-carboxylic adcids, cyclic esters of ω-hydroxy-C₁-C₆-carboxylic acids, such as CH₃C(O)OCH₂CH₃, CH₃C(O)OCH₃, CH₃C(O)OCH₂CH₂CH₂CH₃, CH₃C(O)OCH(CH₃)CH₂CH₃, CH₃C(O)OC(CH₃), CH₃CH₂CH₂C(O)OCH₂CH₃, CH₃CH(OH)C(O)OCH₂CH₃, CH₃CH(OH)C(O)OCH₃, CH₃C(O)OCH₂CH(CH₃)₂, CH₃C(O)OCH(CH₃)₂, CH₃CH₂C(O)OCH₃, benzyl benzoate, and γ-butyrolactone; carbonates, such as ethylene carbonate, propylene carbonate, CH₃CH₂OC(O)OCH₂CH₃, and CH₃OC(O)OCH₃; nitriles, preferably C₁-C₆-nitriles, such as CH₃CN, and CH₃CH₂CN; ketones, preferably C₁-C₆-alkyl-C₁-C₆-alkyl ketones, such as CH₃C(O)CH₃, CH₃C(O)CH₂CH₃, CH₃CH₂C(O)CH₂CH₃, and CH₃C(O)C(CH₃)₃ (MTBK); alcohols, preferably C₁-C₄-alcohols, such as CH₃OH, CH₃CH₂OH, CH₃CH₂CH₂OH, CH₃CH(OH)CH₃, CH₃(CH₂)₃OH, and C(CH₃)₃OH; amides and urea derivatives, preferably dimethyl formamide (DMF), N-methyl-2-pyrrolidone (NMP), dimethyl acetamide (DMA), 1,3-dimethyl-2-imidazolidinone (DMI), 1,3-dimethyl-3,4,5,6-tetrahydro-2(1H)-pyrimidinone (DMPU), hexamethylphosphamide (HMPA); moreover dimethyl sulfoxide (DMSO), sulfolane, and mixtures thereof. The reactants are typically applied in equimolar amounts. However, the molar ration of 1-bromobutane-2,3-dione to compound of formula (1b) may also be above 1:1, e.g. 2.1 or 3:1.

In a subsequent Process 3, compounds of formulat (1c) may then be halogenated to yield compounds of formula (1d) 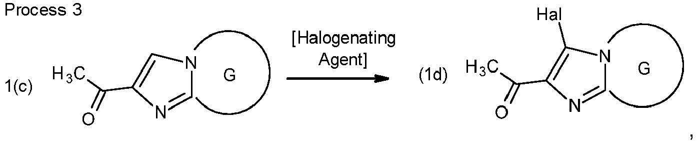 wherein the variable Hal means a halogen atom, e.g. CI, and wherein all orther variables have a meaning as defined for formula (I). This transformation is usually carried out at temperatures of from 10 °C to 40 °C in an inert solvent. Suitable inert solvents are for example aliphatic hydrocarbons, preferably an aliphatic C₅-C₁₆-hydrocarbon, more preferably a C₅-C₁₆-alkane, or C₅-C₁₆-cycloalkane, such as pentane, hexane, cyclohexane, or petrol ether; halogenated hydrocarbons, preferably halogenated aliphatic C₁-C₆-alkanes, or halogenated aromatic C₆-C₁₀-hydrocarbons, such as CH₂Cl₂, CHCl₃, CCl₄, CH₂ClCH₂Cl, CCl₃CH₃, CHCl₂CH₂Cl, CCl₂CCl₂, or chlorobenzene;.

Halogenating agents are well known to the skilled person in the art. Typical examples include 2-chloro-1,3-bis(methoxycarbonyl)guanidine, N-iodosuccinimide, N-bromosuccinimide, and N-chlorosuccinimide. The reactants are typically applied in equimolar amounts. However, the molar ration of the halogenating agent to compounds of formula (Ic) may also be above 1:1, e.g. 2.1 or 3:1.

Subsequently, compounds of formula (Id) may be reacted in Process 4 with a thiolate agent R^{W}-S⁻, which may be applied in the form of its sodium salt Na [R^{W}-S], to yield compunds of formula (1e) 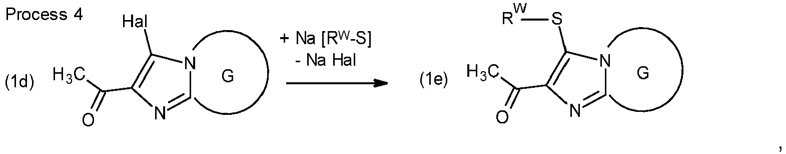 wherein Hal means halogen, and wherein all other variables have a meaning as defined for formula (I). This transformation is usually carried out at temperatures of from -10 °C to 20 °C, in an inert solvent. Suitable inert solvents are aliphatic hydrocarbons, preferably an aliphatic C₅-C₁₆-hydrocarbon, more preferably a C₅-C₁₆-alkane, or C₅-C₁₆-cycloalkane, such as pentane, hexane, cyclohexane, or petrol ether; aromatic hydrocarbons, preferably an aromatic C₆-C₁₀-hydrocarbons, such as benzene, toluene, o-, m-, and p-xylene; ethers, preferably C₁-C₆-cycloalkyl ethers, C₁-C₆-alkyl-C₁-C₆-alkyl ethers and C₁-C₆-alkyl-C₆-C₁₀-aryl ethers, such as CH₃CH₂OCH₂CH₃, (CH₃)₂CHOCH(CH₃)₂, CH₃OC(CH₃)₃ (MTBE), CH₃OCH₃ (DME), CH₃OCH₂CH₂OCH₃, dioxane, anisole, and tetrahydrofurane (THF). The reactants are typically applied in equimolar amounts. However, the molar ration of the thiolate to compounds of formula (1d) may also be above 1:1, e.g. 2.1 or 3:1.

In case m>0, compounds of formula (1e) may be oxidized by addition of an oxizidizing agent as depicted under Process 5 to yield compounds of formula (1f), 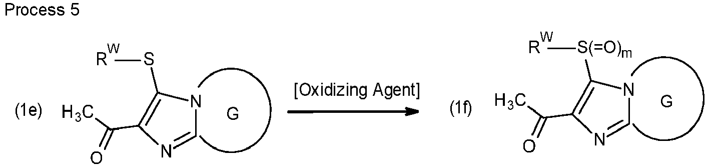 wherein m is 1 or 2, and wherein all variables have a meaning as defined for formula (I). This transformation is usually carried out at temperatures of from -10 °C to 50 °C, preferably from -5 °C to 30 °C, in an inert solvent. Typical Oxidizing Agents include meta-chloroperoxybenzoic acid and H₂O₂. Alternatively, O₂ can be used in the presence of a catalyst as described by Wei, Lian-Qiang; Ye, Bao-Hui, ACS Applied Materials & Interfaces, volume 11, issue 44, pages 41448-41457, DOI 10.1021/acsami.91b15646.

Suitable inert solvents are aliphatic hydrocarbons, preferably an aliphatic C₅-C₁₆-hydrocarbon, more preferably a C₅-C₁₆-alkane, or C₅-C₁₆-cycloalkane, such as pentane, hexane, cyclohexane, or petrol ether; aromatic hydrocarbons, preferably an aromatic C₆-C₁₀-hydrocarbons, such as benzene, toluene, o-, m-, and p-xylene; halogenated hydrocarbons, preferably halogenated aliphatic C₁-C₆-alkanes, or halogenated aromatic C₆-C₁₀-hydrocarbons, such as CH₂Cl₂, CHCl₃, CCl₄, CH₂ClCH₂Cl, CCl₃CH₃, CHCl₂CH₂Cl, CCl₂CCl₂, or chlorobenzene; ethers, preferably C₁-C₆-cycloalkyl ethers, C₁-C₆-alkyl-C₁-C₆-alkyl ethers and C₁-C₆-alkyl-C₆-C₁₀-aryl ethers, such as CH₃CH₂OCH₂CH₃, (CH₃)₂CHOCH(CH₃)₂, CH₃OC(CH₃)₃ (MTBE), CH₃OCH₃ (DME), CH₃OCH₂CH₂OCH₃, dioxane, anisole, and tetrahydrofurane (THF); esters, preferably esters of alliphatic C₁-C₆-alcohols with aliphatic C₁-C₆-carboxylic acids, esters of aromatic C₆-C₁₀-alcohols with aromatic C₆-C₁₀-carboxylic adcids, cyclic esters of ω-hydroxy-C₁-C₆-carboxylic acids, such as CH₃C(O)OCH₂CH₃, CH₃C(O)OCH₃, CH₃C(O)OCH₂CH₂CH₂CH₃, CH₃C(O)OCH(CH₃)CH₂CH₃, CH₃C(O)OC(CH₃), CH₃CH₂CH₂C(O)OCH₂CH₃, CH₃CH(OH)C(O)OCH₂CH₃, CH₃CH(OH)C(O)OCH₃, CH₃C(O)OCH₂CH(CH₃)₂, CH₃C(O)OCH(CH₃)₂, CH₃CH₂C(O)OCH₃, benzyl benzoate, and γ-butyrolactone; carbonates, such as ethylene carbonate, propylene carbonate, CH₃CH₂OC(O)OCH₂CH₃, and CH₃OC(O)OCH₃; nitriles, preferably C₁-C₆-nitriles, such as CH₃CN, and CH₃CH₂CN; ketones, preferably C₁-C₆-alkyl-C₁-C₆-alkyl ketones, such as CH₃C(O)CH₃, CH₃C(O)CH₂CH₃, CH₃CH₂C(O)CH₂CH₃, and CH₃C(O)C(CH₃)₃ (MTBK). Typically, the Oxidizing Agent is used in excess as compared to compounds of formula (1e), e.g. in a ratio of above 2, like above 3.

Compounds of formula (1f) or compounds of formula (1e) may then be brominated to yield compounds of formula (1a) as depicted under Process 6 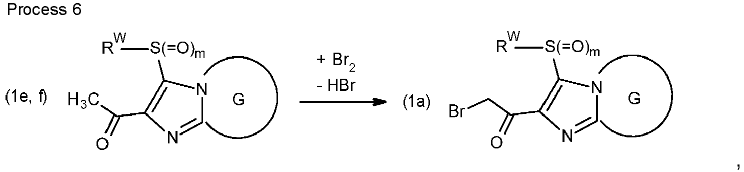 wherein all variables have a meaning as defined for formula (I). This transformation is usually carried out at temperatures of from -10 °C to 50 °C, preferably from 0 °C to 30 °C, optionally in an inert solvent, in the presence of an acid. Typically, the acid is used as the solvent for the raction. Suitable solvents are aliphatic hydrocarbons, such as pentane, hexane, cyclohexane, or petrol ether; ethers, such as CH₃CH₂OCH₂CH₃, (CH₃)₂CHOCH(CH₃)₂, CH₃OC(CH₃)₃ (MTBE), CH₃OCH₃ (DME), CH₃OCH₂CH₂OCH₃, CH₃OC(CH₃)₂CH₂CH₃, dioxane, anisole, 2-methyltetrahydrofuran, tetrahydrofurane (THF), and and diethylene glycol;. Mixtures of the above solvents are also possible. Suitable acids are HBr, HCl, or CH₃COOH, which act as a catalyst in the bromination reaction.

By another way of example, compounds of formula (1g), falling under the definition of formula (1) may for example be prepared as follows.

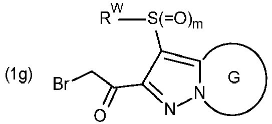

In a first step depicted under Process 7, the compound dimethyl but-2-ynedioate is reacted with a compound of formula (1h) to yield imidazole derifatives of formula (1k) 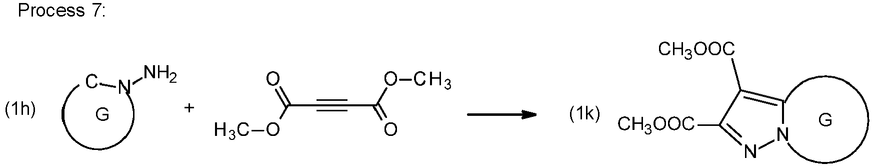 wherein all variables have a meaning as defined for formula (I). This transformation is usually carried out at temperatures of from -10 °C to 40 °C, preferably in the presence of a base, in an inert solvent.

Suitable solvents are aliphatic hydrocarbons, preferably an aliphatic C₅-C₁₆-hydrocarbon, more preferably a C₅-C₁₆-alkane, or C₅-C₁₆-cycloalkane, such as pentane, hexane, cyclohexane, or petrol ether; aromatic hydrocarbons, preferably an aromatic C₆-C₁₀-hydrocarbons, such as benzene, toluene, o-, m-, and p-xylene; halogenated hydrocarbons, preferably halogenated aliphatic C₁-C₆-alkanes, or halogenated aromatic C₆-C₁₀-hydrocarbons, such as CH₂Cl₂, CHCl₃, CCl₄, CH₂ClCH₂Cl, CCl₃CH₃, CHCl₂CH₂Cl, CCl₂CCl₂, or chlorobenzene; ethers, preferably C₁-C₆-cycloalkyl ethers, C₁-C₆-alkyl-C₁-C₆-alkyl ethers and C₁-C₆-alkyl-C₆-C₁₀-aryl ethers, such as CH₃CH₂OCH₂CH₃, (CH₃)₂CHOCH(CH₃)₂, CH₃OC(CH₃)₃ (MTBE), CH₃OCH₃ (DME), CH₃OCH₂CH₂OCH₃, dioxane, anisole, and tetrahydrofurane (THF); esters, preferably esters of aliphtic C₁-C₆-alcohols with aliphatic C₁-C₆-carboxylic acids, esters of aromatic C₆-C₁₀-alcohols with aromatic C₆-C₁₀-carboxylic adcids, cyclic esters of ω-hydroxy-C₁-C₆-carboxylic acids, such as CH₃C(O)OCH₂CH₃, CH₃C(O)OCH₃, CH₃C(O)OCH₂CH₂CH₂CH₃, CH₃C(O)OCH(CH₃)CH₂CH₃, CH₃C(O)OC(CH₃), CH₃CH₂CH₂C(O)OCH₂CH₃, CH₃CH(OH)C(O)OCH₂CH₃, CH₃CH(OH)C(O)OCH₃, CH₃C(O)OCH₂CH(CH₃)₂, CH₃C(O)OCH(CH₃)₂, CH₃CH₂C(O)OCH₃, benzyl benzoate, and γ-butyrolactone; carbonates, such as ethylene carbonate, propylene carbonate, CH₃CH₂OC(O)OCH₂CH₃, and CH₃OC(O)OCH₃; nitriles, preferably C₁-C₆-nitriles, such as CH₃CN, and CH₃CH₂CN; ketones, preferably C₁-C₆-alkyl-C₁-C₆-alkyl ketones, such as CH₃C(O)CH₃, CH₃C(O)CH₂CH₃, CH₃CH₂C(O)CH₂CH₃, and CH₃C(O)C(CH₃)₃ (MTBK); alcohols, preferably C₁-C₄-alcohols, such as CH₃OH, CH₃CH₂OH, CH₃CH₂CH₂OH, CH₃CH(OH)CH₃, CH₃(CH₂)₃OH, and C(CH₃)₃OH; amides and urea derivatives, preferably dimethyl formamide (DMF), N-methyl-2-pyrrolidone (NMP), dimethyl acetamide (DMA), 1,3-dimethyl-2-imidazolidinone (DMI), 1,3-dimethyl-3,4,5,6-tetrahydro-2(1H)-pyrimidinone (DMPU), hexamethylphosphamide (HMPA); moreover dimethyl sulfoxide (DMSO), sulfolane, and mixtures thereof. Suitable bases are, in general, inorganic bases, such as alkali metal and alkaline earth metal hydroxides, such as LiOH, NaOH, KOH, and Ca(OH)₂; alkali metal and alkaline earth metal oxides, such as Li₂O, Na₂O, CaO, and MgO; alkali metal and alkaline earth metal hydrides, such as LiH, NaH, KH and CaH₂; alkali metal and alkaline earth metal carbonates, such as Li₂CO₃, K₂CO₃ and CaCO₃; and alkali metal bicarbonates, such as NaHCO₃.

Compound of formula (1h) and dimethyl but-2-ynedioate may usually be applied in equimolar amounts, i.e. in a molar ratio of approximately 1:1. However, in order to optimize yields, an excess of one of the two compounds may be employed.

Compounds of formula (1k) may then be transformed into compounds of formula (1m) as depicted under Process 8 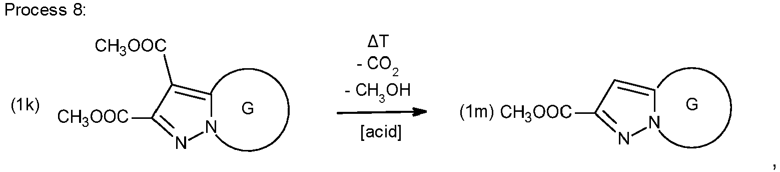 wherein all variables have a meaning as defined for formula (I). This transformation is usually carried out at elevated temperatures of from 50 °C to 150 °C, preferably from 100 °C to 120 °C, in an inert solvent, in the presence of an acid as a catalyst. Suitable solvents are aliphatic hydrocarbons, preferably an aliphatic C₅-C₁₆-hydrocarbon, more preferably a C₅-C₁₆-alkane, or C₅-C₁₆-cycloalkane, such as pentane, hexane, cyclohexane, or petrol ether; aromatic hydrocarbons, preferably an aromatic C₆-C₁₀-hydrocarbons, such as benzene, toluene, o-, m-, and p-xylene; halogenated hydrocarbons, preferably halogenated aliphatic C₁-C₆-alkanes, or halogenated aromatic C₆-C₁₀-hydrocarbons, such as CH₂Cl₂, CHCl₃, CCl₄, CH₂ClCH₂Cl, CCl₃CH₃, CHCl₂CH₂Cl, CCl₂CCl₂, or chlorobenzene; ethers, preferably C₁-C₆-cycloalkyl ethers, C₁-C₆-alkyl-C₁-C₆-alkyl ethers and C₁-C₆-alkyl-C₆-C₁₀-aryl ethers, such as CH₃CH₂OCH₂CH₃, (CH₃)₂CHOCH(CH₃)₂, CH₃OC(CH₃)₃ (MTBE), CH₃OCH₃ (DME), CH₃OCH₂CH₂OCH₃, dioxane, anisole, and tetrahydrofurane (THF); and water. Suitable acids are in general anorganic acids such as HF, HCl, HBr, H₂SO₄, moreover organic acids such as formic acid, acetic acid, propionic acid, oxalic acid, toluene sulphonic acid, benzene sulphonic acid, camphor sulphonic acid, citric acid, and trifluoro acetic acid.

Compounds of formula (1m) may then be halogenated and reacted with a thiolate agent as described analogously in Processes 3, 4, and optionally 5 above if m>0 to yield compounds of formula (1n) 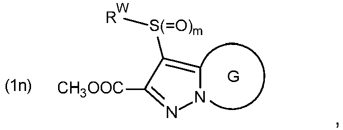 wherein all variables are defined as for formula (I).

Compounds of formula (1n) may then be converted into compounds of formula (1g) by a series of steps that are standard operations in organic chemistry. First, compounds of formula (1n) are saponified under catalysis of a base to yield compounds of formula (1o) as depicted under Process 9 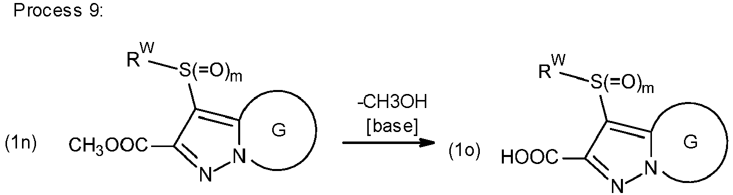 wherein all variables have a meaning as defined for formula (I).

Typical bases that may be employed are inorganic bases as described under Process 1. Typical solvents are water and aqueous compositions comprising water and an additional inert solvent. The reaction is typically carried out at temperatures of from 0 to 150 °C, preferably from 60 to 120 °C.

Subsequently, carboxylic acid compounds of formula (1o) are converted to their corresponding Weinreb-amides of formula (1p) as depicted under Process 10 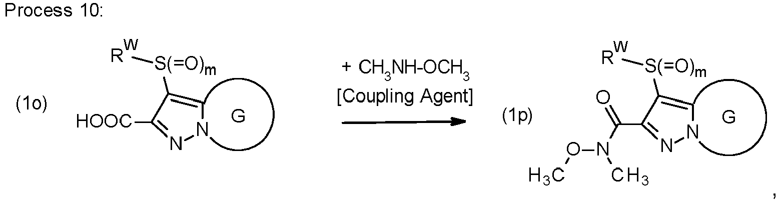 wherein all variables have a meaning as defined for formula (I). The reaction is typically carried out at a temperature of from -5 to 30 °C in an inert solvent in the presence of a Coupling Agent and a base. Typical Coupling Agents are hexafluorophosphate azabenzotriazole tetramethyl uronium (HATU), 3-[Bis(dimethylamino)methyliumyl]-3*H*-benzotriazol-1-oxide hexafluorophosphate (HBTU), O-(1*H*-6-Chlorobenzotriazole-1-yl)-1,1,3,3-tetramethyluronium hexafluorophosphate (HCTU), and propanephosphonic acid anhydride. Typical bases are organic bases as described under Process 1. Suitable inert solvents are are aliphatic hydrocarbons, preferably an aliphatic C₅-C₁₆-hydrocarbon, more preferably a C₅-C₁₆-alkane, or C₅-C₁₆-cycloalkane, such as pentane, hexane, cyclohexane, or petrol ether; aromatic hydrocarbons, preferably an aromatic C₆-C₁₀-hydrocarbons, such as benzene, toluene, o-, m-, and p-xylene; halogenated hydrocarbons, preferably halogenated aliphatic C₁-C₆-alkanes, or halogenated aromatic C₆-C₁₀-hydrocarbons, such as CH₂Cl₂, CHCl₃, CCl₄, CH₂ClCH₂Cl, CCl₃CH₃, CHCl₂CH₂Cl, CCl₂CCl₂, or chlorobenzene; ethers, preferably C₁-C₆-cycloalkyl ethers, C₁-C₆-alkyl-C₁-C₆-alkyl ethers and C₁-C₆-alkyl-C₆-C₁₀-aryl ethers, such as CH₃CH₂OCH₂CH₃, (CH₃)₂CHOCH(CH₃)₂, CH₃OC(CH₃)₃ (MTBE), CH₃OCH₃ (DME), CH₃OCH₂CH₂OCH₃, dioxane, anisole, and tetrahydrofurane (THF); esters, preferably esters of aliphtic C₁-C₆-alcohols with aliphatic C₁-C₆-carboxylic acids, esters of aromatic C₆-C₁₀-alcohols with aromatic C₆-C₁₀-carboxylic adcids, cyclic esters of ω-hydroxy-C₁-C₆-carboxylic acids, such as CH₃C(O)OCH₂CH₃, CH₃C(O)OCH₃, CH₃C(O)OCH₂CH₂CH₂CH₃, CH₃C(O)OCH(CH₃)CH₂CH₃, CH₃C(O)OC(CH₃), CH₃CH₂CH₂C(O)OCH₂CH₃, CH₃CH(OH)C(O)OCH₂CH₃, CH₃CH(OH)C(O)OCH₃, CH₃C(O)OCH₂CH(CH₃)₂, CH₃C(O)OCH(CH₃)₂, CH₃CH₂C(O)OCH₃, benzyl benzoate, and γ-butyrolactone; carbonates, such as ethylene carbonate, propylene carbonate, CH₃CH₂OC(O)OCH₂CH₃, and CH₃OC(O)OCH₃; nitriles, preferably C₁-C₆-nitriles, such as CH₃CN, and CH₃CH₂CN; ketones, preferably C₁-C₆-alkyl-C₁-C₆-alkyl ketones, such as CH₃C(O)CH₃, CH₃C(O)CH₂CH₃, CH₃CH₂C(O)CH₂CH₃, and CH₃C(O)C(CH₃)₃ (MTBK); alcohols, preferably C₁-C₄-alcohols, such as CH₃OH, CH₃CH₂OH, CH₃CH₂CH₂OH, CH₃CH(OH)CH₃, CH₃(CH₂)₃OH, and C(CH₃)₃OH; amides and urea derivatives, preferably dimethyl formamide (DMF), N-methyl-2-pyrrolidone (NMP), dimethyl acetamide (DMA), 1,3-dimethyl-2-imidazolidinone (DMI), 1,3-dimethyl-3,4,5,6-tetrahydro-2(1H)-pyrimidinone (DMPU), hexamethylphosphamide (HMPA); moreover dimethyl sulfoxide (DMSO), sulfolane, and mixtures thereof. Typically, an excess of the coupling agent and the Weinreb amine CH₃NH-OCH₃ are applied.

Weinreb amides of formula (1p) are then reacted with a Grignard reagent to ketones of formula (1q) as depicted under Process 11 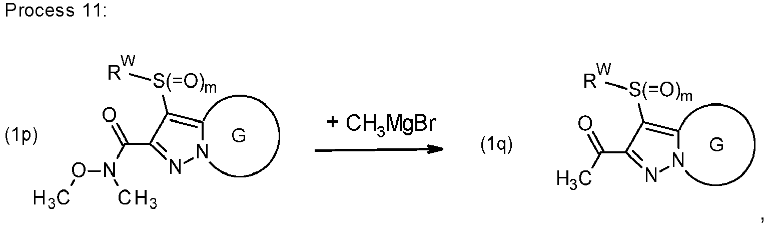 wherein all variables have a meaning as defined for formula (I). Such reactions are carried out at temperatures of from -10 to 30 °C in an inert solvent, typically a polar solvent. Typical solvents include aliphatic hydrocarbons, preferably an aliphatic C₅-C₁₆-hydrocarbon, more preferably a C₅-C₁₆-alkane, or C₅-C₁₆-cycloalkane, such as pentane, hexane, cyclohexane, or petrol ether; aromatic hydrocarbons, preferably an aromatic C₆-C₁₀-hydrocarbons, such as benzene, toluene, o-, m-, and p-xylene; ethers, preferably C₁-C₆-cycloalkyl ethers, C₁-C₆-alkyl-C₁-C₆-alkyl ethers and C₁-C₆-alkyl-C₆-C₁₀-aryl ethers, such as CH₃CH₂OCH₂CH₃, (CH₃)₂CHOCH(CH₃)₂, CH₃OC(CH₃)₃ (MTBE), CH₃OCH₃ (DME), CH₃OCH₂CH₂OCH₃, dioxane, anisole, and tetrahydrofurane (THF).

Finally, compounds of formula 1q may be brominated in Process 12 to yield compounds of formula (1g) 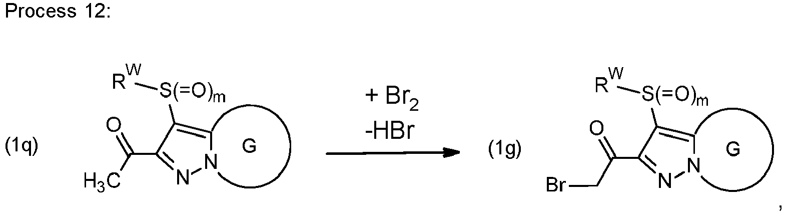 wherein all variables have a meaning as defined for formula (I). Reaction conditions for this type of transformation have been described above for Process 6.

Compounds of formula (III), corresponding to compounds of formula (I) wherein Q is N(R⁵) can be prepared by reaction of compounds of formula (3) with compounds of formula (4), as shown under Process 13 below. 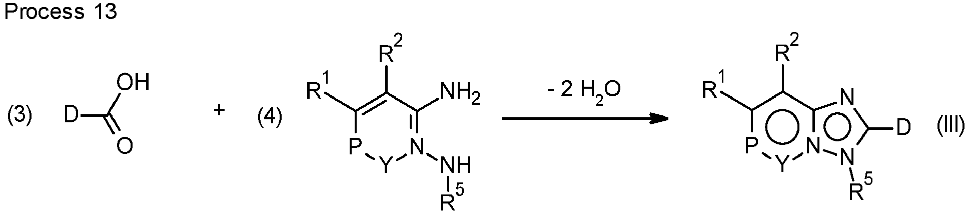 , wherein all variables in formulae (3), (4), and (III) have a meaning as defined for formula (I).

Reactions of this type have been described in EP3257853A1, e.g. p.33-34, and WO2016162318A1, p.90, which passage is incorporated herein by reference. The reaction is typically carried out at elevated temperatures, e.g. 60 to 160 °C, in an inert solvent, optionally in the presence of an acid, or a coupling agent and a base. Suitable solvents are aliphatic hydrocarbons, such as pentane, hexane, cyclohexane, or petrol ether; aromatic hydrocarbons, such as benzene, toluene, o-, m-, and p-xylene; halogenated hydrocarbons, or halogenated aromatic C₆-C₁₀-hydrocarbons, such as CH₂Cl₂, CHCl₃, CCl₄, CH₂ClCH₂Cl, CCl₃CH₃, CHCl₂CH₂Cl, CCl₂CCl₂, or chlorobenzene; ethers, such as CH₃CH₂OCH₂CH₃, (CH₃)₂CHOCH(CH₃)₂, CH₃OC(CH₃)₃ (MTBE), CH₃OCH₃ (DME), CH₃OCH₂CH₂OCH₃, CH₃OC(CH₃)₂CH₂CH₃, dioxane, anisole, 2-methyltetrahydrofuran, tetrahydrofurane (THF), and diethylene glycol; nitriles, such as CH₃CN, and CH₃CH₂CN; alcohols, such as CH₃OH, CH₃CH₂OH, CH₃CH₂CH₂OH, CH₃CH(OH)CH₃, CH₃(CH₂)₃OH, and C(CH₃)₃OH, CH₂(OH)CH₂(OH), and CH₃CH(OH)CH₂OH.. Mixtures of the above solvents are also possible.

Suitable acids are in general inorganic acids such as HF, HCl, HBr, H₂SO₄ and HClO₄; Lewis acids, such as BF₃, AlCl₃, FeCl₃, SnCl₄, TiCl₄ and ZnCl₂, moreover organic acids such as HCOOH, CH₃COOH, CH₃CH₂COOH, oxalic acid, toluene sulphonic acid, benzene sulphonic acid, camphor sulphonic acid, citric acid, and CF₃COOH.

Suitable coupling agents are selected from carbodiimides, such as DCC (dicyclohexylcarbodiimide) and DIC (diisopropylcarbodiimide), benzotriazole derivatives, such as HATU (O-(7-azabenzotriazol-1-yl)-N,N,N',N'-tetramethyluronium hexafluorophosphate), HBTU ((Obenzotriazol-1-yl)-N,N,N',N'-tetramethyluronium hexafluorophosphate) and HCTU (1H-benzotriazolium-1-[bis(dimethylamino)methylene]-5-chloro tetrafluoroborate) and phosphonium-derived activators, such as BOP ((benzotriazol-1-yloxy)-tris(dimethylamino) phosphonium hexafluorophosphate), PyBOP ((benzotriazol-1-yloxy)-tripyrrolidinphosphonium hexafluorophosphate) and PyBrOP (bromotripyrrolidinphosphonium hexafluorophosphate).

Alternatively, compounds of formula (3) may be replaced by their corresponding carbonic acid halogenides, e.g. acid chlorides. In this case the reaction is typically carried out in the presence of a base. Suitable bases are those listed for Process 1 above.

Compounds of formula (3) and compounds of formula (4) are typically reacted with one another in equimolar amounts. In terms of yield, it may be advantageous to employ an excess of compounds of formula (3).

Compounds of formula (3) may be prepared as described in EP3257853A1, p.51-53. Compounds of formula (4) can be prepared as described in WO2017/167832A1, Example C-4, or Bashandy et al. Journal of Enzyme Inhibition and Medicinal Chemistry, 29(5), 619-627, 2014.

Other Compounds of formulae (1) and (3) than those described in EP3257853A1 are available by routine methods of organic chemistry, or are commercially available.

Alternatively, Compounds of formula (III) may be prepared by a two-step reaction, comprising of a reaction of compounds of formula (5) with compounds of formula (2) to yield compounds of formula (6) as displayed under Process 14

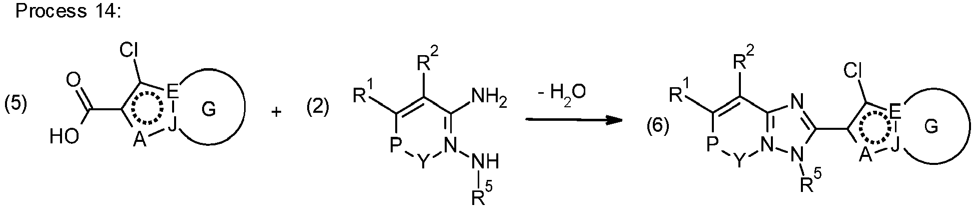

All variables in formulae (2), (5) and (6) have a meaning as defined for formula (I). This reaction may be carried out under the same conditions as described for Process 13 above. Compounds of formula (5) are commercially available, or may be prepared as described in EP3257853A1, [0247] - [0266]

In a second step, compounds of formula (6) are then reacted with a compound of formula (7) to yield compounds of formula (III), falling under the definition of compounds of formula (I), as displayed under Process 15:

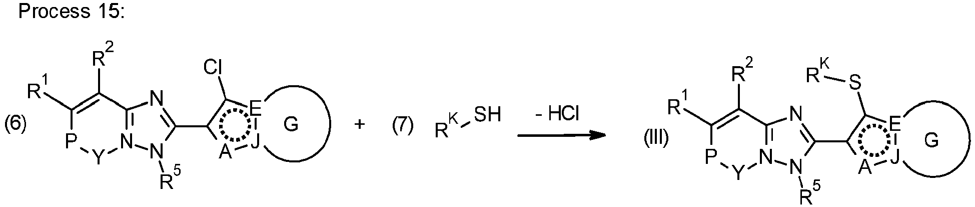

All variables in formulae (6), (7) and (III) have a meaning as defined for formula (I). Reactions of this type have been described in WO2016162318A1, p.89. The reaction is typically carried out at a temperature of from 15 to 60 °C in an inert solvent in the presence of a base. Suitable solvents are aliphatic hydrocarbons, such as pentane, hexane, cyclohexane, or petrol ether; or aromatic hydrocarbons, such as benzene, toluene, o-, m-, and p-xylene;. Mixtures of the above solvents are also possible. Suitable bases are, in general, inorganic bases, preferably alkali metal and alkaline earth metal hydrides, such as LiH, NaH, KH and CaH₂; organic bases, preferably secondary amines, such as pyrrolidine; or tertiary amines, such as diisopropylethylamine, trimethylamine, triethylamine, triisopropylamine and N-methylpiperidine, imidazol, pyridine; substituted pyridines, such as collidine, lutidine and 4-dimethylaminopyridine, and polycyclic amides and amidines, such as 1,8-diazabicycloundec-7-ene (DBU), 1,4-Diazabicyclo[2.2.2]octane (DABCO); or alkali metal salts of secondary amines, such as alkali diisopropylamide, alkali bis(trimethylsilyl)amide, alkali tetramethylpiperidene; alcoholates, such as alkali methanolate, alkali ethanolate, alkali isopropanolate, alkali tert-butanolate; alkali metal - alkyl, and alkali metal - aryl salts, such as n-butyl lithium, tert-butyl lithium, phenyl lithium. The base is typically reacted with compounds of formula (7) before compounds of formula (6) are added to form the thiolate anion.

The bases are generally employed in catalytic amounts; however, they can also be used in equimolar amounts, in excess or, if appropriate, as solvent.

Alternatively, compounds of formula (III) may also be prepared in a Dimroth-like rearrangement of compounds of formula (8), as displayed under Process 16, 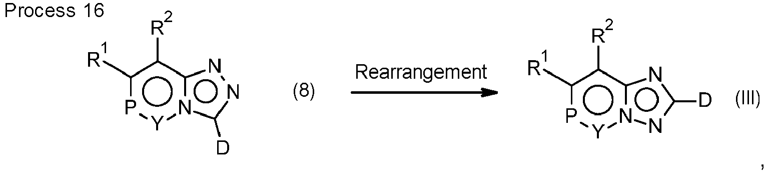 wherein all variables in formulae (8) and (III) have a meaning as defined for formula (I). Reactions of this type have been described in Potts K.T., Surapaneni C.R., 1970, Journal of Heterocyclic Chemistry, or Nagamatsu T., Fujita T., 2002, Heterocycles, 57(4), 631-636. The reaction is typically carried out in the presence of a catalyst, usually an acid or a base, such as NaOH or formic acid, in an inert organic solvent or water at a temperature of from 0 to 80 °C. If no catalyst is used, the reaction may be carried out at elevated temperatrues, e.g. from 30 to 100 °C.

Compounds of formula (8) may be prepared by reaction of hydrazine compounds of formula (9) with orthoesters, as displayed under Process 17, 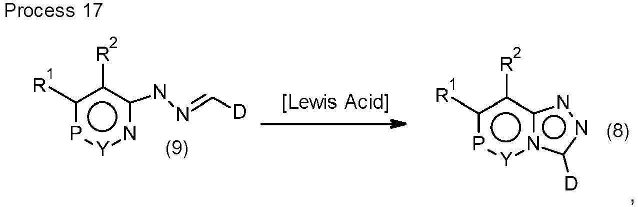 wherein all variables in formulae (8) and (9) have a meaning as defined form formula (I). Reactions of this type have been described by Glushkov V.A. et al., 1998, Pharmaceutical Chemistry Journal, vol.32(5), p.29-32, or WO2012148808, p.143. The reaction is typically performed in the presence of a Lwis acid, such as FeCl₃ or AlCl₃, at elevated temperatures of from 50 to 150 °C in an inert organic solvent. The reaction may be carried out in the presence of an oxidizing agent, e.g. H₂O₂ or CuCl₂.

Compounds of formula (9) are accessible by reaction of hydrazine compounds of formula (10) with aldehyde compounds of formula (11), as displayed under Process 18 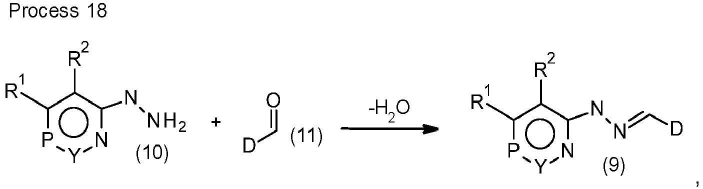 wherein the variables in formulae (9), (10), and (11) have a meaning as defined for formula (I). The reaction is typically carried out in the presence of an acid catalyst, such as toluene sulfonic acid, in an inert organic solvent. Suitable solvents are aliphatic hydrocarbons, such as pentane, hexane, cyclohexane, or petrol ether; or aromatic hydrocarbons, such as benzene, toluene, o-, m-, and p-xylene;. Mixtures of the above solvents are also possible

Hydrazine derivatives of formula (10) are commercially available or may be derived from commercially available compounds. Alternatively, compounds of formula (10) may also be prepared by reaction of hydrazine with compound of formula (12), as displayed under Process 19 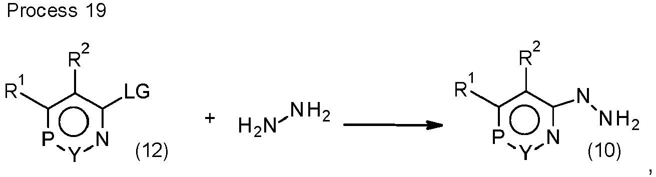 wherein LG is a leaving group, and wherein all other variables have a meaning as defined for formula (I). Typical leaving groups are triflate, iodide and chloride. Reactions of this type have been described in Mao, Y. et al, 2014, Journal of Heterocyclic Chemistry, 51(3), p.594-597. The reaction is typically carried out in a polar solvent, such as CH₃CH₂OH under elevated temperatures, such as from 50 to 100 °C. Compounds of formula (12) are commercially available or can be prepared by standard methods of organic chemistry.

The reaction mixtures are worked up in a customary manner, for example by mixing with water, separating the phases and, if appropriate, chromatographic purification of the crude products. Some of the intermediates and end products are obtained in the form of colorless or slightly brownish viscous oils which are purified or freed from volatile components under reduced pressure and at moderately elevated temperature. If the intermediates and end products are obtained as solids, purification can also be carried out by recrystallization or digestion.

The N-oxides may be prepared from the inventive compounds according to conventional oxidation methods, e. g. by treating compounds I with an organic peracid such as metachloroperbenzoic acid (cf. WO 03/64572 or J. Med. Chem. 38(11), 1892-903, 1995); or with inorganic oxidizing agents such as hydrogen peroxide (cf. J. Heterocyc. Chem. 18(7), 1305-8, 1981) or oxone (cf. J. Am. Chem. Soc. 123(25), 5962-5973, 2001). The oxidation may lead to pure mono-N-oxides or to a mixture of different N-oxides, which can be separated by conventional methods such as chromatography.

If the synthesis yields mixtures of isomers, a separation is generally not necessarily required since in some cases the individual isomers can be interconverted during work-up for use or during application (for example under the action of light, acids or bases). Such conversions may also take place after use, for example in the treatment of plants in the treated plant, or in the harmful fungus to be controlled.

A skilled person will readily understand that the preferences for the substituents, also in particular the ones given in the tables below for the respective substituents, given herein in connection with compounds I apply for the intermediates accordingly. Thereby, the substituents in each case have independently of each other or more preferably in combination the meanings as defined herein.

### Preferences

The variables have, each on their own and in combination, the following preferred meanings. In one embodiment, X is O. In another embodiment of compounds of formula (I), X is S.

In one embodiment of compounds of formula (I), P is NR³ and Q is CR⁶. In another embodiment of compounds of formula (I), P is CR⁴ and Q is NR⁵.

In one embodiment of the present invention the compound of formula (I) is compound of formula (I.A), a compound (I.B) or a compound of formula (I.C).

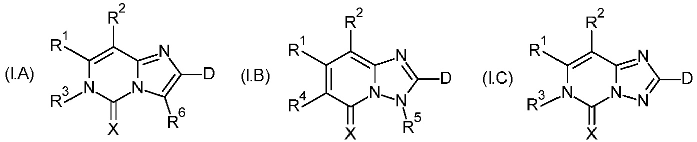

In one embodiment the compounds of formula (I) are compounds of formula (I.A). In another embodiment, the compounds of formula (I) are compounds of formula (I.B). In another embodiment, the compounds of formula (I) are compounds of formula (I.C).

R¹ is H, halogen, C₁-C₆-alkyl, C₂-C₆-alkenyl, C₂-C₆-alkynyl, C₁-C₆-alkoxy, C₁-C₆-alkoxy-C₁-C₆-alkyl, C₃-C₆-cycloalkyl, C₃-C₆-cycloalkoxy, C₁-C₆-sulfenyl, C₁-C₆-sulfinyl, or C₁-C₆-sulfonyl, which groups are unsubstituted or halogenated. In one embodiment, R¹ is H, C₁-C₃-alkyl, C₂-C₃-alkenyl, C₂-C₃-alkynyl, C₁-C₃-alkoxy, C₁-C₃-alkoxy-C₁-C₃-alkyl, C₃-C₅-cycloalkyl, C₃-C₅-cycloalkoxy, which groups are unsubstituted or halogenate. In one embodiment, R¹ is H, C₁-C₃-alkyl, or C₁-C₃-alkoxy, which groups are unsubstituted or halogenated. In another embodiment, R¹ is H, C₁-C₃-alkyl, C₂-C₃-alkenyl, C₂-C₃-alkynyl, C₁-C₃-alkoxy, which groups are unsubstituted or halogenated. In another embodiment, R¹ is C₁-C₃-alkyl, C₁-C₃-alkoxy, which groups are unsubstituted or halogenate. In another embodiment, R¹ is C₁-C₃-haloalkyl, preferably CF₃.

R², R⁴, R⁶ are independently H, halogen, N₃, CN, NO₂, SCN, SF₅; C₁-C₆-alkyl, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxyx-C₁-C₄-alkyl, which groups are unsubstituted or substituted with halogen; C(=O)OR^{A}, NR^{B}R^{C}, NOR^{A}, ONR^{B}R^{C}, C₁-C₆-alkylen-NR^{B}R^{C}, O-C₁-C₆-alkylen-NR^{B}R^{C}, C₁-C₆-alkylen-CN, NH-C₁-C₆-alkylen-NR^{B}R^{C}, C(=O)NR^{B}R^{C}, C(=O)R^{D}, C(=S)R^{D}, SO₂NR^{B}R^{C}, S(=O)ₙR^{E}; phenyl or benzyl, wherein the phenyl ring is unsubstituted or substituted with one or more, same or different substituents R^{F}.

In one embodiment, R² is H, halogen; C₁-C₃-alkyl, C₁-C₃-alkoxy, C₂-C₃-alkenyl, tri-C₁-C₆-alkylsilyl, C₂-C₆-alkynyl, C₁-C₃-alkoxy-C₁-C₂-alkyl, C₁-C₃-alkoxy-C₁-C₂-alkoxy, C₃-C₅-cycloalkyl, C₃-C₅-cycloalkoxy, C₃-C₅-cycloalkyl-C₁-C₂-alkyl, C₃-C₅-cycloalkoxyx-C₁-C₂-alkyl, which groups are unsubstituted or substituted with halogen. In another embodiment, R² is H, halogen; C₁-C₃-alkyl, C₁-C₃-alkoxy, C₂-C₃-alkenyl, C₂-C₆-alkynyl, which groups are unsubstituted or substituted with halogen. In another embodiment, R² is H, halogen; C₁-C₃-alkyl, C₁-C₃-alkoxy, which groups are unsubstituted or substituted with halogen. In another embodiment, R² is H, or C₁-C₃-alkyl.

In one embodiment, R⁴ is H, halogen; C₁-C₃-alkyl, C₁-C₃-alkoxy, C₂-C₃-alkenyl, tri-C₁-C₆-alkylsilyl, C₂-C₆-alkynyl, C₂-C₃-alkoxy-C₁-C₂-alkyl, C₁-C₃-alkoxy-C₁-C₂-alkoxy, C₃-C₅-cycloalkyl, C₃-C₅-cycloalkoxy, C₃-C₅-cycloalkyl-C₁-C₂-alkyl, C₃-C₅-cycloalkoxyx-C₁-C₂-alkyl, which groups are unsubstituted or substituted with halogen. In another embodiment, R⁴ is H, halogen; C₁-C₃-alkyl, C₁-C₃-alkoxy, C₂-C₃-alkenyl, C₂-C₆-alkynyl, which groups are unsubstituted or substituted with halogen. In another embodiment, R⁴ is H, halogen; C₁-C₃-alkyl, C₁-C₃-alkoxy, which groups are unsubstituted or substituted with halogen. In another embodiment, R⁴ is H; C₁-C₃-alkyl, or C₁-C₃-haloalkyl. In another embodiment, R⁴ is H, or C₁-C₃-alkyl. In another embodiment, R⁴ is H.

In one embodiment, R⁶ is H, halogen; C₁-C₃-alkyl, C₁-C₃-alkoxy, C₂-C₃-alkenyl, tri-C₁-C₆-alkylsilyl, C₂-C₆-alkynyl, C₂-C₃-alkoxy-C₁-C₂-alkyl, C₁-C₃-alkoxy-C₁-C₂-alkoxy, C₃-C₅-cycloalkyl, C₃-C₅-cycloalkoxy, C₃-C₅-cycloalkyl-C₁-C₂-alkyl, C₃-C₅-cycloalkoxyx-C₁-C₂-alkyl, which groups are unsubstituted or substituted with halogen. In another embodiment, R⁶ is H, halogen; C₁-C₃-alkyl, C₁-C₃-alkoxy, C₂-C₃-alkenyl, C₂-C₆-alkynyl, which groups are unsubstituted or substituted with halogen. In another embodiment, R⁶ is H, halogen; C₁-C₃-alkyl, C₁-C₃-alkoxy, which groups are unsubstituted or substituted with halogen. In another embodiment, R⁶ is H, C₁-C₃-alkyl, C₁-C₃-haloalkyl. In another embodiment, R⁶ is H, or C₁-C₃-alkyl.

R³, R⁵ are independently C₁-C₆-alkyl, C₁-C₆-alkoxy, C₂-C₆-alkenyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkyl-C₁-C₄-alkyl, or C₃-C₆-cycloalkoxy-C₁-C₄-alkyl, which are unsubstituted or halogenated; C(=O)OR^{A}, NR^{B}R^{C}, C₁-C₆-alkylen-NR^{B}R^{C}, O-C₁-C₆-alkylen-NR^{B}R^{C}, C₁-C₆-alkylen-CN, NH-C₁-C₆-alkylen-NR^{B}R^{C}, C(=O)NR^{B}R^{C}, C(=O)R^{D}, C(=S)R^{D}, SO₂NR^{B}R^{C}, S(=O)ₙR^{E}; phenyl or benzyl, wherein the phenyl ring is unsubstituted or substituted with one or more, same or different substituents R^{F}.

In one embodiment, R³ is C₁-C₆-alkyl, C₁-C₆-alkoxy, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, or C₁-C₃-alkoxy-C₁-C₃-alkyl which are unsubstituted or halogenated; phenyl or benzyl, wherein the phenyl ring is unsubstituted or substituted with one or more, same or different substituents R^{F}. In another embodiment embodiment, R³ is C₁-C₄-alkyl, C₂-C₄-alkenyl, C₂-C₄-alkynyl, C₃-C₆-cycloalkyl, C₃-C₆-cycloalkyl-C₁-C₂-alkyl, or C₁-C₃-alkoxy-C₁-C₃-alkyl, which are unsubstituted or halogenated; phenyl or benzyl, wherein the phenyl ring is unsubstituted or substituted with one or more, same or different substituents R^{F}. In another embodiment, R³ is C₁-C₄-alkyl, C₂-C₄-alkenyl, C₂-C₄-alkynyl, C₃-C₆-cycloalkyl, C₃-C₆-cycloalkyl-C₁-C₂-alkyl, which are unsubstituted or halogenated; phenyl or benzyl, wherein the phenyl ring is unsubstituted or halogenated. In another embodiment, R³ is C₁-C₃-alkyl, C₂-C₃-alkenyl, C₂-C₃-alkynyl, C₃-C₆-cycloalkyl, C₃-C₆-cycloalkyl-C₁-C₂-alkyl, which are unsubstituted or halogenated. In another embodiment, R³ is C₁-C₃-alkyl, C₂-C₃-alkynyl, C₃-C₆-cycloalkyl, C₃-C₆-cycloalkyl-C₁-C₂-alkyl, which are unsubstituted or halogenated. In another embodiment, R³ is C₁-C₃-alkyl, preferably methyl, which are unsubstituted or halogenated.

In one embodiment, R⁵ isC₁-C₆-alkyl, C₁-C₆-alkoxy, C₂-C₆-alkenyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkyl-C₁-C₄-alkyl, or C₃-C₆-cycloalkoxy-C₁-C₄-alkyl, which are unsubstituted or halogenated. In another embodiment embodiment, R⁵ is C₁-C₃-alkyl, C₁-C₃-alkoxy, C₂-C₃-alkenyl, C₂-C₃-alkynyl, C₃-C₆-cycloalkyl, C₃-C₆-cycloalkyl-C₁-C₂-alkyl, which are unsubstituted or halogenated. In another embodiment, R⁵ is C₁-C₃-alkyl, C₂-C₃-alkenyl, C₂-C₃-alkynyl, which are unsubstituted or halogenated. In another embodiment, R⁵ is C₁-C₃-alkyl, or C₁-C₃-haloalkyl. In another embodiment, R⁵ is C₁-C₃-alkyl, preferably methyl, which are unsubstituted or halogenated.

The variable D is a fused bicyclic ring of the following formula 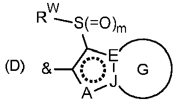 wherein the "&"-symbol signifies the connection to the remainder of formula (I), wherein the dotted circle in the 5-membered ring means that the 5-membered ring may be saturated, partially unsaturated, or fully unsaturated, and wherein the variables have a meaning as defined herein.

The variable A is N, S, O, CR⁷, or NR⁸. In one embodiment, A is N, S, or NR⁸. In another embodiment, A is N. In another embodiment, A is S. In another embodiment, A is NR^{B}. In another embodiment, A is O.

The variables E, J are independently C or N, wherein at least one of the variables selected from E and J is C. In one embodiment, E is N and J is C. In another embodiment, E is C and J is N. Accordingly, in one embodiment, E and A are N and J is C. In another embodiment, J and A are N and E is C.

R⁷ is H, halogen, OH, CN, NC, NO₂, N₃, SCN, NCS, NCO, SF₅; C₁-C₆-alkyl, C₃-C₆-cycloalkyl, C₂-C₆-alkenyl, C₃-C₆-cycloalkenyl, C₂-C₆-alkynyl, which groups are unsubstituted, or substituted with one or more, same or different substituents R^{G}; a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents R^{H}, and wherein said N- and S-atoms are independently oxidized, or non-oxidized;
phenyl, which is unsubstituted, or substituted with one or more, same or different substituents R^{J}; OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, or Si(R^{S})₂R^{T}.

In one embodiment, R⁷ is H, halogen, OH, CN, NC, NO₂, N₃, SF₅; C₁-C₃-alkyl, C₁-C₃-alkoxy, C₃-C₆-cycloalkyl, C₂-C₃-alkenyl, C₃-C₆-cycloalkenyl, C₂-C₃-alkynyl, which groups are unsubstituted or halogenated. In another embodiment, R⁷ is H, halogen; C₁-C₃-alkyl, C₁-C₃-alkoxy, which goups are unsubstituted or halogenated.

R⁸ is H, halogen, CN; C₁-C₆-alkyl, C₃-C₆-cycloalkyl, C₂-C₆-alkenyl, C₃-C₆-cycloalkenyl, C₂-C₆-alkynyl, which groups are unsubstituted, or substituted with one or more, same or different substituents R^{G};
a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents R^{H}, and wherein said N- and S-atoms are independently oxidized, or non-oxidized; phenyl, which is unsubstituted, or substituted with one or more, same or different substituents R^{J}; OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, or Si(R^{S})₂R^{T}.

In one embodiment, R⁸ is H, halogen, OH, CN, NC, NO₂, N₃, SF₅; C₁-C₃-alkyl, C₁-C₃-alkoxy, C₃-C₆-cycloalkyl, C₂-C₃-alkenyl, C₃-C₆-cycloalkenyl, C₂-C₃-alkynyl, which groups are unsubstituted or halogenated. In another embodiment, R⁸ is H, halogen; C₁-C₃-alkyl, C₁-C₃-alkoxy, which goups are unsubstituted or halogenated.

Each R⁹ is independently H, halogen, OH, CN, NC, NO₂, N₃, SCN, NCS, NCO, SF₅; C₁-C₆-alkyl, C₃-C₆-cycloalkyl, C₂-C₆-alkenyl, C₃-C₆-cycloalkenyl, C₂-C₆-alkynyl, C₃-C₆-cycloalkyl-C₁-C₃-alkyl, which groups are unsubstituted, or substituted with one or more, same or different substituents R^{G}; a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents R^{H}, and wherein said N- and S-atoms are independently oxidized, or non-oxidized; phenyl, which is unsubstituted, or substituted with one or more, same or different substituents R^{J}; OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T}; or two substituents R⁹ form, together with the ring members of ring G to which they are bound, a 5- or 6- membered saturated, partially unsaturated, or fully unsaturated carbo- or heterocycle, which carbo- or heterocycle is unsubstituted, or substituted with one or more, same or different substituents R^{J}, and wherein said heterocycle comprises one or more, same or different heteroatoms O, N, or S.

In one embodiment, each R⁹ is independently H, halogen, OH, CN, NO₂, SF₅; C₁-C₃-alkyl, C₃-C₆-cycloalkyl, C₂-C₃-alkenyl, C₃-C₆-cycloalkenyl, C₂-C₃-alkynyl, C₃-C₆-cycloalkyl-C₁-C₂-alkyl, which groups are unsubstituted, or substituted with one or more, same or different substituents R^{G}; a 5- to 6-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents R^{H}, and wherein said N- and S-atoms are independently oxidized, or non-oxidized; phenyl, which is unsubstituted, or substituted with one or more, same or different substituents R^{J}; OR^{K}, SR^{K}, OC(=O)R^{K}, OC(=O)OR^{K}, OC(=O)NR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, C(=O)R^{P}, C(=O)NR^{L}R^{M}, C(=O)OR^{K}; or two substituents R⁹ form, together with the ring members of ring G to which they are bound, a 5- or 6- membered saturated, partially unsaturated, or fully unsaturated carbo- or heterocycle, which carbo- or heterocycle is unsubstituted, or substituted with one or more, same or different substituents R^{J}, and wherein said heterocycle comprises one or more, same or different heteroatoms O, N, or S.

In another embodiment, each R⁹ is independently H, halogen, OH, CN; C₁-C₃-alkyl, C₃-C₆-cycloalkyl, C₂-C₃-alkenyl, C₃-C₆-cycloalkenyl, C₂-C₃-alkynyl, C₃-C₆-cycloalkyl-C₁-C₂-alkyl, which groups are unsubstituted, or substituted with one or more, same or different substituents R^{G}; phenyl, which is unsubstituted, or substituted with one or more, same or different substituents R^{J}; OR^{K}, SR^{K}, OC(=O)R^{K}, OC(=O)OR^{K}, OC(=O)NR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, C(=O)R^{P}, C(=O)NR^{L}R^{M}, or C(=O)OR^{K}.

In another embodiment, each R⁹ is independently H, halogen, OH, CN; C₁-C₃-alkyl, C₁-C₃-alkoxy, C₂-C₃-alkenyl, or C₂-C₃-alkynyl, which groups are unsubstituted, or halogenated.

In another embodiment, each R⁹ is independently H, halogen, OH, CN; C₁-C₃-alkyl, C₁-C₃-alkoxy, C₂-C₃-alkenyl, or C₂-C₃-alkynyl, which groups are unsubstituted, or halogenated, wherein at least one substituent R⁹ is C₁-C₃-alkyl, or C₃-C₆-cylcloalkyl, which groups are substituted with CN, e.g. 1-cyano-cyclopropyl and 1-cyanoisopropyl. In another embodiment, R⁹ is H, C₁-C₃-alkyl, or C₁-C₃-haloalkyl.

In another embodiment, each R⁹ is independently H, halogen, OH, CN; C₁-C₃-alkyl, C₁-C₃-alkoxy, C₂-C₃-alkenyl, or C₂-C₃-alkynyl, which groups are unsubstituted, or halogenated, wherein at least one substituent R⁹ is -C(CN)R⁹¹ R⁹²; or C₃-C₆-cycloalkyl, which is substituted with CN and which either does not have any further substituents, or which is further substituted with one or more, same or different substituents R⁹³.

In another embodiment, at least one R⁹ is -C(CN)R⁹¹R⁹²; or C₃-C₆-cycloalkyl, which is substituted with CN and which either does not have any further substituents, or which is further substituted with one or more, same or different substituents R⁹³.

Each of R⁹¹, R⁹² are independently halogen, CN, C₁-C₆-alkyl, C₁-C₆-haloalkyl, C₃-C₆-cycloalkyl, C₁-C₃-alkoxy, C₁-C₄-alkoxy-C₁-C₄-alkyl, C₁-C₄-alkylsulfanyl, or C₁-C₄-alkoxycarbonyl. In one embodiment, each of R⁹¹ and R⁹² are independently halogen, CN, C₁-C₆-alkyl, or C₁-C₆-haloalkyl. Typically, R⁹¹ and R⁹² are both not H. Preferably, R⁹¹ and R⁹² are independently selected C₁-C₆-alkyl, C₁-C₆-haloalkyl, C₃-C₆-cycloalkyl, C₁-C₄-alkoxy, and C₁-C₄-alkoxy-C₁-C₄-alkyl, most preferably from C₁-C₆-alkyl, C₁-C₆-haloalkyl, C₃-C₆-cycloalkyl, and C₁-C₄-alkoxy, especially preferably from C₁-C₆-alkyl and C₁-C₆-haloalkyl, in particular from C₁-C₃-alkyl and C₁-C₃-haloalkyl, such as from C₁-C₃-alkyl. In a particularly preferred embodiment, both R⁹¹ and R⁹² are CH₃.

Each R⁹³ is independently halogen, CN, C(=O)H, OH, C₃-C₆-cycloalkyl, C(=O)OH, C(=O)NH₂, C₁-C₄-haloalkoxy, C₁-C₄-alkoxy, C₁-C₄-haloalkylsulfanyl, C₁-C₄-haloalkylsulfinyl, C₁-C₄-haloalkylsulfonyl, C₁-C₄-alkoxycarbonyl, C₁-C₄-haloalkoxycarbonyl, C₁-C₄-alkylcarbonyl, C₁-C₄-haloalkylcarbonyl, di-(C₁-C₄)alkylaminocarbonyl, C₁-C₄-alkylaminocarbonyl, C₁-C₄-alkylcarbonylamino, di-(C₁-C₄)alkylcarbonylamino, C₁-C₄-alkoxycarbonylamino, C₁-C₄-alkyl which is unsubstituted or halogenated. In one embodiment, R⁹³ is halogen, CN, C₁-C₃-alkyl, or C₁-C₃-cyanoalkyl.

In another embodiment, two substituents R⁹ form, together with the ring members of ring G to which they are bound, a 5- or 6- membered saturated, partially unsaturated, or fully unsaturated carbo- or heterocycle, which carbo- or heterocycle is unsubstituted, or substituted with one or more, same or different substituents R^{J}, and wherein said heterocycle comprises one or more, same or different heteroatoms O, N, or S.

Each R^{A} is independently H; C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; C₁-C₆-alkylen-NR^{b}R^{c}, C₁-C₆-alkylen-CN; or phenyl or benzyl, wherein the phenyl ring is unsubstituted or substituted with one or more, same or different substitutents R^{F}.

In one embodiment, each R^{A} is independently H; C₁-C₃-alkyl, C₂-C₃-alkenyl, C₂-C₃-alkynyl, C₁-C₃-alkoxy-C₁-C₂-alkyl, C₃-C₆-cycloalkyl, C₃-C₆-cycloalkyl-C₁-C₂-alkyl, C₃-C₆-cycloalkoxy-C₁-C₂-alkyl, which groups are unsubstituted or substituted with halogen; or phenyl or benzyl, wherein the phenyl ring is unsubstituted or substituted with one or more, same or different substitutents R^{F}.

In one embodiment, each R^{A} is independently H; C₁-C₃-alkyl, C₂-C₃-alkenyl, C₂-C₃-alkynyl, C₃-C₆-cycloalkyl, which groups are unsubstituted or substituted with halogen; or phenyl or benzyl, wherein the phenyl ring is unsubstituted or substituted with one or more, same or different substitutents selected from halogen, C₁-C₃-alkyl, C₁-C₃-alkoxy, C₁-C₃-haloalkoxy, and C₁-C₃-haloalkyl.

Each R^{B} is independently H; C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; C₁-C₆-alkylen-CN; phenyl and benzyl, which groups are unsubstituted or substituted with one or more, same or different substituents R^{F}.

In one embodiment, each R^{B} is independently H; C₁-C₃-alkyl, C₂-C₃-alkenyl, C₂-C₃-alkynyl, C₃-C₆-cycloalkyl, C₃-C₆-cycloalkyl-C₁-C₄-alkyl, which groups are unsubstituted or substituted with halogen; phenyl or benzyl, which groups are unsubstituted or substituted with one or more, same or different substituents R^{F}.

In another embodiment, each R^{B} is independently H; C₁-C₃-alkyl, C₂-C₃-alkenyl, C₂-C₃-alkynyl, C₃-C₆-cycloalkyl, which groups are unsubstituted or substituted with halogen; phenyl or benzyl, which groups are unsubstituted or substituted with one or more, same or different substituents selected from halogen, C₁-C₃-alkyl, C₁-C₃-alkoxy, C₁-C₃-haloalkoxy, and C₁-C₃-haloalkyl.

Each R^{C} is independently H; C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; C₁-C₆-alkylen-CN; or phenyl or benzyl, wherein the phenyl ring is unsubstituted or substituted with one or more, same or different substituents R^{F}.

In one embodiment, each R^{C} is independently H; C₁-C₃-alkyl, C₂-C₃-alkenyl, C₂-C₃-alkynyl, C₃-C₆-cycloalkyl, C₃-C₆-cycloalkyl-C₁-C₄-alkyl, which groups are unsubstituted or substituted with halogen; phenyl or benzyl, which groups are unsubstituted or substituted with one or more, same or different substituents R^{F}.

In another embodiment, each R^{C} is independently H; C₁-C₃-alkyl, C₂-C₃-alkenyl, C₂-C₃-alkynyl, C₃-C₆-cycloalkyl, which groups are unsubstituted or substituted with halogen; phenyl or benzyl, which groups are unsubstituted or substituted with one or more, same or different substituents selected from halogen, C₁-C₃-alkyl, C₁-C₃-alkoxy, C₁-C₃-haloalkoxy, and C₁-C₃-haloalkyl.

Alternatively, each moiety NR^{B}R^{C} may also form an N-bound, saturated 5- to 8-membered heterocycle, which in addition to the nitrogen atom may have 1 or 2 further heteroatoms or heteroatom moieties selected from O, S(=O)ₘ and N-R', wherein R' is H or C₁-C₆-alkyl and wherein the N-bound heterocycle is unsubstituted or substituted with one or more, same or different substituents selected from halogen, C₁-C₄-alkyl, C₁-C₄-haloalkyl, C₁-C₄-alkoxy and C₁₋C₄-haloalkoxy. In one embodiment, each moiety NR^{B}R^{C} may also form an N-bound, saturated 5- to 6-membered heterocycle, wherein the N-bound heterocycle is unsubstituted or substituted with one or more, same or different substituents selected from halogen, C₁-C₃-alkyl, C₁-C₃-haloalkyl, C₁-C₃-alkoxy and C₁-C₃-haloalkoxy.

Each R^{D} is independently H; C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; phenyl or benzyl, wherein the phenyl ring is unsubstituted or substituted with one or more, same or different substituents R^{F}.

In one embodiment, each R^{D} is independently C₁-C₃-alkyl, C₂-C₃-alkenyl, C₂-C₃-alkynyl, C₃-C₆-cycloalkyl, C₃-C₆-cycloalkyl-C₁-C₄-alkyl, which groups are unsubstituted or substituted with halogen; phenyl or benzyl, which groups are unsubstituted or substituted with one or more, same or different substituents R^{F}. In another embodiment, each R^{D} is independently C₁-C₃-alkyl, C₂-C₃-alkenyl, C₂-C₃-alkynyl, C₃-C₆-cycloalkyl, which groups are unsubstituted or substituted with halogen; phenyl or benzyl, which groups are unsubstituted or substituted with one or more, same or different substituents selected from halogen, C₁-C₃-alkyl, C₁-C₃-alkoxy, C₁₋C₃-haloalkoxy, and C₁-C₃-haloalkyl.

Each R^{E} is indepentently C₁-C₆-alkyl, C₃-C₆-cycloalkyl, C₃-C₆-cycloalkyl-C₁-C₄-alkyl, which are unsubstituted or substituted with halogen; or phenyl or benzyl, wherein the phenyl ring is unsubstituted or substituted with R^{F}.

In one embodiment, each R^{E} is indepentently C₁-C₃-alkyl, C₃-C₆-cycloalkyl, C₃-C₆-cycloalkyl-C₁-C₂-alkyl, which are unsubstituted or substituted with halogen; or phenyl or benzyl, wherein the phenyl ring is unsubstituted or substituted with one or more, same or different substituents selected from halogen, C₁-C₃-alkyl, C₁-C₃-alkoxy, C₁-C₃-haloalkoxy, and C₁-C₃-haloalkyl. In one embodiment, each R^{E} is indepentently C₁-C₃-alkyl, or C₁-C₃-haloalkyl.

Each R^{F} is independently halogen, N₃, OH, CN, NO₂, SCN, SF₅; C₁-C₆ alkyl, C₁-C₆ alkoxy, C₂-C₆ alkenyl, C₂-C₆ alkynyl, C₁-C₆ alkoxy-C₁-C₄ alkyl, C₁-C₆ alkoxy-C₁-C₄ alkoxy, C₃-C₆ cycloalkyl, C₃-C₆ cycloalkoxy, C₃-C₆ cycloalkyl-C₁-C₄ alkyl, C₃-C₆ cycloalkoxy-C₁-C₄ alkyl, which groups are unsubstituted or substituted with halogen. In one embdodiment, each R^{F} is independently halogen, OH, CN, NO₂; C₁-C₃-alkyl, C₁-C₃-alkoxy, C₂-C₃ alkenyl, C₂-C₃-alkynyl, C₃-C₆-cycloalkyl, which groups are unsubstituted or substituted with halogen. In another embdodiment, each R^{F} is independently halogen; C₁-C₃-alkyl, C₁-C₃-alkoxy, C₂-C₃ alkenyl, C₂-C₃-alkynyl, which groups are unsubstituted or substituted with halogen. In another embodiment, each R^{F} is independently halogen, C₁-C₃-alkyl, or C₁-C₃-haloalkyl.

Each R^{G} is independently halogen, OH, CN, NC, NO₂; C₁-C₆-alkyl, C₃-C₆-cycloalkyl, C₃-C₆-cycloalkenyl, which groups are unsubstituted, or substituted with one or more, same or different substituents selected from halogen, OH, CN, C₁-C₃-alkoxy, C₁-C₃-haloalkoxy, and C₁-C₃-alkyl-carbonyl; a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents selected from halogen, OH, CN, C₁-C₃-alkoxy, C₁-C₃-haloalkoxy, and C₁-C₃-alkyl-carbonyl, and wherein said N- and S-atoms are independently oxidized, or non-oxidized; phenyl, which is unsubstituted, or substituted with one or more, same or different substituents selected from halogen, OH, CN, NO₂, C₁-C₃-alkyl, C₁-C₃-haloalkyl, C₁-C₃-alkoxy, C₁-C₃-haloalkoxy, and C₁-C₃-alkyl-carbonyl; OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T}.

In one embodiment, each R^{G} is independently halogen, OH, CN; C₁-C₃-alkyl, C₃-C₆-cycloalkyl, C₃-C₆-cycloalkenyl, which groups are unsubstituted, or substituted with one or more, same or different substituents selected from halogen, OH, CN, C₁-C₃-alkoxy, C₁-C₃-haloalkoxy, and C₁-C₃-alkylcarbonyl; a 5- to 6-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents selected from halogen, OH, CN, C₁-C₃-alkoxy, C₁-C₃-haloalkoxy, and C₁-C₃-alkyl-carbonyl, and wherein said N- and S-atoms are independently oxidized, or non-oxidized; phenyl, which is unsubstituted, or substituted with one or more, same or different substituents selected from halogen, OH, CN, NO₂, C₁-C₃-alkyl, C₁₋C₃-haloalkyl, C₁₋C₃-alkoxy, C₁-C₃-haloalkoxy, and C₁-C₃-alkyl-carbonyl; OR^{K}, SR^{K}, OC(=O)R^{K}, OC(=O)OR^{K}, OC(=O)NR^{L}R^{M}, ONR^{L}R^{M}, ON=CR^{N}R^{O}, NR^{L}R^{M}, NOR^{K}, ONR^{L}R^{M}, NNR^{L}, N(R^{L})C(=O)R^{K}, N(R^{L})C(=O)OR^{K}, C(=O)R^{P}, C(=S)R^{P}, C(=O)NR^{L}R^{M}, C(=O)OR^{K}. In one embodiment, each R^{G} is independently halogen, CN; C₁-C₃-alkyl, C₁-C₃-alkoxy, C₁-C₃-haloalkyl, C₁-C₃-haloalkoxy, or phenyl. In another embodiment, each R^{G} is independently halogen, C₁-C₃-alkyl, C₁-C₃-alkoxy, C₁-C₃-haloalkyl, or C₁-C₃-haloalkoxy. In another embodiment, each R^{G} is independently halogen.

Each R^{H} is independently halogen, CN, NC, NO₂, SCN, NCS, NCO; C₁-C₆-alkyl, C₃-C₆-cycloalkyl, C₃-C₆-cycloalkenyl, which groups are unsubstituted, or substituted with one or more, same or different substituents selected from halogen, OH, CN, C₁₋C₁₀-alkoxy, C₁-C₃-haloalkoxy, and C₁-C₃-alkyl-carbonyl;
phenyl, which is unsubstituted, or substituted with one or more, same or different substituents selected from halogen, OH, CN, NO₂, C₁-C₃-alkyl, C₁-C₃-haloalkyl, OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T}; or two geminal substituents R^{H} form together with the atom to which they are bound a group =O, =S, or=NR^{L}. In one embodiment, each R^{H} is independently halogen, CN; C₁-C₃-alkyl, C₁-C₃-haloalkyl, C₁-C₃-alkoxy, or C₁-C₃-haloalkoxy.

Each R^{J} is independently halogen, CN, NC, NO₂, SCN, NCS, NCO; C₁-C₆-alkyl, C₃-C₆-cycloalkyl, C₃-C₆-cycloalkenyl, which groups are unsubstituted, or substituted with one or more, same or different substituents selected from halogen, OH, CN, C₁-C₁₀-alkoxy, C₁-C₃-haloalkoxy, and C₁-C₃-alkyl-carbonyl; phenyl, which is unsubstituted, or substituted with one or more, same or different substituents selected from halogen, OH, CN, NO₂, C₁-C₃-alkyl, C₁-C₃-haloalkyl, OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R},or Si(R^{S})₂R^{T}. In one embodiment, each R^{J} is independently halogen, CN; C₁-C₃-alkyl, C₁-C₃-haloalkyl, C₁-C₃-alkoxy, or C₁-C₃-haloalkoxy.

Each R^{K} is independently H; C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with one or more, same or different substituents selected from halogen, CN, NR^{M}R^{N}; C(=O)NR^{M}R^{N}, C(=O)R^{T}; or phenyl or benzyl, wherein the phenyl ring is unsubstituted or substituted with one or more, same or different substitutents R^{X}.

In one embodiment, each R^{K} is independently C₁-C₃-alkyl, C₂-C₃-alkenyl, C₂-C₃-alkynyl, C₃-C₆-cycloalkyl, C₃-C₆-cycloalkyl-C₁-C₄-alkyl, which groups are unsubstituted or substituted with halogen; phenyl or benzyl, which groups are unsubstituted or substituted with one or more, same or different substituents R^{X}. In another embodiment, each R^{K} is independently C₁-C₃-alkyl, C₂-C₃-alkenyl, C₂-C₃-alkynyl, C₃-C₆-cycloalkyl, which groups are unsubstituted or substituted with halogen; phenyl or benzyl, which groups are unsubstituted or substituted with one or more, same or different substituents selected from halogen, C₁-C₃-alkyl, C₁-C₃-alkoxy, C₁-C₃-haloalkoxy, and C₁-C₃-haloalkyl.

Each R^{L} is independently H; C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; C₁-C₆-alkylen-CN; phenyl and benzyl, which groups are unsubstituted or substituted with one or more, same or different substituents R^{X}.

In one embodiment, each R^{L} is independently H; C₁-C₃-alkyl, C₂-C₃-alkenyl, C₂-C₃-alkynyl, C₃-C₆-cycloalkyl, C₃-C₆-cycloalkyl-C₁-C₄-alkyl, which groups are unsubstituted or substituted with halogen; phenyl or benzyl, which groups are unsubstituted or substituted with one or more, same or different substituents R^{X}. In another embodiment, each R^{L} is independently H; C₁-C₃-alkyl, C₂-C₃-alkenyl, C₂-C₃-alkynyl, C₃-C₆-cycloalkyl, which groups are unsubstituted or substituted with halogen; phenyl or benzyl, which groups are unsubstituted or substituted with one or more, same or different substituents selected from halogen, C₁-C₃-alkyl, C₁-C₃-alkoxy, C₁-C₃-haloalkoxy, and C₁-C₃-haloalkyl.

Each R^{M}, R^{R} is independently H; C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; C₁-C₆-alkylen-CN; or phenyl or benzyl, wherein the phenyl ring is unsubstituted or substituted with one or more, same or different substituents R^{X}.

In one embodiment, each R^{M}, R^{R} is independently H; C₁-C₃-alkyl, C₂-C₃-alkenyl, C₂-C₃-alkynyl, C₃-C₆-cycloalkyl, C₃-C₆-cycloalkyl-C₁-C₄-alkyl, which groups are unsubstituted or substituted with halogen; phenyl or benzyl, which groups are unsubstituted or substituted with one or more, same or different substituents R^{X}. In another embodiment, each R^{M}, R^{R} is independently H; C₁-C₃-alkyl, C₂-C₃-alkenyl, C₂-C₃-alkynyl, C₃-C₆-cycloalkyl, which groups are unsubstituted or substituted with halogen; phenyl or benzyl, which groups are unsubstituted or substituted with one or more, same or different substituents selected from halogen, C₁-C₃-alkyl, C₁-C₃-alkoxy, C₁-C₃-haloalkoxy, and C₁-C₃-haloalkyl.

Alternatively, each moiety NR^{M}R^{R}, or NR^{L}R^{M} may also form an N-bound, saturated 5- to 8-membered heterocycle, which in addition to the nitrogen atom may have 1 or 2 further heteroatoms or heteroatom moieties selected from O, S(=O)ₘ and N-R', wherein R' is H or C₁-C₆-alkyl and wherein the N-bound heterocycle is unsubstituted or substituted with one or more, same or different substituents selected from halogen, C₁-C₄-alkyl, C₁-C₄-haloalkyl, C₁-C₄-alkoxy and C₁-C₄-haloalkoxy. In one embodiment, each moiety NR^{M}R^{R}, or NR^{L}R^{M} may also form an N-bound, saturated 5- to 6-membered heterocycle, wherein the N-bound heterocycle is unsubstituted or substituted with one or more, same or different substituents selected from halogen, C₁-C₃-alkyl, C₁-C₃-haloalkyl, C₁-C₃-alkoxy and C₁-C₃-haloalkoxy.

Each R^{N} is independently H, halogen, CN, NO₂, SCN; C₁-C₁₀-alkyl, C₃-C₈-cycloalkyl, C₂-C₁₀-alkenyl, C₃-C₈-cycloalkenyl, C₂-C₁₀-alkynyl, which groups are unsubstituted, or substituted with one or more, same or different substituents selected from halogen, C₁-C₆-alkyl, C₁-C₆-alkoxy, C₁-C₆-haloalkyl, and C₁-C₆-haloalkoxy; a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents selected from halogen, C₁-C₃-alkyl, C₁-C₃-alkoxy, C₁-C₃-haloalkyl, and C₁-C₃-haloalkoxy, and wherein said N- and S-atoms are independently oxidized, or non-oxidized; phenyl, which is unsubstituted, or substituted with one or more, same or different substituents selected from halogen, C₁-C₃-alkyl, C₁-C₃-alkoxy, C₁-C₃-haloalkyl, and C₁-C₃-haloalkoxy.

In one embodiment, each R^{N} is independently C₁-C₃-alkyl, C₂-C₃-alkenyl, C₂-C₃-alkynyl, C₃-C₆-cycloalkyl, which groups are unsubstituted or substituted with halogen; phenyl, which is unsubstituted or substituted with one or more, same or different substituents selected from halogen, C₁-C₃-alkyl, C₁-C₃-alkoxy, C₁-C₃-haloalkyl, and C₁-C₃-haloalkoxy. In another embodiment, each R^{N} is independently C₁-C₃-alkyl, C₂-C₃-alkenyl, C₂-C₃-alkynyl, C₃-C₆-cycloalkyl, which groups are unsubstituted or substituted with halogen; phenyl, which is unsubstituted or substituted with one or more, same or different substituents selected from halogen, C₁-C₃-alkyl, C₁-C₃-alkoxy, C₁-C₃-haloalkyl, and C₁-C₃-haloalkoxy.

Each R^{O} is independently H, C₁-C₄-alkyl, C₁-C₆-cycloalkyl, C₁-C₂-alkoxy-C₁-C₂-alkyl, phenyl, or benzyl;
In one embodiment, each R° is independently H, or C₁-C₃-alkyl.

Each R^{P} is independently H; C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; phenyl or benzyl, wherein the phenyl ring is unsubstituted or substituted with one or more, same or different substituents R^{X}.

In one embodiment, each R^{P} is independently C₁-C₃-alkyl, C₂-C₃-alkenyl, C₂-C₃-alkynyl, C₃-C₆-cycloalkyl, C₃-C₆-cycloalkyl-C₁-C₄-alkyl, which groups are unsubstituted or substituted with halogen; phenyl or benzyl, which groups are unsubstituted or substituted with one or more, same or different substituents R^{X}. In another embodiment, each R^{P} is independently C₁-C₃-alkyl, C₂-C₃-alkenyl, C₂-C₃-alkynyl, C₃-C₆-cycloalkyl, which groups are unsubstituted or substituted with halogen; phenyl or benzyl, which groups are unsubstituted or substituted with one or more, same or different substituents selected from halogen, C₁-C₃-alkyl, C₁-C₃-alkoxy, C₁-C₃-haloalkoxy, and C₁-C₃-haloalkyl.

Each R^{S}, R^{T} is independently H, C₁-C₁₀-alkyl, C₁-C₆-haloalkyl, C₁-C₁₀-alkoxy, C₁-C₄-alkoxy-C₁-C₄-alkyl, C₃-C₈-cycloalkyl, C₃-C₈-halocycloalkyl, C₁-C₄-haloalkoxy-C₁-C₄-alkyl, or phenyl. In one embodiment, each ach R^{S}, R^{T} is independently H, C₁-C₃-alkyl, or C₁-C₃-haloalkyl.

Each R^{V} is indepentently C₁-C₆-alkyl, C₃-C₆-cycloalkyl, C₃-C₆-cycloalkyl-C₁-C₄-alkyl, which are unsubstituted or substituted with halogen; or phenyl or benzyl, wherein the phenyl ring is unsubstituted or substituted with R^{X}. In one embodiment, each R^{V} is indepentently C₁-C₃-alkyl, C₁-C₃-haloalkyl; or phenyl or benzyl, wherein the phenyl ring is unsubstituted or halogenated.

R^{W} is is indepentently C₁-C₆-alkyl, C₃-C₆-cycloalkyl, C₃-C₆-cycloalkyl-C₁-C₄-alkyl, which are unsubstituted or substituted with halogen; or phenyl or benzyl, wherein the phenyl ring is unsubstituted or substituted with one or more, same or different substituents R^{X}. In one embodiment, each R^{W} is indepentently C₁-C₃-alkyl, C₃-C₆-cycloalkyl, C₃-C₆-cycloalkyl-C₁-C₂-alkyl, which are unsubstituted or substituted with halogen; or phenyl or benzyl, wherein the phenyl ring is unsubstituted or substituted with one or more, same or different substituents selected from halogen, C₁-C₃-alkyl, C₁-C₃-alkoxy, C₁-C₃-haloalkoxy, and C₁-C₃-haloalkyl. In one embodiment, each R^{W} is indepentently C₁-C₃-alkyl, or C₁-C₃-haloalkyl.

Each R^{X} is independently halogen, N₃, OH, CN, NO₂, SCN, SF₅; C₁-C₆ alkyl, C₁-C₆ alkoxy, C₂-C₆ alkenyl, C₂-C₆ alkynyl, C₁-C₆ alkoxy-C₁-C₄ alkyl, C₁-C₆ alkoxy-C₁-C₄ alkoxy, C₃-C₆ cycloalkyl, C₃-C₆ cycloalkoxy, C₃-C₆ cycloalkyl-C₁-C₄ alkyl, C₃-C₆ cycloalkoxy-C₁-C₄ alkyl, which groups are unsubstituted or substituted with halogen. In one embdodiment, each R^{X} is independently halogen, OH, CN, NO₂; C₁-C₃-alkyl, C₁-C₃-alkoxy, C₂-C₃ alkenyl, C₂-C₃-alkynyl, C₃-C₆-cycloalkyl, which groups are unsubstituted or substituted with halogen. In another embdodiment, each R^{X} is independently halogen; C₁-C₃-alkyl, C₁-C₃-alkoxy, C₂-C₃ alkenyl, C₂-C₃-alkynyl, which groups are unsubstituted or substituted with halogen. In another embodiment, each R^{X} is independently halogen, C₁-C₃-alkyl, or C₁-C₃-haloalkyl.

The index m is 0, 1, or 2. In one embodiment, the index m is 2. In another embodiment, the index m is 0. The index n is 0, 1, 2, or 3. In one embodiment, the index n is 2. In another embodiment, the index n is 3.

The variable G represents G is a 5- or 6-membered saturated, partially unsaturated, or fully unsaturated carbo- or heterocycle, which carbo- or heterocycle includes the atoms E and J as ring members and is unsubstituted, or substituted with one or more, same or different substituents R⁹, and wherein said heterocycle comprises no, one or more, same or different heteroatoms O, N, or S as ring members in addition to those that may be present as ring members E and J.

In one embodiment, the variable G represents a 6-membered saturated, partially unsaturated, or fully unsaturated carbo- or heterocycle, which carbo- or heterocycle includes the atoms E and J as ring members and is unsubstituted, or substituted with one or more, same or different substituents R⁹, and wherein said heterocycle comprises no, one or more, same or different heteroatoms O, N, or S as ring members in addition to those that may be present as ring members E and J. In one embodiment, the variable G represents a 6-membered saturated, partially unsaturated, or fully unsaturated carbo- or heterocycle, which carbo- or heterocycle includes the atoms E and J as ring members and is unsubstituted, or substituted with no, one or more, same or different substituents R⁹, and wherein said heterocycle comprises no, one or more N-atoms as ring members in addition to those that may be present as ring members E and J.

In one embodiment, the variable G represents a 6-membered saturated, partially unsaturated, or fully unsaturated carbo- or heterocycle, which carbo- or heterocycle includes the atoms E and J as ring members and is unsubstituted, or substituted with no, one or more, same or different substituents R⁹, and wherein said heterocycle comprises no or one more N-atoms as ring members in addition to those that may be present as ring members E and J.

In another embodiment, the variable G represents a 6-membered partially or fully unsaturated carbocycle, which carbocycle includes the atoms E and J as ring members and is unsubstituted, or substituted with one or more, same or different substituents R⁹.

In another embodiment, the variable G represents a 6-membered partially or fully unsaturated heterocycle, which heterocycle includes the atoms E and J as ring members and is unsubstituted, or substituted with one or more, same or different substituents R⁹, and wherein said heterocycle comprises no, one or more, same or different heteroatoms O, N, or S as ring members in addition to those that may be present as ring members E and J.

In one embodiment, the variable G represents a 5-membered saturated, partially unsaturated, or fully unsaturated carbo- or heterocycle, which carbo- or heterocycle includes the atoms E and J as ring members and is unsubstituted, or substituted with one or more, same or different substituents R⁹, and wherein said heterocycle comprises no, one or more, same or different heteroatoms O, N, or S in addition to those that may be present as ring members E and J. In another embodiment, the variable G represents a 5-membered partially or fully unsaturated carbocycle, which carbocycle includes the atoms E and J as ring members and is unsubstituted, or substituted with one or more, same or different substituents R⁹. In another embodiment, the variable G represents a 5-membered partially or fully unsaturated heterocycle, which heterocycle includes the atoms E and J as ring members and is unsubstituted, or substituted with one or more, same or different substituents R⁹, and wherein said heterocycle comprises no, one or more, same or different heteroatoms O, N, or S in addition to those that may be present as ring members E and J.

The variable A is N, S, O, CR⁷, or NR⁸. In one embodiment, the variable A is N. In another embodiment, the variable A is NR⁸. In another embodiment, the variable A is O. In another embodiment, the variable A is S.

The variables E, J are independently C or N, wherein at least one of the variables selected from E and J is C. In one embodiment, E is N and J is C. In another embodiment, J is N and E is C.

In another embodiment, A and E are N, and J is C. In another embodiment, A and J are N, and E is C.

Accordingly, the fused bicyclic ring D may be presented by a formula D1 to D51 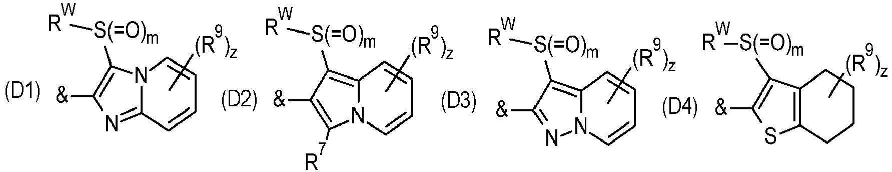 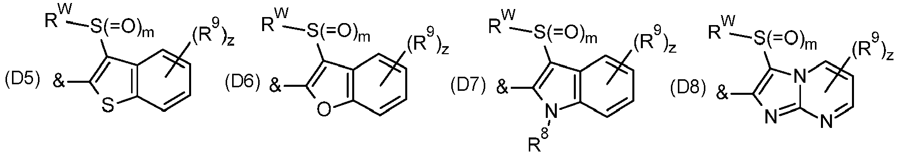 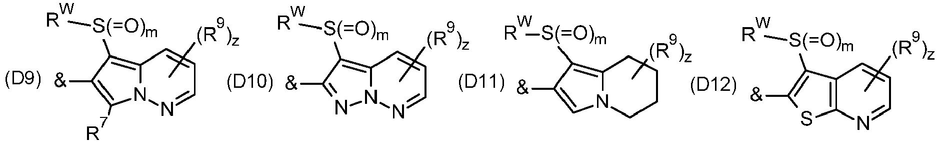 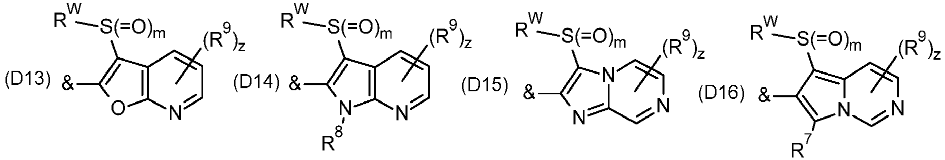 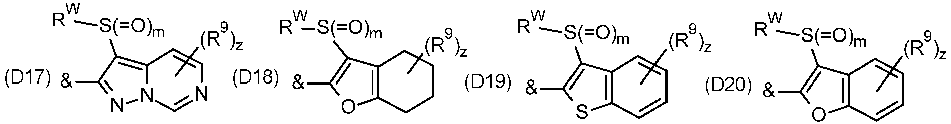 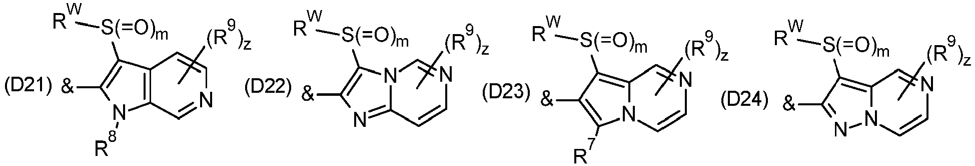 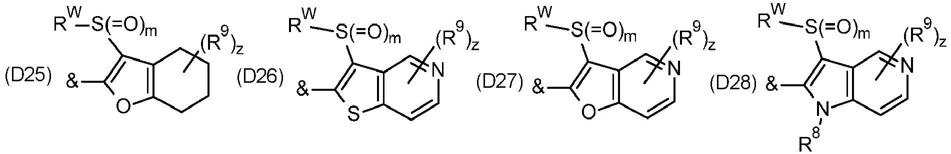 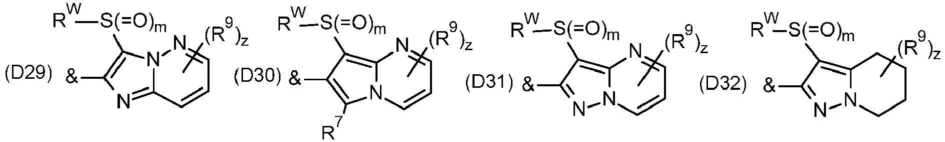 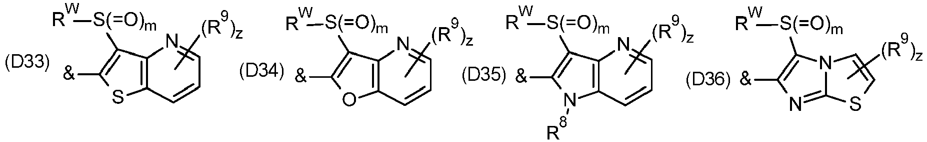 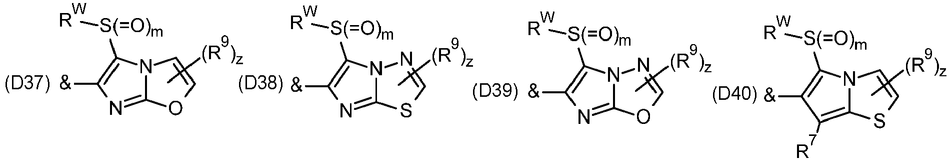 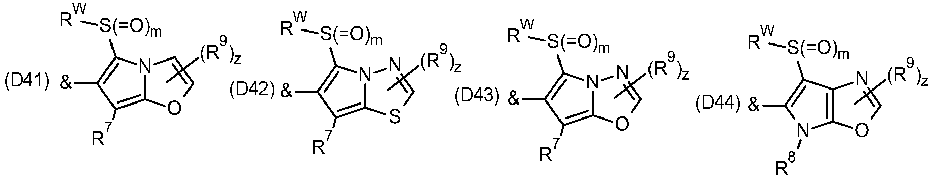 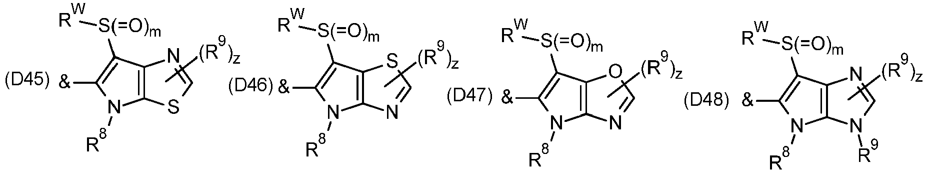 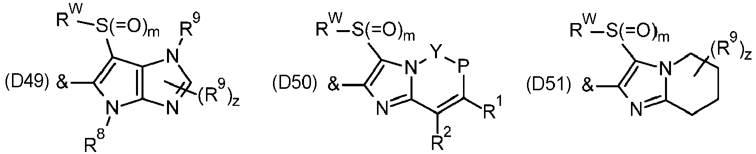 wherein the index z is 0, 1, 2, 3, or 4, preferably 1, and wherein all other variables have a meaning as defined for formula (I). For the avoidance of doubt: substituent(s) R⁹ are bound to a ring member of ring G. In one embodiment, D is selected from D1, D3, D8, and D51. In another embodiment, D is D1. In another embodiment, D is D3. In another embodiment, D is D8. In another embodiment, D is D51.

The position of R⁹ may be described by the following scheme: Formulae (D.A) and (D.B) display the alternatives of the ring G being either a 6-membered or 5-membered ring, respectively 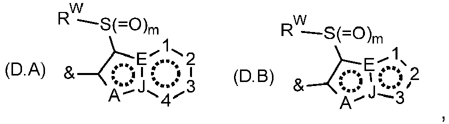 wherein the numbers 1, 2, 3, and 4 each independently denominate the position of a specific ring member, wherein the identity of said ring members is as described herein for formula (I), wherein the "&"-symbol signifies the connection to the remainder of formula (I), wherein the dotted circles in the fused rings means that fused rings may be saturated, partially unsaturated, or fully unsaturated; and wherein the other variables are defined as for formula (I).

Accordingly, the position x of a substituent R⁹ of a ring D1 to D51 will be indicated by the respective suffic ".x", such as D1.1, D1.2, D1.3, or D1.4.

For example, a fused bicyclic ring D1 having one substituent R⁹ at position 2 would correspond to the ring (D1.2) 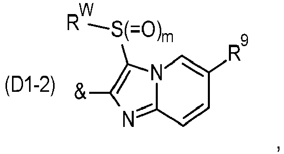 wherein all variables have a meaning as defined for formula (I).

The following Tables 1 to 266 corresponds to specific ring systems as combinations of bicyclic fused rings D with bicyclic fused rings (I-A) or (I-B):
Table 1: compounds of formula (I.A), wherein the variable D is of formula (D1.1).
Table 2: compounds of formula (I.A), wherein the variable D is of formula (D1.2).
Table 3: compounds of formula (I.A), wherein the variable D is of formula (D1.3).
Table 4: compounds of formula (I.A), wherein the variable D is of formula (D1.4).
Table 5: compounds of formula (I.A), wherein the variable D is of formula (D2.1).
Table 6: compounds of formula (I.A), wherein the variable D is of formula (D2.2).
Table 7: compounds of formula (I.A), wherein the variable D is of formula (D2.3).
Table 8: compounds of formula (I.A), wherein the variable D is of formula (D2.4).
Table 9: compounds of formula (I.A), wherein the variable D is of formula (D3.1).
Table 10: compounds of formula (I.A), wherein the variable D is of formula (D3.2).
Table 11: compounds of formula (I.A), wherein the variable D is of formula (D3.3).
Table 12: compounds of formula (I.A), wherein the variable D is of formula (D3.4).
Table 13: compounds of formula (I.A), wherein the variable D is of formula (D4.1).
Table 14: compounds of formula (I.A), wherein the variable D is of formula (D4.2).
Table 15: compounds of formula (I.A), wherein the variable D is of formula (D4.3).
Table 16: compounds of formula (I.A), wherein the variable D is of formula (D4.4).
Table 17: compounds of formula (I.A), wherein the variable D is of formula (D5.1).
Table 18: compounds of formula (I.A), wherein the variable D is of formula (D5.2).
Table 19: compounds of formula (I.A), wherein the variable D is of formula (D5.3).
Table 20: compounds of formula (I.A), wherein the variable D is of formula (D5.4).
Table 21: compounds of formula (I.A), wherein the variable D is of formula (D6.1).
Table 22: compounds of formula (I.A), wherein the variable D is of formula (D6.2).
Table 23: compounds of formula (I.A), wherein the variable D is of formula (D6.3).
Table 24: compounds of formula (I.A), wherein the variable D is of formula (D6.4).
Table 25: compounds of formula (I.A), wherein the variable D is of formula (D7.1).
Table 26: compounds of formula (I.A), wherein the variable D is of formula (D7.2).
Table 27: compounds of formula (I.A), wherein the variable D is of formula (D7.3).
Table 28: compounds of formula (I.A), wherein the variable D is of formula (D7.4).
Table 29: compounds of formula (I.A), wherein the variable D is of formula (D8.1).
Table 30: compounds of formula (I.A), wherein the variable D is of formula (D8.2).
Table 31: compounds of formula (I.A), wherein the variable D is of formula (D8.3).
Table 32: compounds of formula (I.A), wherein the variable D is of formula (D9.1).
Table 33: compounds of formula (I.A), wherein the variable D is of formula (D9.2).
Table 34: compounds of formula (I.A), wherein the variable D is of formula (D9.3).
Table 35: compounds of formula (I.A), wherein the variable D is of formula (D10.1).
Table 36: compounds of formula (I.A), wherein the variable D is of formula (D10.2).
Table 37: compounds of formula (I.A), wherein the variable D is of formula (D10.3).
Table 38: compounds of formula (I.A), wherein the variable D is of formula (D11.1).
Table 39: compounds of formula (I.A), wherein the variable D is of formula (D11.2).
Table 40: compounds of formula (I.A), wherein the variable D is of formula (D11.3).
Table 41: compounds of formula (I.A), wherein the variable D is of formula (D11.4).
Table 42: compounds of formula (I.A), wherein the variable D is of formula (D12.1).
Table 43: compounds of formula (I.A), wherein the variable D is of formula (D12.2).
Table 44: compounds of formula (I.A), wherein the variable D is of formula (D12.3).
Table 45: compounds of formula (I.A), wherein the variable D is of formula (D13.1).
Table 46: compounds of formula (I.A), wherein the variable D is of formula (D13.2).
Table 47: compounds of formula (I.A), wherein the variable D is of formula (D13.3).
Table 48: compounds of formula (I.A), wherein the variable D is of formula (D14.1).
Table 49: compounds of formula (I.A), wherein the variable D is of formula (D14.2).
Table 50: compounds of formula (I.A), wherein the variable D is of formula (D14.3).
Table 51: compounds of formula (I.A), wherein the variable D is of formula (D15.1).
Table 52: compounds of formula (I.A), wherein the variable D is of formula (D15.2).
Table 53: compounds of formula (I.A), wherein the variable D is of formula (D15.4).
Table 54: compounds of formula (I.A), wherein the variable D is of formula (D16.1).
Table 55: compounds of formula (I.A), wherein the variable D is of formula (D16.2).
Table 56: compounds of formula (I.A), wherein the variable D is of formula (D16.4).
Table 57: compounds of formula (I.A), wherein the variable D is of formula (D17.1).
Table 58: compounds of formula (I.A), wherein the variable D is of formula (D17.2).
Table 59: compounds of formula (I.A), wherein the variable D is of formula (D17.4).
Table 60: compounds of formula (I.A), wherein the variable D is of formula (D18.1).
Table 61: compounds of formula (I.A), wherein the variable D is of formula (D18.2).
Table 62: compounds of formula (I.A), wherein the variable D is of formula (D18.3).
Table 63: compounds of formula (I.A), wherein the variable D is of formula (D18.4).
Table 64: compounds of formula (I.A), wherein the variable D is of formula (D19.1).
Table 65: compounds of formula (I.A), wherein the variable D is of formula (D19.2).
Table 66: compounds of formula (I.A), wherein the variable D is of formula (D19.3).
Table 67: compounds of formula (I.A), wherein the variable D is of formula (D19.4).
Table 68: compounds of formula (I.A), wherein the variable D is of formula (D20.1).
Table 69: compounds of formula (I.A), wherein the variable D is of formula (D20.2).
Table 70: compounds of formula (I.A), wherein the variable D is of formula (D20.3).
Table 71: compounds of formula (I.A), wherein the variable D is of formula (D20.4).
Table 72: compounds of formula (I.A), wherein the variable D is of formula (D21.1).
Table 73: compounds of formula (I.A), wherein the variable D is of formula (D21.2).
Table 74: compounds of formula (I.A), wherein the variable D is of formula (D21.4).
Table 75: compounds of formula (I.A), wherein the variable D is of formula (D22.1).
Table 76: compounds of formula (I.A), wherein the variable D is of formula (D22.3).
Table 77: compounds of formula (I.A), wherein the variable D is of formula (D22.4).
Table 78: compounds of formula (I.A), wherein the variable D is of formula (D23.1).
Table 79: compounds of formula (I.A), wherein the variable D is of formula (D23.3).
Table 80: compounds of formula (I.A), wherein the variable D is of formula (D23.4).
Table 81: compounds of formula (I.A), wherein the variable D is of formula (D24.1).
Table 82: compounds of formula (I.A), wherein the variable D is of formula (D24.3).
Table 83: compounds of formula (I.A), wherein the variable D is of formula (D24.4).
Table 84: compounds of formula (I.A), wherein the variable D is of formula (D25.1).
Table 85: compounds of formula (I.A), wherein the variable D is of formula (D25.2).
Table 86: compounds of formula (I.A), wherein the variable D is of formula (D25.3).
Table 87: compounds of formula (I.A), wherein the variable D is of formula (D25.4).
Table 88: compounds of formula (I.A), wherein the variable D is of formula (D26.1).
Table 89: compounds of formula (I.A), wherein the variable D is of formula (D26.3).
Table 90: compounds of formula (I.A), wherein the variable D is of formula (D26.4).
Table 91: compounds of formula (I.A), wherein the variable D is of formula (D27.1).
Table 92: compounds of formula (I.A), wherein the variable D is of formula (D27.3).
Table 93: compounds of formula (I.A), wherein the variable D is of formula (D27.4).
Table 94: compounds of formula (I.A), wherein the variable D is of formula (D28.1).
Table 95: compounds of formula (I.A), wherein the variable D is of formula (D28.3).
Table 96: compounds of formula (I.A), wherein the variable D is of formula (D28.4).
Table 97: compounds of formula (I.A), wherein the variable D is of formula (D29.2).
Table 98: compounds of formula (I.A), wherein the variable D is of formula (D29.3).
Table 99: compounds of formula (I.A), wherein the variable D is of formula (D29.4).
Table 100: compounds of formula (I.A), wherein the variable D is of formula (D30.2).
Table 101: compounds of formula (I.A), wherein the variable D is of formula (D30.3).
Table 102: compounds of formula (I.A), wherein the variable D is of formula (D30.4).
Table 103: compounds of formula (I.A), wherein the variable D is of formula (D31.2).
Table 104: compounds of formula (I.A), wherein the variable D is of formula (D31.3).
Table 105: compounds of formula (I.A), wherein the variable D is of formula (D31.4).
Table 106: compounds of formula (I.A), wherein the variable D is of formula (D32.1).
Table 107: compounds of formula (I.A), wherein the variable D is of formula (D32.2).
Table 108: compounds of formula (I.A), wherein the variable D is of formula (D32.3).
Table 109: compounds of formula (I.A), wherein the variable D is of formula (D32.4).
Table 110: compounds of formula (I.A), wherein the variable D is of formula (D33.2).
Table 111: compounds of formula (I.A), wherein the variable D is of formula (D33.3).
Table 112: compounds of formula (I.A), wherein the variable D is of formula (D33.4).
Table 113: compounds of formula (I.A), wherein the variable D is of formula (D34.2).
Table 114: compounds of formula (I.A), wherein the variable D is of formula (D34.3).
Table 115: compounds of formula (I.A), wherein the variable D is of formula (D34.4).
Table 116: compounds of formula (I.A), wherein the variable D is of formula (D35.2).
Table 117: compounds of formula (I.A), wherein the variable D is of formula (D35.3).
Table 118: compounds of formula (I.A), wherein the variable D is of formula (D35.4).
Table 119: compounds of formula (I.A), wherein the variable D is of formula (D36.1).
Table 120: compounds of formula (I.A), wherein the variable D is of formula (D36.2).
Table 121: compounds of formula (I.A), wherein the variable D is of formula (D37.1).
Table 122: compounds of formula (I.A), wherein the variable D is of formula (D37.2).
Table 123: compounds of formula (I.A), wherein the variable D is of formula (D38.2).
Table 124: compounds of formula (I.A), wherein the variable D is of formula (D39.2).
Table 125: compounds of formula (I.A), wherein the variable D is of formula (D40.1).
Table 126: compounds of formula (I.A), wherein the variable D is of formula (D40.2).
Table 127: compounds of formula (I.A), wherein the variable D is of formula (D41.1).
Table 128: compounds of formula (I.A), wherein the variable D is of formula (D41.2).
Table 129: compounds of formula (I.A), wherein the variable D is of formula (D42.2).
Table 130: compounds of formula (I.A), wherein the variable D is of formula (D43.2).
Table 131: compounds of formula (I.A), wherein the variable D is of formula (D44.2).
Table 132: compounds of formula (I.A), wherein the variable D is of formula (D45.2).
Table 133: compounds of formula (I.A), wherein the variable D is of formula (D46.2).
Table 134: compounds of formula (I.A), wherein the variable D is of formula (D47.2).
Table 135: compounds of formula (I.A), wherein the variable D is of formula (D48.2).
Table 136: compounds of formula (I.A), wherein the variable D is of formula (D49.2).
Table 137: compounds of formula (I.A), wherein the variable D is of formula (D50).
Table 138: compounds of formula (I.A), wherein the variable D is of formula (D51.1).
Table 139: compounds of formula (I.A), wherein the variable D is of formula (D51.2).
Table 140: compounds of formula (I.A), wherein the variable D is of formula (D51.3).
Table 141: compounds of formula (I.A), wherein the variable D is of formula (D51.4).
Table 142: compounds of formula (I.B), wherein the variable D is of formula (D1.1).
Table 143: compounds of formula (I.B), wherein the variable D is of formula (D1.2).
Table 144: compounds of formula (I.B), wherein the variable D is of formula (D1.3).
Table 145: compounds of formula (I.B), wherein the variable D is of formula (D1.4).
Table 146: compounds of formula (I.B), wherein the variable D is of formula (D2.1).
Table 147: compounds of formula (I.B), wherein the variable D is of formula (D2.2).
Table 148: compounds of formula (I.B), wherein the variable D is of formula (D2.3).
Table 149: compounds of formula (I.B), wherein the variable D is of formula (D2.4).
Table 150: compounds of formula (I.B), wherein the variable D is of formula (D3.1).
Table 151: compounds of formula (I.B), wherein the variable D is of formula (D3.2).
Table 152: compounds of formula (I.B), wherein the variable D is of formula (D3.3).
Table 153: compounds of formula (I.B), wherein the variable D is of formula (D3.4).
Table 154: compounds of formula (I.B), wherein the variable D is of formula (D4.1).
Table 155: compounds of formula (I.B), wherein the variable D is of formula (D4.2).
Table 156: compounds of formula (I.B), wherein the variable D is of formula (D4.3).
Table 157: compounds of formula (I.B), wherein the variable D is of formula (D4.4).
Table 158: compounds of formula (I.B), wherein the variable D is of formula (D5.1).
Table 159: compounds of formula (I.B), wherein the variable D is of formula (D5.2).
Table 160: compounds of formula (I.B), wherein the variable D is of formula (D5.3).
Table 161: compounds of formula (I.B), wherein the variable D is of formula (D5.4).
Table 162: compounds of formula (I.B), wherein the variable D is of formula (D6.1).
Table 163: compounds of formula (I.B), wherein the variable D is of formula (D6.2).
Table 164: compounds of formula (I.B), wherein the variable D is of formula (D6.3).
Table 165: compounds of formula (I.B), wherein the variable D is of formula (D6.4).
Table 166: compounds of formula (I.B), wherein the variable D is of formula (D7.1).
Table 167: compounds of formula (I.B), wherein the variable D is of formula (D7.2).
Table 168: compounds of formula (I.B), wherein the variable D is of formula (D7.3).
Table 169: compounds of formula (I.B), wherein the variable D is of formula (D7.4).
Table 170: compounds of formula (I.B), wherein the variable D is of formula (D8.1).
Table 171: compounds of formula (I.B), wherein the variable D is of formula (D8.2).
Table 172: compounds of formula (I.B), wherein the variable D is of formula (D8.3).
Table 173: compounds of formula (I.B), wherein the variable D is of formula (D9.1).
Table 174: compounds of formula (I.B), wherein the variable D is of formula (D9.2).
Table 175: compounds of formula (I.B), wherein the variable D is of formula (D9.3).
Table 176: compounds of formula (I.B), wherein the variable D is of formula (D10.1).
Table 177: compounds of formula (I.B), wherein the variable D is of formula (D10.2).
Table 178: compounds of formula (I.B), wherein the variable D is of formula (D10.3).
Table 179: compounds of formula (I.B), wherein the variable D is of formula (D11.1).
Table 180: compounds of formula (I.B), wherein the variable D is of formula (D11.2).
Table 181: compounds of formula (I.B), wherein the variable D is of formula (D11.3).
Table 182: compounds of formula (I.B), wherein the variable D is of formula (D11.4).
Table 183: compounds of formula (I.B), wherein the variable D is of formula (D12.1).
Table 184: compounds of formula (I.B), wherein the variable D is of formula (D12.2).
Table 185: compounds of formula (I.B), wherein the variable D is of formula (D12.3).
Table 186: compounds of formula (I.B), wherein the variable D is of formula (D13.1).
Table 187: compounds of formula (I.B), wherein the variable D is of formula (D13.2).
Table 188: compounds of formula (I.B), wherein the variable D is of formula (D13.3).
Table 189: compounds of formula (I.B), wherein the variable D is of formula (D14.1).
Table 190: compounds of formula (I.B), wherein the variable D is of formula (D14.2).
Table 191: compounds of formula (I.B), wherein the variable D is of formula (D14.3).
Table 192: compounds of formula (I.B), wherein the variable D is of formula (D15.1).
Table 193: compounds of formula (I.B), wherein the variable D is of formula (D15.2).
Table 194: compounds of formula (I.B), wherein the variable D is of formula (D15.4).
Table 195: compounds of formula (I.B), wherein the variable D is of formula (D16.1).
Table 196: compounds of formula (I.B), wherein the variable D is of formula (D16.2).
Table 197: compounds of formula (I.B), wherein the variable D is of formula (D16.4).
Table 198: compounds of formula (I.B), wherein the variable D is of formula (D17.1).
Table 199: compounds of formula (I.B), wherein the variable D is of formula (D17.2).
Table 200: compounds of formula (I.B), wherein the variable D is of formula (D17.4).
Table 201: compounds of formula (I.B), wherein the variable D is of formula (D18.1).
Table 202: compounds of formula (I.B), wherein the variable D is of formula (D18.2).
Table 203: compounds of formula (I.B), wherein the variable D is of formula (D18.3).
Table 204: compounds of formula (I.B), wherein the variable D is of formula (D18.4).
Table 205: compounds of formula (I.B), wherein the variable D is of formula (D19.1).
Table 206: compounds of formula (I.B), wherein the variable D is of formula (D19.2).
Table 207: compounds of formula (I.B), wherein the variable D is of formula (D19.3).
Table 208: compounds of formula (I.B), wherein the variable D is of formula (D19.4).
Table 209: compounds of formula (I.B), wherein the variable D is of formula (D20.1).
Table 210: compounds of formula (I.B), wherein the variable D is of formula (D20.2).
Table 211: compounds of formula (I.B), wherein the variable D is of formula (D20.3).
Table 212: compounds of formula (I.B), wherein the variable D is of formula (D20.4).
Table 213: compounds of formula (I.B), wherein the variable D is of formula (D21.1).
Table 214: compounds of formula (I.B), wherein the variable D is of formula (D21.2).
Table 215: compounds of formula (I.B), wherein the variable D is of formula (D21.4).
Table 216: compounds of formula (I.B), wherein the variable D is of formula (D22.1).
Table 217: compounds of formula (I.B), wherein the variable D is of formula (D22.3).
Table 218: compounds of formula (I.B), wherein the variable D is of formula (D22.4).
Table 219: compounds of formula (I.B), wherein the variable D is of formula (D23.1).
Table 220: compounds of formula (I.B), wherein the variable D is of formula (D23.3).
Table 221: compounds of formula (I.B), wherein the variable D is of formula (D23.4).
Table 222: compounds of formula (I.B), wherein the variable D is of formula (D24.1).
Table 223: compounds of formula (I.B), wherein the variable D is of formula (D24.3).
Table 224: compounds of formula (I.B), wherein the variable D is of formula (D24.4).
Table 225: compounds of formula (I.B), wherein the variable D is of formula (D25.1).
Table 226: compounds of formula (I.B), wherein the variable D is of formula (D25.2).
Table 227: compounds of formula (I.B), wherein the variable D is of formula (D25.3).
Table 228: compounds of formula (I.B), wherein the variable D is of formula (D25.4).
Table 229: compounds of formula (I.B), wherein the variable D is of formula (D26.1).
Table 230: compounds of formula (I.B), wherein the variable D is of formula (D26.3).
Table 231: compounds of formula (I.B), wherein the variable D is of formula (D26.4).
Table 232: compounds of formula (I.B), wherein the variable D is of formula (D27.1).
Table 333: compounds of formula (I.B), wherein the variable D is of formula (D27.3).
Table 234: compounds of formula (I.B), wherein the variable D is of formula (D27.4).
Table 235: compounds of formula (I.B), wherein the variable D is of formula (D28.1).
Table 236: compounds of formula (I.B), wherein the variable D is of formula (D28.3).
Table 237: compounds of formula (I.B), wherein the variable D is of formula (D28.4).
Table 238: compounds of formula (I.B), wherein the variable D is of formula (D29.2).
Table 239: compounds of formula (I.B), wherein the variable D is of formula (D29.3).
Table 240: compounds of formula (I.B), wherein the variable D is of formula (D29.4).
Table 241: compounds of formula (I.B), wherein the variable D is of formula (D30.2).
Table 242: compounds of formula (I.B), wherein the variable D is of formula (D30.3).
Table 243: compounds of formula (I.B), wherein the variable D is of formula (D30.4).
Table 244: compounds of formula (I.B), wherein the variable D is of formula (D31.2).
Table 245: compounds of formula (I.B), wherein the variable D is of formula (D31.3).
Table 246: compounds of formula (I.B), wherein the variable D is of formula (D31.4).
Table 247: compounds of formula (I.B), wherein the variable D is of formula (D32.1).
Table 248: compounds of formula (I.B), wherein the variable D is of formula (D32.2).
Table 249: compounds of formula (I.B), wherein the variable D is of formula (D32.3).
Table 250: compounds of formula (I.B), wherein the variable D is of formula (D32.4).
Table 251: compounds of formula (I.B), wherein the variable D is of formula (D33.2).
Table 252: compounds of formula (I.B), wherein the variable D is of formula (D33.3).
Table 253: compounds of formula (I.B), wherein the variable D is of formula (D33.4).
Table 254: compounds of formula (I.B), wherein the variable D is of formula (D34.2).
Table 255: compounds of formula (I.B), wherein the variable D is of formula (D34.3).
Table 256: compounds of formula (I.B), wherein the variable D is of formula (D34.4).
Table 257: compounds of formula (I.B), wherein the variable D is of formula (D35.2).
Table 258: compounds of formula (I.B), wherein the variable D is of formula (D35.3).
Table 259: compounds of formula (I.B), wherein the variable D is of formula (D35.4).
Table 260: compounds of formula (I.B), wherein the variable D is of formula (D36.1).
Table 261: compounds of formula (I.B), wherein the variable D is of formula (D36.2).
Table 262: compounds of formula (I.B), wherein the variable D is of formula (D37.1).
Table 263: compounds of formula (I.B), wherein the variable D is of formula (D37.2).
Table 264: compounds of formula (I.B), wherein the variable D is of formula (D38.2).
Table 265: compounds of formula (I.B), wherein the variable D is of formula (D39.2).
Table 266: compounds of formula (I.B), wherein the variable D is of formula (D40.1).
Table 267: compounds of formula (I.B), wherein the variable D is of formula (D40.2).
Table 268: compounds of formula (I.B), wherein the variable D is of formula (D41.1).
Table 269: compounds of formula (I.B), wherein the variable D is of formula (D41.2).
Table 270: compounds of formula (I.B), wherein the variable D is of formula (D42.2).
Table 271: compounds of formula (I.B), wherein the variable D is of formula (D43.2).
Table 272: compounds of formula (I.B), wherein the variable D is of formula (D44.2).
Table 273: compounds of formula (I.B), wherein the variable D is of formula (D45.2).
Table 274: compounds of formula (I.B), wherein the variable D is of formula (D46.2).
Table 275: compounds of formula (I.B), wherein the variable D is of formula (D47.2).
Table 276: compounds of formula (I.B), wherein the variable D is of formula (D48.2).
Table 277: compounds of formula (I.B), wherein the variable D is of formula (D49.2).
Table 278: compounds of formula (I.B), wherein the variable D is of formula (D50).
Table 279: compounds of formula (I.B), wherein the variable D is of formula (D51.1).
Table 280: compounds of formula (I.B), wherein the variable D is of formula (D51.2).
Table 281: compounds of formula (I.B), wherein the variable D is of formula (D51.3).
Table 282: compounds of formula (I.B), wherein the variable D is of formula (D51.4).
Table 283: compounds of formula (I.C), wherein the variable D is of formula (D1.1).
Table 284: compounds of formula (I.C), wherein the variable D is of formula (D1.2).
Table 285: compounds of formula (I.C), wherein the variable D is of formula (D1.3).
Table 286: compounds of formula (I.C), wherein the variable D is of formula (D1.4).
Table 287: compounds of formula (I.C), wherein the variable D is of formula (D2.1).
Table 288: compounds of formula (I.C), wherein the variable D is of formula (D2.2).
Table 289: compounds of formula (I.C), wherein the variable D is of formula (D2.3).
Table 290: compounds of formula (I.C), wherein the variable D is of formula (D2.4).
Table 291: compounds of formula (I.C), wherein the variable D is of formula (D3.1).
Table 292: compounds of formula (I.C), wherein the variable D is of formula (D3.2).
Table 293: compounds of formula (I.C), wherein the variable D is of formula (D3.3).
Table 294: compounds of formula (I.C), wherein the variable D is of formula (D3.4).
Table 295: compounds of formula (I.C), wherein the variable D is of formula (D4.1).
Table 296: compounds of formula (I.C), wherein the variable D is of formula (D4.2).
Table 297: compounds of formula (I.C), wherein the variable D is of formula (D4.3).
Table 298: compounds of formula (I.C), wherein the variable D is of formula (D4.4).
Table 299: compounds of formula (I.C), wherein the variable D is of formula (D5.1).
Table 300: compounds of formula (I.C), wherein the variable D is of formula (D5.2).
Table 301: compounds of formula (I.C), wherein the variable D is of formula (D5.3).
Table 302: compounds of formula (I.C), wherein the variable D is of formula (D5.4).
Table 303: compounds of formula (I.C), wherein the variable D is of formula (D6.1).
Table 304: compounds of formula (I.C), wherein the variable D is of formula (D6.2).
Table 305: compounds of formula (I.C), wherein the variable D is of formula (D6.3).
Table 306: compounds of formula (I.C), wherein the variable D is of formula (D6.4).
Table 307: compounds of formula (I.C), wherein the variable D is of formula (D7.1).
Table 308: compounds of formula (I.C), wherein the variable D is of formula (D7.2).
Table 309: compounds of formula (I.C), wherein the variable D is of formula (D7.3).
Table 310: compounds of formula (I.C), wherein the variable D is of formula (D7.4).
Table 311: compounds of formula (I.C), wherein the variable D is of formula (D8.1).
Table 312: compounds of formula (I.C), wherein the variable D is of formula (D8.2).
Table 313: compounds of formula (I.C), wherein the variable D is of formula (D8.3).
Table 314: compounds of formula (I.C), wherein the variable D is of formula (D9.1).
Table 315: compounds of formula (I.C), wherein the variable D is of formula (D9.2).
Table 316: compounds of formula (I.C), wherein the variable D is of formula (D9.3).
Table 317: compounds of formula (I.C), wherein the variable D is of formula (D10.1).
Table 318: compounds of formula (I.C), wherein the variable D is of formula (D10.2).
Table 319: compounds of formula (I.C), wherein the variable D is of formula (D10.3).
Table 320: compounds of formula (I.C), wherein the variable D is of formula (D11.1).
Table 321: compounds of formula (I.C), wherein the variable D is of formula (D11.2).
Table 322: compounds of formula (I.C), wherein the variable D is of formula (D11.3).
Table 323: compounds of formula (I.C), wherein the variable D is of formula (D11.4).
Table 324: compounds of formula (I.C), wherein the variable D is of formula (D12.1).
Table 325: compounds of formula (I.C), wherein the variable D is of formula (D12.2).
Table 326: compounds of formula (I.C), wherein the variable D is of formula (D12.3).
Table 327: compounds of formula (I.C), wherein the variable D is of formula (D13.1).
Table 328: compounds of formula (I.C), wherein the variable D is of formula (D13.2).
Table 329: compounds of formula (I.C), wherein the variable D is of formula (D13.3).
Table 330: compounds of formula (I.C), wherein the variable D is of formula (D14.1).
Table 331: compounds of formula (I.C), wherein the variable D is of formula (D14.2).
Table 332: compounds of formula (I.C), wherein the variable D is of formula (D14.3).
Table 333: compounds of formula (I.C), wherein the variable D is of formula (D15.1).
Table 334: compounds of formula (I.C), wherein the variable D is of formula (D15.2).
Table 335: compounds of formula (I.C), wherein the variable D is of formula (D15.4).
Table 336: compounds of formula (I.C), wherein the variable D is of formula (D16.1).
Table 337: compounds of formula (I.C), wherein the variable D is of formula (D16.2).
Table 338: compounds of formula (I.C), wherein the variable D is of formula (D16.4).
Table 339: compounds of formula (I.C), wherein the variable D is of formula (D17.1).
Table 340: compounds of formula (I.C), wherein the variable D is of formula (D17.2).
Table 341: compounds of formula (I.C), wherein the variable D is of formula (D17.4).
Table 342: compounds of formula (I.C), wherein the variable D is of formula (D18.1).
Table 343: compounds of formula (I.C), wherein the variable D is of formula (D18.2).
Table 344: compounds of formula (I.C), wherein the variable D is of formula (D18.3).
Table 345: compounds of formula (I.C), wherein the variable D is of formula (D18.4).
Table 346: compounds of formula (I.C), wherein the variable D is of formula (D19.1).
Table 347: compounds of formula (I.C), wherein the variable D is of formula (D19.2).
Table 348: compounds of formula (I.C), wherein the variable D is of formula (D19.3).
Table 349: compounds of formula (I.C), wherein the variable D is of formula (D19.4).
Table 350: compounds of formula (I.C), wherein the variable D is of formula (D20.1).
Table 351: compounds of formula (I.C), wherein the variable D is of formula (D20.2).
Table 352: compounds of formula (I.C), wherein the variable D is of formula (D20.3).
Table 353: compounds of formula (I.C), wherein the variable D is of formula (D20.4).
Table 354: compounds of formula (I.C), wherein the variable D is of formula (D21.1).
Table 355: compounds of formula (I.C), wherein the variable D is of formula (D21.2).
Table 356: compounds of formula (I.C), wherein the variable D is of formula (D21.4).
Table 357: compounds of formula (I.C), wherein the variable D is of formula (D22.1).
Table 358: compounds of formula (I.C), wherein the variable D is of formula (D22.3).
Table 359: compounds of formula (I.C), wherein the variable D is of formula (D22.4).
Table 360: compounds of formula (I.C), wherein the variable D is of formula (D23.1).
Table 361: compounds of formula (I.C), wherein the variable D is of formula (D23.3).
Table 362: compounds of formula (I.C), wherein the variable D is of formula (D23.4).
Table 363: compounds of formula (I.C), wherein the variable D is of formula (D24.1).
Table 364: compounds of formula (I.C), wherein the variable D is of formula (D24.3).
Table 365: compounds of formula (I.C), wherein the variable D is of formula (D24.4).
Table 366: compounds of formula (I.C), wherein the variable D is of formula (D25.1).
Table 367: compounds of formula (I.C), wherein the variable D is of formula (D25.2).
Table 368: compounds of formula (I.C), wherein the variable D is of formula (D25.3).
Table 369: compounds of formula (I.C), wherein the variable D is of formula (D25.4).
Table 370: compounds of formula (I.C), wherein the variable D is of formula (D26.1).
Table 371: compounds of formula (I.C), wherein the variable D is of formula (D26.3).
Table 372: compounds of formula (I.C), wherein the variable D is of formula (D26.4).
Table 373: compounds of formula (I.C), wherein the variable D is of formula (D27.1).
Table 374: compounds of formula (I.C), wherein the variable D is of formula (D27.3).
Table 375: compounds of formula (I.C), wherein the variable D is of formula (D27.4).
Table 376: compounds of formula (I.C), wherein the variable D is of formula (D28.1).
Table 377: compounds of formula (I.C), wherein the variable D is of formula (D28.3).
Table 378: compounds of formula (I.C), wherein the variable D is of formula (D28.4).
Table 379: compounds of formula (I.C), wherein the variable D is of formula (D29.2).
Table 380: compounds of formula (I.C), wherein the variable D is of formula (D29.3).
Table 381: compounds of formula (I.C), wherein the variable D is of formula (D29.4).
Table 382: compounds of formula (I.C), wherein the variable D is of formula (D30.2).
Table 383: compounds of formula (I.C), wherein the variable D is of formula (D30.3).
Table 384: compounds of formula (I.C), wherein the variable D is of formula (D30.4).
Table 385: compounds of formula (I.C), wherein the variable D is of formula (D31.2).
Table 386: compounds of formula (I.C), wherein the variable D is of formula (D31.3).
Table 387: compounds of formula (I.C), wherein the variable D is of formula (D31.4).
Table 388: compounds of formula (I.C), wherein the variable D is of formula (D32.1).
Table 389: compounds of formula (I.C), wherein the variable D is of formula (D32.2).
Table 390: compounds of formula (I.C), wherein the variable D is of formula (D32.3).
Table 391: compounds of formula (I.C), wherein the variable D is of formula (D32.4).
Table 392: compounds of formula (I.C), wherein the variable D is of formula (D33.2).
Table 393: compounds of formula (I.C), wherein the variable D is of formula (D33.3).
Table 394: compounds of formula (I.C), wherein the variable D is of formula (D33.4).
Table 395: compounds of formula (I.C), wherein the variable D is of formula (D34.2).
Table 396: compounds of formula (I.C), wherein the variable D is of formula (D34.3).
Table 397: compounds of formula (I.C), wherein the variable D is of formula (D34.4).
Table 398: compounds of formula (I.C), wherein the variable D is of formula (D35.2).
Table 399: compounds of formula (I.C), wherein the variable D is of formula (D35.3).
Table 400: compounds of formula (I.C), wherein the variable D is of formula (D35.4).
Table 401: compounds of formula (I.C), wherein the variable D is of formula (D36.1).
Table 402: compounds of formula (I.C), wherein the variable D is of formula (D36.2).
Table 403: compounds of formula (I.C), wherein the variable D is of formula (D37.1).
Table 404: compounds of formula (I.C), wherein the variable D is of formula (D37.2).
Table 405: compounds of formula (I.C), wherein the variable D is of formula (D38.2).
Table 406: compounds of formula (I.C), wherein the variable D is of formula (D39.2).
Table 407: compounds of formula (I.C), wherein the variable D is of formula (D40.1).
Table 408: compounds of formula (I.C), wherein the variable D is of formula (D40.2).
Table 409: compounds of formula (I.C), wherein the variable D is of formula (D41.1).
Table 410: compounds of formula (I.C), wherein the variable D is of formula (D41.2).
Table 411: compounds of formula (I.C), wherein the variable D is of formula (D42.2).
Table 412: compounds of formula (I.C), wherein the variable D is of formula (D43.2).
Table 413: compounds of formula (I.C), wherein the variable D is of formula (D44.2).
Table 414: compounds of formula (I.C), wherein the variable D is of formula (D45.2).
Table 415: compounds of formula (I.C), wherein the variable D is of formula (D46.2).
Table 416: compounds of formula (I.C), wherein the variable D is of formula (D47.2).
Table 417: compounds of formula (I.C), wherein the variable D is of formula (D48.2).
Table 418: compounds of formula (I.C), wherein the variable D is of formula (D49.2).
Table 419: compounds of formula (I.C), wherein the variable D is of formula (D50).
Table 420: compounds of formula (I.C), wherein the variable D is of formula (D50.1).
Table 421: compounds of formula (I.C), wherein the variable D is of formula (D50.2).
Table 422: compounds of formula (I.C), wherein the variable D is of formula (D50.3).
Table 423: compounds of formula (I.C), wherein the variable D is of formula (D50.4).

The following Table A represents specific combinations of definitions for substituents X, R², R³, and R⁹ in lines A1 to A480. Each combination individually and all combinations collectively represent preferred embodiments.

**Table A: lines A1 to A480 represent definitions for combinations of the variables R¹, R², R³, R⁹, and X. "cPr" is cyclopropyl; "1-CN-cPr" is 1-cyano-cyclopropyl; "1-CN-iPr" is 1-cyano-isopropyl; "Bn" is benzyl; "Cy" is cyclohexyl; "cBu" is cyclobutyl.**

| Line | X | R² | R³ | R⁹ |
|---|---|---|---|---|
| A1 | O | H | CH₃ | CH₃ |
| A2 | O | H | CH₃ | CF₃ |
| A3 | O | H | CH₃ | OCH₃ |
| A4 | O | H | CH₃ | OCF₃ |
| A5 | O | H | CH₃ | F |
| A6 | O | H | CH₃ | Cl |
| A7 | O | H | CH₃ | Br |
| A8 | O | H | CH₃ | 1-CN-cPr |
| A9 | O | H | CH₃ | 1-CN-iPr |
| A10 | O | H | CH₃ | H |
| A11 | O | H | CH₂CH₃ | CH₃ |
| A12 | O | H | CH₂CH₃ | CF₃ |
| A13 | O | H | CH₂CH₃ | OCH₃ |
| A14 | O | H | CH₂CH₃ | OCF₃ |
| A15 | O | H | CH₂CH₃ | F |
| A16 | O | H | CH₂CH₃ | Br |
| A17 | O | H | CH₂CH₃ | 1-CN-cPr |
| A18 | O | H | CH₂CH₃ | 1-CN-iPr |
| A19 | O | H | CH₂CH₃ | Cl |
| A20 | O | H | CH₂CH₃ | H |
| A21 | O | H | cPr | CH₃ |
| A22 | O | H | cPr | CF₃ |
| A23 | O | H | cPr | OCH₃ |
| A24 | O | H | cPr | OCF₃ |
| A25 | O | H | cPr | F |
| A26 | O | H | cPr | Cl |
| A27 | O | H | cPr | Br |
| A28 | O | H | cPr | 1-CN-cPr |
| A29 | O | H | cPr | 1-CN-iPr |
| A30 | O | H | cPr | H |
| A31 | O | H | Bn | CH₃ |
| A32 | O | H | Bn | CF₃ |
| A33 | O | H | Bn | OCH₃ |
| A34 | O | H | Bn | OCF₃ |
| A35 | O | H | Bn | F |
| A36 | O | H | Bn | Cl |
| A37 | O | H | Bn | Br |
| A38 | O | H | Bn | 1-CN-cPr |
| A39 | O | H | Bn | 1-CN-iPr |
| A40 | O | H | Bn | H |
| A41 | O | H | -CH₂-cPr | CH₃ |
| A42 | O | H | -CH₂-cPr | CF₃ |
| A43 | O | H | -CH₂-cPr | OCH₃ |
| A44 | O | H | -CH₂-cPr | OCF₃ |
| A45 | O | H | -CH₂-cPr | F |
| A46 | O | H | -CH₂-cPr | Cl |
| A47 | O | H | -CH₂-cPr | Br |
| A48 | O | H | -CH₂-cPr | 1-CN-cPr |
| A49 | O | H | -CH₂-cPr | 1-CN-iPr |
| A50 | O | H | -CH₂-cPr | H |
| A51 | O | H | -CH₂CHCH₂ | CH₃ |
| A52 | O | H | -CH₂CHCH₂ | CF₃ |
| A53 | O | H | -CH₂CHCH₂ | OCH₃ |
| A54 | O | H | -CH₂CHCH₂ | OCF₃ |
| A55 | O | H | -CH₂CHCH₂ | F |
| A56 | O | H | -CH₂CHCH₂ | Cl |
| A57 | O | H | -CH₂CHCH₂ | Br |
| A58 | O | H | -CH₂CHCH₂ | 1-CN-cPr |
| A59 | O | H | -CH₂CHCH₂ | 1-CN-iPr |
| A60 | O | H | -CH₂CHCH₂ | H |
| A61 | O | H | CH₂CH(CH₃)₂ | CH₃ |
| A62 | O | H | CH₂CH(CH₃)₂ | CF₃ |
| A63 | O | H | CH₂CH(CH₃)₂ | OCH₃ |
| A64 | O | H | CH₂CH(CH₃)₂ | OCF₃ |
| A65 | O | H | CH₂CH(CH₃)₂ | F |
| A66 | O | H | CH₂CH(CH₃)₂ | Cl |
| A67 | O | H | CH₂CH(CH₃)₂ | Br |
| A68 | O | H | CH₂CH(CH₃)₂ | 1-CN-cPr |
| A69 | O | H | CH₂CH(CH₃)₂ | 1-CN-iPr |
| A70 | O | H | CH₂CH(CH₃)₂ | H |
| A71 | O | H | -CH₂-Cy | CH₃ |
| A72 | O | H | -CH₂-Cy | CF₃ |
| A73 | O | H | -CH₂-Cy | OCH₃ |
| A74 | O | H | -CH₂-Cy | OCF₃ |
| A75 | O | H | -CH₂-Cy | F |
| A76 | O | H | -CH₂-Cy | Cl |
| A77 | O | H | -CH₂-Cy | Br |
| A78 | O | H | -CH₂-Cy | 1-CN-cPr |
| A79 | O | H | -CH₂-Cy | 1-CN-iPr |
| A80 | O | H | -CH₂-Cy | H |
| A81 | O | H | -CH₂CCH | CH₃ |
| A82 | O | H | -CH₂CCH | CF₃ |
| A83 | O | H | -CH₂CCH | OCH₃ |
| A84 | O | H | -CH₂CCH | OCF₃ |
| A85 | O | H | -CH₂CCH | F |
| A86 | O | H | -CH₂CCH | Cl |
| A87 | O | H | -CH₂CCH | Br |
| A88 | O | H | -CH₂CCH | 1-CN-cPr |
| A89 | O | H | -CH₂CCH | 1-CN-iPr |
| A90 | O | H | -CH₂CCH | H |
| A91 | O | H | -CH₂CF₃ | CH₃ |
| A92 | O | H | -CH₂CF₃ | CF₃ |
| A93 | O | H | -CH₂CF₃ | OCH₃ |
| A94 | O | H | -CH₂CF₃ | OCF₃ |
| A95 | O | H | -CH₂CF₃ | F |
| A96 | O | H | -CH₂CF₃ | Cl |
| A97 | O | H | -CH₂CF₃ | Br |
| A98 | O | H | -CH₂CF₃ | 1-CN-cPr |
| A99 | O | H | -CH₂CF₃ | 1-CN-iPr |
| A100 | O | H | -CH₂CF₃ | H |
| A101 | O | H | CH₂CH₂OCH 3 | CH₃ |
| A102 | O | H | CH₂CH₂OCH 3 | CF₃ |
| A103 | O | H | CH₂CH₂OCH 3 | OCH₃ |
| A104 | O | H | CH₂CH₂OCH 3 | OCF₃ |
| A105 | O | H | CH₂CH₂OCH 3 | F |
| A106 | O | H | CH₂CH₂OCH 3 | Cl |
| A107 | O | H | CH₂CH₂OCH 3 | Br |
| A108 | O | H | CH₂CH₂OCH 3 | 1-CN-cPr |
| A109 | O | H | CH₂CH₂OCH 3 | 1-CN-iPr |
| A110 | O | H | CH₂CH₂OCH 3 | H |
| A111 | O | H | -CH₂-cBu | CH₃ |
| A112 | O | H | -CH₂-cBu | CF₃ |
| A113 | O | H | -CH₂-cBu | OCH₃ |
| A114 | O | H | -CH₂-cBu | OCF₃ |
| A115 | O | H | -CH₂-cBu | F |
| A116 | O | H | -CH₂-cBu | Cl |
| A117 | O | H | -CH₂-cBu | Br |
| A118 | O | H | -CH₂-cBu | 1-CN-cPr |
| A119 | O | H | -CH₂-cBu | 1-CN-iPr |
| A120 | O | H | -CH₂-cBu | H |
| A121 | S | H | CH₃ | CH₃ |
| A122 | S | H | CH₃ | CF₃ |
| A123 | S | H | CH₃ | OCH₃ |
| A124 | S | H | CH₃ | OCF₃ |
| A125 | S | H | CH₃ | F |
| A126 | S | H | CH₃ | Cl |
| A127 | S | H | CH₃ | Br |
| A128 | S | H | CH₃ | 1-CN-cPr |
| A129 | S | H | CH₃ | 1-CN-iPr |
| A130 | S | H | CH₃ | H |
| A131 | S | H | CH₂CH₃ | CH₃ |
| A132 | S | H | CH₂CH₃ | CF₃ |
| A133 | S | H | CH₂CH₃ | OCH₃ |
| A134 | S | H | CH₂CH₃ | OCF₃ |
| A135 | S | H | CH₂CH₃ | F |
| A136 | S | H | CH₂CH₃ | Cl |
| A137 | S | H | CH₂CH₃ | Br |
| A138 | S | H | CH₂CH₃ | 1-CN-cPr |
| A139 | S | H | CH₂CH₃ | 1-CN-iPr |
| A140 | S | H | CH₂CH₃ | H |
| A141 | S | H | cPr | CH₃ |
| A142 | S | H | cPr | CF₃ |
| A143 | S | H | cPr | OCH₃ |
| A144 | S | H | cPr | OCF₃ |
| A145 | S | H | cPr | F |
| A146 | S | H | cPr | Cl |
| A147 | S | H | cPr | Br |
| A148 | S | H | cPr | 1-CN-cPr |
| A149 | S | H | cPr | 1-CN-iPr |
| A150 | S | H | cPr | H |
| A151 | S | H | Bn | CH₃ |
| A152 | S | H | Bn | CF₃ |
| A153 | S | H | Bn | OCH₃ |
| A154 | S | H | Bn | OCF₃ |
| A155 | S | H | Bn | F |
| A156 | S | H | Bn | Cl |
| A157 | S | H | Bn | Br |
| A158 | S | H | Bn | 1-CN-cPr |
| A159 | S | H | Bn | 1-CN-iPr |
| A160 | S | H | Bn | H |
| A161 | S | H | -CH₂-cPr | CH₃ |
| A162 | S | H | -CH₂-cPr | CF₃ |
| A163 | S | H | -CH₂-cPr | OCH₃ |
| A164 | S | H | -CH₂-cPr | OCF₃ |
| A165 | S | H | -CH₂-cPr | F |
| A166 | S | H | -CH₂-cPr | Cl |
| A167 | S | H | -CH₂-cPr | Br |
| A168 | S | H | -CH₂-cPr | 1-CN-cPr |
| A169 | S | H | -CH₂-cPr | 1-CN-iPr |
| A170 | S | H | -CH₂-cPr | H |
| A171 | S | H | -CH₂CHCH₂ | CH₃ |
| A172 | S | H | -CH₂CHCH₂ | CF₃ |
| A173 | S | H | -CH₂CHCH₂ | OCH₃ |
| A174 | S | H | -CH₂CHCH₂ | OCF₃ |
| A175 | S | H | -CH₂CHCH₂ | F |
| A176 | S | H | -CH₂CHCH₂ | Cl |
| A177 | S | H | -CH₂CHCH₂ | Br |
| A178 | S | H | -CH₂CHCH₂ | 1-CN-cPr |
| A179 | S | H | -CH₂CHCH₂ | 1-CN-iPr |
| A180 | S | H | -CH₂CHCH₂ | H |
| A181 | S | H | CH₂CH(CH₃)₂ | CH₃ |
| A182 | S | H | CH₂CH(CH₃)₂ | CF₃ |
| A183 | S | H | CH₂CH(CH₃)₂ | OCH₃ |
| A184 | S | H | CH₂CH(CH₃)₂ | OCF₃ |
| A185 | S | H | CH₂CH(CH₃)₂ | F |
| A186 | S | H | CH₂CH(CH₃)₂ | Cl |
| A187 | S | H | CH₂CH(CH₃)₂ | Br |
| A188 | S | H | CH₂CH(CH₃)₂ | 1-CN-cPr |
| A189 | S | H | CH₂CH(CH₃)₂ | 1-CN-iPr |
| A190 | S | H | CH₂CH(CH₃)₂ | H |
| A191 | S | H | -CH₂-Cy | CH₃ |
| A192 | S | H | -CH₂-Cy | CF₃ |
| A193 | S | H | -CH₂-Cy | OCH₃ |
| A194 | S | H | -CH₂-Cy | OCF₃ |
| A195 | S | H | -CH₂-Cy | F |
| A196 | S | H | -CH₂-Cy | Cl |
| A197 | S | H | -CH₂-Cy | Br |
| A198 | S | H | -CH₂-Cy | 1-CN-cPr |
| A199 | S | H | -CH₂-Cy | 1-CN-iPr |
| A200 | S | H | -CH₂-Cy | H |
| A201 | S | H | -CH₂CCH | CH₃ |
| A202 | S | H | -CH₂CCH | CF₃ |
| A203 | S | H | -CH₂CCH | OCH₃ |
| A204 | S | H | -CH₂CCH | OCF₃ |
| A205 | S | H | -CH₂CCH | F |
| A206 | S | H | -CH₂CCH | Cl |
| A207 | S | H | -CH₂CCH | Br |
| A208 | S | H | -CH₂CCH | 1-CN-cPr |
| A209 | S | H | -CH₂CCH | 1-CN-iPr |
| A210 | S | H | -CH₂CCH | H |
| A211 | S | H | -CH₂CF₃ | CH₃ |
| A212 | S | H | -CH₂CF₃ | CF₃ |
| A213 | S | H | -CH₂CF₃ | OCH₃ |
| A214 | S | H | -CH₂CF₃ | OCF₃ |
| A215 | S | H | -CH₂CF₃ | F |
| A216 | S | H | -CH₂CF₃ | Cl |
| A217 | S | H | -CH₂CF₃ | Br |
| A218 | S | H | -CH₂CF₃ | 1-CN-cPr |
| A219 | S | H | -CH₂CF₃ | 1-CN-iPr |
| A220 | S | H | -CH₂CF₃ | H |
| A221 | S | H | CH₂CH₂OCH ₃ | CH₃ |
| A222 | S | H | CH₂CH₂OCH ₃ | CF₃ |
| A223 | S | H | CH₂CH₂OCH ₃ | OCH₃ |
| A224 | S | H | CH₂CH₂OCH ₃ | OCF₃ |
| A225 | S | H | CH₂CH₂OCH ₃ | F |
| A226 | S | H | CH₂CH₂OCH ₃ | Cl |
| A227 | S | H | CH₂CH₂OCH ₃ | Br |
| A228 | S | H | CH₂CH₂OCH ₃ | 1-CN-cPr |
| A229 | S | H | CH₂CH₂OCH ₃ | 1-CN-iPr |
| A230 | S | H | CH₂CH₂OCH ₃ | H |
| A231 | S | H | -CH₂-cBu | CH₃ |
| A232 | S | H | -CH₂-cBu | CF₃ |
| A233 | S | H | -CH₂-cBu | OCH₃ |
| A234 | S | H | -CH₂-cBu | OCF₃ |
| A235 | S | H | -CH₂-cBu | F |
| A236 | S | H | -CH₂-cBu | Cl |
| A237 | S | H | -CH₂-cBu | Br |
| A238 | S | H | -CH₂-cBu | 1-CN-cPr |
| A239 | S | H | -CH₂-cBu | 1-CN-iPr |
| A240 | S | H | -CH₂-cBu | H |
| A241 | O | CH₃ | CH₃ | CH₃ |
| A242 | O | CH₃ | CH₃ | CF₃ |
| A243 | O | CH₃ | CH₃ | OCH₃ |
| A244 | O | CH₃ | CH₃ | OCF₃ |
| A245 | O | CH₃ | CH₃ | F |
| A246 | O | CH₃ | CH₃ | Cl |
| A247 | O | CH₃ | CH₃ | Br |
| A248 | O | CH₃ | CH₃ | 1-CN-cPr |
| A249 | O | CH₃ | CH₃ | 1-CN-iPr |
| A250 | O | CH₃ | CH₃ | H |
| A251 | O | CH₃ | CH₂CH₃ | CH₃ |
| A252 | O | CH₃ | CH₂CH₃ | CF₃ |
| A253 | O | CH₃ | CH₂CH₃ | OCH₃ |
| A254 | O | CH₃ | CH₂CH₃ | OCF₃ |
| A255 | O | CH₃ | CH₂CH₃ | F |
| A256 | O | CH₃ | CH₂CH₃ | Cl |
| A257 | O | CH₃ | CH₂CH₃ | Br |
| A258 | O | CH₃ | CH₂CH₃ | 1-CN-cPr |
| A259 | O | CH₃ | CH₂CH₃ | 1-CN-iPr |
| A260 | O | CH₃ | CH₂CH₃ | H |
| A261 | O | CH₃ | cPr | CH₃ |
| A262 | O | CH₃ | cPr | CF₃ |
| A263 | O | CH₃ | cPr | OCH₃ |
| A264 | O | CH₃ | cPr | OCF₃ |
| A265 | O | CH₃ | cPr | F |
| A266 | O | CH₃ | cPr | Cl |
| A267 | O | CH₃ | cPr | Br |
| A268 | O | CH₃ | cPr | 1-CN-cPr |
| A269 | O | CH₃ | cPr | 1-CN-iPr |
| A270 | O | CH₃ | cPr | H |
| A271 | O | CH₃ | Bn | CH₃ |
| A272 | O | CH₃ | Bn | CF₃ |
| A273 | O | CH₃ | Bn | OCH₃ |
| A274 | O | CH₃ | Bn | OCF₃ |
| A275 | O | CH₃ | Bn | F |
| A276 | O | CH₃ | Bn | Cl |
| A277 | O | CH₃ | Bn | Br |
| A278 | O | CH₃ | Bn | 1-CN-cPr |
| A279 | O | CH₃ | Bn | 1-CN-iPr |
| A280 | O | CH₃ | Bn | H |
| A281 | O | CH₃ | -CH₂-cPr | CH₃ |
| A282 | O | CH₃ | -CH₂-cPr | CF₃ |
| A283 | O | CH₃ | -CH₂-cPr | OCH₃ |
| A284 | O | CH₃ | -CH₂-cPr | OCF₃ |
| A285 | O | CH₃ | -CH₂-cPr | F |
| A286 | O | CH₃ | -CH₂-cPr | Cl |
| A287 | O | CH₃ | -CH₂-cPr | Br |
| A288 | O | CH₃ | -CH₂-cPr | 1-CN-cPr |
| A289 | O | CH₃ | -CH₂-cPr | 1-CN-iPr |
| A290 | O | CH₃ | -CH₂-cPr | H |
| A291 | O | CH₃ | -CH₂CHCH₂ | CH₃ |
| A292 | O | CH₃ | -CH₂CHCH₂ | CF₃ |
| A293 | O | CH₃ | -CH₂CHCH₂ | OCH₃ |
| A294 | O | CH₃ | -CH₂CHCH₂ | OCF₃ |
| A295 | O | CH₃ | -CH₂CHCH₂ | F |
| A296 | O | CH₃ | -CH₂CHCH₂ | Cl |
| A297 | O | CH₃ | -CH₂CHCH₂ | Br |
| A298 | O | CH₃ | -CH₂CHCH₂ | 1-CN-cPr |
| A299 | O | CH₃ | -CH₂CHCH₂ | 1-CN-iPr |
| A300 | O | CH₃ | -CH₂CHCH₂ | H |
| A301 | O | CH₃ | CH₂CH(CH₃)₂ | CH₃ |
| A302 | O | CH₃ | CH₂CH(CH₃)₂ | CF₃ |
| A303 | O | CH₃ | CH₂CH(CH₃)₂ | OCH₃ |
| A304 | O | CH₃ | CH₂CH(CH₃)₂ | OCF₃ |
| A305 | O | CH₃ | CH₂CH(CH₃)₂ | F |
| A306 | O | CH₃ | CH₂CH(CH₃)₂ | Cl |
| A307 | O | CH₃ | CH₂CH(CH₃)₂ | Br |
| A308 | O | CH₃ | CH₂CH(CH₃)₂ | 1-CN-cPr |
| A309 | O | CH₃ | CH₂CH(CH₃)₂ | 1-CN-iPr |
| A310 | O | CH₃ | CH₂CH(CH₃)₂ | H |
| A311 | O | CH₃ | -CH₂-Cy | CH₃ |
| A312 | O | CH₃ | -CH₂-Cy | CF₃ |
| A313 | O | CH₃ | -CH₂-Cy | OCH₃ |
| A314 | O | CH₃ | -CH₂-Cy | OCF₃ |
| A315 | O | CH₃ | -CH₂-Cy | F |
| A316 | O | CH₃ | -CH₂-Cy | Cl |
| A317 | O | CH₃ | -CH₂-Cy | Br |
| A318 | O | CH₃ | -CH₂-Cy | 1-CN-cPr |
| A319 | O | CH₃ | -CH₂-Cy | 1-CN-iPr |
| A320 | O | CH₃ | -CH₂-Cy | H |
| A321 | O | CH₃ | -CH₂CCH | CH₃ |
| A322 | O | CH₃ | -CH₂CCH | CF₃ |
| A323 | O | CH₃ | -CH₂CCH | OCH₃ |
| A324 | O | CH₃ | -CH₂CCH | OCF₃ |
| A325 | O | CH₃ | -CH₂CCH | F |
| A326 | O | CH₃ | -CH₂CCH | Cl |
| A327 | O | CH₃ | -CH₂CCH | Br |
| A328 | O | CH₃ | -CH₂CCH | 1-CN-cPr |
| A329 | O | CH₃ | -CH₂CCH | 1-CN-iPr |
| A330 | O | CH₃ | -CH₂CCH | H |
| A331 | O | CH₃ | -CH₂CF₃ | CH₃ |
| A332 | O | CH₃ | -CH₂CF₃ | CF₃ |
| A333 | O | CH₃ | -CH₂CF₃ | OCH₃ |
| A334 | O | CH₃ | -CH₂CF₃ | OCF₃ |
| A335 | O | CH₃ | -CH₂CF₃ | F |
| A336 | O | CH₃ | -CH₂CF₃ | Cl |
| A337 | O | CH₃ | -CH₂CF₃ | Br |
| A338 | O | CH₃ | -CH₂CF₃ | 1-CN-cPr |
| A339 | O | CH₃ | -CH₂CF₃ | 1-CN-iPr |
| A340 | O | CH₃ | -CH₂CF₃ | H |
| A341 | O | CH₃ | CH₂CH₂OCH ₃ | CH₃ |
| A342 | O | CH₃ | CH₂CH₂OCH ₃ | CF₃ |
| A343 | O | CH₃ | CH₂CH₂OCH ₃ | OCH₃ |
| A344 | O | CH₃ | CH₂CH₂OCH ₃ | OCF₃ |
| A345 | O | CH₃ | CH₂CH₂OCH ₃ | F |
| A346 | O | CH₃ | CH₂CH₂OCH ₃ | Cl |
| A347 | O | CH₃ | CH₂CH₂OCH ₃ | Br |
| A348 | O | CH₃ | CH₂CH₂OCH ₃ | 1-CN-cPr |
| A349 | O | CH₃ | CH₂CH₂OCH ₃ | 1-CN-iPr |
| A350 | O | CH₃ | CH₂CH₂OCH ₃ | H |
| A351 | O | CH₃ | -CH₂-cBu | CH₃ |
| A352 | O | CH₃ | -CH₂-cBu | CF₃ |
| A353 | O | CH₃ | -CH₂-cBu | OCH₃ |
| A354 | O | CH₃ | -CH₂-cBu | OCF₃ |
| A355 | O | CH₃ | -CH₂-cBu | F |
| A356 | O | CH₃ | -CH₂-cBu | Cl |
| A357 | O | CH₃ | -CH₂-cBu | Br |
| A358 | O | CH₃ | -CH₂-cBu | 1-CN-cPr |
| A359 | O | CH₃ | -CH₂-cBu | 1-CN-iPr |
| A360 | O | CH₃ | -CH₂-cBu | H |
| A361 | S | CH₃ | CH₃ | CH₃ |
| A362 | S | CH₃ | CH₃ | CF₃ |
| A363 | S | CH₃ | CH₃ | OCH₃ |
| A364 | S | CH₃ | CH₃ | OCF₃ |
| A365 | S | CH₃ | CH₃ | F |
| A366 | S | CH₃ | CH₃ | Cl |
| A367 | S | CH₃ | CH₃ | Br |
| A368 | S | CH₃ | CH₃ | 1-CN-cPr |
| A369 | S | CH₃ | CH₃ | 1-CN-iPr |
| A370 | S | CH₃ | CH₃ | H |
| A371 | S | CH₃ | CH₂CH₃ | CH₃ |
| A372 | S | CH₃ | CH₂CH₃ | CF₃ |
| A373 | S | CH₃ | CH₂CH₃ | OCH₃ |
| A374 | S | CH₃ | CH₂CH₃ | OCF₃ |
| A375 | S | CH₃ | CH₂CH₃ | F |
| A376 | S | CH₃ | CH₂CH₃ | Cl |
| A377 | S | CH₃ | CH₂CH₃ | Br |
| A378 | S | CH₃ | CH₂CH₃ | 1-CN-cPr |
| A379 | S | CH₃ | CH₂CH₃ | 1-CN-iPr |
| A380 | S | CH₃ | CH₂CH₃ | H |
| A381 | S | CH₃ | cPr | CH₃ |
| A382 | S | CH₃ | cPr | CF₃ |
| A383 | S | CH₃ | cPr | OCH₃ |
| A384 | S | CH₃ | cPr | OCF₃ |
| A385 | S | CH₃ | cPr | F |
| A386 | S | CH₃ | cPr | Cl |
| A387 | S | CH₃ | cPr | Br |
| A388 | S | CH₃ | cPr | 1-CN-cPr |
| A389 | S | CH₃ | cPr | 1-CN-iPr |
| A390 | S | CH₃ | cPr | H |
| A391 | S | CH₃ | Bn | CH₃ |
| A392 | S | CH₃ | Bn | CF₃ |
| A393 | S | CH₃ | Bn | OCH₃ |
| A394 | S | CH₃ | Bn | OCF₃ |
| A395 | S | CH₃ | Bn | F |
| A396 | S | CH₃ | Bn | Cl |
| A397 | S | CH₃ | Bn | Br |
| A398 | S | CH₃ | Bn | 1-CN-cPr |
| A399 | S | CH₃ | Bn | 1-CN-iPr |
| A400 | S | CH₃ | Bn | H |
| A401 | S | CH₃ | -CH₂-cPr | CH₃ |
| A402 | S | CH₃ | -CH₂-cPr | CF₃ |
| A403 | S | CH₃ | -CH₂-cPr | OCH₃ |
| A404 | S | CH₃ | -CH₂-cPr | OCF₃ |
| A405 | S | CH₃ | -CH₂-cPr | F |
| A406 | S | CH₃ | -CH₂-cPr | Cl |
| A407 | S | CH₃ | -CH₂-cPr | Br |
| A408 | S | CH₃ | -CH₂-cPr | 1-CN-cPr |
| A409 | S | CH₃ | -CH₂-cPr | 1-CN-iPr |
| A410 | S | CH₃ | -CH₂-cPr | H |
| A411 | S | CH₃ | -CH₂CHCH₂ | CH₃ |
| A412 | S | CH₃ | -CH₂CHCH₂ | CF₃ |
| A413 | S | CH₃ | -CH₂CHCH₂ | OCH₃ |
| A414 | S | CH₃ | -CH₂CHCH₂ | OCF₃ |
| A415 | S | CH₃ | -CH₂CHCH₂ | F |
| A416 | S | CH₃ | -CH₂CHCH₂ | Cl |
| A417 | S | CH₃ | -CH₂CHCH₂ | Br |
| A418 | S | CH₃ | -CH₂CHCH₂ | 1-CN-cPr |
| A419 | S | CH₃ | -CH₂CHCH₂ | 1-CN-iPr |
| A420 | S | CH₃ | -CH₂CHCH₂ | H |
| A421 | S | CH₃ | CH₂CH(CH₃)₂ | CH₃ |
| A422 | S | CH₃ | CH₂CH(CH₃)₂ | CF₃ |
| A423 | S | CH₃ | CH₂CH(CH₃)₂ | OCH₃ |
| A424 | S | CH₃ | CH₂CH(CH₃)₂ | OCF₃ |
| A425 | S | CH₃ | CH₂CH(CH₃)₂ | F |
| A426 | S | CH₃ | CH₂CH(CH₃)₂ | Cl |
| A427 | S | CH₃ | CH₂CH(CH₃)₂ | Br |
| A428 | S | CH₃ | CH₂CH(CH₃)₂ | 1-CN-cPr |
| A429 | S | CH₃ | CH₂CH(CH₃)₂ | 1-CN-iPr |
| A430 | S | CH₃ | CH₂CH(CH₃)₂ | H |
| A431 | S | CH₃ | -CH₂-Cy | CH₃ |
| A432 | S | CH₃ | -CH₂-Cy | CF₃ |
| A433 | S | CH₃ | -CH₂-Cy | OCH₃ |
| A434 | S | CH₃ | -CH₂-Cy | OCF₃ |
| A435 | S | CH₃ | -CH₂-Cy | F |
| A436 | S | CH₃ | -CH₂-Cy | Cl |
| A437 | S | CH₃ | -CH₂-Cy | Br |
| A438 | S | CH₃ | -CH₂-Cy | 1-CN-cPr |
| A439 | S | CH₃ | -CH₂-Cy | 1-CN-iPr |
| A440 | S | CH₃ | -CH₂-Cy | H |
| A441 | S | CH₃ | -CH₂CCH | CH₃ |
| A442 | S | CH₃ | -CH₂CCH | CF₃ |
| A443 | S | CH₃ | -CH₂CCH | OCH₃ |
| A444 | S | CH₃ | -CH₂CCH | OCF₃ |
| A445 | S | CH₃ | -CH₂CCH | F |
| A446 | S | CH₃ | -CH₂CCH | Cl |
| A447 | S | CH₃ | -CH₂CCH | Br |
| A448 | S | CH₃ | -CH₂CCH | 1-CN-cPr |
| A449 | S | CH₃ | -CH₂CCH | 1-CN-iPr |
| A450 | S | CH₃ | -CH₂CCH | H |
| A451 | S | CH₃ | -CH₂CF₃ | CH₃ |
| A452 | S | CH₃ | -CH₂CF₃ | CF₃ |
| A453 | S | CH₃ | -CH₂CF₃ | OCH₃ |
| A454 | S | CH₃ | -CH₂CF₃ | OCF₃ |
| A455 | S | CH₃ | -CH₂CF₃ | F |
| A456 | S | CH₃ | -CH₂CF₃ | Cl |
| A457 | S | CH₃ | -CH₂CF₃ | Br |
| A458 | S | CH₃ | -CH₂CF₃ | 1-CN-cPr |
| A459 | S | CH₃ | -CH₂CF₃ | 1-CN-iPr |
| A460 | S | CH₃ | -CH₂CF₃ | H |
| A461 | S | CH₃ | CH₂CH₂OCH ₃ | CH₃ |
| A462 | S | CH₃ | CH₂CH₂OCH ₃ | CF₃ |
| A463 | S | CH₃ | CH₂CH₂OCH ₃ | OCH₃ |
| A464 | S | CH₃ | CH₂CH₂OCH ₃ | OCF₃ |
| A465 | S | CH₃ | CH₂CH₂OCH ₃ | F |
| A466 | S | CH₃ | CH₂CH₂OCH ₃ | Cl |
| A467 | S | CH₃ | CH₂CH₂OCH ₃ | Br |
| A468 | S | CH₃ | CH₂CH₂OCH ₃ | 1-CN-cPr |
| A469 | S | CH₃ | CH₂CH₂OCH ₃ | 1-CN-iPr |
| A470 | S | CH₃ | CH₂CH₂OCH ₃ | H |
| A471 | S | CH₃ | -CH₂-cBu | CH₃ |
| A472 | S | CH₃ | -CH₂-cBu | CF₃ |
| A473 | S | CH₃ | -CH₂-cBu | OCH₃ |
| A474 | S | CH₃ | -CH₂-cBu | OCF₃ |
| A475 | S | CH₃ | -CH₂-cBu | F |
| A476 | S | CH₃ | -CH₂-cBu | Cl |
| A477 | S | CH₃ | -CH₂-cBu | Br |
| A478 | S | CH₃ | -CH₂-cBu | 1-CN-cPr |
| A479 | S | CH₃ | -CH₂-cBu | 1-CN-iPr |
| A480 | S | CH₃ | -CH₂-cBu | H |

The following Embodiments 1 to 574 represent specific combinations of definitions for all substituents and Tables. Each combination individually and all combinations collectively represent preferred embodiments.

Embodiment 1: R¹ is CF₃, R⁶ is CH₃; R^{W} is CH₂CH₃, the index m is 2, R⁷ and R⁸ - if present - are H, the ring system is represented by Table 1 and R², R³, X and R⁹ have a meaning as defined in a line A1 to A480 in Table A.

Embodiment 2: R¹ is CF₃, R⁶ is CH₃; R^{W} is CH₂CH₃, the index m is 2, R⁷ and R⁸ - if present - are H, the ring system is represented by Table 2 and R², R³, X and R⁹ have a meaning as defined in a line A1 to A480 in Table A.

Embodiment 3: R¹ is CF₃, R⁶ is CH₃; R^{W} is CH₂CH₃, the index m is 2, R⁷ and R⁸ - if present - are H, the ring system is represented by Table 3 and R², R³, X and R⁹ have a meaning as defined in a line A1 to A480 in Table A.

Embodiment 4: R¹ is CF₃, R⁶ is CH₃; R^{W} is CH₂CH₃, the index m is 2, R⁷ and R⁸ - if present - are H, the ring system is represented by Table 4 and R², R³, X and R⁹ have a meaning as defined in a line A1 to A480 in Table A.

Embodiment 5: R¹ is CF₃, R⁶ is CH₃; R^{W} is CH₂CH₃, the index m is 2, R⁷ and R⁸ - if present - are H, the ring system is represented by Table 5 and R², R³, X and R⁹ have a meaning as defined in a line A1 to A480 in Table A.

Embodiment 6: R¹ is CF₃, R⁶ is CH₃; R^{W} is CH₂CH₃, the index m is 2, R⁷ and R⁸ - if present - are H, the ring system is represented by Table 6 and R², R³, X and R⁹ have a meaning as defined in a line A1 to A480 in Table A.

Embodiment 7: R¹ is CF₃, R⁶ is CH₃; R^{W} is CH₂CH₃, the index m is 2, R⁷ and R⁸ - if present - are H, the ring system is represented by Table 7 and R², R³, X and R⁹ have a meaning as defined in a line A1 to A480 in Table A.

Embodiment 8: R¹ is CF₃, R⁶ is CH₃; R^{W} is CH₂CH₃, the index m is 2, R⁷ and R⁸ - if present - are H, the ring system is represented by Table 8 and R², R³, X and R⁹ have a meaning as defined in a line A1 to A480 in Table A.

Embodiment 9: R¹ is CF₃, R⁶ is CH₃; R^{W} is CH₂CH₃, the index m is 2, R⁷ and R⁸ - if present - are H, the ring system is represented by Table 9 and R², R³, X and R⁹ have a meaning as defined in a line A1 to A480 in Table A.

Embodiment 10: R¹ is CF₃, R⁶ is CH₃; R^{W} is CH₂CH₃, the index m is 2, R⁷ and R⁸ - if present - are H, the ring system is represented by Table 10 and R², R³, X and R⁹ have a meaning as defined in a line A1 to A480 in Table A.

Embodiment 11: R¹ is CF₃, R⁶ is CH₃; R^{W} is CH₂CH₃, the index m is 2, R⁷ and R⁸ - if present - are H, the ring system is represented by Table 11 and R², R³, X and R⁹ have a meaning as defined in a line A1 to A480 in Table A.

Embodiment 12: R¹ is CF₃, R⁶ is CH₃; R^{W} is CH₂CH₃, the index m is 2, R⁷ and R⁸ - if present - are H, the ring system is represented by Table 12 and R², R³, X and R⁹ have a meaning as defined in a line A1 to A480 in Table A.

Embodiment 13: R¹ is CF₃, R⁶ is CH₃; R^{W} is CH₂CH₃, the index m is 2, R⁷ and R⁸ - if present - are H, the ring system is represented by Table 13 and R², R³, X and R⁹ have a meaning as defined in a line A1 to A480 in Table A.

Embodiment 14: R¹ is CF₃, R⁶ is CH₃; R^{W} is CH₂CH₃, the index m is 2, R⁷ and R⁸ - if present - are H, the ring system is represented by Table 14 and R², R³, X and R⁹ have a meaning as defined in a line A1 to A480 in Table A.

Embodiment 15: R¹ is CF₃, R⁶ is CH₃; R^{W} is CH₂CH₃, the index m is 2, R⁷ and R⁸ - if present - are H, the ring system is represented by Table 15 and R², R³, X and R⁹ have a meaning as defined in a line A1 to A480 in Table A.

Embodiment 16: R¹ is CF₃, R⁶ is CH₃; R^{W} is CH₂CH₃, the index m is 2, R⁷ and R⁸ - if present - are H, the ring system is represented by Table 16 and R², R³, X and R⁹ have a meaning as defined in a line A1 to A480 in Table A.

Embodiment 17: R¹ is CF₃, R⁶ is CH₃; R^{W} is CH₂CH₃, the index m is 2, R⁷ and R⁸ - if present - are H, the ring system is represented by Table 17 and R², R³, X and R⁹ have a meaning as defined in a line A1 to A480 in Table A.

Embodiment 18: R¹ is CF₃, R⁶ is CH₃; R^{W} is CH₂CH₃, the index m is 2, R⁷ and R⁸ - if present - are H, the ring system is represented by Table 18 and R², R³, X and R⁹ have a meaning as defined in a line A1 to A480 in Table A.

Embodiment 19: R¹ is CF₃, R⁶ is CH₃; R^{W} is CH₂CH₃, the index m is 2, R⁷ and R⁸ - if present - are H, the ring system is represented by Table 19 and R², R³, X and R⁹ have a meaning as defined in a line A1 to A480 in Table A.

Embodiment 20: R¹ is CF₃, R⁶ is CH₃; R^{W} is CH₂CH₃, the index m is 2, R⁷ and R⁸ - if present - are H, the ring system is represented by Table 20 and R², R³, X and R⁹ have a meaning as defined in a line A1 to A480 in Table A.

Embodiment 21: R¹ is CF₃, R⁶ is CH₃; R^{W} is CH₂CH₃, the index m is 2, R⁷ and R⁸ - if present - are H, the ring system is represented by Table 21 and R², R³, X and R⁹ have a meaning as defined in a line A1 to A480 in Table A.

Embodiment 22: R¹ is CF₃, R⁶ is CH₃; R^{W} is CH₂CH₃, the index m is 2, R⁷ and R⁸ - if present - are H, the ring system is represented by Table 22 and R², R³, X and R⁹ have a meaning as defined in a line A1 to A480 in Table A.

Embodiment 23: R¹ is CF₃, R⁶ is CH₃; R^{W} is CH₂CH₃, the index m is 2, R⁷ and R⁸ - if present - are H, the ring system is represented by Table 23 and R², R³, X and R⁹ have a meaning as defined in a line A1 to A480 in Table A.

Embodiment 24: R¹ is CF₃, R⁶ is CH₃; R^{W} is CH₂CH₃, the index m is 2, R⁷ and R⁸ - if present - are H, the ring system is represented by Table 24 and R², R³, X and R⁹ have a meaning as defined in a line A1 to A480 in Table A.

Embodiment 25: R¹ is CF₃, R⁶ is CH₃; R^{W} is CH₂CH₃, the index m is 2, R⁷ and R⁸ - if present - are H, the ring system is represented by Table 25 and R², R³, X and R⁹ have a meaning as defined in a line A1 to A480 in Table A.

Embodiment 26: R¹ is CF₃, R⁶ is CH₃; R^{W} is CH₂CH₃, the index m is 2, R⁷ and R⁸ - if present - are H, the ring system is represented by Table 26 and R², R³, X and R⁹ have a meaning as defined in a line A1 to A480 in Table A.

Embodiment 27: R¹ is CF₃, R⁶ is CH₃; R^{W} is CH₂CH₃, the index m is 2, R⁷ and R⁸ - if present - are H, the ring system is represented by Table 27 and R², R³, X and R⁹ have a meaning as defined in a line A1 to A480 in Table A.

Embodiment 28: R¹ is CF₃, R⁶ is CH₃; R^{W} is CH₂CH₃, the index m is 2, R⁷ and R⁸ - if present - are H, the ring system is represented by Table 28 and R², R³, X and R⁹ have a meaning as defined in a line A1 to A480 in Table A.

Embodiment 29: R¹ is CF₃, R⁶ is CH₃; R^{W} is CH₂CH₃, the index m is 2, R⁷ and R⁸ - if present - are H, the ring system is represented by Table 29 and R², R³, X and R⁹ have a meaning as defined in a line A1 to A480 in Table A.

Embodiment 30: R¹ is CF₃, R⁶ is CH₃; R^{W} is CH₂CH₃, the index m is 2, R⁷ and R⁸ - if present - are H, the ring system is represented by Table 30 and R², R³, X and R⁹ have a meaning as defined in a line A1 to A480 in Table A.

Embodiment 31: R¹ is CF₃, R⁶ is CH₃; R^{W} is CH₂CH₃, the index m is 2, R⁷ and R⁸ - if present - are H, the ring system is represented by Table 31 and R², R³, X and R⁹ have a meaning as defined in a line A1 to A480 in Table A.

Embodiment 32: R¹ is CF₃, R⁶ is CH₃; R^{W} is CH₂CH₃, the index m is 2, R⁷ and R⁸ - if present - are H, the ring system is represented by Table 32 and R², R³, X and R⁹ have a meaning as defined in a line A1 to A480 in Table A.

Embodiment 33: R¹ is CF₃, R⁶ is CH₃; R^{W} is CH₂CH₃, the index m is 2, R⁷ and R⁸ - if present - are H, the ring system is represented by Table 33 and R², R³, X and R⁹ have a meaning as defined in a line A1 to A480 in Table A.

Embodiment 34: R¹ is CF₃, R⁶ is CH₃; R^{W} is CH₂CH₃, the index m is 2, R⁷ and R⁸ - if present - are H, the ring system is represented by Table 34 and R², R³, X and R⁹ have a meaning as defined in a line A1 to A480 in Table A.

Embodiment 35: R¹ is CF₃, R⁶ is CH₃; R^{W} is CH₂CH₃, the index m is 2, R⁷ and R⁸ - if present - are H, the ring system is represented by Table 35 and R², R³, X and R⁹ have a meaning as defined in a line A1 to A480 in Table A.

Embodiment 36: R¹ is CF₃, R⁶ is CH₃; R^{W} is CH₂CH₃, the index m is 2, R⁷ and R⁸ - if present - are H, the ring system is represented by Table 36 and R², R³, X and R⁹ have a meaning as defined in a line A1 to A480 in Table A.

Embodiment 37: R¹ is CF₃, R⁶ is CH₃; R^{W} is CH₂CH₃, the index m is 2, R⁷ and R⁸ - if present - are H, the ring system is represented by Table 37 and R², R³, X and R⁹ have a meaning as defined in a line A1 to A480 in Table A.

Embodiment 38: R¹ is CF₃, R⁶ is CH₃; R^{W} is CH₂CH₃, the index m is 2, R⁷ and R⁸ - if present - are H, the ring system is represented by Table 38 and R², R³, X and R⁹ have a meaning as defined in a line A1 to A480 in Table A.

Embodiment 39: R¹ is CF₃, R⁶ is CH₃; R^{W} is CH₂CH₃, the index m is 2, R⁷ and R⁸ - if present - are H, the ring system is represented by Table 39 and R², R³, X and R⁹ have a meaning as defined in a line A1 to A480 in Table A.

Embodiment 40: R¹ is CF₃, R⁶ is CH₃; R^{W} is CH₂CH₃, the index m is 2, R⁷ and R⁸ - if present - are H, the ring system is represented by Table 40 and R², R³, X and R⁹ have a meaning as defined in a line A1 to A480 in Table A.

Embodiment 41: R¹ is CF₃, R⁶ is CH₃; R^{W} is CH₂CH₃, the index m is 2, R⁷ and R⁸ - if present - are H, the ring system is represented by Table 41 and R², R³, X and R⁹ have a meaning as defined in a line A1 to A480 in Table A.

Embodiment 42: R¹ is CF₃, R⁶ is CH₃; R^{W} is CH₂CH₃, the index m is 2, R⁷ and R⁸ - if present - are H, the ring system is represented by Table 42 and R², R³, X and R⁹ have a meaning as defined in a line A1 to A480 in Table A.

Embodiment 43: R¹ is CF₃, R⁶ is CH₃; R^{W} is CH₂CH₃, the index m is 2, R⁷ and R⁸ - if present - are H, the ring system is represented by Table 43 and R², R³, X and R⁹ have a meaning as defined in a line A1 to A480 in Table A.

Embodiment 44: R¹ is CF₃, R⁶ is CH₃; R^{W} is CH₂CH₃, the index m is 2, R⁷ and R⁸ - if present - are H, the ring system is represented by Table 44 and R², R³, X and R⁹ have a meaning as defined in a line A1 to A480 in Table A.

Embodiment 45: R¹ is CF₃, R⁶ is CH₃; R^{W} is CH₂CH₃, the index m is 2, R⁷ and R⁸ - if present - are H, the ring system is represented by Table 45 and R², R³, X and R⁹ have a meaning as defined in a line A1 to A480 in Table A.

Embodiment 46: R¹ is CF₃, R⁶ is CH₃; R^{W} is CH₂CH₃, the index m is 2, R⁷ and R⁸ - if present - are H, the ring system is represented by Table 46 and R², R³, X and R⁹ have a meaning as defined in a line A1 to A480 in Table A.

Embodiment 47: R¹ is CF₃, R⁶ is CH₃; R^{W} is CH₂CH₃, the index m is 2, R⁷ and R⁸ - if present - are H, the ring system is represented by Table 47 and R², R³, X and R⁹ have a meaning as defined in a line A1 to A480 in Table A.

Embodiment 48: R¹ is CF₃, R⁶ is CH₃; R^{W} is CH₂CH₃, the index m is 2, R⁷ and R⁸ - if present - are H, the ring system is represented by Table 48 and R², R³, X and R⁹ have a meaning as defined in a line A1 to A480 in Table A.

Embodiment 49: R¹ is CF₃, R⁶ is CH₃; R^{W} is CH₂CH₃, the index m is 2, R⁷ and R⁸ - if present - are H, the ring system is represented by Table 49 and R², R³, X and R⁹ have a meaning as defined in a line A1 to A480 in Table A.

Embodiment 50: R¹ is CF₃, R⁶ is CH₃; R^{W} is CH₂CH₃, the index m is 2, R⁷ and R⁸ - if present - are H, the ring system is represented by Table 50 and R², R³, X and R⁹ have a meaning as defined in a line A1 to A480 in Table A.

Embodiment 51: R¹ is CF₃, R⁶ is CH₃; R^{W} is CH₂CH₃, the index m is 2, R⁷ and R⁸ - if present - are H, the ring system is represented by Table 51 and R², R³, X and R⁹ have a meaning as defined in a line A1 to A480 in Table A.

Embodiment 52: R¹ is CF₃, R⁶ is CH₃; R^{W} is CH₂CH₃, the index m is 2, R⁷ and R⁸ - if present - are H, the ring system is represented by Table 52 and R², R³, X and R⁹ have a meaning as defined in a line A1 to A480 in Table A.

Embodiment 53: R¹ is CF₃, R⁶ is CH₃; R^{W} is CH₂CH₃, the index m is 2, R⁷ and R⁸ - if present - are H, the ring system is represented by Table 53 and R², R³, X and R⁹ have a meaning as defined in a line A1 to A480 in Table A.

Embodiment 54: R¹ is CF₃, R⁶ is CH₃; R^{W} is CH₂CH₃, the index m is 2, R⁷ and R⁸ - if present - are H, the ring system is represented by Table 54 and R², R³, X and R⁹ have a meaning as defined in a line A1 to A480 in Table A.

Embodiment 55: R¹ is CF₃, R⁶ is CH₃; R^{W} is CH₂CH₃, the index m is 2, R⁷ and R⁸ - if present - are H, the ring system is represented by Table 55 and R², R³, X and R⁹ have a meaning as defined in a line A1 to A480 in Table A.

Embodiment 56: R¹ is CF₃, R⁶ is CH₃; R^{W} is CH₂CH₃, the index m is 2, R⁷ and R⁸ - if present - are H, the ring system is represented by Table 56 and R², R³, X and R⁹ have a meaning as defined in a line A1 to A480 in Table A.

Embodiment 57: R¹ is CF₃, R⁶ is CH₃; R^{W} is CH₂CH₃, the index m is 2, R⁷ and R⁸ - if present - are H, the ring system is represented by Table 57 and R², R³, X and R⁹ have a meaning as defined in a line A1 to A480 in Table A.

Embodiment 58: R¹ is CF₃, R⁶ is CH₃; R^{W} is CH₂CH₃, the index m is 2, R⁷ and R⁸ - if present - are H, the ring system is represented by Table 58 and R², R³, X and R⁹ have a meaning as defined in a line A1 to A480 in Table A.

Embodiment 59: R¹ is CF₃, R⁶ is CH₃; R^{W} is CH₂CH₃, the index m is 2, R⁷ and R⁸ - if present - are H, the ring system is represented by Table 59 and R², R³, X and R⁹ have a meaning as defined in a line A1 to A480 in Table A.

Embodiment 60: R¹ is CF₃, R⁶ is CH₃; R^{W} is CH₂CH₃, the index m is 2, R⁷ and R⁸ - if present - are H, the ring system is represented by Table 60 and R², R³, X and R⁹ have a meaning as defined in a line A1 to A480 in Table A.

Embodiment 61: R¹ is CF₃, R⁶ is CH₃; R^{W} is CH₂CH₃, the index m is 2, R⁷ and R⁸ - if present - are H, the ring system is represented by Table 61 and R², R³, X and R⁹ have a meaning as defined in a line A1 to A480 in Table A.

Embodiment 62: R¹ is CF₃, R⁶ is CH₃; R^{W} is CH₂CH₃, the index m is 2, R⁷ and R⁸ - if present - are H, the ring system is represented by Table 62 and R², R³, X and R⁹ have a meaning as defined in a line A1 to A480 in Table A.

Embodiment 63: R¹ is CF₃, R⁶ is CH₃; R^{W} is CH₂CH₃, the index m is 2, R⁷ and R⁸ - if present - are H, the ring system is represented by Table 63 and R², R³, X and R⁹ have a meaning as defined in a line A1 to A480 in Table A.

Embodiment 64: R¹ is CF₃, R⁶ is CH₃; R^{W} is CH₂CH₃, the index m is 2, R⁷ and R⁸ - if present - are H, the ring system is represented by Table 64 and R², R³, X and R⁹ have a meaning as defined in a line A1 to A480 in Table A.

Embodiment 65: R¹ is CF₃, R⁶ is CH₃; R^{W} is CH₂CH₃, the index m is 2, R⁷ and R⁸ - if present - are H, the ring system is represented by Table 65 and R², R³, X and R⁹ have a meaning as defined in a line A1 to A480 in Table A.

Embodiment 66: R¹ is CF₃, R⁶ is CH₃; R^{W} is CH₂CH₃, the index m is 2, R⁷ and R⁸ - if present - are H, the ring system is represented by Table 66 and R², R³, X and R⁹ have a meaning as defined in a line A1 to A480 in Table A.

Embodiment 67: R¹ is CF₃, R⁶ is CH₃; R^{W} is CH₂CH₃, the index m is 2, R⁷ and R⁸ - if present - are H, the ring system is represented by Table 67 and R², R³, X and R⁹ have a meaning as defined in a line A1 to A480 in Table A.

Embodiment 68: R¹ is CF₃, R⁶ is CH₃; R^{W} is CH₂CH₃, the index m is 2, R⁷ and R⁸ - if present - are H, the ring system is represented by Table 68 and R², R³, X and R⁹ have a meaning as defined in a line A1 to A480 in Table A.

Embodiment 69: R¹ is CF₃, R⁶ is CH₃; R^{W} is CH₂CH₃, the index m is 2, R⁷ and R⁸ - if present - are H, the ring system is represented by Table 69 and R², R³, X and R⁹ have a meaning as defined in a line A1 to A480 in Table A.

Embodiment 70: R¹ is CF₃, R⁶ is CH₃; R^{W} is CH₂CH₃, the index m is 2, R⁷ and R⁸ - if present - are H, the ring system is represented by Table 70 and R², R³, X and R⁹ have a meaning as defined in a line A1 to A480 in Table A.

Embodiment 71: R¹ is CF₃, R⁶ is CH₃; R^{W} is CH₂CH₃, the index m is 2, R⁷ and R⁸ - if present - are H, the ring system is represented by Table 71 and R², R³, X and R⁹ have a meaning as defined in a line A1 to A480 in Table A.

Embodiment 72: R¹ is CF₃, R⁶ is CH₃; R^{W} is CH₂CH₃, the index m is 2, R⁷ and R⁸ - if present - are H, the ring system is represented by Table 72 and R², R³, X and R⁹ have a meaning as defined in a line A1 to A480 in Table A.

Embodiment 73: R¹ is CF₃, R⁶ is CH₃; R^{W} is CH₂CH₃, the index m is 2, R⁷ and R⁸ - if present - are H, the ring system is represented by Table 73 and R², R³, X and R⁹ have a meaning as defined in a line A1 to A480 in Table A.

Embodiment 74: R¹ is CF₃, R⁶ is CH₃; R^{W} is CH₂CH₃, the index m is 2, R⁷ and R⁸ - if present - are H, the ring system is represented by Table 74 and R², R³, X and R⁹ have a meaning as defined in a line A1 to A480 in Table A.

Embodiment 75: R¹ is CF₃, R⁶ is CH₃; R^{W} is CH₂CH₃, the index m is 2, R⁷ and R⁸ - if present - are H, the ring system is represented by Table 75 and R², R³, X and R⁹ have a meaning as defined in a line A1 to A480 in Table A.

Embodiment 76: R¹ is CF₃, R⁶ is CH₃; R^{W} is CH₂CH₃, the index m is 2, R⁷ and R⁸ - if present - are H, the ring system is represented by Table 76 and R², R³, X and R⁹ have a meaning as defined in a line A1 to A480 in Table A.

Embodiment 77: R¹ is CF₃, R⁶ is CH₃; R^{W} is CH₂CH₃, the index m is 2, R⁷ and R⁸ - if present - are H, the ring system is represented by Table 77 and R², R³, X and R⁹ have a meaning as defined in a line A1 to A480 in Table A.

Embodiment 78: R¹ is CF₃, R⁶ is CH₃; R^{W} is CH₂CH₃, the index m is 2, R⁷ and R⁸ - if present - are H, the ring system is represented by Table 78 and R², R³, X and R⁹ have a meaning as defined in a line A1 to A480 in Table A.

Embodiment 79: R¹ is CF₃, R⁶ is CH₃; R^{W} is CH₂CH₃, the index m is 2, R⁷ and R⁸ - if present - are H, the ring system is represented by Table 79 and R², R³, X and R⁹ have a meaning as defined in a line A1 to A480 in Table A.

Embodiment 80: R¹ is CF₃, R⁶ is CH₃; R^{W} is CH₂CH₃, the index m is 2, R⁷ and R⁸ - if present - are H, the ring system is represented by Table 80 and R², R³, X and R⁹ have a meaning as defined in a line A1 to A480 in Table A.

Embodiment 81: R¹ is CF₃, R⁶ is CH₃; R^{W} is CH₂CH₃, the index m is 2, R⁷ and R⁸ - if present - are H, the ring system is represented by Table 81 and R², R³, X and R⁹ have a meaning as defined in a line A1 to A480 in Table A.

Embodiment 82: R¹ is CF₃, R⁶ is CH₃; R^{W} is CH₂CH₃, the index m is 2, R⁷ and R⁸ - if present - are H, the ring system is represented by Table 82 and R², R³, X and R⁹ have a meaning as defined in a line A1 to A480 in Table A.

Embodiment 83: R¹ is CF₃, R⁶ is CH₃; R^{W} is CH₂CH₃, the index m is 2, R⁷ and R⁸ - if present - are H, the ring system is represented by Table 83 and R², R³, X and R⁹ have a meaning as defined in a line A1 to A480 in Table A.

Embodiment 84: R¹ is CF₃, R⁶ is CH₃; R^{W} is CH₂CH₃, the index m is 2, R⁷ and R⁸ - if present - are H, the ring system is represented by Table 84 and R², R³, X and R⁹ have a meaning as defined in a line A1 to A480 in Table A.

Embodiment 85: R¹ is CF₃, R⁶ is CH₃; R^{W} is CH₂CH₃, the index m is 2, R⁷ and R⁸ - if present - are H, the ring system is represented by Table 85 and R², R³, X and R⁹ have a meaning as defined in a line A1 to A480 in Table A.

Embodiment 86: R¹ is CF₃, R⁶ is CH₃; R^{W} is CH₂CH₃, the index m is 2, R⁷ and R⁸ - if present - are H, the ring system is represented by Table 86 and R², R³, X and R⁹ have a meaning as defined in a line A1 to A480 in Table A.

Embodiment 87: R¹ is CF₃, R⁶ is CH₃; R^{W} is CH₂CH₃, the index m is 2, R⁷ and R⁸ - if present - are H, the ring system is represented by Table 87 and R², R³, X and R⁹ have a meaning as defined in a line A1 to A480 in Table A.

Embodiment 88: R¹ is CF₃, R⁶ is CH₃; R^{W} is CH₂CH₃, the index m is 2, R⁷ and R⁸ - if present - are H, the ring system is represented by Table 88 and R², R³, X and R⁹ have a meaning as defined in a line A1 to A480 in Table A.

Embodiment 89: R¹ is CF₃, R⁶ is CH₃; R^{W} is CH₂CH₃, the index m is 2, R⁷ and R⁸ - if present - are H, the ring system is represented by Table 89 and R², R³, X and R⁹ have a meaning as defined in a line A1 to A480 in Table A.

Embodiment 90: R¹ is CF₃, R⁶ is CH₃; R^{W} is CH₂CH₃, the index m is 2, R⁷ and R⁸ - if present - are H, the ring system is represented by Table 90 and R², R³, X and R⁹ have a meaning as defined in a line A1 to A480 in Table A.

Embodiment 91: R¹ is CF₃, R⁶ is CH₃; R^{W} is CH₂CH₃, the index m is 2, R⁷ and R⁸ - if present - are H, the ring system is represented by Table 91 and R², R³, X and R⁹ have a meaning as defined in a line A1 to A480 in Table A.

Embodiment 92: R¹ is CF₃, R⁶ is CH₃; R^{W} is CH₂CH₃, the index m is 2, R⁷ and R⁸ - if present - are H, the ring system is represented by Table 92 and R², R³, X and R⁹ have a meaning as defined in a line A1 to A480 in Table A.

Embodiment 93: R¹ is CF₃, R⁶ is CH₃; R^{W} is CH₂CH₃, the index m is 2, R⁷ and R⁸ - if present - are H, the ring system is represented by Table 93 and R², R³, X and R⁹ have a meaning as defined in a line A1 to A480 in Table A.

Embodiment 94: R¹ is CF₃, R⁶ is CH₃; R^{W} is CH₂CH₃, the index m is 2, R⁷ and R⁸ - if present - are H, the ring system is represented by Table 94 and R², R³, X and R⁹ have a meaning as defined in a line A1 to A480 in Table A.

Embodiment 95: R¹ is CF₃, R⁶ is CH₃; R^{W} is CH₂CH₃, the index m is 2, R⁷ and R⁸ - if present - are H, the ring system is represented by Table 95 and R², R³, X and R⁹ have a meaning as defined in a line A1 to A480 in Table A.

Embodiment 96: R¹ is CF₃, R⁶ is CH₃; R^{W} is CH₂CH₃, the index m is 2, R⁷ and R⁸ - if present - are H, the ring system is represented by Table 96 and R², R³, X and R⁹ have a meaning as defined in a line A1 to A480 in Table A.

Embodiment 97: R¹ is CF₃, R⁶ is CH₃; R^{W} is CH₂CH₃, the index m is 2, R⁷ and R⁸ - if present - are H, the ring system is represented by Table 97 and R², R³, X and R⁹ have a meaning as defined in a line A1 to A480 in Table A.

Embodiment 98: R¹ is CF₃, R⁶ is CH₃; R^{W} is CH₂CH₃, the index m is 2, R⁷ and R⁸ - if present - are H, the ring system is represented by Table 98 and R², R³, X and R⁹ have a meaning as defined in a line A1 to A480 in Table A.

Embodiment 99: R¹ is CF₃, R⁶ is CH₃; R^{W} is CH₂CH₃, the index m is 2, R⁷ and R⁸ - if present - are H, the ring system is represented by Table 99 and R², R³, X and R⁹ have a meaning as defined in a line A1 to A480 in Table A.

Embodiment 100: R¹ is CF₃, R⁶ is CH₃; R^{W} is CH₂CH₃, the index m is 2, R⁷ and R⁸ - if present - are H, the ring system is represented by Table 100 and R², R³, X and R⁹ have a meaning as defined in a line A1 to A480 in Table A.

Embodiment 101: R¹ is CF₃, R⁶ is CH₃; R^{W} is CH₂CH₃, the index m is 2, R⁷ and R⁸ - if present - are H, the ring system is represented by Table 101 and R², R³, X and R⁹ have a meaning as defined in a line A1 to A480 in Table A.

Embodiment 102: R¹ is CF₃, R⁶ is CH₃; R^{W} is CH₂CH₃, the index m is 2, R⁷ and R⁸ - if present - are H, the ring system is represented by Table 102 and R², R³, X and R⁹ have a meaning as defined in a line A1 to A480 in Table A.

Embodiment 103: R¹ is CF₃, R⁶ is CH₃; R^{W} is CH₂CH₃, the index m is 2, R⁷ and R⁸ - if present - are H, the ring system is represented by Table 103 and R², R³, X and R⁹ have a meaning as defined in a line A1 to A480 in Table A.

Embodiment 104: R¹ is CF₃, R⁶ is CH₃; R^{W} is CH₂CH₃, the index m is 2, R⁷ and R⁸ - if present - are H, the ring system is represented by Table 104 and R², R³, X and R⁹ have a meaning as defined in a line A1 to A480 in Table A.

Embodiment 105: R¹ is CF₃, R⁶ is CH₃; R^{W} is CH₂CH₃, the index m is 2, R⁷ and R⁸ - if present - are H, the ring system is represented by Table 105 and R², R³, X and R⁹ have a meaning as defined in a line A1 to A480 in Table A.

Embodiment 106: R¹ is CF₃, R⁶ is CH₃; R^{W} is CH₂CH₃, the index m is 2, R⁷ and R⁸ - if present - are H, the ring system is represented by Table 106 and R², R³, X and R⁹ have a meaning as defined in a line A1 to A480 in Table A.

Embodiment 107: R¹ is CF₃, R⁶ is CH₃; R^{W} is CH₂CH₃, the index m is 2, R⁷ and R⁸ - if present - are H, the ring system is represented by Table 107 and R², R³, X and R⁹ have a meaning as defined in a line A1 to A480 in Table A.

Embodiment 108: R¹ is CF₃, R⁶ is CH₃; R^{W} is CH₂CH₃, the index m is 2, R⁷ and R⁸ - if present - are H, the ring system is represented by Table 108 and R², R³, X and R⁹ have a meaning as defined in a line A1 to A480 in Table A.

Embodiment 109: R¹ is CF₃, R⁶ is CH₃; R^{W} is CH₂CH₃, the index m is 2, R⁷ and R⁸- if present - are H, the ring system is represented by Table 109 and R², R³, X and R⁹ have a meaning as defined in a line A1 to A480 in Table A.

Embodiment 110: R¹ is CF₃, R⁶ is CH₃; R^{W} is CH₂CH₃, the index m is 2, R⁷ and R⁸ - if present - are H, the ring system is represented by Table 110 and R², R³, X and R⁹ have a meaning as defined in a line A1 to A480 in Table A.

Embodiment 111: R¹ is CF₃, R⁶ is CH₃; R^{W} is CH₂CH₃, the index m is 2, R⁷ and R⁸ - if present - are H, the ring system is represented by Table 111 and R², R³, X and R⁹ have a meaning as defined in a line A1 to A480 in Table A.

Embodiment 112: R¹ is CF₃, R⁶ is CH₃; R^{W} is CH₂CH₃, the index m is 2, R⁷ and R⁸ - if present - are H, the ring system is represented by Table 112 and R², R³, X and R⁹ have a meaning as defined in a line A1 to A480 in Table A.

Embodiment 113: R¹ is CF₃, R⁶ is CH₃; R^{W} is CH₂CH₃, the index m is 2, R⁷ and R⁸ - if present - are H, the ring system is represented by Table 113 and R², R³, X and R⁹ have a meaning as defined in a line A1 to A480 in Table A.

Embodiment 114: R¹ is CF₃, R⁶ is CH₃; R^{W} is CH₂CH₃, the index m is 2, R⁷ and R⁸ - if present - are H, the ring system is represented by Table 114 and R², R³, X and R⁹ have a meaning as defined in a line A1 to A480 in Table A.

Embodiment 115: R¹ is CF₃, R⁶ is CH₃; R^{W} is CH₂CH₃, the index m is 2, R⁷ and R⁸ - if present - are H, the ring system is represented by Table 115 and R², R³, X and R⁹ have a meaning as defined in a line A1 to A480 in Table A.

Embodiment 116: R¹ is CF₃, R⁶ is CH₃; R^{W} is CH₂CH₃, the index m is 2, R⁷ and R⁸ - if present - are H, the ring system is represented by Table 116 and R², R³, X and R⁹ have a meaning as defined in a line A1 to A480 in Table A.

Embodiment 117: R¹ is CF₃, R⁶ is CH₃; R^{W} is CH₂CH₃, the index m is 2, R⁷ and R⁸ - if present - are H, the ring system is represented by Table 117 and R², R³, X and R⁹ have a meaning as defined in a line A1 to A480 in Table A.

Embodiment 118: R¹ is CF₃, R⁶ is CH₃; R^{W} is CH₂CH₃, the index m is 2, R⁷ and R⁸ - if present - are H, the ring system is represented by Table 118 and R², R³, X and R⁹ have a meaning as defined in a line A1 to A480 in Table A.

Embodiment 119: R¹ is CF₃, R⁶ is CH₃; R^{W} is CH₂CH₃, the index m is 2, R⁷ and R⁸ - if present - are H, the ring system is represented by Table 119 and R², R³, X and R⁹ have a meaning as defined in a line A1 to A480 in Table A.

Embodiment 120: R¹ is CF₃, R⁶ is CH₃; R^{W} is CH₂CH₃, the index m is 2, R⁷ and R⁸ - if present - are H, the ring system is represented by Table 120 and R², R³, X and R⁹ have a meaning as defined in a line A1 to A480 in Table A.

Embodiment 121: R¹ is CF₃, R⁶ is CH₃; R^{W} is CH₂CH₃, the index m is 2, R⁷ and R⁸ - if present - are H, the ring system is represented by Table 121 and R², R³, X and R⁹ have a meaning as defined in a line A1 to A480 in Table A.

Embodiment 122: R¹ is CF₃, R⁶ is CH₃; R^{W} is CH₂CH₃, the index m is 2, R⁷ and R⁸- if present - are H, the ring system is represented by Table 122 and R², R³, X and R⁹ have a meaning as defined in a line A1 to A480 in Table A.

Embodiment 123: R¹ is CF₃, R⁶ is CH₃; R^{W} is CH₂CH₃, the index m is 2, R⁷ and R⁸ - if present - are H, the ring system is represented by Table 123 and R², R³, X and R⁹ have a meaning as defined in a line A1 to A480 in Table A.

Embodiment 124: R¹ is CF₃, R⁶ is CH₃; R^{W} is CH₂CH₃, the index m is 2, R⁷ and R⁸ - if present - are H, the ring system is represented by Table 124 and R², R³, X and R⁹ have a meaning as defined in a line A1 to A480 in Table A.

Embodiment 125: R¹ is CF₃, R⁶ is CH₃; R^{W} is CH₂CH₃, the index m is 2, R⁷ and R⁸ - if present - are H, the ring system is represented by Table 125 and R², R³, X and R⁹ have a meaning as defined in a line A1 to A480 in Table A.

Embodiment 126: R¹ is CF₃, R⁶ is CH₃; R^{W} is CH₂CH₃, the index m is 2, R⁷ and R⁸ - if present - are H, the ring system is represented by Table 126 and R², R³, X and R⁹ have a meaning as defined in a line A1 to A480 in Table A.

Embodiment 127: R¹ is CF₃, R⁶ is CH₃; R^{W} is CH₂CH₃, the index m is 2, R⁷ and R⁸ - if present - are H, the ring system is represented by Table 127 and R², R³, X and R⁹ have a meaning as defined in a line A1 to A480 in Table A.

Embodiment 128: R¹ is CF₃, R⁶ is CH₃; R^{W} is CH₂CH₃, the index m is 2, R⁷ and R⁸ - if present - are H, the ring system is represented by Table 128 and R², R³, X and R⁹ have a meaning as defined in a line A1 to A480 in Table A.

Embodiment 129: R¹ is CF₃, R⁶ is CH₃; R^{W} is CH₂CH₃, the index m is 2, R⁷ and R⁸ - if present - are H, the ring system is represented by Table 129 and R², R³, X and R⁹ have a meaning as defined in a line A1 to A480 in Table A.

Embodiment 130: R¹ is CF₃, R⁶ is CH₃; R^{W} is CH₂CH₃, the index m is 2, R⁷ and R⁸ - if present - are H, the ring system is represented by Table 130 and R², R³, X and R⁹ have a meaning as defined in a line A1 to A480 in Table A.

Embodiment 131: R¹ is CF₃, R⁶ is CH₃; R^{W} is CH₂CH₃, the index m is 2, R⁷ and R⁸ - if present - are H, the ring system is represented by Table 131 and R², R³, X and R⁹ have a meaning as defined in a line A1 to A480 in Table A.

Embodiment 132: R¹ is CF₃, R⁶ is CH₃; R^{W} is CH₂CH₃, the index m is 2, R⁷ and R⁸ - if present - are H, the ring system is represented by Table 132 and R², R³, X and R⁹ have a meaning as defined in a line A1 to A480 in Table A.

Embodiment 133: R¹ is CF₃, R⁶ is CH₃; R^{W} is CH₂CH₃, the index m is 2, R⁷ and R⁸- if present - are H, the ring system is represented by Table 133 and R², R³, X and R⁹ have a meaning as defined in a line A1 to A480 in Table A.

Embodiment 134: R¹ is CF₃, R⁶ is CH₃; R^{W} is CH₂CH₃, the index m is 2, R⁷ and R⁸ - if present - are H, the ring system is represented by Table 134 and R², R³, X and R⁹ have a meaning as defined in a line A1 to A480 in Table A.

Embodiment 135: R¹ is CF₃, R⁶ is CH₃; R^{W} is CH₂CH₃, the index m is 2, R⁷ and R⁸ - if present - are H, the ring system is represented by Table 135 and R², R³, X and R⁹ have a meaning as defined in a line A1 to A480 in Table A.

Embodiment 136: R¹ is CF₃, R⁶ is CH₃; R^{W} is CH₂CH₃, the index m is 2, R⁷ and R⁸ - if present - are H, the ring system is represented by Table 136 and R², R³, X and R⁹ have a meaning as defined in a line A1 to A480 in Table A.

Embodiment 137: R¹ is CF₃, R⁶ is CH₃; R^{W} is CH₂CH₃, the index m is 2, R⁷ and R⁸ - if present - are H, the ring system is represented by Table 137 and R², R³, X and R⁹ have a meaning as defined in a line A1 to A480 in Table A.

Embodiment 138: R¹ is CF₃, R⁶ is CH₃; R^{W} is CH₂CH₃, the index m is 2, R⁷ and R⁸ - if present - are H, the ring system is represented by Table 138 and R², R³, X and R⁹ have a meaning as defined in a line A1 to A480 in Table A.

Embodiment 139: R¹ is CF₃, R⁶ is CH₃; R^{W} is CH₂CH₃, the index m is 2, R⁷ and R⁸ - if present - are H, the ring system is represented by Table 139 and R², R³, X and R⁹ have a meaning as defined in a line A1 to A480 in Table A.

Embodiment 140: R¹ is CF₃, R⁶ is CH₃; R^{W} is CH₂CH₃, the index m is 2, R⁷ and R⁸ - if present - are H, the ring system is represented by Table 140 and R², R³, X and R⁹ have a meaning as defined in a line A1 to A480 in Table A.

Embodiment 141: R¹ is CF₃, R⁶ is CH₃; R^{W} is CH₂CH₃, the index m is 2, R⁷ and R⁸ - if present - are H, the ring system is represented by Table 141 and R², R³, X and R⁹ have a meaning as defined in a line A1 to A480 in Table A.

Embodiment 142: R¹ is CF₃, R⁶ is H; R^{W} is CH₂CH₃, the index m is 2, R⁷ and R⁸ - if present - are H, the ring system is represented by Table 1 and R², R³, X and R⁹ have a meaning as defined in a line A1 to A480 in Table A.

Embodiment 143: R¹ is CF₃, R⁶ is H; R^{W} is CH₂CH₃, the index m is 2, R⁷ and R⁸ - if present - are H, the ring system is represented by Table 2 and R², R³, X and R⁹ have a meaning as defined in a line A1 to A480 in Table A.

Embodiment 144: R¹ is CF₃, R⁶ is H; R^{W} is CH₂CH₃, the index m is 2, R⁷ and R⁸ - if present - are H, the ring system is represented by Table 3 and R², R³, X and R⁹ have a meaning as defined in a line A1 to A480 in Table A.

Embodiment 145: R¹ is CF₃, R⁶ is H; R^{W} is CH₂CH₃, the index m is 2, R⁷ and R⁸ - if present - are H, the ring system is represented by Table 4 and R², R³, X and R⁹ have a meaning as defined in a line A1 to A480 in Table A.

Embodiment 146: R¹ is CF₃, R⁶ is H; R^{W} is CH₂CH₃, the index m is 2, R⁷ and R⁸ - if present - are H, the ring system is represented by Table 5 and R², R³, X and R⁹ have a meaning as defined in a line A1 to A480 in Table A.

Embodiment 147: R¹ is CF₃, R⁶ is H; R^{W} is CH₂CH₃, the index m is 2, R⁷ and R⁸ - if present - are H, the ring system is represented by Table 6 and R², R³, X and R⁹ have a meaning as defined in a line A1 to A480 in Table A.

Embodiment 148: R¹ is CF₃, R⁶ is H; R^{W} is CH₂CH₃, the index m is 2, R⁷ and R⁸ - if present - are H, the ring system is represented by Table 7 and R², R³, X and R⁹ have a meaning as defined in a line A1 to A480 in Table A.

Embodiment 149: R¹ is CF₃, R⁶ is H; R^{W} is CH₂CH₃, the index m is 2, R⁷ and R⁸ - if present - are H, the ring system is represented by Table 8 and R², R³, X and R⁹ have a meaning as defined in a line A1 to A480 in Table A.

Embodiment 150: R¹ is CF₃, R⁶ is H; R^{W} is CH₂CH₃, the index m is 2, R⁷ and R⁸ - if present - are H, the ring system is represented by Table 9 and R², R³, X and R⁹ have a meaning as defined in a line A1 to A480 in Table A.

Embodiment 151: R¹ is CF₃, R⁶ is H; R^{W} is CH₂CH₃, the index m is 2, R⁷ and R⁸ - if present - are H, the ring system is represented by Table 10 and R², R³, X and R⁹ have a meaning as defined in a line A1 to A480 in Table A.

Embodiment 152: R¹ is CF₃, R⁶ is H; R^{W} is CH₂CH₃, the index m is 2, R⁷ and R⁸ - if present - are H, the ring system is represented by Table 11 and R², R³, X and R⁹ have a meaning as defined in a line A1 to A480 in Table A.

Embodiment 153: R¹ is CF₃, R⁶ is H; R^{W} is CH₂CH₃, the index m is 2, R⁷ and R⁸ - if present - are H, the ring system is represented by Table 12 and R², R³, X and R⁹ have a meaning as defined in a line A1 to A480 in Table A.

Embodiment 154: R¹ is CF₃, R⁶ is H; R^{W} is CH₂CH₃, the index m is 2, R⁷ and R⁸ - if present - are H, the ring system is represented by Table 13 and R², R³, X and R⁹ have a meaning as defined in a line A1 to A480 in Table A.

Embodiment 155: R¹ is CF₃, R⁶ is H; R^{W} is CH₂CH₃, the index m is 2, R⁷ and R⁸ - if present - are H, the ring system is represented by Table 14 and R², R³, X and R⁹ have a meaning as defined in a line A1 to A480 in Table A.

Embodiment 156: R¹ is CF₃, R⁶ is H; R^{W} is CH₂CH₃, the index m is 2, R⁷ and R⁸ - if present - are H, the ring system is represented by Table 15 and R², R³, X and R⁹ have a meaning as defined in a line A1 to A480 in Table A.

Embodiment 157: R¹ is CF₃, R⁶ is H; R^{W} is CH₂CH₃, the index m is 2, R⁷ and R⁸ - if present - are H, the ring system is represented by Table 16 and R², R³, X and R⁹ have a meaning as defined in a line A1 to A480 in Table A.

Embodiment 158: R¹ is CF₃, R⁶ is H; R^{W} is CH₂CH₃, the index m is 2, R⁷ and R⁸ - if present - are H, the ring system is represented by Table 17 and R², R³, X and R⁹ have a meaning as defined in a line A1 to A480 in Table A.

Embodiment 159: R¹ is CF₃, R⁶ is H; R^{W} is CH₂CH₃, the index m is 2, R⁷ and R⁸ - if present - are H, the ring system is represented by Table 18 and R², R³, X and R⁹ have a meaning as defined in a line A1 to A480 in Table A.

Embodiment 160: R¹ is CF₃, R⁶ is H; R^{W} is CH₂CH₃, the index m is 2, R⁷ and R⁸ - if present - are H, the ring system is represented by Table 19 and R², R³, X and R⁹ have a meaning as defined in a line A1 to A480 in Table A.

Embodiment 161: R¹ is CF₃, R⁶ is H; R^{W} is CH₂CH₃, the index m is 2, R⁷ and R⁸ - if present - are H, the ring system is represented by Table 20 and R², R³, X and R⁹ have a meaning as defined in a line A1 to A480 in Table A.

Embodiment 162: R¹ is CF₃, R⁶ is H; R^{W} is CH₂CH₃, the index m is 2, R⁷ and R⁸ - if present - are H, the ring system is represented by Table 21 and R², R³, X and R⁹ have a meaning as defined in a line A1 to A480 in Table A.

Embodiment 163: R¹ is CF₃, R⁶ is H; R^{W} is CH₂CH₃, the index m is 2, R⁷ and R⁸ - if present - are H, the ring system is represented by Table 22 and R², R³, X and R⁹ have a meaning as defined in a line A1 to A480 in Table A.

Embodiment 164: R¹ is CF₃, R⁶ is H; R^{W} is CH₂CH₃, the index m is 2, R⁷ and R⁸ - if present - are H, the ring system is represented by Table 23 and R², R³, X and R⁹ have a meaning as defined in a line A1 to A480 in Table A.

Embodiment 165: R¹ is CF₃, R⁶ is H; R^{W} is CH₂CH₃, the index m is 2, R⁷ and R⁸ - if present - are H, the ring system is represented by Table 24 and R², R³, X and R⁹ have a meaning as defined in a line A1 to A480 in Table A.

Embodiment 166: R¹ is CF₃, R⁶ is H; R^{W} is CH₂CH₃, the index m is 2, R⁷ and R⁸ - if present - are H, the ring system is represented by Table 25 and R², R³, X and R⁹ have a meaning as defined in a line A1 to A480 in Table A.

Embodiment 167: R¹ is CF₃, R⁶ is H; R^{W} is CH₂CH₃, the index m is 2, R⁷ and R⁸ - if present - are H, the ring system is represented by Table 26 and R², R³, X and R⁹ have a meaning as defined in a line A1 to A480 in Table A.

Embodiment 168: R¹ is CF₃, R⁶ is H; R^{W} is CH₂CH₃, the index m is 2, R⁷ and R⁸ - if present - are H, the ring system is represented by Table 27 and R², R³, X and R⁹ have a meaning as defined in a line A1 to A480 in Table A.

Embodiment 169: R¹ is CF₃, R⁶ is H; R^{W} is CH₂CH₃, the index m is 2, R⁷ and R⁸ - if present - are H, the ring system is represented by Table 28 and R², R³, X and R⁹ have a meaning as defined in a line A1 to A480 in Table A.

Embodiment 170: R¹ is CF₃, R⁶ is H; R^{W} is CH₂CH₃, the index m is 2, R⁷ and R⁸ - if present - are H, the ring system is represented by Table 29 and R², R³, X and R⁹ have a meaning as defined in a line A1 to A480 in Table A.

Embodiment 171: R¹ is CF₃, R⁶ is H; R^{W} is CH₂CH₃, the index m is 2, R⁷ and R⁸ - if present - are H, the ring system is represented by Table 30 and R², R³, X and R⁹ have a meaning as defined in a line A1 to A480 in Table A.

Embodiment 172: R¹ is CF₃, R⁶ is H; R^{W} is CH₂CH₃, the index m is 2, R⁷ and R⁸ - if present - are H, the ring system is represented by Table 31 and R², R³, X and R⁹ have a meaning as defined in a line A1 to A480 in Table A.

Embodiment 173: R¹ is CF₃, R⁶ is H; R^{W} is CH₂CH₃, the index m is 2, R⁷ and R⁸ - if present - are H, the ring system is represented by Table 32 and R², R³, X and R⁹ have a meaning as defined in a line A1 to A480 in Table A.

Embodiment 174: R¹ is CF₃, R⁶ is H; R^{W} is CH₂CH₃, the index m is 2, R⁷ and R⁸ - if present - are H, the ring system is represented by Table 33 and R², R³, X and R⁹ have a meaning as defined in a line A1 to A480 in Table A.

Embodiment 175: R¹ is CF₃, R⁶ is H; R^{W} is CH₂CH₃, the index m is 2, R⁷ and R⁸ - if present - are H, the ring system is represented by Table 34 and R², R³, X and R⁹ have a meaning as defined in a line A1 to A480 in Table A.

Embodiment 176: R¹ is CF₃, R⁶ is H; R^{W} is CH₂CH₃, the index m is 2, R⁷ and R⁸ - if present - are H, the ring system is represented by Table 35 and R², R³, X and R⁹ have a meaning as defined in a line A1 to A480 in Table A.

Embodiment 177: R¹ is CF₃, R⁶ is H; R^{W} is CH₂CH₃, the index m is 2, R⁷ and R⁸ - if present - are H, the ring system is represented by Table 36 and R², R³, X and R⁹ have a meaning as defined in a line A1 to A480 in Table A.

Embodiment 178: R¹ is CF₃, R⁶ is H; R^{W} is CH₂CH₃, the index m is 2, R⁷ and R⁸ - if present - are H, the ring system is represented by Table 37 and R², R³, X and R⁹ have a meaning as defined in a line A1 to A480 in Table A.

Embodiment 179: R¹ is CF₃, R⁶ is H; R^{W} is CH₂CH₃, the index m is 2, R⁷ and R⁸ - if present - are H, the ring system is represented by Table 38 and R², R³, X and R⁹ have a meaning as defined in a line A1 to A480 in Table A.

Embodiment 180: R¹ is CF₃, R⁶ is H; R^{W} is CH₂CH₃, the index m is 2, R⁷ and R⁸ - if present - are H, the ring system is represented by Table 39 and R², R³, X and R⁹ have a meaning as defined in a line A1 to A480 in Table A.

Embodiment 181: R¹ is CF₃, R⁶ is H; R^{W} is CH₂CH₃, the index m is 2, R⁷ and R⁸ - if present - are H, the ring system is represented by Table 40 and R², R³, X and R⁹ have a meaning as defined in a line A1 to A480 in Table A.

Embodiment 182: R¹ is CF₃, R⁶ is H; R^{W} is CH₂CH₃, the index m is 2, R⁷ and R⁸ - if present - are H, the ring system is represented by Table 41 and R², R³, X and R⁹ have a meaning as defined in a line A1 to A480 in Table A.

Embodiment 183: R¹ is CF₃, R⁶ is H; R^{W} is CH₂CH₃, the index m is 2, R⁷ and R⁸ - if present - are H, the ring system is represented by Table 42 and R², R³, X and R⁹ have a meaning as defined in a line A1 to A480 in Table A.

Embodiment 184: R¹ is CF₃, R⁶ is H; R^{W} is CH₂CH₃, the index m is 2, R⁷ and R⁸ - if present - are H, the ring system is represented by Table 43 and R², R³, X and R⁹ have a meaning as defined in a line A1 to A480 in Table A.

Embodiment 185: R¹ is CF₃, R⁶ is H; R^{W} is CH₂CH₃, the index m is 2, R⁷ and R⁸ - if present - are H, the ring system is represented by Table 44 and R², R³, X and R⁹ have a meaning as defined in a line A1 to A480 in Table A.

Embodiment 186: R¹ is CF₃, R⁶ is H; R^{W} is CH₂CH₃, the index m is 2, R⁷ and R⁸ - if present - are H, the ring system is represented by Table 45 and R², R³, X and R⁹ have a meaning as defined in a line A1 to A480 in Table A.

Embodiment 187: R¹ is CF₃, R⁶ is H; R^{W} is CH₂CH₃, the index m is 2, R⁷ and R⁸ - if present - are H, the ring system is represented by Table 46 and R², R³, X and R⁹ have a meaning as defined in a line A1 to A480 in Table A.

Embodiment 188: R¹ is CF₃, R⁶ is H; R^{W} is CH₂CH₃, the index m is 2, R⁷ and R⁸ - if present - are H, the ring system is represented by Table 47 and R², R³, X and R⁹ have a meaning as defined in a line A1 to A480 in Table A.

Embodiment 189: R¹ is CF₃, R⁶ is H; R^{W} is CH₂CH₃, the index m is 2, R⁷ and R⁸ - if present - are H, the ring system is represented by Table 48 and R², R³, X and R⁹ have a meaning as defined in a line A1 to A480 in Table A.

Embodiment 190: R¹ is CF₃, R⁶ is H; R^{W} is CH₂CH₃, the index m is 2, R⁷ and R⁸ - if present - are H, the ring system is represented by Table 49 and R², R³, X and R⁹ have a meaning as defined in a line A1 to A480 in Table A.

Embodiment 191: R¹ is CF₃, R⁶ is H; R^{W} is CH₂CH₃, the index m is 2, R⁷ and R⁸ - if present - are H, the ring system is represented by Table 50 and R², R³, X and R⁹ have a meaning as defined in a line A1 to A480 in Table A.

Embodiment 192: R¹ is CF₃, R⁶ is H; R^{W} is CH₂CH₃, the index m is 2, R⁷ and R⁸ - if present - are H, the ring system is represented by Table 51 and R², R³, X and R⁹ have a meaning as defined in a line A1 to A480 in Table A.

Embodiment 193: R¹ is CF₃, R⁶ is H; R^{W} is CH₂CH₃, the index m is 2, R⁷ and R⁸ - if present - are H, the ring system is represented by Table 52 and R², R³, X and R⁹ have a meaning as defined in a line A1 to A480 in Table A.

Embodiment 194: R¹ is CF₃, R⁶ is H; R^{W} is CH₂CH₃, the index m is 2, R⁷ and R⁸ - if present - are H, the ring system is represented by Table 53 and R², R³, X and R⁹ have a meaning as defined in a line A1 to A480 in Table A.

Embodiment 195: R¹ is CF₃, R⁶ is H; R^{W} is CH₂CH₃, the index m is 2, R⁷ and R⁸ - if present - are H, the ring system is represented by Table 54 and R², R³, X and R⁹ have a meaning as defined in a line A1 to A480 in Table A.

Embodiment 196: R¹ is CF₃, R⁶ is H; R^{W} is CH₂CH₃, the index m is 2, R⁷ and R⁸ - if present - are H, the ring system is represented by Table 55 and R², R³, X and R⁹ have a meaning as defined in a line A1 to A480 in Table A.

Embodiment 197: R¹ is CF₃, R⁶ is H; R^{W} is CH₂CH₃, the index m is 2, R⁷ and R⁸ - if present - are H, the ring system is represented by Table 56 and R², R³, X and R⁹ have a meaning as defined in a line A1 to A480 in Table A.

Embodiment 198: R¹ is CF₃, R⁶ is H; R^{W} is CH₂CH₃, the index m is 2, R⁷ and R⁸ - if present - are H, the ring system is represented by Table 57 and R², R³, X and R⁹ have a meaning as defined in a line A1 to A480 in Table A.

Embodiment 199: R¹ is CF₃, R⁶ is H; R^{W} is CH₂CH₃, the index m is 2, R⁷ and R⁸ - if present - are H, the ring system is represented by Table 58 and R², R³, X and R⁹ have a meaning as defined in a line A1 to A480 in Table A.

Embodiment 200: R¹ is CF₃, R⁶ is H; R^{W} is CH₂CH₃, the index m is 2, R⁷ and R⁸ - if present - are H, the ring system is represented by Table 59 and R², R³, X and R⁹ have a meaning as defined in a line A1 to A480 in Table A.

Embodiment 201: R¹ is CF₃, R⁶ is H; R^{W} is CH₂CH₃, the index m is 2, R⁷ and R⁸ - if present - are H, the ring system is represented by Table 60 and R², R³, X and R⁹ have a meaning as defined in a line A1 to A480 in Table A.

Embodiment 202: R¹ is CF₃, R⁶ is H; R^{W} is CH₂CH₃, the index m is 2, R⁷ and R⁸ - if present - are H, the ring system is represented by Table 61 and R², R³, X and R⁹ have a meaning as defined in a line A1 to A480 in Table A.

Embodiment 203: R¹ is CF₃, R⁶ is H; R^{W} is CH₂CH₃, the index m is 2, R⁷ and R⁸ - if present - are H, the ring system is represented by Table 62 and R², R³, X and R⁹ have a meaning as defined in a line A1 to A480 in Table A.

Embodiment 204: R¹ is CF₃, R⁶ is H; R^{W} is CH₂CH₃, the index m is 2, R⁷ and R⁸ - if present - are H, the ring system is represented by Table 63 and R², R³, X and R⁹ have a meaning as defined in a line A1 to A480 in Table A.

Embodiment 205: R¹ is CF₃, R⁶ is H; R^{W} is CH₂CH₃, the index m is 2, R⁷ and R⁸ - if present - are H, the ring system is represented by Table 64 and R², R³, X and R⁹ have a meaning as defined in a line A1 to A480 in Table A.

Embodiment 206: R¹ is CF₃, R⁶ is H; R^{W} is CH₂CH₃, the index m is 2, R⁷ and R⁸ - if present - are H, the ring system is represented by Table 65 and R², R³, X and R⁹ have a meaning as defined in a line A1 to A480 in Table A.

Embodiment 207: R¹ is CF₃, R⁶ is H; R^{W} is CH₂CH₃, the index m is 2, R⁷ and R⁸ - if present - are H, the ring system is represented by Table 66 and R², R³, X and R⁹ have a meaning as defined in a line A1 to A480 in Table A.

Embodiment 208: R¹ is CF₃, R⁶ is H; R^{W} is CH₂CH₃, the index m is 2, R⁷ and R⁸ - if present - are H, the ring system is represented by Table 67 and R², R³, X and R⁹ have a meaning as defined in a line A1 to A480 in Table A.

Embodiment 209: R¹ is CF₃, R⁶ is H; R^{W} is CH₂CH₃, the index m is 2, R⁷ and R⁸ - if present - are H, the ring system is represented by Table 68 and R², R³, X and R⁹ have a meaning as defined in a line A1 to A480 in Table A.

Embodiment 210: R¹ is CF₃, R⁶ is H; R^{W} is CH₂CH₃, the index m is 2, R⁷ and R⁸ - if present - are H, the ring system is represented by Table 69 and R², R³, X and R⁹ have a meaning as defined in a line A1 to A480 in Table A.

Embodiment 211: R¹ is CF₃, R⁶ is H; R^{W} is CH₂CH₃, the index m is 2, R⁷ and R⁸ - if present - are H, the ring system is represented by Table 70 and R², R³, X and R⁹ have a meaning as defined in a line A1 to A480 in Table A.

Embodiment 212: R¹ is CF₃, R⁶ is H; R^{W} is CH₂CH₃, the index m is 2, R⁷ and R⁸ - if present - are H, the ring system is represented by Table 71 and R², R³, X and R⁹ have a meaning as defined in a line A1 to A480 in Table A.

Embodiment 213: R¹ is CF₃, R⁶ is H; R^{W} is CH₂CH₃, the index m is 2, R⁷ and R⁸ - if present - are H, the ring system is represented by Table 72 and R², R³, X and R⁹ have a meaning as defined in a line A1 to A480 in Table A.

Embodiment 214: R¹ is CF₃, R⁶ is H; R^{W} is CH₂CH₃, the index m is 2, R⁷ and R⁸ - if present - are H, the ring system is represented by Table 73 and R², R³, X and R⁹ have a meaning as defined in a line A1 to A480 in Table A.

Embodiment 215: R¹ is CF₃, R⁶ is H; R^{W} is CH₂CH₃, the index m is 2, R⁷ and R⁸ - if present - are H, the ring system is represented by Table 74 and R², R³, X and R⁹ have a meaning as defined in a line A1 to A480 in Table A.

Embodiment 216: R¹ is CF₃, R⁶ is H; R^{W} is CH₂CH₃, the index m is 2, R⁷ and R⁸ - if present - are H, the ring system is represented by Table 75 and R², R³, X and R⁹ have a meaning as defined in a line A1 to A480 in Table A.

Embodiment 217: R¹ is CF₃, R⁶ is H; R^{W} is CH₂CH₃, the index m is 2, R⁷ and R⁸ - if present - are H, the ring system is represented by Table 76 and R², R³, X and R⁹ have a meaning as defined in a line A1 to A480 in Table A.

Embodiment 218: R¹ is CF₃, R⁶ is H; R^{W} is CH₂CH₃, the index m is 2, R⁷ and R⁸ - if present - are H, the ring system is represented by Table 77 and R², R³, X and R⁹ have a meaning as defined in a line A1 to A480 in Table A.

Embodiment 219: R¹ is CF₃, R⁶ is H; R^{W} is CH₂CH₃, the index m is 2, R⁷ and R⁸ - if present - are H, the ring system is represented by Table 78 and R², R³, X and R⁹ have a meaning as defined in a line A1 to A480 in Table A.

Embodiment 220: R¹ is CF₃, R⁶ is H; R^{W} is CH₂CH₃, the index m is 2, R⁷ and R⁸ - if present - are H, the ring system is represented by Table 79 and R², R³, X and R⁹ have a meaning as defined in a line A1 to A480 in Table A.

Embodiment 221: R¹ is CF₃, R⁶ is H; R^{W} is CH₂CH₃, the index m is 2, R⁷ and R⁸ - if present - are H, the ring system is represented by Table 80 and R², R³, X and R⁹ have a meaning as defined in a line A1 to A480 in Table A.

Embodiment 222: R¹ is CF₃, R⁶ is H; R^{W} is CH₂CH₃, the index m is 2, R⁷ and R⁸ - if present - are H, the ring system is represented by Table 81 and R², R³, X and R⁹ have a meaning as defined in a line A1 to A480 in Table A.

Embodiment 223: R¹ is CF₃, R⁶ is H; R^{W} is CH₂CH₃, the index m is 2, R⁷ and R⁸ - if present - are H, the ring system is represented by Table 82 and R², R³, X and R⁹ have a meaning as defined in a line A1 to A480 in Table A.

Embodiment 224: R¹ is CF₃, R⁶ is H; R^{W} is CH₂CH₃, the index m is 2, R⁷ and R⁸ - if present - are H, the ring system is represented by Table 83 and R², R³, X and R⁹ have a meaning as defined in a line A1 to A480 in Table A.

Embodiment 225: R¹ is CF₃, R⁶ is H; R^{W} is CH₂CH₃, the index m is 2, R⁷ and R⁸ - if present - are H, the ring system is represented by Table 84 and R², R³, X and R⁹ have a meaning as defined in a line A1 to A480 in Table A.

Embodiment 226: R¹ is CF₃, R⁶ is H; R^{W} is CH₂CH₃, the index m is 2, R⁷ and R⁸ - if present - are H, the ring system is represented by Table 85 and R², R³, X and R⁹ have a meaning as defined in a line A1 to A480 in Table A.

Embodiment 227: R¹ is CF₃, R⁶ is H; R^{W} is CH₂CH₃, the index m is 2, R⁷ and R⁸ - if present - are H, the ring system is represented by Table 86 and R², R³, X and R⁹ have a meaning as defined in a line A1 to A480 in Table A.

Embodiment 228: R¹ is CF₃, R⁶ is H; R^{W} is CH₂CH₃, the index m is 2, R⁷ and R⁸ - if present - are H, the ring system is represented by Table 87 and R², R³, X and R⁹ have a meaning as defined in a line A1 to A480 in Table A.

Embodiment 229: R¹ is CF₃, R⁶ is H; R^{W} is CH₂CH₃, the index m is 2, R⁷ and R⁸ - if present - are H, the ring system is represented by Table 88 and R², R³, X and R⁹ have a meaning as defined in a line A1 to A480 in Table A.

Embodiment 230: R¹ is CF₃, R⁶ is H; R^{W} is CH₂CH₃, the index m is 2, R⁷ and R⁸ - if present - are H, the ring system is represented by Table 89 and R², R³, X and R⁹ have a meaning as defined in a line A1 to A480 in Table A.

Embodiment 231: R¹ is CF₃, R⁶ is H; R^{W} is CH₂CH₃, the index m is 2, R⁷ and R⁸ - if present - are H, the ring system is represented by Table 90 and R², R³, X and R⁹ have a meaning as defined in a line A1 to A480 in Table A.

Embodiment 232: R¹ is CF₃, R⁶ is H; R^{W} is CH₂CH₃, the index m is 2, R⁷ and R⁸ - if present - are H, the ring system is represented by Table 91 and R², R³, X and R⁹ have a meaning as defined in a line A1 to A480 in Table A.

Embodiment 233: R¹ is CF₃, R⁶ is H; R^{W} is CH₂CH₃, the index m is 2, R⁷ and R⁸ - if present - are H, the ring system is represented by Table 92 and R², R³, X and R⁹ have a meaning as defined in a line A1 to A480 in Table A.

Embodiment 234: R¹ is CF₃, R⁶ is H; R^{W} is CH₂CH₃, the index m is 2, R⁷ and R⁸ - if present - are H, the ring system is represented by Table 93 and R², R³, X and R⁹ have a meaning as defined in a line A1 to A480 in Table A.

Embodiment 235: R¹ is CF₃, R⁶ is H; R^{W} is CH₂CH₃, the index m is 2, R⁷ and R⁸ - if present - are H, the ring system is represented by Table 94 and R², R³, X and R⁹ have a meaning as defined in a line A1 to A480 in Table A.

Embodiment 236: R¹ is CF₃, R⁶ is H; R^{W} is CH₂CH₃, the index m is 2, R⁷ and R⁸ - if present - are H, the ring system is represented by Table 95 and R², R³, X and R⁹ have a meaning as defined in a line A1 to A480 in Table A.

Embodiment 237: R¹ is CF₃, R⁶ is H; R^{W} is CH₂CH₃, the index m is 2, R⁷ and R⁸ - if present - are H, the ring system is represented by Table 96 and R², R³, X and R⁹ have a meaning as defined in a line A1 to A480 in Table A.

Embodiment 238: R¹ is CF₃, R⁶ is H; R^{W} is CH₂CH₃, the index m is 2, R⁷ and R⁸ - if present - are H, the ring system is represented by Table 97 and R², R³, X and R⁹ have a meaning as defined in a line A1 to A480 in Table A.

Embodiment 239: R¹ is CF₃, R⁶ is H; R^{W} is CH₂CH₃, the index m is 2, R⁷ and R⁸ - if present - are H, the ring system is represented by Table 98 and R², R³, X and R⁹ have a meaning as defined in a line A1 to A480 in Table A.

Embodiment 240: R¹ is CF₃, R⁶ is H; R^{W} is CH₂CH₃, the index m is 2, R⁷ and R⁸ - if present - are H, the ring system is represented by Table 99 and R², R³, X and R⁹ have a meaning as defined in a line A1 to A480 in Table A.

Embodiment 241: R¹ is CF₃, R⁶ is H; R^{W} is CH₂CH₃, the index m is 2, R⁷ and R⁸ - if present - are H, the ring system is represented by Table 100 and R², R³, X and R⁹ have a meaning as defined in a line A1 to A480 in Table A.

Embodiment 242: R¹ is CF₃, R⁶ is H; R^{W} is CH₂CH₃, the index m is 2, R⁷ and R⁸ - if present - are H, the ring system is represented by Table 101 and R², R³, X and R⁹ have a meaning as defined in a line A1 to A480 in Table A.

Embodiment 243: R¹ is CF₃, R⁶ is H; R^{W} is CH₂CH₃, the index m is 2, R⁷ and R⁸ - if present - are H, the ring system is represented by Table 102 and R², R³, X and R⁹ have a meaning as defined in a line A1 to A480 in Table A.

Embodiment 244: R¹ is CF₃, R⁶ is H; R^{W} is CH₂CH₃, the index m is 2, R⁷ and R⁸ - if present - are H, the ring system is represented by Table 103 and R², R³, X and R⁹ have a meaning as defined in a line A1 to A480 in Table A.

Embodiment 245: R¹ is CF₃, R⁶ is H; R^{W} is CH₂CH₃, the index m is 2, R⁷ and R⁸ - if present - are H, the ring system is represented by Table 104 and R², R³, X and R⁹ have a meaning as defined in a line A1 to A480 in Table A.

Embodiment 246: R¹ is CF₃, R⁶ is H; R^{W} is CH₂CH₃, the index m is 2, R⁷ and R⁸ - if present - are H, the ring system is represented by Table 105 and R², R³, X and R⁹ have a meaning as defined in a line A1 to A480 in Table A.

Embodiment 247: R¹ is CF₃, R⁶ is H; R^{W} is CH₂CH₃, the index m is 2, R⁷ and R⁸ - if present - are H, the ring system is represented by Table 106 and R², R³, X and R⁹ have a meaning as defined in a line A1 to A480 in Table A.

Embodiment 248: R¹ is CF₃, R⁶ is H; R^{W} is CH₂CH₃, the index m is 2, R⁷ and R⁸ - if present - are H, the ring system is represented by Table 107 and R², R³, X and R⁹ have a meaning as defined in a line A1 to A480 in Table A.

Embodiment 249: R¹ is CF₃, R⁶ is H; R^{W} is CH₂CH₃, the index m is 2, R⁷ and R⁸ - if present - are H, the ring system is represented by Table 108 and R², R³, X and R⁹ have a meaning as defined in a line A1 to A480 in Table A.

Embodiment 250: R¹ is CF₃, R⁶ is H; R^{W} is CH₂CH₃, the index m is 2, R⁷ and R⁸- if present - are H, the ring system is represented by Table 109 and R², R³, X and R⁹ have a meaning as defined in a line A1 to A480 in Table A.

Embodiment 251: R¹ is CF₃, R⁶ is H; R^{W} is CH₂CH₃, the index m is 2, R⁷ and R⁸ - if present - are H, the ring system is represented by Table 110 and R², R³, X and R⁹ have a meaning as defined in a line A1 to A480 in Table A.

Embodiment 252: R¹ is CF₃, R⁶ is H; R^{W} is CH₂CH₃, the index m is 2, R⁷ and R⁸- if present - are H, the ring system is represented by Table 111 and R², R³, X and R⁹ have a meaning as defined in a line A1 to A480 in Table A.

Embodiment 253: R¹ is CF₃, R⁶ is H; R^{W} is CH₂CH₃, the index m is 2, R⁷ and R⁸- if present - are H, the ring system is represented by Table 112 and R², R³, X and R⁹ have a meaning as defined in a line A1 to A480 in Table A.

Embodiment 254: R¹ is CF₃, R⁶ is H; R^{W} is CH₂CH₃, the index m is 2, R⁷ and R⁸ - if present - are H, the ring system is represented by Table 113 and R², R³, X and R⁹ have a meaning as defined in a line A1 to A480 in Table A.

Embodiment 255: R¹ is CF₃, R⁶ is H; R^{W} is CH₂CH₃, the index m is 2, R⁷ and R⁸- if present - are H, the ring system is represented by Table 114 and R², R³, X and R⁹ have a meaning as defined in a line A1 to A480 in Table A.

Embodiment 256: R¹ is CF₃, R⁶ is H; R^{W} is CH₂CH₃, the index m is 2, R⁷ and R⁸- if present - are H, the ring system is represented by Table 115 and R², R³, X and R⁹ have a meaning as defined in a line A1 to A480 in Table A.

Embodiment 257: R¹ is CF₃, R⁶ is H; R^{W} is CH₂CH₃, the index m is 2, R⁷ and R⁸- if present - are H, the ring system is represented by Table 116 and R², R³, X and R⁹ have a meaning as defined in a line A1 to A480 in Table A.

Embodiment 258: R¹ is CF₃, R⁶ is H; R^{W} is CH₂CH₃, the index m is 2, R⁷ and R⁸- if present - are H, the ring system is represented by Table 117 and R², R³, X and R⁹ have a meaning as defined in a line A1 to A480 in Table A.

Embodiment 259: R¹ is CF₃, R⁶ is H; R^{W} is CH₂CH₃, the index m is 2, R⁷ and R⁸- if present - are H, the ring system is represented by Table 118 and R², R³, X and R⁹ have a meaning as defined in a line A1 to A480 in Table A.

Embodiment 260: R¹ is CF₃, R⁶ is H; R^{W} is CH₂CH₃, the index m is 2, R⁷ and R⁸- if present - are H, the ring system is represented by Table 119 and R², R³, X and R⁹ have a meaning as defined in a line A1 to A480 in Table A.

Embodiment 261: R¹ is CF₃, R⁶ is H; R^{W} is CH₂CH₃, the index m is 2, R⁷ and R⁸ - if present - are H, the ring system is represented by Table 120 and R², R³, X and R⁹ have a meaning as defined in a line A1 to A480 in Table A.

Embodiment 262: R¹ is CF₃, R⁶ is H; R^{W} is CH₂CH₃, the index m is 2, R⁷ and R⁸- if present - are H, the ring system is represented by Table 121 and R², R³, X and R⁹ have a meaning as defined in a line A1 to A480 in Table A.

Embodiment 263: R¹ is CF₃, R⁶ is H; R^{W} is CH₂CH₃, the index m is 2, R⁷ and R⁸- if present - are H, the ring system is represented by Table 122 and R², R³, X and R⁹ have a meaning as defined in a line A1 to A480 in Table A.

Embodiment 264: R¹ is CF₃, R⁶ is H; R^{W} is CH₂CH₃, the index m is 2, R⁷ and R⁸- if present - are H, the ring system is represented by Table 123 and R², R³, X and R⁹ have a meaning as defined in a line A1 to A480 in Table A.

Embodiment 265: R¹ is CF₃, R⁶ is H; R^{W} is CH₂CH₃, the index m is 2, R⁷ and R⁸- if present - are H, the ring system is represented by Table 124 and R², R³, X and R⁹ have a meaning as defined in a line A1 to A480 in Table A.

Embodiment 266: R¹ is CF₃, R⁶ is H; R^{W} is CH₂CH₃, the index m is 2, R⁷ and R⁸- if present - are H, the ring system is represented by Table 125 and R², R³, X and R⁹ have a meaning as defined in a line A1 to A480 in Table A.

Embodiment 267: R¹ is CF₃, R⁶ is H; R^{W} is CH₂CH₃, the index m is 2, R⁷ and R⁸ - if present - are H, the ring system is represented by Table 126 and R², R³, X and R⁹ have a meaning as defined in a line A1 to A480 in Table A.

Embodiment 268: R¹ is CF₃, R⁶ is H; R^{W} is CH₂CH₃, the index m is 2, R⁷ and R⁸- if present - are H, the ring system is represented by Table 127 and R², R³, X and R⁹ have a meaning as defined in a line A1 to A480 in Table A.

Embodiment 269: R¹ is CF₃, R⁶ is H; R^{W} is CH₂CH₃, the index m is 2, R⁷ and R⁸- if present - are H, the ring system is represented by Table 128 and R², R³, X and R⁹ have a meaning as defined in a line A1 to A480 in Table A.

Embodiment 270: R¹ is CF₃, R⁶ is H; R^{W} is CH₂CH₃, the index m is 2, R⁷ and R⁸- if present - are H, the ring system is represented by Table 129 and R², R³, X and R⁹ have a meaning as defined in a line A1 to A480 in Table A.

Embodiment 271: R¹ is CF₃, R⁶ is H; R^{W} is CH₂CH₃, the index m is 2, R⁷ and R⁸- if present - are H, the ring system is represented by Table 130 and R², R³, X and R⁹ have a meaning as defined in a line A1 to A480 in Table A.

Embodiment 272: R¹ is CF₃, R⁶ is H; R^{W} is CH₂CH₃, the index m is 2, R⁷ and R⁸- if present - are H, the ring system is represented by Table 131 and R², R³, X and R⁹ have a meaning as defined in a line A1 to A480 in Table A.

Embodiment 273: R¹ is CF₃, R⁶ is H; R^{W} is CH₂CH₃, the index m is 2, R⁷ and R⁸- if present - are H, the ring system is represented by Table 132 and R², R³, X and R⁹ have a meaning as defined in a line A1 to A480 in Table A.

Embodiment 274: R¹ is CF₃, R⁶ is H; R^{W} is CH₂CH₃, the index m is 2, R⁷ and R⁸- if present - are H, the ring system is represented by Table 133 and R², R³, X and R⁹ have a meaning as defined in a line A1 to A480 in Table A.

Embodiment 275: R¹ is CF₃, R⁶ is H; R^{W} is CH₂CH₃, the index m is 2, R⁷ and R⁸- if present - are H, the ring system is represented by Table 134 and R², R³, X and R⁹ have a meaning as defined in a line A1 to A480 in Table A.

Embodiment 276: R¹ is CF₃, R⁶ is H; R^{W} is CH₂CH₃, the index m is 2, R⁷ and R⁸- if present - are H, the ring system is represented by Table 135 and R², R³, X and R⁹ have a meaning as defined in a line A1 to A480 in Table A.

Embodiment 277: R¹ is CF₃, R⁶ is H; R^{W} is CH₂CH₃, the index m is 2, R⁷ and R⁸- if present - are H, the ring system is represented by Table 136 and R², R³, X and R⁹ have a meaning as defined in a line A1 to A480 in Table A.

Embodiment 278: R¹ is CF₃, R⁶ is H; R^{W} is CH₂CH₃, the index m is 2, R⁷ and R⁸- if present - are H, the ring system is represented by Table 137 and R², R³, X and R⁹ have a meaning as defined in a line A1 to A480 in Table A.

Embodiment 279: R¹ is CF₃, R⁶ is H; R^{W} is CH₂CH₃, the index m is 2, R⁷ and R⁸- if present - are H, the ring system is represented by Table 138 and R², R³, X and R⁹ have a meaning as defined in a line A1 to A480 in Table A.

Embodiment 280: R¹ is CF₃, R⁶ is H; R^{W} is CH₂CH₃, the index m is 2, R⁷ and R⁸ - if present - are H, the ring system is represented by Table 139 and R², R³, X and R⁹ have a meaning as defined in a line A1 to A480 in Table A.

Embodiment 281: R¹ is CF₃, R⁶ is H; R^{W} is CH₂CH₃, the index m is 2, R⁷ and R⁸ - if present - are H, the ring system is represented by Table 140 and R², R³, X and R⁹ have a meaning as defined in a line A1 to A480 in Table A.

Embodiment 282: R¹ is CF₃, R⁶ is H; R^{W} is CH₂CH₃, the index m is 2, R⁷ and R⁸- if present - are H, the ring system is represented by Table 141 and R², R³, X and R⁹ have a meaning as defined in a line A1 to A480 in Table A.

Embodiment 283: R¹ is CF₃, R² is H, R⁴ is H, R⁵ is H, X is O, R^{W} is CH₂CH₃, the index m is 2, R⁷ and R⁸- if present - are H, R⁹ is CH₃, and the ring system is represented by a Table 142 to 282.

Embodiment 284: R¹ is CF₃, R² is CH₃, R⁴ is H, R⁵ is H, X is O, R^{W} is CH₂CH₃, the index m is 2, R⁷ and R⁸- if present - are H, R⁹ is CH₃, and the ring system is represented by a Table 142 to 282.

Embodiment 285: R¹ is CF₃, R² is H, R⁴ is H, R⁵ is CH₃, X is O, R^{W} is CH₂CH₃, the index m is 2, R⁷ and R⁸- if present - are H, R⁹ is CH₃, and the ring system is represented by a Table 142 to 282.

Embodiment 286: R¹ is CF₃, R² is CH₃, R⁴ is H, R⁵ is CH₃, X is O, R^{W} is CH₂CH₃, the index m is 2, R⁷ and R⁸- if present - are H, R⁹ is CH₃, and the ring system is represented by a Table 142 to 282.

Embodiment 287: R¹ is CF₃, R² is H, R⁴ is H, R⁵ is H, X is S, R^{W} is CH₂CH₃, the index m is 2, R⁷ and R⁸- if present - are H, R⁹ is CH₃, and the ring system is represented by a Table 142 to 282.

Embodiment 288: R¹ is CF₃, R² is CH₃, R⁴ is H, R⁵ is H, X is S, R^{W} is CH₂CH₃, the index m is 2, R⁷ and R⁸- if present - are H, R⁹ is CH₃, and the ring system is represented by a Table 142 to 282.

Embodiment 289: R¹ is CF₃, R² is H, R⁴ is H, R⁵ is CH₃, X is S, R^{W} is CH₂CH₃, the index m is 2, R⁷ and R⁸- if present - are H, R⁹ is CH₃, and the ring system is represented by a Table 142 to 282.

Embodiment 290: R¹ is CF₃, R² is CH₃, R⁴ is H, R⁵ is CH₃, X is S, R^{W} is CH₂CH₃, the index m is 2, R⁷ and R⁸- if present - are H, R⁹ is CH₃, and the ring system is represented by a Table 142 to 282.

Embodiment 291: R¹ is CF₃, R² is H, R⁴ is H, R⁵ is H, X is O, R^{W} is CH₂CH₃, the index m is 2, R⁷ and R⁸- if present - are H, R⁹ is CF₃, and the ring system is represented by a Table 142 to 282.

Embodiment 292: R¹ is CF₃, R² is CH₃, R⁴ is H, R⁵ is H, X is O, R^{W} is CH₂CH₃, the index m is 2, R⁷ and R⁸- if present - are H, R⁹ is CF₃, and the ring system is represented by a Table 142 to 282.

Embodiment 293: R¹ is CF₃, R² is H, R⁴ is H, R⁵ is CH₃, X is O, R^{W} is CH₂CH₃, the index m is 2, R⁷ and R⁸- if present - are H, R⁹ is CF₃, and the ring system is represented by a Table 142 to 282.

Embodiment 294: R¹ is CF₃, R² is CH₃, R⁴ is H, R⁵ is CH₃, X is O, R^{W} is CH₂CH₃, the index m is 2, R⁷ and R⁸- if present - are H, R⁹ is CF₃, and the ring system is represented by a Table 142 to 282.

Embodiment 295: R¹ is CF₃, R² is H, R⁴ is H, R⁵ is H, X is S, R^{W} is CH₂CH₃, the index m is 2, R⁷ and R⁸- if present - are H, R⁹ is FH₃, and the ring system is represented by a Table 142 to 282.

Embodiment 296: R¹ is CF₃, R² is CH₃, R⁴ is H, R⁵ is H, X is S, R^{W} is CH₂CH₃, the index m is 2, R⁷ and R⁸- if present - are H, R⁹ is CF₃, and the ring system is represented by a Table 142 to 282.

Embodiment 297: R¹ is CF₃, R² is H, R⁴ is H, R⁵ is CH₃, X is S, R^{W} is CH₂CH₃, the index m is 2, R⁷ and R⁸- if present - are H, R⁹ is CF₃, and the ring system is represented by a Table 142 to 282.

Embodiment 298: R¹ is CF₃, R² is CH₃, R⁴ is H, R⁵ is CH₃, X is S, R^{W} is CH₂CH₃, the index m is 2, R⁷ and R⁸- if present - are H, R⁹ is CF₃, and the ring system is represented by a Table 142 to 282.

Embodiment 299: R¹ is CF₃, R² is H, R⁴ is H, R⁵ is H, X is O, R^{W} is CH₂CH₃, the index m is 2, R⁷ and R⁸- if present - are H, R⁹ is F, and the ring system is represented by a Table 142 to 282.

Embodiment 300: R¹ is CF₃, R² is CH₃, R⁴ is H, R⁵ is H, X is O, R^{W} is CH₂CH₃, the index m is 2, R⁷ and R⁸- if present - are H, R⁹ is F, and the ring system is represented by a Table 142 to 282.

Embodiment 301: R¹ is CF₃, R² is H, R⁴ is H, R⁵ is CH₃, X is O, R^{W} is CH₂CH₃, the index m is 2, R⁷ and R⁸- if present - are H, R⁹ is F, and the ring system is represented by a Table 142 to 282.

Embodiment 302: R¹ is CF₃, R² is CH₃, R⁴ is H, R⁵ is CH₃, X is O, R^{W} is CH₂CH₃, the index m is 2, R⁷ and R⁸- if present - are H, R⁹ is F, and the ring system is represented by a Table 142 to 282.

Embodiment 303: R¹ is CF₃, R² is H, R⁴ is H, R⁵ is H, X is S, R^{W} is CH₂CH₃, the index m is 2, R⁷ and R⁸- if present - are H, R⁹ is F, and the ring system is represented by a Table 142 to 282.

Embodiment 304: R¹ is CF₃, R² is CH₃, R⁴ is H, R⁵ is H, X is S, R^{W} is CH₂CH₃, the index m is 2, R⁷ and R⁸- if present - are H, R⁹ is F, and the ring system is represented by a Table 142 to 282.

Embodiment 305: R¹ is CF₃, R² is H, R⁴ is H, R⁵ is CH₃, X is S, R^{W} is CH₂CH₃, the index m is 2, R⁷ and R⁸- if present - are H, R⁹ is F, and the ring system is represented by a Table 142 to 282.

Embodiment 306: R¹ is CF₃, R² is CH₃, R⁴ is H, R⁵ is CH₃, X is S, R^{W} is CH₂CH₃, the index m is 2, R⁷ and R⁸- if present - are H, R⁹ is F, and the ring system is represented by a Table 142 to 282.

Embodiment 307: R¹ is CF₃, R² is H, R⁴ is H, R⁵ is H, X is O, R^{W} is CH₂CH₃, the index m is 2, R⁷ and R⁸- if present - are H, R⁹ is Cl, and the ring system is represented by a Table 142 to 282.

Embodiment 308: R¹ is CF₃, R² is CH₃, R⁴ is H, R⁵ is H, X is O, R^{W} is CH₂CH₃, the index m is 2, R⁷ and R⁸- if present - are H, R⁹ is Cl, and the ring system is represented by a Table 142 to 282.

Embodiment 309: R¹ is CF₃, R² is H, R⁴ is H, R⁵ is CH₃, X is O, R^{W} is CH₂CH₃, the index m is 2, R⁷ and R⁸- if present - are H, R⁹ is Cl, and the ring system is represented by a Table 142 to 282.

Embodiment 310: R¹ is CF₃, R² is CH₃, R⁴ is H, R⁵ is CH₃, X is O, R^{W} is CH₂CH₃, the index m is 2, R⁷ and R⁸- if present - are H, R⁹ is Cl, and the ring system is represented by a Table 142 to 282.

Embodiment 311: R¹ is CF₃, R² is H, R⁴ is H, R⁵ is H, X is S, R^{W} is CH₂CH₃, the index m is 2, R⁷ and R⁸- if present - are H, R⁹ is Cl, and the ring system is represented by a Table 142 to 282.

Embodiment 312: R¹ is CF₃, R² is CH₃, R⁴ is H, R⁵ is H, X is S, R^{W} is CH₂CH₃, the index m is 2, R⁷ and R⁸- if present - are H, R⁹ is Cl, and the ring system is represented by a Table 142 to 282.

Embodiment 313: R¹ is CF₃, R² is H, R⁴ is H, R⁵ is CH₃, X is S, R^{W} is CH₂CH₃, the index m is 2, R⁷ and R⁸- if present - are H, R⁹ is Cl, and the ring system is represented by a Table 142 to 282.

Embodiment 314: R¹ is CF₃, R² is CH₃, R⁴ is H, R⁵ is CH₃, X is S, R^{W} is CH₂CH₃, the index m is 2, R⁷ and R⁸- if present - are H, R⁹ is Cl, and the ring system is represented by a Table 142 to 282.

Embodiment 315: R¹ is CF₃, R² is H, R⁴ is H, R⁵ is H, X is O, R^{W} is CH₂CH₃, the index m is 2, R⁷ and R⁸- if present - are H, R⁹ is Br, and the ring system is represented by a Table 142 to 282.

Embodiment 316: R¹ is CF₃, R² is CH₃, R⁴ is H, R⁵ is H, X is O, R^{W} is CH₂CH₃, the index m is 2, R⁷ and R⁸- if present - are H, R⁹ is Br, and the ring system is represented by a Table 142 to 282.

Embodiment 317: R¹ is CF₃, R² is H, R⁴ is H, R⁵ is CH₃, X is O, R^{W} is CH₂CH₃, the index m is 2, R⁷ and R⁸- if present - are H, R⁹ is Br, and the ring system is represented by a Table 142 to 282.

Embodiment 318: R¹ is CF₃, R² is CH₃, R⁴ is H, R⁵ is CH₃, X is O, R^{W} is CH₂CH₃, the index m is 2, R⁷ and R⁸- if present - are H, R⁹ is Br, and the ring system is represented by a Table 142 to 282.

Embodiment 319: R¹ is CF₃, R² is H, R⁴ is H, R⁵ is H, X is S, R^{W} is CH₂CH₃, the index m is 2, R⁷ and R⁸- if present - are H, R⁹ is Br, and the ring system is represented by a Table 142 to 282.

Embodiment 320: R¹ is CF₃, R² is CH₃, R⁴ is H, R⁵ is H, X is S, R^{W} is CH₂CH₃, the index m is 2, R⁷ and R⁸- if present - are H, R⁹ is Br, and the ring system is represented by a Table 142 to 282.

Embodiment 321: R¹ is CF₃, R² is H, R⁴ is H, R⁵ is CH₃, X is S, R^{W} is CH₂CH₃, the index m is 2, R⁷ and R⁸- if present - are H, R⁹ is Br, and the ring system is represented by a Table 142 to 282.

Embodiment 322: R¹ is CF₃, R² is CH₃, R⁴ is H, R⁵ is CH₃, X is S, R^{W} is CH₂CH₃, the index m is 2, R⁷ and R⁸- if present - are H, R⁹ is Br, and the ring system is represented by a Table 142 to 282.

Embodiment 323: R¹ is CF₃, R² is H, R⁴ is H, R⁵ is H, X is O, R^{W} is CH₂CH₃, the index m is 2, R⁷ and R⁸- if present - are H, R⁹ is 1-cyanocyclopropyl, and the ring system is represented by a Table 142 to 282.

Embodiment 324: R¹ is CF₃, R² is CH₃, R⁴ is H, R⁵ is H, X is O, R^{W} is CH₂CH₃, the index m is 2, R⁷ and R⁸- if present - are H, R⁹ is 1-cyanocyclopropyl, and the ring system is represented by a Table 142 to 282.

Embodiment 325: R¹ is CF₃, R² is H, R⁴ is H, R⁵ is CH₃, X is O, R^{W} is CH₂CH₃, the index m is 2, R⁷ and R⁸- if present - are H, R⁹ is 1-cyanocyclopropyl, and the ring system is represented by a Table 142 to 282.

Embodiment 326: R¹ is CF₃, R² is CH₃, R⁴ is H, R⁵ is CH₃, X is O, R^{W} is CH₂CH₃, the index m is 2, R⁷ and R⁸- if present - are H, R⁹ is 1-cyanocyclopropyl, and the ring system is represented by a Table 142 to 282.

Embodiment 327: R¹ is CF₃, R² is H, R⁴ is H, R⁵ is H, X is S, R^{W} is CH₂CH₃, the index m is 2, R⁷ and R⁸- if present - are H, R⁹ is 1-cyanocyclopropyl, and the ring system is represented by a Table 142 to 282.

Embodiment 328: R¹ is CF₃, R² is CH₃, R⁴ is H, R⁵ is H, X is S, R^{W} is CH₂CH₃, the index m is 2, R⁷ and R⁸- if present - are H, R⁹ is 1-cyanocyclopropyl, and the ring system is represented by a Table 142 to 282.

Embodiment 329: R¹ is CF₃, R² is H, R⁴ is H, R⁵ is CH₃, X is S, R^{W} is CH₂CH₃, the index m is 2, R⁷ and R⁸- if present - are H, R⁹ is 1-cyanocyclopropyl, and the ring system is represented by a Table 142 to 282.

Embodiment 330: R¹ is CF₃, R² is CH₃, R⁴ is H, R⁵ is CH₃, X is S, R^{W} is CH₂CH₃, the index m is 2, R⁷ and R⁸- if present - are H, R⁹ is 1-cyanocyclopropyl, and the ring system is represented by a Table 142 to 282.

Embodiment 331: R¹ is CF₃, R² is H, R⁴ is H, R⁵ is H, X is O, R^{W} is CH₂CH₃, the index m is 2, R⁷ and R⁸- if present - are H, R⁹ is 1-cyanoisopropyl, and the ring system is represented by a Table 142 to 282.

Embodiment 332: R¹ is CF₃, R² is CH₃, R⁴ is H, R⁵ is H, X is O, R^{W} is CH₂CH₃, the index m is 2, R⁷ and R⁸- if present - are H, R⁹ is 1-cyanoisopropyl, and the ring system is represented by a Table 142 to 282.

Embodiment 333: R¹ is CF₃, R² is H, R⁴ is H, R⁵ is CH₃, X is O, R^{W} is CH₂CH₃, the index m is 2, R⁷ and R⁸- if present - are H, R⁹ is 1-cyanoisopropyl, and the ring system is represented by a Table 142 to 282.

Embodiment 334: R¹ is CF₃, R² is CH₃, R⁴ is H, R⁵ is CH₃, X is O, R^{W} is CH₂CH₃, the index m is 2, R⁷ and R⁸- if present - are H, R⁹ is 1-cyanoisopropyl, and the ring system is represented by a Table 142 to 282.

Embodiment 335: R¹ is CF₃, R² is H, R⁴ is H, R⁵ is H, X is S, R^{W} is CH₂CH₃, the index m is 2, R⁷ and R⁸- if present - are H, R⁹ is 1-cyanoisopropyl, and the ring system is represented by a Table 142 to 282.

Embodiment 336: R¹ is CF₃, R² is CH₃, R⁴ is H, R⁵ is H, X is S, R^{W} is CH₂CH₃, the index m is 2, R⁷ and R⁸- if present - are H, R⁹ is 1-cyanoisopropyl, and the ring system is represented by a Table 142 to 282.

Embodiment 337: R¹ is CF₃, R² is H, R⁴ is H, R⁵ is CH₃, X is S, R^{W} is CH₂CH₃, the index m is 2, R⁷ and R⁸- if present - are H, R⁹ is 1-cyanoisopropyl, and the ring system is represented by a Table 142 to 282.

Embodiment 338: R¹ is CF₃, R² is CH₃, R⁴ is H, R⁵ is CH₃, X is S, R^{W} is CH₂CH₃, the index m is 2, R⁷ and R⁸- if present - are H, R⁹ is 1-cyanoisopropyl, and the ring system is represented by a Table 142 to 282.

Embodiment 339: R¹ is CF₃, R² is H, R⁴ is H, R⁵ is H, X is O, R^{W} is CH₂CH₃, the index m is 2, R⁷ and R⁸- if present - are H, R⁹ is CH₂CH₃, and the ring system is represented by a Table 142 to 282.

Embodiment 340: R¹ is CF₃, R² is CH₃, R⁴ is H, R⁵ is H, X is O, R^{W} is CH₂CH₃, the index m is 2, R⁷ and R⁸- if present - are H, R⁹ is CH₂CH₃, and the ring system is represented by a Table 142 to 282.

Embodiment 341: R¹ is CF₃, R² is H, R⁴ is H, R⁵ is CH₃, X is O, R^{W} is CH₂CH₃, the index m is 2, R⁷ and R⁸- if present - are H, R⁹ is CH₂CH₃, and the ring system is represented by a Table 142 to 282.

Embodiment 342: R¹ is CF₃, R² is CH₃, R⁴ is H, R⁵ is CH₃, X is O, R^{W} is CH₂CH₃, the index m is 2, R⁷ and R⁸- if present - are H, R⁹ is CH₂CH₃, and the ring system is represented by a Table 142 to 282.

Embodiment 343: R¹ is CF₃, R² is H, R⁴ is H, R⁵ is H, X is S, R^{W} is CH₂CH₃, the index m is 2, R⁷ and R⁸- if present - are H, R⁹ is CH₂CH₃, and the ring system is represented by a Table 142 to 282.

Embodiment 344: R¹ is CF₃, R² is CH₃, R⁴ is H, R⁵ is H, X is S, R^{W} is CH₂CH₃, the index m is 2, R⁷ and R⁸- if present - are H, R⁹ is CH₂CH₃, and the ring system is represented by a Table 142 to 282.

Embodiment 345: R¹ is CF₃, R² is H, R⁴ is H, R⁵ is CH₃, X is S, R^{W} is CH₂CH₃, the index m is 2, R⁷ and R⁸- if present - are H, R⁹ is CH₂CH₃, and the ring system is represented by a Table 142 to 282.

Embodiment 346: R¹ is CF₃, R² is CH₃, R⁴ is H, R⁵ is CH₃, X is S, R^{W} is CH₂CH₃, the index m is 2, R⁷ and R⁸- if present - are H, R⁹ is CH₂CH₃, and the ring system is represented by a Table 142 to 282.

Embodiment 347: R¹ is CF₃, R² is H, R⁴ is H, R⁵ is H, X is O, R^{W} is CH₂CH₃, the index m is 2, R⁷ and R⁸- if present - are H, R⁹ is cPr, and the ring system is represented by a Table 142 to 282.

Embodiment 348: R¹ is CF₃, R² is CH₃, R⁴ is H, R⁵ is H, X is O, R^{W} is CH₂CH₃, the index m is 2, R⁷ and R⁸- if present - are H, R⁹ is cPr, and the ring system is represented by a Table 142 to 282.

Embodiment 349: R¹ is CF₃, R² is H, R⁴ is H, R⁵ is CH₃, X is O, R^{W} is CH₂CH₃, the index m is 2, R⁷ and R⁸- if present - are H, R⁹ is cPr, and the ring system is represented by a Table 142 to 282.

Embodiment 350: R¹ is CF₃, R² is CH₃, R⁴ is H, R⁵ is CH₃, X is O, R^{W} is CH₂CH₃, the index m is 2, R⁷ and R⁸- if present - are H, R⁹ is cPr, and the ring system is represented by a Table 142 to 282.

Embodiment 351: R¹ is CF₃, R² is H, R⁴ is H, R⁵ is H, X is S, R^{W} is CH₂CH₃, the index m is 2, R⁷ and R⁸- if present - are H, R⁹ is cPr, and the ring system is represented by a Table 142 to 282.

Embodiment 352: R¹ is CF₃, R² is CH₃, R⁴ is H, R⁵ is H, X is S, R^{W} is CH₂CH₃, the index m is 2, R⁷ and R⁸- if present - are H, R⁹ is cPr, and the ring system is represented by a Table 142 to 282.

Embodiment 353: R¹ is CF₃, R² is H, R⁴ is H, R⁵ is CH₃, X is S, R^{W} is CH₂CH₃, the index m is 2, R⁷ and R⁸- if present - are H, R⁹ is cPr, and the ring system is represented by a Table 142 to 282.

Embodiment 354: R¹ is CF₃, R² is CH₃, R⁴ is H, R⁵ is CH₃, X is S, R^{W} is CH₂CH₃, the index m is 2, R⁷ and R⁸- if present - are H, R⁹ is cPr, and the ring system is represented by a Table 142 to 282.

Embodiment 355: R¹ is CF₃, R² is H, R⁴ is H, R⁵ is H, X is O, R^{W} is CH₂CH₃, the index m is 2, R⁷ and R⁸- if present - are H, R⁹ is benzyl, and the ring system is represented by a Table 142 to 282.

Embodiment 356: R¹ is CF₃, R² is CH₃, R⁴ is H, R⁵ is H, X is O, R^{W} is CH₂CH₃, the index m is 2, R⁷ and R⁸- if present - are H, R⁹ is benzyl, and the ring system is represented by a Table 142 to 282.

Embodiment 357: R¹ is CF₃, R² is H, R⁴ is H, R⁵ is CH₃, X is O, R^{W} is CH₂CH₃, the index m is 2, R⁷ and R⁸- if present - are H, R⁹ is benzyl, and the ring system is represented by a Table 142 to 282.

Embodiment 358: R¹ is CF₃, R² is CH₃, R⁴ is H, R⁵ is CH₃, X is O, R^{W} is CH₂CH₃, the index m is 2, R⁷ and R⁸- if present - are H, R⁹ is benzyl, and the ring system is represented by a Table 142 to 282.

Embodiment 359: R¹ is CF₃, R² is H, R⁴ is H, R⁵ is H, X is S, R^{W} is CH₂CH₃, the index m is 2, R⁷ and R⁸- if present - are H, R⁹ is benzyl, and the ring system is represented by a Table 142 to 282.

Embodiment 360: R¹ is CF₃, R² is CH₃, R⁴ is H, R⁵ is H, X is S, R^{W} is CH₂CH₃, the index m is 2, R⁷ and R⁸- if present - are H, R⁹ is benzyl, and the ring system is represented by a Table 142 to 282.

Embodiment 361: R¹ is CF₃, R² is H, R⁴ is H, R⁵ is CH₃, X is S, R^{W} is CH₂CH₃, the index m is 2, R⁷ and R⁸- if present - are H, R⁹ is benzyl, and the ring system is represented by a Table 142 to 282.

Embodiment 362: R¹ is CF₃, R² is CH₃, R⁴ is H, R⁵ is CH₃, X is S, R^{W} is CH₂CH₃, the index m is 2, R⁷ and R⁸- if present - are H, R⁹ is benzyl, and the ring system is represented by a Table 142 to 282.

Embodiment 363: R¹ is CF₃, R² is H, R⁴ is H, R⁵ is H, X is O, R^{W} is CH₂CH₃, the index m is 2, R⁷ and R⁸- if present - are H, R⁹ is cyclopropylmethyl, and the ring system is represented by a Table 142 to 282.

Embodiment 364: R¹ is CF₃, R² is CH₃, R⁴ is H, R⁵ is H, X is O, R^{W} is CH₂CH₃, the index m is 2, R⁷ and R⁸- if present - are H, R⁹ is cyclopropylmethyl, and the ring system is represented by a Table 142 to 282.

Embodiment 365: R¹ is CF₃, R² is H, R⁴ is H, R⁵ is CH₃, X is O, R^{W} is CH₂CH₃, the index m is 2, R⁷ and R⁸- if present - are H, R⁹ is cyclopropylmethyl, and the ring system is represented by a Table 142 to 282.

Embodiment 366: R¹ is CF₃, R² is CH₃, R⁴ is H, R⁵ is CH₃, X is O, R^{W} is CH₂CH₃, the index m is 2, R⁷ and R⁸- if present - are H, R⁹ is cyclopropylmethyl, and the ring system is represented by a Table 142 to 282.

Embodiment 367: R¹ is CF₃, R² is H, R⁴ is H, R⁵ is H, X is S, R^{W} is CH₂CH₃, the index m is 2, R⁷ and R⁸- if present - are H, R⁹ is cyclopropylmethyl, and the ring system is represented by a Table 142 to 282.

Embodiment 368: R¹ is CF₃, R² is CH₃, R⁴ is H, R⁵ is H, X is S, R^{W} is CH₂CH₃, the index m is 2, R⁷ and R⁸- if present - are H, R⁹ is cyclopropylmethyl, and the ring system is represented by a Table 142 to 282.

Embodiment 369: R¹ is CF₃, R² is H, R⁴ is H, R⁵ is CH₃, X is S, R^{W} is CH₂CH₃, the index m is 2, R⁷ and R⁸- if present - are H, R⁹ is cyclopropylmethyl, and the ring system is represented by a Table 142 to 282.

Embodiment 370: R¹ is CF₃, R² is CH₃, R⁴ is H, R⁵ is CH₃, X is S, R^{W} is CH₂CH₃, the index m is 2, R⁷ and R⁸- if present - are H, R⁹ is cyclopropylmethyl, and the ring system is represented by a Table 142 to 282.

Embodiment 371: R¹ is CF₃, R² is H, R⁴ is H, R⁵ is H, X is O, R^{W} is CH₂CH₃, the index m is 2, R⁷ and R⁸- if present - are H, R⁹ is 2-propynyl, and the ring system is represented by a Table 142 to 282.

Embodiment 372: R¹ is CF₃, R² is CH₃, R⁴ is H, R⁵ is H, X is O, R^{W} is CH₂CH₃, the index m is 2, R⁷ and R⁸- if present - are H, R⁹ is 2-propynyl, and the ring system is represented by a Table 142 to 282.

Embodiment 373: R¹ is CF₃, R² is H, R⁴ is H, R⁵ is CH₃, X is O, R^{W} is CH₂CH₃, the index m is 2, R⁷ and R⁸- if present - are H, R⁹ is 2-propynyl, and the ring system is represented by a Table 142 to 282.

Embodiment 374: R¹ is CF₃, R² is CH₃, R⁴ is H, R⁵ is CH₃, X is O, R^{W} is CH₂CH₃, the index m is 2, R⁷ and R⁸- if present - are H, R⁹ is 2-propynyl, and the ring system is represented by a Table 142 to 282.

Embodiment 375: R¹ is CF₃, R² is H, R⁴ is H, R⁵ is H, X is S, R^{W} is CH₂CH₃, the index m is 2, R⁷ and R⁸- if present - are H, R⁹ is 2-propynyl, and the ring system is represented by a Table 142 to 282.

Embodiment 376: R¹ is CF₃, R² is CH₃, R⁴ is H, R⁵ is H, X is S, R^{W} is CH₂CH₃, the index m is 2, R⁷ and R⁸- if present - are H, R⁹ is 2-propynyl, and the ring system is represented by a Table 142 to 282.

Embodiment 377: R¹ is CF₃, R² is H, R⁴ is H, R⁵ is CH₃, X is S, R^{W} is CH₂CH₃, the index m is 2, R⁷ and R⁸- if present - are H, R⁹ is 2-propynyl, and the ring system is represented by a Table 142 to 282.

Embodiment 378: R¹ is CF₃, R² is CH₃, R⁴ is H, R⁵ is CH₃, X is S, R^{W} is CH₂CH₃, the index m is 2, R⁷ and R⁸- if present - are H, R⁹ is 2-propynyl, and the ring system is represented by a Table 142 to 282.

Embodiment 379: R¹ is CF₃, R² is H, R⁴ is H, R⁵ is H, X is O, R^{W} is CH₂CH₃, the index m is 2, R⁷ and R⁸- if present - are H, R⁹ is isobutyl, and the ring system is represented by a Table 142 to 282.

Embodiment 380: R¹ is CF₃, R² is CH₃, R⁴ is H, R⁵ is H, X is O, R^{W} is CH₂CH₃, the index m is 2, R⁷ and R⁸- if present - are H, R⁹ is isobutyl, and the ring system is represented by a Table 142 to 282.

Embodiment 381: R¹ is CF₃, R² is H, R⁴ is H, R⁵ is CH₃, X is O, R^{W} is CH₂CH₃, the index m is 2, R⁷ and R⁸- if present - are H, R⁹ is isobutyl, and the ring system is represented by a Table 142 to 282.

Embodiment 382: R¹ is CF₃, R² is CH₃, R⁴ is H, R⁵ is CH₃, X is O, R^{W} is CH₂CH₃, the index m is 2, R⁷ and R⁸- if present - are H, R⁹ is isobutyl, and the ring system is represented by a Table 142 to 282.

Embodiment 383: R¹ is CF₃, R² is H, R⁴ is H, R⁵ is H, X is S, R^{W} is CH₂CH₃, the index m is 2, R⁷ and R⁸- if present - are H, R⁹ is isobutyl, and the ring system is represented by a Table 142 to 282.

Embodiment 384: R¹ is CF₃, R² is CH₃, R⁴ is H, R⁵ is H, X is S, R^{W} is CH₂CH₃, the index m is 2, R⁷ and R⁸- if present - are H, R⁹ is isobutyl, and the ring system is represented by a Table 142 to 282.

Embodiment 385: R¹ is CF₃, R² is H, R⁴ is H, R⁵ is CH₃, X is S, R^{W} is CH₂CH₃, the index m is 2, R⁷ and R⁸- if present - are H, R⁹ is isobutyl, and the ring system is represented by a Table 142 to 282.

Embodiment 386: R¹ is CF₃, R² is CH₃, R⁴ is H, R⁵ is CH₃, X is S, R^{W} is CH₂CH₃, the index m is 2, R⁷ and R⁸- if present - are H, R⁹ is isobutyl, and the ring system is represented by a Table 142 to 282.

Embodiment 387: R¹ is CF₃, R² is H, R⁴ is H, R⁵ is H, X is O, R^{W} is CH₂CH₃, the index m is 2, R⁷ and R⁸- if present - are H, R⁹ is cyclohexylmethyl, and the ring system is represented by a Table 142 to 282.

Embodiment 388: R¹ is CF₃, R² is CH₃, R⁴ is H, R⁵ is H, X is O, R^{W} is CH₂CH₃, the index m is 2, R⁷ and R⁸- if present - are H, R⁹ is cyclohexylmethyl, and the ring system is represented by a Table 142 to 282.

Embodiment 389: R¹ is CF₃, R² is H, R⁴ is H, R⁵ is CH₃, X is O, R^{W} is CH₂CH₃, the index m is 2, R⁷ and R⁸- if present - are H, R⁹ is cyclohexylmethyl, and the ring system is represented by a Table 142 to 282.

Embodiment 390: R¹ is CF₃, R² is CH₃, R⁴ is H, R⁵ is CH₃, X is O, R^{W} is CH₂CH₃, the index m is 2, R⁷ and R⁸- if present - are H, R⁹ is cyclohexylmethyl, and the ring system is represented by a Table 142 to 282.

Embodiment 391: R¹ is CF₃, R² is H, R⁴ is H, R⁵ is H, X is S, R^{W} is CH₂CH₃, the index m is 2, R⁷ and R⁸- if present - are H, R⁹ is cyclohexylmethyl, and the ring system is represented by a Table 142 to 282.

Embodiment 392: R¹ is CF₃, R² is CH₃, R⁴ is H, R⁵ is H, X is S, R^{W} is CH₂CH₃, the index m is 2, R⁷ and R⁸- if present - are H, R⁹ is cyclohexylmethyl, and the ring system is represented by a Table 142 to 282.

Embodiment 393: R¹ is CF₃, R² is H, R⁴ is H, R⁵ is CH₃, X is S, R^{W} is CH₂CH₃, the index m is 2, R⁷ and R⁸- if present - are H, R⁹ is cyclohexylmethyl, and the ring system is represented by a Table 142 to 282.

Embodiment 394: R¹ is CF₃, R² is CH₃, R⁴ is H, R⁵ is CH₃, X is S, R^{W} is CH₂CH₃, the index m is 2, R⁷ and R⁸- if present - are H, R⁹ is , cyclohexylmethyl, and the ring system is represented by a Table 142 to 282.

Embodiment 395: R¹ is CF₃, R² is H, R⁴ is H, R⁵ is H, X is O, R^{W} is CH₂CH₃, the index m is 2, R⁷ and R⁸- if present - are H, R⁹ is 2-propenyl, and the ring system is represented by a Table 142 to 282.

Embodiment 396: R¹ is CF₃, R² is CH₃, R⁴ is H, R⁵ is H, X is O, R^{W} is CH₂CH₃, the index m is 2, R⁷ and R⁸- if present - are H, R⁹ is 2-propenyl, and the ring system is represented by a Table 142 to 282.

Embodiment 397: R¹ is CF₃, R² is H, R⁴ is H, R⁵ is CH₃, X is O, R^{W} is CH₂CH₃, the index m is 2, R⁷ and R⁸- if present - are H, R⁹ is 2-propenyl, and the ring system is represented by a Table 142 to 282.

Embodiment 398: R¹ is CF₃, R² is CH₃, R⁴ is H, R⁵ is CH₃, X is O, R^{W} is CH₂CH₃, the index m is 2, R⁷ and R⁸- if present - are H, R⁹ is 2-propenyl, and the ring system is represented by a Table 142 to 282.

Embodiment 399: R¹ is CF₃, R² is H, R⁴ is H, R⁵ is H, X is S, R^{W} is CH₂CH₃, the index m is 2, R⁷ and R⁸- if present - are H, R⁹ is 2-propenyl, and the ring system is represented by a Table 142 to 282.

Embodiment 400: R¹ is CF₃, R² is CH₃, R⁴ is H, R⁵ is H, X is S, R^{W} is CH₂CH₃, the index m is 2, R⁷ and R⁸- if present - are H, R⁹ is 2-propenyl, and the ring system is represented by a Table 142 to 282.

Embodiment 401: R¹ is CF₃, R² is H, R⁴ is H, R⁵ is CH₃, X is S, R^{W} is CH₂CH₃, the index m is 2, R⁷ and R⁸- if present - are H, R⁹ is 2-propenyl, and the ring system is represented by a Table 142 to 282.

Embodiment 402: R¹ is CF₃, R² is CH₃, R⁴ is H, R⁵ is CH₃, X is S, R^{W} is CH₂CH₃, the index m is 2, R⁷ and R⁸- if present - are H, R⁹ is 2-propenyl, and the ring system is represented by a Table 142 to 282.

Embodiment 403: R¹ is CF₃, R² is H, R⁴ is H, R⁵ is H, X is O, R^{W} is CH₂CH₃, the index m is 2, R⁷ and R⁸- if present - are H, R⁹ is CH₂CF₃, and the ring system is represented by a Table 142 to 282.

Embodiment 404: R¹ is CF₃, R² is CH₃, R⁴ is H, R⁵ is H, X is O, R^{W} is CH₂CH₃, the index m is 2, R⁷ and R⁸- if present - are H, R⁹ is CH₂CF₃, and the ring system is represented by a Table 142 to 282.

Embodiment 405: R¹ is CF₃, R² is H, R⁴ is H, R⁵ is CH₃, X is O, R^{W} is CH₂CH₃, the index m is 2, R⁷ and R⁸- if present - are H, R⁹ is CH₂CF₃, and the ring system is represented by a Table 142 to 282.

Embodiment 406: R¹ is CF₃, R² is CH₃, R⁴ is H, R⁵ is CH₃, X is O, R^{W} is CH₂CH₃, the index m is 2, R⁷ and R⁸- if present - are H, R⁹ is CH₂CF₃, and the ring system is represented by a Table 142 to 282.

Embodiment 407: R¹ is CF₃, R² is H, R⁴ is H, R⁵ is H, X is S, R^{W} is CH₂CH₃, the index m is 2, R⁷ and R⁸- if present - are H, R⁹ is CH₂CF₃, and the ring system is represented by a Table 142 to 282.

Embodiment 408: R¹ is CF₃, R² is CH₃, R⁴ is H, R⁵ is H, X is S, R^{W} is CH₂CH₃, the index m is 2, R⁷ and R⁸- if present - are H, R⁹ is CH₂CF₃, and the ring system is represented by a Table 142 to 282.

Embodiment 409: R¹ is CF₃, R² is H, R⁴ is H, R⁵ is CH₃, X is S, R^{W} is CH₂CH₃, the index m is 2, R⁷ and R⁸- if present - are H, R⁹ is CH₂CF₃, and the ring system is represented by a Table 142 to 282.

Embodiment 410: R¹ is CF₃, R² is CH₃, R⁴ is H, R⁵ is CH₃, X is S, R^{W} is CH₂CH₃, the index m is 2, R⁷ and R⁸- if present - are H, R⁹ is CH₂CF₃, and the ring system is represented by a Table 142 to 282.

Embodiment 411: R¹ is CF₃, R² is H, R⁴ is H, R⁵ is H, X is O, R^{W} is CH₂CH₃, the index m is 2, R⁷ and R⁸- if present - are H, R⁹ is CH₂CH₂OCH₃, and the ring system is represented by a Table 142 to 282.

Embodiment 412: R¹ is CF₃, R² is CH₃, R⁴ is H, R⁵ is H, X is O, R^{W} is CH₂CH₃, the index m is 2, R⁷ and R⁸- if present - are H, R⁹ is CH₂CH₂OCH₃, and the ring system is represented by a Table 142 to 282.

Embodiment 413: R¹ is CF₃, R² is H, R⁴ is H, R⁵ is CH₃, X is O, R^{W} is CH₂CH₃, the index m is 2, R⁷ and R⁸- if present - are H, R⁹ is CH₂CH₂OCH₃, and the ring system is represented by a Table 142 to 282.

Embodiment 414: R¹ is CF₃, R² is CH₃, R⁴ is H, R⁵ is CH₃, X is O, R^{W} is CH₂CH₃, the index m is 2, R⁷ and R⁸- if present - are H, R⁹ is CH₂CH₂OCH₃, and the ring system is represented by a Table 142 to 282.

Embodiment 415: R¹ is CF₃, R² is H, R⁴ is H, R⁵ is H, X is S, R^{W} is CH₂CH₃, the index m is 2, R⁷ and R⁸- if present - are H, R⁹ is CH₂CH₂OCH₃, and the ring system is represented by a Table 142 to 282.

Embodiment 416: R¹ is CF₃, R² is CH₃, R⁴ is H, R⁵ is H, X is S, R^{W} is CH₂CH₃, the index m is 2, R⁷ and R⁸- if present - are H, R⁹ is CH₂CH₂OCH₃, and the ring system is represented by a Table 142 to 282.

Embodiment 417: R¹ is CF₃, R² is H, R⁴ is H, R⁵ is CH₃, X is S, R^{W} is CH₂CH₃, the index m is 2, R⁷ and R⁸- if present - are H, R⁹ is CH₂CH₂OCH₃, and the ring system is represented by a Table 142 to 282.

Embodiment 418: R¹ is CF₃, R² is CH₃, R⁴ is H, R⁵ is CH₃, X is S, R^{W} is CH₂CH₃, the index m is 2, R⁷ and R⁸- if present - are H, R⁹ is CH₂CH₂OCH₃, and the ring system is represented by a Table 142 to 282.

Embodiment 419: R¹ is CF₃, R² is H, R⁴ is H, R⁵ is H, X is O, R^{W} is CH₂CH₃, the index m is 2, R⁷ and R⁸- if present - are H, R⁹ is cyclobutylmethyl, and the ring system is represented by a Table 142 to 282.

Embodiment 420: R¹ is CF₃, R² is CH₃, R⁴ is H, R⁵ is H, X is O, R^{W} is CH₂CH₃, the index m is 2, R⁷ and R⁸- if present - are H, R⁹ is cyclobutylmethyl, and the ring system is represented by a Table 142 to 282.

Embodiment 421: R¹ is CF₃, R² is H, R⁴ is H, R⁵ is CH₃, X is O, R^{W} is CH₂CH₃, the index m is 2, R⁷ and R⁸- if present - are H, R⁹ is cyclobutylmethyl, and the ring system is represented by a Table 142 to 282.

Embodiment 422: R¹ is CF₃, R² is CH₃, R⁴ is H, R⁵ is CH₃, X is O, R^{W} is CH₂CH₃, the index m is 2, R⁷ and R⁸- if present - are H, R⁹ is cyclobutylmethyl, and the ring system is represented by a Table 142 to 282.

Embodiment 423: R¹ is CF₃, R² is H, R⁴ is H, R⁵ is H, X is S, R^{W} is CH₂CH₃, the index m is 2, R⁷ and R⁸- if present - are H, R⁹ is cyclobutylmethyl, and the ring system is represented by a Table 142 to 282.

Embodiment 424: R¹ is CF₃, R² is CH₃, R⁴ is H, R⁵ is H, X is S, R^{W} is CH₂CH₃, the index m is 2, R⁷ and R⁸- if present - are H, R⁹ is cyclobutylmethyl, and the ring system is represented by a Table 142 to 282.

Embodiment 425: R¹ is CF₃, R² is H, R⁴ is H, R⁵ is CH₃, X is S, R^{W} is CH₂CH₃, the index m is 2, R⁷ and R⁸- if present - are H, R⁹ is cyclobutylmethyl, and the ring system is represented by a Table 142 to 282.

Embodiment 426: R¹ is CF₃, R² is CH₃, R⁴ is H, R⁵ is CH₃, X is S, R^{W} is CH₂CH₃, the index m is 2, R⁷ and R⁸- if present - are H, R⁹ is cyclobutylmethyl, and the ring system is represented by a Table 142 to 282.

Embodiment 427: R¹ is CF₃, R² is H, R⁴ is H, R⁵ is H, X is O, R^{W} is CH₂CH₃, the index m is 2, R⁷ and R⁸- if present - are H, R⁹ is H, and the ring system is represented by a Table 142 to 282.

Embodiment 428: R¹ is CF₃, R² is CH₃, R⁴ is H, R⁵ is H, X is O, R^{W} is CH₂CH₃, the index m is 2, R⁷ and R⁸- if present - are H, R⁹ is H, and the ring system is represented by a Table 142 to 282.

Embodiment 429: R¹ is CF₃, R² is H, R⁴ is H, R⁵ is CH₃, X is O, R^{W} is CH₂CH₃, the index m is 2, R⁷ and R⁸- if present - are H, R⁹ is H, and the ring system is represented by a Table 142 to 282.

Embodiment 430: R¹ is CF₃, R² is CH₃, R⁴ is H, R⁵ is CH₃, X is O, R^{W} is CH₂CH₃, the index m is 2, R⁷ and R⁸- if present - are H, R⁹ is H, and the ring system is represented by a Table 142 to 282.

Embodiment 431: R¹ is CF₃, R² is H, R⁴ is H, R⁵ is H, X is S, R^{W} is CH₂CH₃, the index m is 2, R⁷ and R⁸- if present - are H, R⁹ is H, and the ring system is represented by a Table 142 to 282.

Embodiment 432: R¹ is CF₃, R² is CH₃, R⁴ is H, R⁵ is H, X is S, R^{W} is CH₂CH₃, the index m is 2, R⁷ and R⁸- if present - are H, R⁹ is H, and the ring system is represented by a Table 142 to 282.

Embodiment 433: R¹ is CF₃, R² is H, R⁴ is H, R⁵ is CH₃, X is S, R^{W} is CH₂CH₃, the index m is 2, R⁷ and R⁸- if present - are H, R⁹ is H, and the ring system is represented by a Table 142 to 282.

Embodiment 434: R¹ is CF₃, R² is CH₃, R⁴ is H, R⁵ is CH₃, X is S, R^{W} is CH₂CH₃, the index m is 2, R⁷ and R⁸- if present - are H, R⁹ is H, and the ring system is represented by a Table 142 to 282.

Embodiment 435: R¹ is CF₃; R^{W} is CH₂CH₃, the index m is 2, R⁷ and R⁸ - if present - are H, the ring system is represented by Table 283 and R², R³, X and R⁹ have a meaning as defined in a line A1 to A480 in Table A.

Embodiment 436: R¹ is CF₃; R^{W} is CH₂CH₃, the index m is 2, R⁷ and R⁸ - if present - are H, the ring system is represented by Table 284 and R², R³, X and R⁹ have a meaning as defined in a line A1 to A480 in Table A.

Embodiment 437: R¹ is CF₃; R^{W} is CH₂CH₃, the index m is 2, R⁷ and R⁸ - if present - are H, the ring system is represented by Table 285 and R², R³, X and R⁹ have a meaning as defined in a line A1 to A480 in Table A.

Embodiment 438: R¹ is CF₃; R^{W} is CH₂CH₃, the index m is 2, R⁷ and R⁸ - if present - are H, the ring system is represented by Table 286 and R², R³, X and R⁹ have a meaning as defined in a line A1 to A480 in Table A.

Embodiment 439: R¹ is CF₃; R^{W} is CH₂CH₃, the index m is 2, R⁷ and R⁸- if present - are H, the ring system is represented by Table 287 and R², R³, X and R⁹ have a meaning as defined in a line A1 to A480 in Table A.

Embodiment 440: R¹ is CF₃; R^{W} is CH₂CH₃, the index m is 2, R⁷ and R⁸- if present - are H, the ring system is represented by Table 288 and R², R³, X and R⁹ have a meaning as defined in a line A1 to A480 in Table A.

Embodiment 441: R¹ is CF₃; R^{W} is CH₂CH₃, the index m is 2, R⁷ and R⁸- if present - are H, the ring system is represented by Table 289 and R², R³, X and R⁹ have a meaning as defined in a line A1 to A480 in Table A.

Embodiment 442: R¹ is CF₃; R^{W} is CH₂CH₃, the index m is 2, R⁷ and R⁸- if present - are H, the ring system is represented by Table 290 and R², R³, X and R⁹ have a meaning as defined in a line A1 to A480 in Table A.

Embodiment 443: R¹ is CF₃, R^{W} is CH₂CH₃, the index m is 2, R⁷ and R⁸- if present - are H, the ring system is represented by Table 291 and R², R³, X and R⁹ have a meaning as defined in a line A1 to A480 in Table A.

Embodiment 444: R¹ is CF₃, R^{W} is CH₂CH₃, the index m is 2, R⁷ and R⁸- if present - are H, the ring system is represented by Table 292 and R², R³, X and R⁹ have a meaning as defined in a line A1 to A480 in Table A.

Embodiment 445: R¹ is CF₃, R^{W} is CH₂CH₃, the index m is 2, R⁷ and R⁸- if present - are H, the ring system is represented by Table 293 and R², R³, X and R⁹ have a meaning as defined in a line A1 to A480 in Table A.

Embodiment 446: R¹ is CF₃, R^{W} is CH₂CH₃, the index m is 2, R⁷ and R⁸- if present - are H, the ring system is represented by Table 294 and R², R³, X and R⁹ have a meaning as defined in a line A1 to A480 in Table A.

Embodiment 447: R¹ is CF₃, R^{W} is CH₂CH₃, the index m is 2, R⁷ and R⁸- if present - are H, the ring system is represented by Table 295 and R², R³, X and R⁹ have a meaning as defined in a line A1 to A480 in Table A.

Embodiment 448: R¹ is CF₃, R^{W} is CH₂CH₃, the index m is 2, R⁷ and R⁸- if present - are H, the ring system is represented by Table 296 and R², R³, X and R⁹ have a meaning as defined in a line A1 to A480 in Table A.

Embodiment 449: R¹ is CF₃, R^{W} is CH₂CH₃, the index m is 2, R⁷ and R⁸- if present - are H, the ring system is represented by Table 297 and R², R³, X and R⁹ have a meaning as defined in a line A1 to A480 in Table A.

Embodiment 450: R¹ is CF₃, R^{W} is CH₂CH₃, the index m is 2, R⁷ and R⁸- if present - are H, the ring system is represented by Table 298 and R², R³, X and R⁹ have a meaning as defined in a line A1 to A480 in Table A.

Embodiment 451: R¹ is CF₃, R^{W} is CH₂CH₃, the index m is 2, R⁷ and R⁸- if present - are H, the ring system is represented by Table 299 and R², R³, X and R⁹ have a meaning as defined in a line A1 to A480 in Table A.

Embodiment 452: R¹ is CF₃, R^{W} is CH₂CH₃, the index m is 2, R⁷ and R⁸- if present - are H, the ring system is represented by Table 300 and R², R³, X and R⁹ have a meaning as defined in a line A1 to A480 in Table A.

Embodiment 453: R¹ is CF₃, R^{W} is CH₂CH₃, the index m is 2, R⁷ and R⁸- if present - are H, the ring system is represented by Table 301 and R², R³, X and R⁹ have a meaning as defined in a line A1 to A480 in Table A.

Embodiment 454: R¹ is CF₃, R^{W} is CH₂CH₃, the index m is 2, R⁷ and R⁸- if present - are H, the ring system is represented by Table 302 and R², R³, X and R⁹ have a meaning as defined in a line A1 to A480 in Table A.

Embodiment 455: R¹ is CF₃, R^{W} is CH₂CH₃, the index m is 2, R⁷ and R⁸- if present - are H, the ring system is represented by Table 303 and R², R³, X and R⁹ have a meaning as defined in a line A1 to A480 in Table A.

Embodiment 456: R¹ is CF₃, R^{W} is CH₂CH₃, the index m is 2, R⁷ and R⁸- if present - are H, the ring system is represented by Table 304 and R², R³, X and R⁹ have a meaning as defined in a line A1 to A480 in Table A.

Embodiment 457: R¹ is CF₃, R^{W} is CH₂CH₃, the index m is 2, R⁷ and R⁸- if present - are H, the ring system is represented by Table 305 and R², R³, X and R⁹ have a meaning as defined in a line A1 to A480 in Table A.

Embodiment 458: R¹ is CF₃, R^{W} is CH₂CH₃, the index m is 2, R⁷ and R⁸- if present - are H, the ring system is represented by Table 306 and R², R³, X and R⁹ have a meaning as defined in a line A1 to A480 in Table A.

Embodiment 459: R¹ is CF₃, R^{W} is CH₂CH₃, the index m is 2, R⁷ and R⁸- if present - are H, the ring system is represented by Table 307 and R², R³, X and R⁹ have a meaning as defined in a line A1 to A480 in Table A.

Embodiment 460: R¹ is CF₃, R^{W} is CH₂CH₃, the index m is 2, R⁷ and R⁸- if present - are H, the ring system is represented by Table 308 and R², R³, X and R⁹ have a meaning as defined in a line A1 to A480 in Table A.

Embodiment 461: R¹ is CF₃, R^{W} is CH₂CH₃, the index m is 2, R⁷ and R⁸- if present - are H, the ring system is represented by Table 309 and R², R³, X and R⁹ have a meaning as defined in a line A1 to A480 in Table A.

Embodiment 462: R¹ is CF₃, R^{W} is CH₂CH₃, the index m is 2, R⁷ and R⁸- if present - are H, the ring system is represented by Table 310 and R², R³, X and R⁹ have a meaning as defined in a line A1 to A480 in Table A.

Embodiment 463: R¹ is CF₃, R^{W} is CH₂CH₃, the index m is 2, R⁷ and R⁸- if present - are H, the ring system is represented by Table 311 and R², R³, X and R⁹ have a meaning as defined in a line A1 to A480 in Table A.

Embodiment 464: R¹ is CF₃, R^{W} is CH₂CH₃, the index m is 2, R⁷ and R⁸- if present - are H, the ring system is represented by Table 312 and R², R³, X and R⁹ have a meaning as defined in a line A1 to A480 in Table A.

Embodiment 465: R¹ is CF₃, R^{W} is CH₂CH₃, the index m is 2, R⁷ and R⁸- if present - are H, the ring system is represented by Table 313 and R², R³, X and R⁹ have a meaning as defined in a line A1 to A480 in Table A.

Embodiment 466: R¹ is CF₃, R^{W} is CH₂CH₃, the index m is 2, R⁷ and R⁸- if present - are H, the ring system is represented by Table 314 and R², R³, X and R⁹ have a meaning as defined in a line A1 to A480 in Table A.

Embodiment 467: R¹ is CF₃, R^{W} is CH₂CH₃, the index m is 2, R⁷ and R⁸- if present - are H, the ring system is represented by Table 315 and R², R³, X and R⁹ have a meaning as defined in a line A1 to A480 in Table A.

Embodiment 468: R¹ is CF₃, R^{W} is CH₂CH₃, the index m is 2, R⁷ and R⁸- if present - are H, the ring system is represented by Table 316 and R², R³, X and R⁹ have a meaning as defined in a line A1 to A480 in Table A.

Embodiment 469: R¹ is CF₃, R^{W} is CH₂CH₃, the index m is 2, R⁷ and R⁸- if present - are H, the ring system is represented by Table 317 and R², R³, X and R⁹ have a meaning as defined in a line A1 to A480 in Table A.

Embodiment 470: R¹ is CF₃, R^{W} is CH₂CH₃, the index m is 2, R⁷ and R⁸- if present - are H, the ring system is represented by Table 318 and R², R³, X and R⁹ have a meaning as defined in a line A1 to A480 in Table A.

Embodiment 471: R¹ is CF₃, R^{W} is CH₂CH₃, the index m is 2, R⁷ and R⁸- if present - are H, the ring system is represented by Table 319 and R², R³, X and R⁹ have a meaning as defined in a line A1 to A480 in Table A.

Embodiment 472: R¹ is CF₃, R^{W} is CH₂CH₃, the index m is 2, R⁷ and R⁸- if present - are H, the ring system is represented by Table 320 and R², R³, X and R⁹ have a meaning as defined in a line A1 to A480 in Table A.

Embodiment 473: R¹ is CF₃, R^{W} is CH₂CH₃, the index m is 2, R⁷ and R⁸ - if present - are H, the ring system is represented by Table 321 and R², R³, X and R⁹ have a meaning as defined in a line A1 to A480 in Table A.

Embodiment 474: R¹ is CF₃, R^{W} is CH₂CH₃, the index m is 2, R⁷ and R⁸ - if present - are H, the ring system is represented by Table 322 and R², R³, X and R⁹ have a meaning as defined in a line A1 to A480 in Table A.

Embodiment 475: R¹ is CF₃, R^{W} is CH₂CH₃, the index m is 2, R⁷ and R⁸ - if present - are H, the ring system is represented by Table 323 and R², R³, X and R⁹ have a meaning as defined in a line A1 to A480 in Table A.

Embodiment 476: R¹ is CF₃, R^{W} is CH₂CH₃, the index m is 2, R⁷ and R⁸ - if present - are H, the ring system is represented by Table 324 and R², R³, X and R⁹ have a meaning as defined in a line A1 to A480 in Table A.

Embodiment 477: R¹ is CF₃, R^{W} is CH₂CH₃, the index m is 2, R⁷ and R⁸ - if present - are H, the ring system is represented by Table 325 and R², R³, X and R⁹ have a meaning as defined in a line A1 to A480 in Table A.

Embodiment 478: R¹ is CF₃, R^{W} is CH₂CH₃, the index m is 2, R⁷ and R⁸ - if present - are H, the ring system is represented by Table 326 and R², R³, X and R⁹ have a meaning as defined in a line A1 to A480 in Table A.

Embodiment 479: R¹ is CF₃, R^{W} is CH₂CH₃, the index m is 2, R⁷ and R⁸ - if present - are H, the ring system is represented by Table 327 and R², R³, X and R⁹ have a meaning as defined in a line A1 to A480 in Table A.

Embodiment 480: R¹ is CF₃, R^{W} is CH₂CH₃, the index m is 2, R⁷ and R⁸ - if present - are H, the ring system is represented by Table 328 and R², R³, X and R⁹ have a meaning as defined in a line A1 to A480 in Table A.

Embodiment 481: R¹ is CF₃, R^{W} is CH₂CH₃, the index m is 2, R⁷ and R⁸ - if present - are H, the ring system is represented by Table 329 and R², R³, X and R⁹ have a meaning as defined in a line A1 to A480 in Table A.

Embodiment 482: R¹ is CF₃, R^{W} is CH₂CH₃, the index m is 2, R⁷ and R⁸ - if present - are H, the ring system is represented by Table 330 and R², R³, X and R⁹ have a meaning as defined in a line A1 to A480 in Table A.

Embodiment 483: R¹ is CF₃, R^{W} is CH₂CH₃, the index m is 2, R⁷ and R⁸ - if present - are H, the ring system is represented by Table 331 and R², R³, X and R⁹ have a meaning as defined in a line A1 to A480 in Table A.

Embodiment 484: R¹ is CF₃, R^{W} is CH₂CH₃, the index m is 2, R⁷ and R⁸ - if present - are H, the ring system is represented by Table 332 and R², R³, X and R⁹ have a meaning as defined in a line A1 to A480 in Table A.

Embodiment 485: R¹ is CF₃, R^{W} is CH₂CH₃, the index m is 2, R⁷ and R⁸ - if present - are H, the ring system is represented by Table 333 and R², R³, X and R⁹ have a meaning as defined in a line A1 to A480 in Table A.

Embodiment 486: R¹ is CF₃, R^{W} is CH₂CH₃, the index m is 2, R⁷ and R⁸ - if present - are H, the ring system is represented by Table 334 and R², R³, X and R⁹ have a meaning as defined in a line A1 to A480 in Table A.

Embodiment 487: R¹ is CF₃, R^{W} is CH₂CH₃, the index m is 2, R⁷ and R⁸ - if present - are H, the ring system is represented by Table 335 and R², R³, X and R⁹ have a meaning as defined in a line A1 to A480 in Table A.

Embodiment 488: R¹ is CF₃, R^{W} is CH₂CH₃, the index m is 2, R⁷ and R⁸ - if present - are H, the ring system is represented by Table 336 and R², R³, X and R⁹ have a meaning as defined in a line A1 to A480 in Table A.

Embodiment 489: R¹ is CF₃, R^{W} is CH₂CH₃, the index m is 2, R⁷ and R⁸ - if present - are H, the ring system is represented by Table 338 and R², R³, X and R⁹ have a meaning as defined in a line A1 to A480 in Table A.

Embodiment 490: R¹ is CF₃, R^{W} is CH₂CH₃, the index m is 2, R⁷ and R⁸ - if present - are H, the ring system is represented by Table 339 and R², R³, X and R⁹ have a meaning as defined in a line A1 to A480 in Table A.

Embodiment 491: R¹ is CF₃, R^{W} is CH₂CH₃, the index m is 2, R⁷ and R⁸ - if present - are H, the ring system is represented by Table 340 and R², R³, X and R⁹ have a meaning as defined in a line A1 to A480 in Table A.

Embodiment 492: R¹ is CF₃, R^{W} is CH₂CH₃, the index m is 2, R⁷ and R⁸ - if present - are H, the ring system is represented by Table 341 and R², R³, X and R⁹ have a meaning as defined in a line A1 to A480 in Table A.

Embodiment 493: R¹ is CF₃, R^{W} is CH₂CH₃, the index m is 2, R⁷ and R⁸ - if present - are H, the ring system is represented by Table 342 and R², R³, X and R⁹ have a meaning as defined in a line A1 to A480 in Table A.

Embodiment 494: R¹ is CF₃, R^{W} is CH₂CH₃, the index m is 2, R⁷ and R⁸ - if present - are H, the ring system is represented by Table 343 and R², R³, X and R⁹ have a meaning as defined in a line A1 to A480 in Table A.

Embodiment 495: R¹ is CF₃, R^{W} is CH₂CH₃, the index m is 2, R⁷ and R⁸ - if present - are H, the ring system is represented by Table 344 and R², R³, X and R⁹ have a meaning as defined in a line A1 to A480 in Table A.

Embodiment 496: R¹ is CF₃; R^{W} is CH₂CH₃, the index m is 2, R⁷ and R⁸ - if present - are H, the ring system is represented by Table 345 and R², R³, X and R⁹ have a meaning as defined in a line A1 to A480 in Table A.

Embodiment 497: R¹ is CF₃, R^{W} is CH₂CH₃, the index m is 2, R⁷ and R⁸ - if present - are H, the ring system is represented by Table 346 and R², R³, X and R⁹ have a meaning as defined in a line A1 to A480 in Table A.

Embodiment 498: R¹ is CF₃, R^{W} is CH₂CH₃, the index m is 2, R⁷ and R⁸ - if present - are H, the ring system is represented by Table 347 and R², R³, X and R⁹ have a meaning as defined in a line A1 to A480 in Table A.

Embodiment 499: R¹ is CF₃, R^{W} is CH₂CH₃, the index m is 2, R⁷ and R⁸ - if present - are H, the ring system is represented by Table 348 and R², R³, X and R⁹ have a meaning as defined in a line A1 to A480 in Table A.

Embodiment 500: R¹ is CF₃, R^{W} is CH₂CH₃, the index m is 2, R⁷ and R⁸ - if present - are H, the ring system is represented by Table 349 and R², R³, X and R⁹ have a meaning as defined in a line A1 to A480 in Table A.

Embodiment 501: R¹ is CF₃, R^{W} is CH₂CH₃, the index m is 2, R⁷ and R⁸ - if present - are H, the ring system is represented by Table 350 and R², R³, X and R⁹ have a meaning as defined in a line A1 to A480 in Table A.

Embodiment 502: R¹ is CF₃, R^{W} is CH₂CH₃, the index m is 2, R⁷ and R⁸ - if present - are H, the ring system is represented by Table 351 and R², R³, X and R⁹ have a meaning as defined in a line A1 to A480 in Table A.

Embodiment 503: R¹ is CF₃, R^{W} is CH₂CH₃, the index m is 2, R⁷ and R⁸ - if present - are H, the ring system is represented by Table 352 and R², R³, X and R⁹ have a meaning as defined in a line A1 to A480 in Table A.

Embodiment 504: R¹ is CF₃, R^{W} is CH₂CH₃, the index m is 2, R⁷ and R⁸ - if present - are H, the ring system is represented by Table 353 and R², R³, X and R⁹ have a meaning as defined in a line A1 to A480 in Table A.

Embodiment 505: R¹ is CF₃, R^{W} is CH₂CH₃, the index m is 2, R⁷ and R⁸ - if present - are H, the ring system is represented by Table 354 and R², R³, X and R⁹ have a meaning as defined in a line A1 to A480 in Table A.

Embodiment 506: R¹ is CF₃, R^{W} is CH₂CH₃, the index m is 2, R⁷ and R⁸ - if present - are H, the ring system is represented by Table 355 and R², R³, X and R⁹ have a meaning as defined in a line A1 to A480 in Table A.

Embodiment 507: R¹ is CF₃, R^{W} is CH₂CH₃, the index m is 2, R⁷ and R⁸ - if present - are H, the ring system is represented by Table 356 and R², R³, X and R⁹ have a meaning as defined in a line A1 to A480 in Table A.

Embodiment 508: R¹ is CF₃, R^{W} is CH₂CH₃, the index m is 2, R⁷ and R⁸ - if present - are H, the ring system is represented by Table 357 and R², R³, X and R⁹ have a meaning as defined in a line A1 to A480 in Table A.

Embodiment 509: R¹ is CF₃, R^{W} is CH₂CH₃, the index m is 2, R⁷ and R⁸ - if present - are H, the ring system is represented by Table 358 and R², R³, X and R⁹ have a meaning as defined in a line A1 to A480 in Table A.

Embodiment 510: R¹ is CF₃, R^{W} is CH₂CH₃, the index m is 2, R⁷ and R⁸ - if present - are H, the ring system is represented by Table 359 and R², R³, X and R⁹ have a meaning as defined in a line A1 to A480 in Table A.

Embodiment 511: R¹ is CF₃, R^{W} is CH₂CH₃, the index m is 2, R⁷ and R⁸ - if present - are H, the ring system is represented by Table 360 and R², R³, X and R⁹ have a meaning as defined in a line A1 to A480 in Table A.

Embodiment 512: R¹ is CF₃, R^{W} is CH₂CH₃, the index m is 2, R⁷ and R⁸ - if present - are H, the ring system is represented by Table 361 and R², R³, X and R⁹ have a meaning as defined in a line A1 to A480 in Table A.

Embodiment 513: R¹ is CF₃, R^{W} is CH₂CH₃, the index m is 2, R⁷ and R⁸ - if present - are H, the ring system is represented by Table 362 and R², R³, X and R⁹ have a meaning as defined in a line A1 to A480 in Table A.

Embodiment 514: R¹ is CF₃, R^{W} is CH₂CH₃, the index m is 2, R⁷ and R⁸ - if present - are H, the ring system is represented by Table 363 and R², R³, X and R⁹ have a meaning as defined in a line A1 to A480 in Table A.

Embodiment 515: R¹ is CF₃, R^{W} is CH₂CH₃, the index m is 2, R⁷ and R⁸ - if present - are H, the ring system is represented by Table 364 and R², R³, X and R⁹ have a meaning as defined in a line A1 to A480 in Table A.

Embodiment 516: R¹ is CF₃, R^{W} is CH₂CH₃, the index m is 2, R⁷ and R⁸ - if present - are H, the ring system is represented by Table 365 and R², R³, X and R⁹ have a meaning as defined in a line A1 to A480 in Table A.

Embodiment 517: R¹ is CF₃, R^{W} is CH₂CH₃, the index m is 2, R⁷ and R⁸ - if present - are H, the ring system is represented by Table 366 and R², R³, X and R⁹ have a meaning as defined in a line A1 to A480 in Table A.

Embodiment 518: R¹ is CF₃, R^{W} is CH₂CH₃, the index m is 2, R⁷ and R⁸ - if present - are H, the ring system is represented by Table 367 and R², R³, X and R⁹ have a meaning as defined in a line A1 to A480 in Table A.

Embodiment 519: R¹ is CF₃, R^{W} is CH₂CH₃, the index m is 2, R⁷ and R⁸ - if present - are H, the ring system is represented by Table 368 and R², R³, X and R⁹ have a meaning as defined in a line A1 to A480 in Table A.

Embodiment 520: R¹ is CF₃, R^{W} is CH₂CH₃, the index m is 2, R⁷ and R⁸ - if present - are H, the ring system is represented by Table 369 and R², R³, X and R⁹ have a meaning as defined in a line A1 to A480 in Table A.

Embodiment 521: R¹ is CF₃, R^{W} is CH₂CH₃, the index m is 2, R⁷ and R⁸ - if present - are H, the ring system is represented by Table 370 and R², R³, X and R⁹ have a meaning as defined in a line A1 to A480 in Table A.

Embodiment 522: R¹ is CF₃, R^{W} is CH₂CH₃, the index m is 2, R⁷ and R⁸ - if present - are H, the ring system is represented by Table 371 and R², R³, X and R⁹ have a meaning as defined in a line A1 to A480 in Table A.

Embodiment 523: R¹ is CF₃, R^{W} is CH₂CH₃, the index m is 2, R⁷ and R⁸ - if present - are H, the ring system is represented by Table 372 and R², R³, X and R⁹ have a meaning as defined in a line A1 to A480 in Table A.

Embodiment 524: R¹ is CF₃, R^{W} is CH₂CH₃, the index m is 2, R⁷ and R⁸ - if present - are H, the ring system is represented by Table 373 and R², R³, X and R⁹ have a meaning as defined in a line A1 to A480 in Table A.

Embodiment 525: R¹ is CF₃, R^{W} is CH₂CH₃, the index m is 2, R⁷ and R⁸ - if present - are H, the ring system is represented by Table 374 and R², R³, X and R⁹ have a meaning as defined in a line A1 to A480 in Table A.

Embodiment 526: R¹ is CF₃, R^{W} is CH₂CH₃, the index m is 2, R⁷ and R⁸ - if present - are H, the ring system is represented by Table 375 and R², R³, X and R⁹ have a meaning as defined in a line A1 to A480 in Table A.

Embodiment 527: R¹ is CF₃, R^{W} is CH₂CH₃, the index m is 2, R⁷ and R⁸ - if present - are H, the ring system is represented by Table 376 and R², R³, X and R⁹ have a meaning as defined in a line A1 to A480 in Table A.

Embodiment 528: R¹ is CF₃, R^{W} is CH₂CH₃, the index m is 2, R⁷ and R⁸ - if present - are H, the ring system is represented by Table 377 and R², R³, X and R⁹ have a meaning as defined in a line A1 to A480 in Table A.

Embodiment 529: R¹ is CF₃, R^{W} is CH₂CH₃, the index m is 2, R⁷ and R⁸ - if present - are H, the ring system is represented by Table 378 and R², R³, X and R⁹ have a meaning as defined in a line A1 to A480 in Table A.

Embodiment 530: R¹ is CF₃, R^{W} is CH₂CH₃, the index m is 2, R⁷ and R⁸ - if present - are H, the ring system is represented by Table 379 and R², R³, X and R⁹ have a meaning as defined in a line A1 to A480 in Table A.

Embodiment 531: R¹ is CF₃, R^{W} is CH₂CH₃, the index m is 2, R⁷ and R⁸ - if present - are H, the ring system is represented by Table 380 and R², R³, X and R⁹ have a meaning as defined in a line A1 to A480 in Table A.

Embodiment 532: R¹ is CF₃, R^{W} is CH₂CH₃, the index m is 2, R⁷ and R⁸ - if present - are H, the ring system is represented by Table 381 and R², R³, X and R⁹ have a meaning as defined in a line A1 to A480 in Table A.

Embodiment 533: R¹ is CF₃, R^{W} is CH₂CH₃, the index m is 2, R⁷ and R⁸ - if present - are H, the ring system is represented by Table 382 and R², R³, X and R⁹ have a meaning as defined in a line A1 to A480 in Table A.

Embodiment 534: R¹ is CF₃, R^{W} is CH₂CH₃, the index m is 2, R⁷ and R⁸ - if present - are H, the ring system is represented by Table 383 and R², R³, X and R⁹ have a meaning as defined in a line A1 to A480 in Table A.

Embodiment 535: R¹ is CF₃, R^{W} is CH₂CH₃, the index m is 2, R⁷ and R⁸ - if present - are H, the ring system is represented by Table 384 and R², R³, X and R⁹ have a meaning as defined in a line A1 to A480 in Table A.

Embodiment 536: R¹ is CF₃, R^{W} is CH₂CH₃, the index m is 2, R⁷ and R⁸ - if present - are H, the ring system is represented by Table 385 and R², R³, X and R⁹ have a meaning as defined in a line A1 to A480 in Table A.

Embodiment 537: R¹ is CF₃, R^{W} is CH₂CH₃, the index m is 2, R⁷ and R⁸ - if present - are H, the ring system is represented by Table 386 and R², R³, X and R⁹ have a meaning as defined in a line A1 to A480 in Table A.

Embodiment 538: R¹ is CF₃, R^{W} is CH₂CH₃, the index m is 2, R⁷ and R⁸ - if present - are H, the ring system is represented by Table 387 and R², R³, X and R⁹ have a meaning as defined in a line A1 to A480 in Table A.

Embodiment 539: R¹ is CF₃, R^{W} is CH₂CH₃, the index m is 2, R⁷ and R⁸ - if present - are H, the ring system is represented by Table 388 and R², R³, X and R⁹ have a meaning as defined in a line A1 to A480 in Table A.

Embodiment 540: R¹ is CF₃, R^{W} is CH₂CH₃, the index m is 2, R⁷ and R⁸ - if present - are H, the ring system is represented by Table 389 and R², R³, X and R⁹ have a meaning as defined in a line A1 to A480 in Table A.

Embodiment 541: R¹ is CF₃, R^{W} is CH₂CH₃, the index m is 2, R⁷ and R⁸ - if present - are H, the ring system is represented by Table 390 and R², R³, X and R⁹ have a meaning as defined in a line A1 to A480 in Table A.

Embodiment 542: R¹ is CF₃, R^{W} is CH₂CH₃, the index m is 2, R⁷ and R⁸ - if present - are H, the ring system is represented by Table 391 and R², R³, X and R⁹ have a meaning as defined in a line A1 to A480 in Table A.

Embodiment 543: R¹ is CF₃, R^{W} is CH₂CH₃, the index m is 2, R⁷ and R⁸ - if present - are H, the ring system is represented by Table 392 and R², R³, X and R⁹ have a meaning as defined in a line A1 to A480 in Table A.

Embodiment 544: R¹ is CF₃, R^{W} is CH₂CH₃, the index m is 2, R⁷ and R⁸ - if present - are H, the ring system is represented by Table 393 and R², R³, X and R⁹ have a meaning as defined in a line A1 to A480 in Table A.

Embodiment 545: R¹ is CF₃, R^{W} is CH₂CH₃, the index m is 2, R⁷ and R⁸ - if present - are H, the ring system is represented by Table 394 and R², R³, X and R⁹ have a meaning as defined in a line A1 to A480 in Table A.

Embodiment 546: R¹ is CF₃, R^{W} is CH₂CH₃, the index m is 2, R⁷ and R⁸ - if present - are H, the ring system is represented by Table 395 and R², R³, X and R⁹ have a meaning as defined in a line A1 to A480 in Table A.

Embodiment 547: R¹ is CF₃, R^{W} is CH₂CH₃, the index m is 2, R⁷ and R⁸ - if present - are H, the ring system is represented by Table 396 and R², R³, X and R⁹ have a meaning as defined in a line A1 to A480 in Table A.

Embodiment 548: R¹ is CF₃, R^{W} is CH₂CH₃, the index m is 2, R⁷ and R⁸ - if present - are H, the ring system is represented by Table 397 and R², R³, X and R⁹ have a meaning as defined in a line A1 to A480 in Table A.

Embodiment 549: R¹ is CF₃, R^{W} is CH₂CH₃, the index m is 2, R⁷ and R⁸ - if present - are H, the ring system is represented by Table 398 and R², R³, X and R⁹ have a meaning as defined in a line A1 to A480 in Table A.

Embodiment 550: R¹ is CF₃, R^{W} is CH₂CH₃, the index m is 2, R⁷ and R⁸ - if present - are H, the ring system is represented by Table 399 and R², R³, X and R⁹ have a meaning as defined in a line A1 to A480 in Table A.

Embodiment 551: R¹ is CF₃, R^{W} is CH₂CH₃, the index m is 2, R⁷ and R⁸ - if present - are H, the ring system is represented by Table 400 and R², R³, X and R⁹ have a meaning as defined in a line A1 to A480 in Table A.

Embodiment 552: R¹ is CF₃, R^{W} is CH₂CH₃, the index m is 2, R⁷ and R⁸ - if present - are H, the ring system is represented by Table 401 and R², R³, X and R⁹ have a meaning as defined in a line A1 to A480 in Table A.

Embodiment 553: R¹ is CF₃, R^{W} is CH₂CH₃, the index m is 2, R⁷ and R⁸ - if present - are H, the ring system is represented by Table 402 and R², R³, X and R⁹ have a meaning as defined in a line A1 to A480 in Table A.

Embodiment 554: R¹ is CF₃, R^{W} is CH₂CH₃, the index m is 2, R⁷ and R⁸ - if present - are H, the ring system is represented by Table 403 and R², R³, X and R⁹ have a meaning as defined in a line A1 to A480 in Table A.

Embodiment 555: R¹ is CF₃, R^{W} is CH₂CH₃, the index m is 2, R⁷ and R⁸ - if present - are H, the ring system is represented by Table 404 and R², R³, X and R⁹ have a meaning as defined in a line A1 to A480 in Table A.

Embodiment 556: R¹ is CF₃, R^{W} is CH₂CH₃, the index m is 2, R⁷ and R⁸ - if present - are H, the ring system is represented by Table 405 and R², R³, X and R⁹ have a meaning as defined in a line A1 to A480 in Table A.

Embodiment 557: R¹ is CF₃, R^{W} is CH₂CH₃, the index m is 2, R⁷ and R⁸ - if present - are H, the ring system is represented by Table 406 and R², R³, X and R⁹ have a meaning as defined in a line A1 to A480 in Table A.

Embodiment 558: R¹ is CF₃, R^{W} is CH₂CH₃, the index m is 2, R⁷ and R⁸ - if present - are H, the ring system is represented by Table 407 and R², R³, X and R⁹ have a meaning as defined in a line A1 to A480 in Table A.

Embodiment 559: R¹ is CF₃, R^{W} is CH₂CH₃, the index m is 2, R⁷ and R⁸ - if present - are H, the ring system is represented by Table 408 and R², R³, X and R⁹ have a meaning as defined in a line A1 to A480 in Table A.

Embodiment 560: R¹ is CF₃, R^{W} is CH₂CH₃, the index m is 2, R⁷ and R⁸ - if present - are H, the ring system is represented by Table 409 and R², R³, X and R⁹ have a meaning as defined in a line A1 to A480 in Table A.

Embodiment 561: R¹ is CF₃, R^{W} is CH₂CH₃, the index m is 2, R⁷ and R⁸ - if present - are H, the ring system is represented by Table 410 and R², R³, X and R⁹ have a meaning as defined in a line A1 to A480 in Table A.

Embodiment 562: R¹ is CF₃, R^{W} is CH₂CH₃, the index m is 2, R⁷ and R⁸ - if present - are H, the ring system is represented by Table 411 and R², R³, X and R⁹ have a meaning as defined in a line A1 to A480 in Table A.

Embodiment 563: R¹ is CF₃, R^{W} is CH₂CH₃, the index m is 2, R⁷ and R⁸ - if present - are H, the ring system is represented by Table 412 and R², R³, X and R⁹ have a meaning as defined in a line A1 to A480 in Table A.

Embodiment 564: R¹ is CF₃, R^{W} is CH₂CH₃, the index m is 2, R⁷ and R⁸ - if present - are H, the ring system is represented by Table 413 and R², R³, X and R⁹ have a meaning as defined in a line A1 to A480 in Table A.

Embodiment 565: R¹ is CF₃, R^{W} is CH₂CH₃, the index m is 2, R⁷ and R⁸ - if present - are H, the ring system is represented by Table 414 and R², R³, X and R⁹ have a meaning as defined in a line A1 to A480 in Table A.

Embodiment 566: R¹ is CF₃, R^{W} is CH₂CH₃, the index m is 2, R⁷ and R⁸ - if present - are H, the ring system is represented by Table 415 and R², R³, X and R⁹ have a meaning as defined in a line A1 to A480 in Table A.

Embodiment 567: R¹ is CF₃, R^{W} is CH₂CH₃, the index m is 2, R⁷ and R⁸ - if present - are H, the ring system is represented by Table 416 and R², R³, X and R⁹ have a meaning as defined in a line A1 to A480 in Table A.

Embodiment 568: R¹ is CF₃, R^{W} is CH₂CH₃, the index m is 2, R⁷ and R⁸ - if present - are H, the ring system is represented by Table 417 and R², R³, X and R⁹ have a meaning as defined in a line A1 to A480 in Table A.

Embodiment 569: R¹ is CF₃, R^{W} is CH₂CH₃, the index m is 2, R⁷ and R⁸ - if present - are H, the ring system is represented by Table 418 and R², R³, X and R⁹ have a meaning as defined in a line A1 to A480 in Table A.

Embodiment 570: R¹ is CF₃, R^{W} is CH₂CH₃, the index m is 2, R⁷ and R⁸ - if present - are H, the ring system is represented by Table 419 and R², R³, X and R⁹ have a meaning as defined in a line A1 to A480 in Table A.

Embodiment 571: R¹ is CF₃, R^{W} is CH₂CH₃, the index m is 2, R⁷ and R⁸ - if present - are H, the ring system is represented by Table 420 and R², R³, X and R⁹ have a meaning as defined in a line A1 to A480 in Table A.

Embodiment 572: R¹ is CF₃, R^{W} is CH₂CH₃, the index m is 2, R⁷ and R⁸ - if present - are H, the ring system is represented by Table 421 and R², R³, X and R⁹ have a meaning as defined in a line A1 to A480 in Table A.

Embodiment 573: R¹ is CF₃, R^{W} is CH₂CH₃, the index m is 2, R⁷ and R⁸ - if present - are H, the ring system is represented by Table 422 and R², R³, X and R⁹ have a meaning as defined in a line A1 to A480 in Table A.

Embodiment 574: R¹ is CF₃, R^{W} is CH₂CH₃, the index m is 2, R⁷ and R⁸ - if present - are H, the ring system is represented by Table 423 and R², R³, X and R⁹ have a meaning as defined in a line A1 to A480 in Table A.

In a preferred embodiment, the compound of formula (I) is a compound of formula (!.A) or (I.B), wherein the bicyclic fused ring D is a ring selected from D1 to D51, and wherein
- R¹: is C₁-C₃-haloalkyl;
- R²: is H, or C₁-C₃-alkyl;
- R³: is C₁-C₃-alkyl, C₂-C₃-alkenyl, C₂-C₃-alkynyl, C₁-C₃-alkoxy-C₁-C₃-alkyl, C₃-C₅-cycloalkyl, phenyl, benzyl, C₃-C₆-cycloalkyl-C₁-C₃-alkyl, which groups are unsubstituted or halogenated;
- R⁴: is C₁-C₃-alkyl or H;
- R⁵: is C₁-C₃-alkyl;
- R⁶: is H, or C₁-C₃-alkl;
- R⁷: is H, or C₁-C₃-alkyl;
- R⁸: is H, or C₁-C₃-alkyl;
- R⁹: is C₁-C₃-alkyl, C₁-C₃-haloalkyl, C₁-C₃-alkoxy, C₁-C₃-haloalkoxy, halogen; or C₃-C₄-cycloalkyl, or C₁-C₃-alkyl, which groups are substituted with CN, preferably 1-cyanocyclopropyl or 1-cyanoisopropyl;
- X: is O or S;
- m: is 0 or 2;
- R^{W}: is CH₂CH₃

In another preferred embodiment, the compound of formula (I) is a compound of formula (LA), (I.B), or (I.C) wherein the bicyclic fused ring D is a ring selected from D1 to D51, and wherein
- R¹: is C₁-C₃-haloalkyl;
- R²: is H, or C₁-C₃-alkyl;
- R³: is C₁-C₃-alkyl, C₂-C₃-alkenyl, C₂-C₃-alkynyl, C₁-C₃-alkoxy-C₁-C₃-alkyl, C₃-C₅-cycloalkyl, phenyl, benzyl, C₃-C₆-cycloalkyl-C₁-C₃-alkyl, which groups are unsubstituted or halogenated;
- R⁴: is C₁-C₃-alkyl or H;
- R⁵: is C₁-C₃-alkyl;
- R⁶: is H, or C₁-C₃-alkl;
- R⁷: is H, or C₁-C₃-alkyl;
- R⁸: is H, or C₁-C₃-alkyl;
- R⁹: is C₁-C₃-alkyl, C₁-C₃-haloalkyl, C₁-C₃-alkoxy, C₁-C₃-haloalkoxy, halogen;
- X: is O or S;
- m: is 0 or 2;
- R^{W}: is CH₂CH₃

In another preferred embodiment, the compound of formula (I) is a compound of formula (LA), wherein the bicyclic fused ring D is a ring selected from D1 to D51, and wherein
- R¹: is C₁-C₃-haloalkyl;
- R²: is H, or C₁-C₃-alkyl;
- R³: is C₁-C₃-alkyl, C₂-C₃-alkenyl, C₂-C₃-alkynyl, C₁-C₃-alkoxy-C₁-C₃-alkyl, C₃-C₅-cycloalkyl, phenyl, benzyl, C₃-C₆-cycloalkyl-C₁-C₃-alkyl, which groups are unsubstituted or halogenated;
- R⁶: is H, or C₁-C₃-alkl;
- R⁷: is H, or C₁-C₃-alkyl;
- R⁸: is H, or C₁-C₃-alkyl;
- R⁹: is C₁-C₃-alkyl, C₁-C₃-haloalkyl, C₁-C₃-alkoxy, C₁-C₃-haloalkoxy, halogen;
- X: is O or S;
- m: is 0 or 2;
- R^{W}: is CH₂CH₃

In another preferred embodiment, the compound of formula (I) is a compound of formula (LA), wherein the bicyclic fused ring D is a ring selected from D1, D3, D8, and D51, and wherein
- R¹: is C₁-C₃-haloalkyl;
- R²: is H;
- R³: is C₁-C₃-alkyl, C₂-C₃-alkenyl, C₂-C₃-alkynyl, C₃-C₅-cycloalkyl, C₃-C₆-cycloalkyl-C₁-C₃-alkyl, which groups are unsubstituted or halogenated;
- R⁶: is H, or C₁-C₃-alkl;
- R⁷: is H, or C₁-C₃-alkyl;
- R⁸: is H, or C₁-C₃-alkyl;
- R⁹: is C₁-C₃-alkyl, C₁-C₃-alkoxy, C₃-C₆-cycloalkyl, which groups are unsubstituted or substituted with halogen or CN;
- X: is O or S;
- m: is 0 or 2;
- R^{W}: is CH₂CH₃

In another preferred embodiment, the compound of formula (I) is a compound of formula (LA), wherein the bicyclic fused ring D is a ring selected from D1, D3, D8, and D51, and wherein
- R¹: is C₁-C₃-haloalkyl;
- R²: is H;
- R³: is C₁-C₃-alkyl, C₂-C₃-alkenyl, C₂-C₃-alkynyl, C₃-C₅-cycloalkyl, C₃-C₆-cycloalkyl-C₁-C₃-alkyl, which groups are unsubstituted or halogenated;
- R⁶: is H, or C₁-C₃-alkl;
- R⁷: is H, or C₁-C₃-alkyl;
- R⁸: is H, or C₁-C₃-alkyl;
- R⁹: is C₁-C₃-alkyl, which is unsubstituted or substituted with halogen or CN;
- X: is O or S;
- m: is 0 or 2;
- R^{W}: is CH₂CH₃

In another preferred embodiment, the compound of formula (I) is a compound of formula (LA), wherein the bicyclic fused ring D is a ring selected from D1, D3, D8, and D51, and wherein
- R¹: is C₁-C₃-haloalkyl;
- R²: is H;
- R³: is C₁-C₃-alkyl, C₂-C₃-alkenyl, C₂-C₃-alkynyl, C₃-C₅-cycloalkyl, C₃-C₆-cycloalkyl-C₁-C₃-alkyl, which groups are unsubstituted or halogenated;
- R⁶: is H, or C₁-C₃-alkl;
- R⁷: is H, or C₁-C₃-alkyl;
- R⁸: is H, or C₁-C₃-alkyl;
- R⁹: is C₁-C₃-alkyl, C₁-C₃-haloalkyl, C₁-C₃-alkoxy, C₁-C₃-haloalkoxy, halogen;
- X: is O or S;
- m: is 0 or 2;
- R^{W}: is CH₂CH₃

In another preferred embodiment, the compound of formula (I) is a compound of formula (LA), wherein the bicyclic fused ring D is a ring D1, and wherein
- R¹: is C₁-C₃-haloalkyl;
- R²: is H;
- R³: is C₁-C₃-alkyl, C₂-C₃-alkenyl, C₂-C₃-alkynyl, C₃-C₅-cycloalkyl, C₃-C₆-cycloalkyl-C₁-C₃-alkyl, which groups are unsubstituted or halogenated;
- R⁶: is H, or C₁-C₃-alkl;
- R⁷: is H, or C₁-C₃-alkyl;
- R⁸: is H, or C₁-C₃-alkyl;
- R⁹: is C₁-C₃-alkyl, or C₁-C₃-haloalkyl;
- X: is O or S;
- m: is 0 or 2;
- R^{W}: is CH₂CH₃

### Mixtures

The present invention also relates to a mixture of at least one compound of the invention with at least one mixing partner as defined herein. Preferred are binary mixtures of one compound of the invention as component I with one mixing partner as defined herein as component II. Preferred weight ratios for such binary mixtures are from 5000:1 to 1:5000, preferably from 1000:1 to 1:1000, more preferably from 100:1 to 1:100, particularly from 10:1 to 1:10. In such binary mixtures, components I and II may be used in equal amounts, or an excess of component I, or an excess of component II may be used.

Mixing partners can be selected from pesticides, in particular insecticides, nematicides, and acaricides, fungicides, herbicides, plant growth regulators, fertilizers. Preferred mixing partners are insecticides, nematicides and fungicides.

### Formulations

The invention also relates to agrochemical compositions comprising an auxiliary and at least one compound of the present invention or a mixture thereof.

An agrochemical composition comprises a pesticidally effective amount of a compound of the present invention or a mixture thereof. The term "pesticidally effective amount" is defined below.

The compounds of the present invention or the mixtures thereof can be converted into customary types of agro-chemical compositions, e. g. solutions, emulsions, suspensions, dusts, powders, pastes, granules, pressings, capsules, and mixtures thereof. Examples for composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further compositions types are defined in the "Catalogue of pesticide formulation types and international coding system", Technical Mono-graph No. 2, 6th Ed. May 2008, CropLife International.

The compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents. Suitable solid carriers or fillers are mineral earths.

Suitable surfactants are surface-active compounds, e.g. anionic, cationic, nonionic, and amphoteric surfactants, block polymers, polyelectrolytes. Such surfactants can be used as emusifier, dispersant, solubilizer, wetter, penetration enhancer, protective colloid, or adjuvant. Surfactants are listed in McCutcheon's, Vol.1: Emulsifiers & Detergents, McCutcheon's Directories, Glen Rock, USA, 2008 (International or North American Ed.). Suitable anionic surfactants are alkali, alkaline earth, or ammonium salts of sulfonates, sulfates, phosphates, carboxylates. Suitable nonionic surfactants are alkoxylates, N-subsituted fatty acid amides, amine oxides, esters, sugar-based surfactants, polymeric surfactants. Suitable cationic surfactants are qua-ternary surfactants. The agrochemical compositions generally comprise between 0.01 and 95%, preferably between 0.1 and 90%, and most preferably between 0.5 and 75%, by weight of active substance. The active substances are employed in a purity of from 90% to 100%, preferably from 95% to 100%.

Various types of oils, wetters, adjuvants, or fertilizer may be added to the active substances or the compositions comprising them as premix or, if appropriate not until immediately prior to use (tank mix). These agents can be admixed with the compositions according to the invention in a weight ratio of 1:100 to 100:1.

The user applies the composition according to the invention usually from a predosage device, a knapsack sprayer, a spray tank, a spray plane, or an irrigation system. Usually, the agro-chemical composition is made up with water, buffer, and/or further auxiliaries to the desired application concentration and the ready-to-use spray liquor or the agrochemical composition according to the invention is thus obtained. Usually, 20 to 2000 liters, of the ready-to-use spray liquor are applied per hectare of agricultural useful area.

The compounds of formula (I) are suitable for use in protecting crops, plants, plant propagation materials, e.g. seeds, or soil or water, in which the plants are growing, from attack or infestation by animal pests. Therefore, the invention also relates to a plant protection method, which comprises contacting crops, plants, plant propagation materials, e.g. seeds, or soil or water, in which the plants are growing, to be protected from attack or infestation by animal pests, with a pesticidally effective amount of a compound of formula (I).

The compounds I are also suitable for use in combating or controlling animal pests. There-fore, the invention also relates to a method of combating or controlling animal pests, which comprises contacting the animal pests, their habitat, breeding ground, or food supply, or the crops, plants, plant propagation materials, e.g. seeds, or soil, or the area, material or environment in which the animal pests are growing or may grow, with a pesticidally effective amount of a compound I.

The compounds I are effective through both contact and ingestion to any and all developmental stages, such as egg, larva, pupa, and adult.

The compounds I can be applied as such or in form of compositions comprising them.

The application can be carried out both before and after the infestation of the crops, plants, plant propagation materials by the pests.

The term "contacting" includes both direct contact (applying the compounds/compositions directly on the animal pest or plant) and indirect contact (applying the compounds/compositions to the locus).

The term "animal pest" includes arthropods, gastropods, and nematodes. Preferred animal pests according to the invention are arthropods, preferably insects and arachnids, in particular insects. The term "plant" includes cereals, e.g. durum and other wheat, rye, barley, triticale, oats, rice, or maize (fodder maize and sugar maize / sweet and field corn); beet, e.g. sugar beet, or fodder beet; fruits, e.g. pomes, stone fruits, or soft fruits, e.g. apples, pears, plums, peaches, nectarines, almonds, cherries, papayas, strawberries, raspberries, blackberries or gooseberries; leguminous plants, e.g. beans, lentils, peas, alfalfa, or soybeans; oil plants, e.g. rapeseed (oilseed rape), turnip rape, mustard, olives, sunflowers, coconut, cocoa beans, castor oil plants, oil palms, ground nuts, or soybeans; cucurbits, e.g. squashes, pumpkins, cucumber or melons; fiber plants, e.g. cotton, flax, hemp, or jute; citrus fruit, e.g. oranges, lemons, grape-fruits or mandarins; vegetables, e.g. eggplant, spinach, lettuce (e.g. iceberg lettuce), chicory, cabbage, asparagus, cabbages, carrots, onions, garlic, leeks, tomatoes, potatoes, cucurbits or sweet peppers; lauraceous plants, e.g. avocados, cinnamon, or camphor; energy and raw material plants, e.g. corn, soybean, rapeseed, sugar cane or oil palm; tobacco; nuts, e.g. walnuts; pistachios; coffee; tea; bananas; vines; hop; sweet leaf (Stevia); natural rubber plants or ornamental and forestry plants, , shrubs, broad-leaved trees or evergreens, eucalyptus; turf; lawn; grass. Preferred plants include potatoes sugar beets, tobacco, wheat, rye, barley, oats, rice, corn, cotton, soybeans, rapeseed, legumes, sunflowers, coffee, or sugar cane; fruits; vines; ornamentals; or vegetables, e.g. cucumbers, tomatoes, beans or squashes.

The term "seed" embraces seeds and plant propagules including true seeds, seed pieces, suckers, corms, bulbs, fruit, tubers, grains, cuttings, cut shoots, and means preferably true seeds. "Pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions e.g. desired pesticidal effect and duration, weather, target species, locus, mode of application.

For use in treating crop plants, e.g. by foliar application, the rate of application of the active ingredients of this invention may be in the range of 0.0001 g to 4000 g per hectare, e.g. from 1 g to 2 kg per hectare or from 1 g to 750 g per hectare, desirably from 1 g to 100 g per hectare.

The compounds I are also suitable for use against non-crop insect pests. For use against said non-crop pests, compounds I can be used as bait composition, gel, general insect spray, aero-sol, as ultra-low volume application and bed net (impregnated or surface applied).

The term "non-crop insect pest" refers to pests, which are particularly relevant for non-crop targets, e.g. ants, termites, wasps, flies, ticks, mosquitoes, bed bugs, crickets, or cockroaches, such as: *Aedes aegypti, Musca domestica, Tribolium* spp.; termites such as *Reticulitermes flavipes, Coptotermes formosanus*; roaches such as *Blatella germanica, Periplaneta Americana;* ants such as *Solenopsis invicta, Linepithema humile,* and *Camponotus pennsylvanicus.*

The bait can be a liquid, a solid or a semisolid preparation (e.g. a gel). For use in bait compositions, the typical content of active ingredient is from 0.001 wt% to 15 wt%, desirably from 0.001 wt% to 5 wt% of active compound.

The compounds I and its compositions can be used for protecting wooden materials such as trees, board fences, sleepers, frames, artistic artifacts, etc. and buildings, but also construction materials, furniture, leathers, fibers, vinyl articles, electric wires and cables etc. from ants, termites and/or wood or textile destroying beetles, and for controlling ants and termites from doing harm to crops or human beings (e.g. when the pests invade into houses and public facilities or nest in yards, orchards or parks).

Customary application rates in the protection of materials are, e.g., from 0.001 g to 2000 g or from 0.01 g to 1000 g of active compound per m² treated material, desirably from 0.1 g to 50 g per m². Insecticidal compositions for use in the impregnation of materials typically contain from 0.001 to 95 wt%, preferably from 0.1 to 45 wt%, and more preferably from 1 to 25 wt% of at least one repellent and/or insecticide.

### Pests

The compounds of the invention are especially suitable for efficiently combating animal pests e.g. arthropods, and nematodes including:
insects from the order of **Lepidoptera,** for example *Achroia grisella, Acleris* spp. such as A. *fimbriana, A. gloverana, A. variana; Acrolepiopsis assectella, Acronicta major, Adoxophyes* spp. such as *A. cyrtosema, A. orana; Aedia leucomelas, Agrotis* spp. such as *A. exclamationis, A. fucosa, A. ipsilon, A. orthogoma, A. segetum, A. subterranea; Alabama argillacea, Aleurodicus dispersus, Alsophila pometaria, Ampelophaga rubiginosa, Amyelois transitella, Anacampsis sarcitella, Anagasta kuehniella, Anarsia lineatella, Anisota senatoria, Antheraea pernyi, Anticarsia (*=*Thermesia)* spp. such as *A. gemmatalis; Apamea* spp., *Aproaerema modicella, Archips* spp. such as *A. argyrospila, A. fuscocupreanus, A. rosana, A. xyloseanus; Argyresthia conjugella, Argyroploce* spp., *Argyrotaenia* spp. such as *A. velutinana; Athetis mindara, Austroasca viridigrisea, Autographa gamma, Autographa nigrisigna, Barathra brassicae, Bedellia* spp., *Bonagota salubricola, Borbo cinnara, Bucculatrix thurberiella, Bupalus piniarius, Busseola* spp., *Cacoecia* spp. such as *C. murinana, C. podana; Cactoblastis cactorum, Cadra cautella, Calingo braziliensis, Caloptilis theivora, Capua reticulana, Carposina* spp. such as *C*. *niponensis, C. sasakii; Cephus* spp., *Chaetocnema aridula, Cheimatobia brumata, Chilo* spp. such as *C. Indicus, C. suppressalis, C. partellus; Choreutis pariana, Choristoneura* spp. such as *C*. *conflictana, C. fumiferana, C. longicellana, C. murinana, C. occidentalis, C*. *rosaceana; Chrysodeixis (*=*Pseudoplusia)* spp. *such* as *C*. *eriosoma, C. includens; Cirphis unipuncta, Clysia ambiguella, Cnaphalocerus* spp., *Cnaphalocrocis medinalis, Cnephasia* spp., *Cochylis hospes, Coleophora* spp., *Colias eurytheme, Conopomorpha* spp., *Conotrachelus* spp., *Copitarsia* spp., *Corcyra cephalonica, Crambus caliginosellus, Crambus teterrellus, Crocidosema (=Epinotia) aporema, Cydalima (=Diaphania) perspectalis, Cydia (*=*Carpocapsa)* spp. such as *C*. *pomonella, C. latiferreana; Dalaca noctuides, Datana integerrima, Dasychira pinicola, Dendrolimus* spp. such as *D. pini, D. spectabilis, D. sibiricus; Desmia funeralis, Diaphania* spp. such as *D. nitidalis, D. hyalinata; Diatraea grandiosella, Diatraea saccharalis, Diphthera festiva, Earias* spp. such as *E*. *insulana, E. vittella; Ecdytolopha aurantianu, Egira (=Xylomyges) curialis, Elasmopalpus lignosellus, Eldana saccharina, Endopiza viteana, Ennomos subsignaria, Eoreuma loftini, Ephestia* spp. such as *E. cautella, E. elutella, E. kuehniella; Epinotia aporema, Epiphyas postvittana, Erannis tiliaria, Erionota thrax, Etiella* spp., *Eulia* spp., *Eupoecilia ambiguella, Euproctis chrysorrhoea, Euxoa* spp., *Evetria bouliana, Faronta albilinea, Feltia* spp. such as *F. subterranean; Galleria mellonella, Gracillaria* spp., *Grapholita* spp. such as *G. funebrana, G. molesta, G. inopinata; Halysidota* spp., *Harrisina americana, Hedylepta* spp., *Helicoverpa* spp. such as *H. armigera (*=*Heliothis armigera), H. zea (*=*Heliothis zea); Heliothis* spp. such as *H. assulta, H. subflexa, H. virescens; Hellula* spp. such as *H. undalis, H. rogatalis; Helocoverpa gelotopoeon, Hemileuca oliviae, Herpetogramma licarsisalis, Hibernia defoliaria, Hofmannophila pseudospretella, Homoeosoma electellum, Homona magnanima, Hypena scabra, Hyphantria cunea, Hyponomeuta padella, Hyponomeuta malinellus, Kakivoria flavofasciata, Keiferia lycopersicella, Lambdina fiscellaria fiscellaria, Lambdina fiscellaria lugubrosa, Lamprosema indicata, Laspeyresia molesta, Leguminivora glycinivorella, Lerodea eufala, Leucinodes orbonalis, Leucoma salicis, Leucoptera* spp. such as *L. coffeella, L. scitella; Leuminivora lycinivorella, Lithocolletis blancardella, Lithophane antennata, Llattia octo (=Amyna axis), Lobesia botrana, Lophocampa* spp., *Loxagrotis albicosta, Loxostege* spp. such as *L. sticticalis, L. cereralis; Lymantria* spp. such as *L. dispar, L. monacha; Lyonetia clerkella, Lyonetia prunifoliella, Malacosoma* spp. such as *M. americanum, M. californicum, M. constrictum, M. neustria; Mamestra* spp. such as *M*. *brassicae, M. configurata; Mamstra brassicae, Manduca* spp. such as *M*. *quinquemaculata, M. sexta; Marasmia spp, Marmara* spp., *Maruca testulalis, Megalopyge lanata, Melanchra picta, Melanitis leda, Mocis* spp. such as *M. lapites, M. repanda; Mocis latipes, Monochroa fragariae, Mythimna separata, Nemapogon cloacella, Neoleucinodes elegantalis, Nepytia* spp., *Nymphula* spp., *Oiketicus* spp., *Omiodes indicata, Omphisa anastomosalis, Operophtera brumata, Orgyia pseudotsugata, Oria* spp., *Orthaga thyrisalis, Ostrinia* spp. such as *O. nubilalis; Oulema oryzae, Paleacrita vernata, Panolis flammea, Parnara* spp., *Papaipema nebris, Papilio cresphontes, Paramyelois transitella, Paranthrene regalis, Paysandisia archon, Pectinophora* spp. such as *P. gossypiella; Peridroma saucia, Perileucoptera* spp., such as *P. coffeella; Phalera bucephala, Phryganidia californica, Phthorimaea* spp. such as *P. operculella; Phyllocnistis citrella, Phyllonorycter* spp. such as *P. blancardella, P. crataegella, P. issikii, P. ringoniella; Pieris* spp. such as *P. brassicae, P. rapae, P. napi; Pilocrocis tripunctata, Plathypena scabra, Platynota* spp. such as *P. flavedana, P. idaeusalis, P. stultana; Platyptilia carduidactyla, Plebejus argus, Plodia interpunctella, Plusia spp, Plutella maculipennis, Plutella xylostella, Pontia protodica, Prays* spp., *Prodenia* spp., *Proxenus lepigone, Pseudaletia* spp. such as *P. sequax, P. unipuncta; Pyrausta nubilalis, Rachiplusia nu, Richia albicosta, Rhizobius ventralis, Rhyacionia frustrana, Sabulodes aegrotata, Schizura concinna, Schoenobius* spp., *Schreckensteinia festaliella, Scirpophaga* spp. such as *S. incertulas, S. innotata; Scotia segetum, Sesamia* spp. such as *S. inferens, Seudyra subflava, Sitotroga cerealella, Sparganothis pilleriana, Spilonota lechriaspis, S. ocellana, Spodoptera (=Lamphygma)* spp. such as *S. cosmoides, S. eridania, S. exigua, S. frugiperda, S. latisfascia, S. littoralis, S. litura, S. omithogalli; Stigmella* spp., *Stomopteryx subsecivella, Strymon bazochii, Sylepta derogata, Synanthedon* spp. such as S. exitiosa, *Tecia solanivora, Telehin licus, Thaumatopoea pityocampa, Thaumatotibia (=Cryptophlebia) leucotreta, Thaumetopoea pityocampa, Theda* spp., *Theresimima ampelophaga, Thyrinteina* spp, *Tildenia inconspicuella, Tinea* spp. such as *T. cloacella, T. pellionella; Tineola bisselliella, Tortrix* spp. such as *T. viridana; Trichophaga tapetzella, Trichoplusia* spp. such as *T. ni; Tuta (=Scrobipalpula) absoluta, Udea* spp. such as *U. rubigalis, U. rubigalis; Virachola* spp., *Yponomeuta padella, and Zeiraphera canadensis;*
insects from the order of **Coleoptera,** for example *Acalymma vittatum, Acanthoscehdes obtectus, Adoretus* spp., *Agelastica alni, Agrilus* spp. such as A. *anxius, A. planipennis, A. sinuatus; Agriotes* spp. such as *A. fuscicollis, A. lineatus, A. obscurus; Alphitobius diaperinus, Amphimallus solstitialis, Anisandrus dispar, Anisoplia austriaca, Anobium punctatum, Anomala corpulenta, Anomala rufocuprea, Anoplophora* spp. such as *A. glabripennis; Anthonomus* spp. such as *A. eugenii, A. grandis, A. pomorum; Anthrenus* spp., *Aphthona euphoridae, Apion* spp., *Apogonia* spp., *Athous haemorrhoidalis, Atomaria* spp. such as *A. linearis; Attagenus* spp., *Aulacophora femoralis, Blastophagus piniperda, Blitophaga undata, Bruchidius obtectus, Bruchus* spp. such as *B. lentis, B. pisorum, B. rufimanus; Byctiscus betulae, Callidiellum rufipenne, Callopistria floridensis, Callosobruchus chinensis, Cameraria ohridella, Cassida nebulosa, Cerotoma trifurcata, Cetonia aurata, Ceuthorhynchus* spp. such as *C. assimilis, C. napi; Chaetocnema tibialis, Cleonus mendicus, Conoderus* spp. such as *C. vespertinus; Conotrachelus nenuphar, Cosmopolites* spp., *Costelytra zealandica, Crioceris asparagi, Cryptolestes ferrugineus, Cryptorhynchus lapathi, Ctenicera* spp. such as *C. destructor; Curculio* spp., *Cylindrocopturus* spp., *Cyclocephala* spp., *Dactylispa balyi, Dectes texanus, Dermestes* spp., *Diabrotica* spp. such as *D. undecimpunctata, D. speciosa, D. longicornis, D. semipunctata, D. virgifera; Diaprepes abbreviates, Dichocrocis* spp., *Dicladispa armigera, Diloboderus abderus, Diocalandra frumenti (Diocalandra stigmaticollis), Enaphalodes rufulus, Epilachna* spp. such as *E. varivestis, E. vigintioctomaculata; Epitrix* spp. such as *E. hirtipennis, E. similaris; Eutheola humilis, Eutinobothrus brasiliensis, Faustinus cubae, Gibbium psylloides, Gnathocerus cornutus, Hellula undalis, Heteronychus arator, Hylamorpha elegans, Hylobius abietis, Hylotrupes bajulus, Hypera* spp. such as *H. brunneipennis, H. postica; Hypomeces squamosus, Hypothenemus* spp., *Ips typographus, Lachnosterna consanguinea, Lasioderma serricorne, Latheticus oryzae, Lathridius* spp., *Lema* spp. such as *L. bilineata, L. melanopus; Leptinotarsa* spp. such as *L. decemlineata; Leptispa pygmaea, Limonius californicus, Lissorhoptrus oryzophilus, Lixus* spp., *Luperodes* spp., *Lyctus* spp. such as *L. bruneus; Liogenys fuscus, Macrodactylus* spp. such as *M. subspinosus; Maladera matrida, Megaplatypus mutates, Megascelis* spp., *Melanotus communis, Meligethes* spp. such as *M. aeneus; Melolontha* spp. such as *M. hippocastani, M. melolontha; Metamasius hemipterus, Microtheca* spp., *Migdolus* spp. such as *M. fryanus, Monochamus* spp. such as *M. alternatus; Naupactus xanthographus, Niptus hololeucus, Oberia brevis, Oemona hirta, Oryctes rhinoceros, Oryzaephilus surinamensis, Oryzaphagus oryzae, Otiorrhynchus sulcatus, Otiorrhynchus ovatus, Otiorrhynchus sulcatus, Oulema melanopus, Oulema oryzae, Oxycetonia jucunda, Phaedon* spp. such as *P. brassicae, P. cochleariae; Phoracantha recurva, Phyllobius pyri, Phyllopertha horticola, Phyllophaga* spp. such as *P. helleri; Phyllotreta* spp. such as *P. chrysocephala, P. nemorum, P. striolata, P. vittula; Phyllopertha horticola, Popillia japonica, Premnotrypes* spp., *Psacothea hilaris, Psylliodes chrysocephala, Prostephanus truncates, Psylliodes* spp., *Ptinus* spp., *Pulga saltona, Rhizopertha dominica, Rhynchophorus* spp. such as *R. billineatus, R. ferrugineus, R. palmarum, R. phoenicis, R. vulneratus; Saperda candida, Scolytus schevyrewi, Scyphophorus acupunctatus, Sitona lineatus, Sitophilus* spp. such as *S. granaria, S. oryzae, S. zeamais; Sphenophorus* spp. such as *S. levis; Stegobium paniceum, Sternechus* spp. such as *S. subsignatus; Strophomorphus ctenotus, Symphyletes* spp., *Tanymecus* spp., *Tenebrio molitor, Tenebrioides mauretanicus, Tribolium* spp. such as *T. castaneum; Trogoderma* spp., *Tychius* spp., *Xylotrechus* spp. such as *X. pyrrhoderus; and, Zabrus* spp. *such* as *Z. tenebrioides;*
insects from the order of **Diptera** e.g. *Aedes* spp. such as *A. aegypti, A. albopictus, A. vexans; Anastrepha ludens, Anopheles* spp. such as *A. albimanus, A. crucians, A. freeborni, A. gambiae, A. leucosphyrus, A. maculipennis, A. minimus, A. quadrimaculatus, A. sinensis; Bactrocera invadens, Bibio hortulanus, Calliphora erythrocephala, Calliphora vicina, Ceratitis capitata, Chrysomyia* spp. such as *C. bezziana, C. hominivorax, C. macellaria; Chrysops atlanticus, Chrysops discalis, Chrysops silacea, Cochliomyia* spp. such as *C. hominivorax; Contarinia* spp. such as *C. sorghicola; Cordylobia anthropophaga, Culex* spp. such as *C. nigripalpus, C. pipiens, C. quinquefasciatus, C. tarsalis, C. tritaeniorhynchus; Culicoides furens, Culiseta inornata, Culiseta melanura, Cuterebra* spp., *Dacus cucurbitae, Dacus oleae, Dasineura brassicae, Dasineura oxycoccana, Delia* spp. such as *D. antique, D. coarctata, D. platura, D. radicum; Dermatobia hominis, Drosophila* spp. such as *D. suzukii, Fannia* spp. such as *F. canicularis; Gastraphilus* spp. such as *G. intestinalis; Geomyza tipunctata, Glossina* spp. such as *G. fuscipes, G. morsitans, G. palpalis, G. tachinoides; Haematobia irritans, Haplodiplosis equestris, Hippelates* spp., Hylemyia spp. such as *H. platura; Hypoderma* spp. such as *H. lineata; Hyppobosca* spp., *Hydrellia philippina, Leptoconops torrens, Liriomyza* spp. such as *L. sativae, L. trifolii; Lucilia* spp. such as *L. caprina, L. cuprina, L. sericata; Lycoria pectoralis, Mansonia titillanus, Mayetiola* spp. such as M. *destructor; Musca* spp. such as *M. autumnalis, M. domestica; Muscina stabulans, Oestrus* spp. such as *O. ovis; Opomyza florum, Oscinella* spp. such as *O. frit; Orseolia oryzae, Pegomya hysocyami, Phlebotomus argentipes, Phorbia* spp. such as *P. antiqua, P. brassicae, P. coarctata; Phytomyza gymnostoma, Prosimulium mixtum, Psila rosae, Psorophora columbiae, Psorophora discolor, Rhagoletis* spp. such as *R. cerasi, R. cingulate, R. indifferens, R. mendax, R. pomonella; Rivellia quadrifasciata, Sarcophaga* spp. such as *S. haemorrhoidalis; Simulium vittatum, Sitodiplosis mosellana, Stomoxys* spp. such as *S. calcitrans; Tabanus* spp. such as *T. atratus, T. bovinus, T. lineola, T. similis; Tannia spp., Thecodiplosis japonensis, Tipula oleracea, Tipula paludosa,* and *Wohlfahrtia* spp;
insects from the order of **Thysanoptera** for example, *Baliothrips biformis, Dichromothrips corbetti, Dichromothrips* ssp., *Echinothrips americanus, Enneothrips flavens, Frankliniella* spp. such as *F. fusca, F. occidentalis, F. tritici; Heliothrips* spp., *Hercinothrips femoralis, Kakothrips* spp., *Microcephalothrips abdominalis, Neohydatothrips samayunkur, Pezothrips kellyanus, Rhipiphorothrips cruentatus, Scirtothrips* spp. such as *S. citri, S. dorsalis, S. perseae; Stenchaetothrips spp, Taeniothrips cardamoni, Taeniothrips inconsequens, Thrips* spp. such as *T. imagines, T. hawaiiensis, T. oryzae, T. palmi, T. parvispinus, T. tabaci;*
insects from the order of **Hemiptera** for example, *Acizzia jamatonica, Acrosternum* spp. such as *A. hilare;* Acyrthosipon spp. such as *A. onobrychis, A. pisum; Adelges laricis, Adelges tsugae, Adelphocoris* spp., such as *A. rapidus, A. superbus; Aeneolamia* spp., *Agonoscena* spp., *Aulacorthum solani, Aleurocanthus woglumi, Aleurodes* spp., *Aleurodicus disperses, Aleurolobus barodensis, Aleurothrixus* spp., *Amrasca* spp., *Anasa tristis, Antestiopsis* spp., *Anuraphis cardui, Aonidiella* spp., *Aphanostigma piri, Aphidula nasturtii, Aphis* spp. such as *A. craccivora, A. fabae, A. forbesi, A. gossypii, A. grossulariae, A. maidiradicis, A. pomi, A. sambuci, A. schneideri, A. spiraecola; Arboridia apicalis, Arilus critatus, Aspidiella* spp., *Aspidiotus* spp., *Atanus* spp., *Aulacaspis yasumatsui, Aulacorthum solani, Bactericera cockerelli (Paratrioza cockerelli), Bemisia* spp. such as *B. argentifolii, B. tabaci (Aleurodes tabaci); Blissus* spp. such as *B. leucopterus; Brachycaudus* spp. such as *B. cardui, B. helichrysi, B. persicae, B. prunicola; Brachycolus* spp., *Brachycorynella asparagi, Brevicoryne brassicae, Cacopsylla* spp. such as *C. fulguralis, C. pyricola (Psylla piri); Calligypona marginata, Calocoris* spp., *Campylomma livida, Capitophorus horni, Carneocephala fulgida, Cavelerius* spp., *Ceraplastes* spp., *Ceratovacuna lanigera, Ceroplastes ceriferus, Cerosipha gossypii, Chaetosiphon fragaefolii, Chionaspis tegalensis, Chlorita onukii, Chromaphis juglandicola, Chrysomphalus ficus, Cicadulina mbila, Cimex* spp. such as *C. hemipterus, C. lectularius; Coccomytilus halli, Coccus* spp. such as *C. hesperidum, C. pseudomagnoliarum; Corythucha arcuata, Creontiades dilutus, Cryptomyzus ribis, Chrysomphalus aonidum, Cryptomyzus ribis, Ctenarytaina spatulata, Cyrtopeltis notatus, Dalbulus* spp., *Dasynus piperis, Dialeurodes* spp. such as *D. citrifolii; Dalbulus maidis, Diaphorina* spp. such as *D. citri; Diaspis* spp. such as *D. bromeliae; Dichelops furcatus, Diconocoris hewetti, Doralis* spp., *Dreyfusia nordmannianae, Dreyfusia piceae, Drosicha* spp., *Dysaphis* spp. such as *D. plantaginea, D. pyri, D. radicola; Dysaulacorthum pseudosolani, Dysdercus* spp. such as *D. cingulatus, D. intermedius; Dysmicoccus* spp., *Edessa* spp., *Geocoris* spp., *Empoasca* spp. such as *E. fabae, E. solana; Epidiaspis leperii, Eriosoma* spp. such as *E. lanigerum, E. pyricola; Erythroneura* spp., *Eurygaster* spp. such as *E. integriceps; Euscelis bilobatus, Euschistus* spp. such as *E. heros, E. impictiventris, E. servus; Fiorinia theae, Geococcus coffeae, Glycaspis brimblecombei, Halyomorpha* spp. such as *H. halys; Heliopeltis* spp., *Homalodisca vitripennis (=H. coagulata), Horcias nobilellus, Hyalopterus pruni, Hyperomyzus lactucae, Icerya* spp. such as *I. purchase; Idiocerus* spp., *Idioscopus* spp., *Laodelphax striatellus, Lecanium* spp., *Lecanoideus floccissimus, Lepidosaphes* spp. such as *L. ulmi; Leptocorisa* spp., *Leptoglossus phyllopus, Lipaphis eηsimi, Lygus* spp. such as *L. hesperus, L. lineolaris, L. pratensis; Maconellicoccus hirsutus, Marchalina hellenica, Macropes excavatus, Macrosiphum* spp. such as *M. rosae, M. avenae, M. euphorbiae; Macrosteles quadrilineatus, Mahanarva fimbriolata, Megacopta cribraria, Megoura viciae, Melanaphis pyrarius, Melanaphis sacchari, Melanocallis (=Tinocallis) caryaefoliae, Metcafiella* spp., *Metopolophium dirhodum, Monellia costalis, Monelliopsis pecanis, Myzocallis coryli, Murgantia* spp., *Myzus* spp. such as *M. ascalonicus, M. cerasi, M. nicotianae, M. persicae, M. varians; Nasonovia ribis-nigri, Neotoxoptera formosana, Neomegalotomus spp, Nephotettix* spp. such as *N. malayanus, N. nigropictus, N. parvus, N. virescens; Nezara* spp. such as *N. viridula; Nilaparvata lugens, Nysius huttoni, Oebalus* spp. such as O. *pugnax; Oncometopia* spp., *Orthezia praelonga, Oxycaraenus hyalinipennis, Parabemisia myricae, Parlatoria* spp., *Parthenolecanium* spp. such as *P. corni, P. persicae; Pemphigus* spp. such as *P. bursarius, P. populivenae; Peregrinus maidis, Perkinsiella saccharicida, Phenacoccus* spp. such as *P. aceris, P. gossypii; Phloeomyzus passerinii, Phorodon humuli, Phylloxera* spp. such as *P. devastatrix, Piesma quadrata, Piezodorus* spp. such as *P. guildinii; Pinnaspis aspidistrae, Planococcus* spp. such as *P. citri, P. ficus; Prosapia bicincta, Protopulvinaria pyriformis, Psallus seriatus, Pseudacysta persea, Pseudaulacaspis pentagona, Pseudococcus* spp. such as *P. comstocki; Psylla* spp. such as *P. mali; Pteromalus* spp., *Pulvinaria amygdali, Pyrilla* spp., *Quadraspidiotus* spp., such as *Q. perniciosus; Quesada gigas, Rastrococcus* spp., *Reduvius senilis, Rhizoecus americanus, Rhodnius* spp., *Rhopalomyzus ascalonicus, Rhopalosiphum* spp. such as *R. pseudobrassicas, R. insertum, R. maidis, R. padi; Sagatodes* spp., *Sahlbergella singularis, Saissetia* spp., *Sappaphis mala, Sappaphis mali, Scaptocoris* spp., *Scaphoides titanus, Schizaphis graminum, Schizoneura lanuginosa, Scotinophora* spp., *Selenaspidus articulatus, Sitobion avenae, Sogata* spp., *Sogatella furcifera, Solubea insularis, Spissistilus festinus (=Stictocephala festina), Stephanitis nashi, Stephanitis pyrioides, Stephanitis takeyai, Tenalaphara malayensis, Tetraleurodes perseae, Therioaphis maculate, Thyanta* spp. such as *T. accerra, T. perditor; Tibraca* spp., *Tomaspis* spp., *Toxoptera* spp. such as *T. aurantii; Trialeurodes* spp. such as *T. abutilonea, T. ricini, T. vaporariorum; Triatoma* spp., *Trioza* spp., *Typhlocyba* spp., *Unaspis* spp. such as *U. citri, U. yanonensis; and Viteus vitifolii,*
Insects from the order **Hymenoptera** for example *Acanthomyops interjectus, Athalia rosae, Atta* spp. *such* as *A. capiguara, A. cephalotes, A. cephalotes, A. laevigata, A. robusta, A. sexdens, A. texana, Bombus* spp., *Brachymyrmex* spp., *Camponotus* spp. such as *C. floridanus, C. pennsylvanicus, C. modoc; Cardiocondyla nuda, Chalibion sp, Crematogaster* spp., *Dasymutilla occidentalis, Diprion* spp., *Dolichovespula maculata, Dorymyrmex* spp., *Dryocosmus kuriphilus, Formica* spp., *Hoplocampa* spp. such as *H. minuta, H. testudinea; Iridomyrmex humilis,* Lasius spp. such as *L. niger, Linepithema humile, Liometopum* spp., *Leptocybe invasa, Monomorium* spp. such as *M. pharaonis, Monomorium, Nylandria fulva, Pachycondyla chinensis, Paratrechina longicornis, Paravespula* spp., such as *P. germanica, P. pennsylvanica, P. vulgaris; Pheidole* spp. such as *P. megacephala; Pogonomyrmex* spp. such as *P. barbatus, P. californicus, Polistes rubiginosa, Prenolepis impairs, Pseudomyrmex gracilis, Schelipron* spp., *Sirex cyaneus, Solenopsis* spp. such as *S. geminata, S.invicta, S. molesta, S. richteri, S. xyloni, Sphecius speciosus, Sphex* spp., *Tapinoma* spp. such as *T. melanocephalum, T. sessile; Tetramorium* spp. such as *T. caespitum, T. bicarinatum, Vespa* spp. such as *V. crabro; Vespula* spp. such as *V. squamosal; Wasmannia auropunctata, Xylocopa* sp;
Insects from the order **Orthoptera** for example *Acheta domesticus, Calliptamus italicus, Chortoicetes terminifera, Ceuthophilus* spp., *Diastrammena asynamora, Dociostaurus maroccanus, Gryllotalpa* spp. such as *G. africana, G. gryllotalpa; Gryllus* spp., *Hieroglyphus daganensis, Kraussaria angulifera, Locusta* spp. such as *L. migratoria, L. pardalina; Melanoplus* spp. such as *M. bivittatus, M. femurrubrum, M. mexicanus, M. sanguinipes, M. spretus; Nomadacris septemfasciata, Oedaleus senegalensis, Scapteriscus* spp., *Schistocerca* spp. such as *S. americana, S. gregaria, Stemopelmatus* spp., *Tachycines asynamorus,* and *Zonozerus variegatus;*
Pests from the Class **Arachnida** for example **Acari,**e.g. of the families Argasidae, Ixodidae and Sarcoptidae, such as *Amblyomma* spp. (e.g. *A. americanum, A. variegatum, A. maculatum),* Argas spp. such as *A. persicu), Boophilus* spp. such as *B. annulatus, B. decoloratus, B. microplus, Dermacentor spp.* such as *D.silvarum, D. andersoni, D. variabilis, Hyalomma* spp. such as *H. truncatum, Ixodes* spp. such as *I. ricinus, I. rubicundus, I. scapularis, I. holocyclus, I. pacificus, Rhipicephalus sanguineus, Ornithodorus* spp. such as *O. moubata, O. hermsi, O. turicata, Ornithonyssus bacoti, Otobius megnini, Dermanyssus gallinae, Psoroptes* spp. such as *P. ovis, Rhipicephalus* spp. such as *R. sanguineus, R. appendiculatus, Rhipicephalus evertsi, Rhizoglyphus* spp., *Sarcoptes* spp. such as *S. Scabiei;* and Family **Eriophyidae** including *Aceria* spp. such as *A. sheldoni, A. anthocoptes, Acallitus* spp., *Aculops* spp. such as *A. lycopersici, A. pelekassi; Aculus* spp. such as *A. schlechtendali; Colomerus vitis, Epitrimerus pyri, Phyllocoptruta oleivora; Eriophytes ribis* and *Eriophyes* spp. such as *Eriophyes sheldoni;* Family **Tarsonemidae** including *Hemitarsonemus* spp., *Phytonemus pallidus and Polyphagotarsonemus latus, Stenotarsonemus* spp. *Steneotarsonemus spinki;* Family **Tenuipalpidae** including Brevipalpus spp. such as *B. phoenicis;* Family **Tetranychidae** including *Eotetranychus* spp., *Eutetranychus* spp., *Oligonychus* spp., *Petrobia latens, Tetranychus* spp. such as *T. cinnabarinus, T. evansi, T. kanzawai, T, pacificus, T. phaseulus, T. telarius* and *T. urticae: Bryobia praetiosa; Panonychus* spp. such as *P. ulmi, P. citri; Metatetranychus* spp. and *Oligonychus* spp. such as *O. pratensis, O. perseae, Vasates lycopersici; Raoiella indica, Family* **Carpoglyphidae** including *Carpoglyphus* spp.; *Penthaleidae* spp. such as *Halotydeus destructor,* Family **Demodicidae** with species such as *Demodex* spp.; Family **Trombicidea** including *Trombicula* spp.; Family **Macronyssidae** including *Ornothonyssus* spp.; Family **Pyemotidae** including *Pyemotes tritici; Tyrophagus putrescentiae;* Family **Acaridae** including *Acarus sire,* Family **Araneida** including *Latrodectus mactans, Tegenaria agrestis, Chiracanthium sp, Lycosa sp Achaearanea tepidariorum* and *Loxosceles reclusa;*
Pests from the Phylum **Nematoda,** for example, plant parasitic nematodes such as root-knot nematodes, *Meloidogyne* spp. such as *M. hapla, M. incognita, M. javanica;* cyst-forming nematodes, *Globodera* spp. such as *G. rostochiensis; Heterodera* spp. such as *H. avenae, H. glyci-nes, H. schachtii, H. trifolii;* Seed gall nematodes, *Anguina* spp.; Stem and foliar nematodes, *Aphelenchoides* spp. such as A. *besseyi;* Sting nematodes, *Belonolaimus* spp. such as *B. longicaudatus;* Pine nematodes, *Bursaphelenchus* spp. such as *B. lignicolus, B. xylophilus;* Ring nematodes, *Criconema* spp., *Criconemella* spp. such as *C. xenoplax* and *C. ornata;* and, *Criconemoides* spp. such as *Criconemoides informis; Mesocriconema* spp.; Stem and bulb nematodes, *Ditylenchus* spp. such as *D. destructor, D. dipsaci;* Awl nematodes, *Dolichodorus* spp.; Spiral nematodes, *Heliocotylenchus multicinctus;* Sheath and sheathoid nematodes, *Hemicycliophora* spp. and *Hemicriconemoides* spp.; *Hirshmanniella* spp.; Lance nematodes, *Hoploaimus* spp.; False rootknot nematodes, *Nacobbus* spp.; Needle nematodes, *Longidorus* spp. such as *L. elongatus;* Lesion nematodes, *Pratylenchus* spp. such as *P. brachyurus, P. neglectus, P. penetrans, P. curvitatus, P. goodeyi;* Burrowing nematodes, *Radopholus* spp. such as *R. similis; Rhadopholus* spp.; *Rhodopholus* spp.; Reniform nematodes, *Rotylenchus* spp. such as *R. robustus, R. reniformis; Scutellonema* spp.; Stubby-root nematode, *Trichodorus* spp. such as *T. obtusus, T. primitivus; Paratrichodorus* spp. such as *P. minor;* Stunt nematodes, *Tylenchorhynchus* spp. such as *T. claytoni, T. dubius;* Citrus nematodes, *Tylenchulus* spp. such as *T. semipenetrans;* Dagger nematodes, *Xiphinema* spp.; and other plant parasitic nematode species;
Insects from the order **Blattodea** for example *Macrotermes* spp. such as *M. natalensis; Cornitermes cumulans, Procornitermes* spp., *Globitermes sulfureus, Neocapritermes* spp. such as *N. opacus, N. parvus; Odontotermes spp., Nasutitermes* spp. such as *N. corniger, Coptotermes* spp. such as *C. formosanus, C. gestroi, C. acinaciformis; Reticulitermes* spp. such as *R. hesperus, R. tibialis, R. speratus, R. flavipes, R. grassei, R. lucifugus, R. virginicus; Heterotermes* spp. such as *H. aureus, H. longiceps, H. tenuis; Cryptotermes* spp. such as *C. brevis, C. cavifrons; Incisitermes* spp. such as *I. minor, I. snyderi; Marginitermes hubbardi, Kalotermes flavicollis, Neotermes* spp. such as *N. castaneus, Zootermopsis* spp. *such* as *Z. angusticollis, Z. nevadensis, Mastotermes* spp. such as *M. darwiniensis; Blatta* spp. such as *B. orientalis, B. lateralis; Blattella* spp. such as *B. asahinae, B. germanica; Rhyparobia maderae, Panchlora nivea, Periplaneta* spp. such as *P. americana, P. australasiae, P. brunnea, P. fuliginosa, P. japonica; Supella longipalpa, Parcoblatta pennsylvanica, Eurycotis floridana, Pycnoscelus surinamensis,*
Insects from the order **Siphonoptera** for example *Cediopsylla simples, Ceratophyllus* spp., *Ctenocephalides* spp. such as *C. felis, C. canis, Xenopsylla cheopis, Pulex irritans, Trichodectes canis, Tunga penetrans,* and *Nosopsyllus fasciatus,*
Insects from the order **Thysanura** for example *Lepisma saccharina* , *Ctenolepisma urbana,* and *Thermobia domestica,*
Pests from the class **Chilopoda** for example *Geophilus* spp., Scutigera spp. such as *Scutigera coleoptrata;*
Pests from the class **Diplopoda** for example *Blaniulus guttulatus, Julus* spp., *Narceus* spp.,
Pests from the class **Symphyla** for example *Scutigerella immaculata,*
Insects from the order **Dermaptera,** for example *Forficula auricularia,*
Insects from the order **Collembola,** for example *Onychiurus* spp., such as *Onychiurus armatus,*
Pests from the order **Isopoda** for example, *Armadillidium vulgare, Oniscus asellus, Porcellio scaber,*
Insects from the order **Phthiraptera,** for example *Damalinia* spp., Pediculus spp. such as *Pediculus humanus capitis, Pediculus humanus corporis, Pediculus humanus humanus; Pthirus pubis,* Haematopinus spp. such as *Haematopinus eurysternus, Haematopinus suis;* Linognathus spp. such as *Linognathus vituli; Bovicola bovis, Menopon gallinae, Menacanthus stramineus* and *Solenopotes capillatus, Trichodectes* spp.,
Examples of further pest species which may be controlled by compounds of fomula (I) include: from the Phylum **Mollusca,** class **Bivalvia,** for example, *Dreissena* spp.; class **Gastropoda,** for example, *Arion* spp., *Biomphalaria* spp., *Bulinus* spp., *Deroceras* spp., *Galba* spp., *Lymnaea* spp., *Oncomelania* spp., *Pomacea canaliclata, Succinea* spp.; from the class of the **helminths,** for example, *Ancylostoma duodenale, Ancylostoma ceylanicum, Acylostoma braziliensis, Ancylostoma* spp., *Ascaris lubricoides, Ascaris spp., Brugia malayi, Brugia timori, Bunostomum* spp., *Chabertia* spp., *Clonorchis* spp., *Cooperia* spp., *Dicrocoelium* spp., *Dictyocaulus filaria, Diphyllobothrium latum, Dracunculus medinensis, Echinococcus granulosus, Echinococcus multilocularis, Enterobius vermicularis, Faciola* spp., Haemonchus spp. such as *Haemonchus contortus; Heterakis* spp., *Hymenolepis nana, Hyostrongulus* spp., *Loa Loa, Nematodirus* spp., *Oesophagostomum* spp., *Opisthorchis* spp., *Onchocerca volvulus, Ostertagia* spp., *Paragonimus* spp., *Schistosomen* spp., *Strongyloides fuellebomi, Strongyloides stercora lis, Stronyloides* spp., *Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.*

### Animal health

The compounds I are suitable for use in treating or protecting animals against infestation or infection by parasites. Therefore, the invention also relates to the use of a compound of the invention for the manufacture of a medicament for the treatment or protection of animals against infestation or infection by parasites. Furthermore, the invention relates to a method of treating or protecting animals against infestation and infection by parasites, which comprises orally, topically or parenterally administering or applying to the animals a parasiticidally effective amount of a compound I.

The invention also relates to the non-therapeutic use of compounds of the invention for treating or protecting animals against infestation and infection by parasites. Moreover, the invention relates to a non-therapeutic method of treating or protecting animals against infestation and infection by parasites, which comprises applying to a locus a parasiticidally effective amount of a compound I.

The compounds of the invention are further suitable for use in combating or controlling parasites in and on animals. Furthermore, the invention relates to a method of combating or controlling parasites in and on animals, which comprises contacting the parasites with a parasitically effective amount of a compound I.

The invention also relates to the non-therapeutic use of compounds I for controlling or combating parasites. Moreover, the invention relates to a non-therapeutic method of combating or controlling parasites, which comprises applying to a locus a parasiticidally effective amount of a compound I.

The compounds I can be effective through both contact (via soil, glass, wall, bed net, carpet, blankets or animal parts) and ingestion (e.g. baits). Furthermore, the compounds I can be applied to any and all developmental stages.

The compounds I can be applied as such or in form of compositions comprising them.

The term "locus" means the habitat, food supply, breeding ground, area, material or environment in which a parasite is growing or may grow outside of the animal.

As used herein, the term "parasites" includes endo- and ectoparasites. In some embodiments of the invention, endoparasites can be preferred. In other embodiments, ectoparasites can be preferred. Infestations in warm-blooded animals and fish include lice, biting lice, ticks, nasal bots, keds, biting flies, muscoid flies, flies, myiasitic fly larvae, chiggers, gnats, mosquitoes and fleas.

The compounds of the invention are especially useful for combating the following parasites: *Cimex lectularius, Rhipicephalus sanguineus,* and *Ctenocephalides felis.*

As used herein, the term "animal" includes warm-blooded animals (including humans) and fish. Preferred are mammals, such as cattle, sheep, swine, camels, deer, horses, pigs, poultry, rabbits, goats, dogs and cats, water buffalo, donkeys, fallow deer and reindeer, and also in furbearing animals such as mink, chinchilla and raccoon, birds such as hens, geese, turkeys and ducks and fish such as fresh- and salt-water fish such as trout, carp and eels. Particularly preferred are domestic animals, such as dogs or cats.

The compounds I may be applied in total amounts of 0.5 mg/kg to 100 mg/kg per day, preferably 1 mg/kg to 50 mg/kg per day.

For oral administration to warm-blooded animals, the compounds I may be formulated as animal feeds, animal feed premixes, animal feed concentrates, pills, solutions, pastes, suspensions, drenches, gels, tablets, boluses and capsules. For oral administration, the dosage form chosen should provide the animal with 0.01 mg/kg to 100 mg/kg of animal body weight per day of the compounds I, preferably with 0.5 mg/kg to 100 mg/kg of animal body weight per day.

Alternatively, the compounds I may be administered to animals parenterally, e.g., by intraruminal, intramuscular, intravenous or subcutaneous injection. The compounds I may be dispersed or dissolved in a physiologically acceptable carrier for subcutaneous injection. Alternatively, the compounds I may be formulated into an implant for subcutaneous administration. In addition the compounds I may be transdermally administered to animals. For parenteral administration, the dosage form chosen should provide the animal with 0.01 mg/kg to 100 mg/kg of animal body weight per day of the compounds I.

The compounds I may also be applied topically to the animals in the form of dips, dusts, powders, collars, medallions, sprays, shampoos, spot-on and pour-on formulations and in ointments or oil-in-water or water-in-oil emulsions. For topical application, dips and sprays usually contain 0.5 ppm to 5,000 ppm and preferably 1 ppm to 3,000 ppm of the compounds I. In addition, the compounds I may be formulated as ear tags for animals, particularly quadrupeds e.g. cattle and sheep.

Oral solutions are administered directly.

Solutions for use on the skin are trickled on, spread on, rubbed in, sprinkled on or sprayed on. Gels are applied to or spread on the skin or introduced into body cavities.

Pour-on formulations are poured or sprayed onto limited areas of the skin, the active compound penetrating the skin and acting systemically. Pour-on formulations are prepared by dissolving, suspending or emulsifying the active compound in suitable skin-compatible solvents or solvent mixtures.

Emulsions can be administered orally, dermally or as injections.

Suspensions can be administered orally or topically/dermally.

Semi-solid preparations can be administered orally or topically/dermally.

For the production of solid preparations, the active compound is mixed with suitable excipients, if appropriate with addition of auxiliaries, and brought into the desired form.

The compositions which can be used in the invention can comprise generally from about 0.001 to 95% of the compound I.

Ready-to-use preparations contain the compounds acting against parasites, preferably ectoparasites, in concentrations of 10 ppm to 80% by weight, preferably from 0.1 to 65% by weight, more preferably from 1 to 50% by weight, most preferably from 5 to 40% by weight.

Preparations which are diluted before use contain the compounds acting against ectoparasites in concentrations of 0.5 to 90% by weight, preferably of 1 to 50% by weight.

Furthermore, the preparations comprise the compounds of formula I against endoparasites in concentrations of 10 ppm to 2% by weight, preferably of 0.05 to 0.9% by weight, very particularly preferably of 0.005 to 0.25% by weight.

Solid formulations which release compounds of the invention may be applied in total amounts of 10 mg/kg to 300 mg/kg, preferably 20 mg/kg to 200 mg/kg, most preferably 25 mg/kg to 160 mg/kg body weight of the treated animal in the course of three weeks.

The following examples illustrate the invention.

### A. Preparation of Compounds

Materials: Unless otherwise noted, reagents and solvents were purchased at highest commercial quality and used without further purification.

All reactions were monitored by thin-layer chromatography (TLC) using Merck silica gel 60 F₂54 pre-coated plates (0.25 mm). Flash chromatography was carried out with Kanto Chemical silica gel (Kanto Chemical, silica gel 60N, spherical neutral, 0.040-0.050 mm, Cat.-No. 37563-84). ¹H NMR spectra were recorded on JEOL JNM-ECA-500 (500 MHz). Chemical shifts are expressed in ppm downfield from the internal solvent peaks for acetone-d₆ (¹H; δ = 2.05 ppm) and CD₃OD (¹H; δ = 3.30 ppm), and *J* values are given in Hertz. The following abbreviations were used to explain the multiplicities: s = singlet, d = doublet, t = triplet, q = quartet, dd = double doublet, ddd = doublet of doublets of doublets, dt = double triplet, td = triplet of doublets m = multiplet, br = broad, J= coupling constant. High-resolution mass spectra were measured on a JEOL JMS-T100LP. Characterization: The compounds were characterized by coupled High Performance Liquid Chromatography with mass spectrometry (HPLC/MS). Method A: UHPLC-MS on Shimadzu Nexera UHPLC & Shimadzu LCMS 20-20 ESI. Analytical UHPLC column: Phenomenex Kinetex 1,7 µm XB-C18 100A; 50 x 2.1 mm; mobile phase: A: water + 0.1% TFA; B: acetonitrile; gradient: 5-100% B in 1.50 minutes; 100% B 0.20 min; flow: 0,8-1,0mL/min in 1,50 minutes at 60°C. MS-method: ESI positive; mass range (m/z) 100-700.

### Synthesis Example A: 2-[3-ethylsulfonyl-6-(trifluoromethyl)imidazo[1,2-alpyridin-2-yl]-6-methyl-7-(trifluoromethyl)imidazo[1,2-c]pyrimidin-5-one (compound 1.1)

### Step 1) Manufacture of 2-bromo-1-[3-ethylsulfanyl-6-(trifluoromethyl)imidazo[1,2-a]pyridin-2-yl]ethanone

A solution of 185 mg 1-[3-ethylsulfanyl-6-(trifluoromethyl)imidazo[1,2-a]pyridin-2-yl]ethanone (as disclosed and synthesized in EP3257853A1, table 30, i-6-004) was dissolved in 3 ml of 25% HBr in CH₃COOH and a solution of 123 mg Br₂ in 3 ml CH3COOH was added at room temperature and stirred for 4 hours. Water was added and the mixture was extracted twice with methyl tert. butyl ether. The combined organic layers were dried and concentrated under reduced pressure to afford a residue. The residue was purified by column chromatography under reversed phase conditions to afford 130 mg of 2-bromo-1-[3-ethylsulfanyl-6-(trifluoromethyl)imidazo[1,2-a]pyridin-2-yl]ethanone LC-MS: mass found 366.8; t_{R}= 1.215 min (t_{R}: retention time).

### Step 2) Manufacture of 2-[3-ethylsulfonyl-6-(trifluoromethyl)imidazo[1,2-alpyridin-2-yll-6-methyl-7-(trifluoromethyl)imidazo[1,2-clpyrimidin-5-one

Step 2a: A mixture of 122 mg of 2-bromo-1-[3-ethylsulfanyl-6-(trifluoromethyl)imidazo[1,2-a]pyridin-2-yl]ethanone and 4-amino-1-methyl-6-(trifluoromethyl)pyrimidin-2-one (77 mg) was dissolved in 3 ml 1,4-dioxan and heated to 140°C for 4 hours in a microwave reactor. The reaction was then cooled to 20 to 25 °C and concentrated *in vacuo* to afford a residue. The residue was purified by column chromatography under reversed phase conditions to afford 51 mg of 2-[3-ethylsulfanyl-6-(trifluoromethyl)imidazo[1,2-a]pyridin-2-yl]-6-methyl-7-(trifluoromethyl) imidazo[1,2-c]pyrimidin-5-one. LC-MS: mass found for C₁₈H₁₃N₅OF₆S [M+H]⁺ = 461.9; t_{R}= 1.186 min (t_{R}: retention time).

Step 2b: To a solution of 2-[3-ethylsulfanyl-6-(trifluoromethyl)imidazo[1,2-a]pyridin-2-yl]-6-methyl-7-(trifluoromethyl) imidazo[1,2-c]pyrimidin-5-one (51 mg) in CH₂Cl₂ (5 mL) at 5°C was added *meta-*chloroperoxybenzoic acid (63 mg), the reaction mixture was then allowed to warm to 20 to 25 °C and stirred for 16 hours. The reaction mixture was then poured into water and extracted with CH₂Cl₂. The combined organic layers were dried, and concentrated under reduced pressure to afford a residue. The residue was purified by column chromatography under reversed phase conditions eluting with water/acetonitrile to afford compound I.1 (20.4 mg, 37% yield). LC-MS: mass found for C₁₈H₁₃N₅O₃F₆S [M+H]⁺ 493.9 ; t_{R}= 1.165 min (t_{R}: retention time).

### Synthesis Example B: 6-cyclopropyl-2-[3-(ethanesulfonyl)imidazo[1.2-alpyridin-2-yl]-7-(trifluoromethyl)imidazo[1,2-c]pyrimidin-5-one (compound 1.2)

### Step 1) Manufacture of ethyl imidazo[1,2-a] pyridine-2-carboxylate

To a stirred composition comprising 2-aminopyridine (10 g) and CH₃CH₂OH (100 mL) was added ethyl bromopyruvate (24.86 g) dropwise over a period a time of 10 min at 20 to 25 °C. The resulting reaction mixture was heated to boiling temperature under refluxation for 5 hours. The solvent was then evaporated under reduced pressure. Work-up of the residue by extraction and column chromatography afforded ethyl imidazo[1,2-a] pyridine-2-carboxylate (10 g, 60% yield).

¹H-NMR (500 MHz, Chloroform-d) δ 8.17 (dt, *J* = 7.0, 1.2 Hz, 1H), 7.71 (dt, *J* = 9.3, 1.1 Hz, 1H), 7.70 (S, 1H), 7.34 (ddd, *J* = 9.2, 6.7, 1.3 Hz, 1H), 7.04 (td, *J* = 6.9, 1.1 Hz, 1H), 4.52 (q, *J* = 7.1 Hz, 2H), 1.48 (t, *J* = 7.1 Hz, 3H). LC-MS: Mass found (M+1) = 190.1.

### Step-2: Manufacture of ethyl 3-chloroimidazo[1,2-a] pyridine-2-carboxylate

Ethyl imidazo[1,2-a] pyridine-2-carboxylate (1g) from step-1 was dissolved in DMF (10 mL), then N-chlorosuccinimide (0.913 g) was added at 20 to 25 °C under nitrogen atmosphere. The reaction mixture was heated to 40°C for 4 hours. The reaction mixture quenched with water and worked-up by extraction to afford a brown colour oil of ethyl 3-chloroimidazo[1,2-a] pyridine-2-carboxylate (1.0 g, 80 % yield). ¹H-NMR (500 MHz, Chloroform-d) δ 8.22 - 8.10 (m, 1H), 7.72 (d, *J* = 9.0 Hz, 1H), 7.35 (ddd, *J* = 9.4, 6.8, 1.4 Hz, 1H), 7.09 - 6.98 (m, 1H), 4.58 - 4.44 (m, 2H), 1.55 - 1.42 (m, 3H). LC-MS: Mass found (M+1) =224.1.

### Step-3: Manufacture of ethyl 3-ethylsulfanylimidazo[1,2-a] pyridine-2-carboxylate

To a stirred composition comprising ethyl 3-chloroimidazo[1,2-a] pyridine-2-carboxylate (4g) from step-2 in DMF (40 mL) was added sodium ethanethiolate (2.24 g) at 20 to 25 °C. The resulting reaction mixture was stirred the reaction mixture at 20 to 25 °C for 2 hours. The reaction mixture was then quenched with water and worked-up by extraction to afford ethyl 3-ethylsulfanylimi-dazo[1,2-a] pyridine-2-carboxylate (3 g, 70 % yield). ¹H-NMR (500 MHz, Chloroform-*d*) δ 8.49 (dt, *J* = 7.0, 1.2 Hz, 1H), 7.64 (dt, *J* = 9.0, 1.1 Hz, 1H), 7.30 - 7.20 (m, 1H), 6.92 (td, *J* = 6.8, 1.2 Hz, 1H), 4.43 (d, *J* = 7.1 Hz, 2H), 2.86 (d, *J =* 7.3 Hz, 2H), 1.40 (t, *J =* 7.1 Hz, 3H), 1.11 (t, *J* = 7.4 Hz, 3H). LC-MS: Mass found (M+1) =250.1.

### Step-4: Manufacture of ethyl 3-ethylsulfonylimidazo[1,2-a] pyridine-2-carboxylate

To a stirred composition comprising ethyl 3-ethylsulfanylimidazo[1,2-a]pyridine-2-carboxylate (13g) from step-3 was added *meta*-Chloroperoxybenzoic acid (3 equivalents) at 0 °C. The reaction mixture was then allowed to warm to 20 to 25 °C and stirred for 16 hours. The reaction was then quenched with water upon which a sodium bisulphite solution was added. Work-up by extraction afforded ethyl 3-ethylsulfonylimidazo[1,2-a] pyridine-2-carboxylate as yellow colour solid (14 g, 90 % yield).

¹H-NMR (500 MHz, Chloroform-d) δ 9.27 (dt, *J* = 7.2, 1.2 Hz, 1H), 7.85 (dt, *J* = 9.0, 1.2 Hz, 1H), 7.53 (ddd, *J* = 9.1, 6.8, 1.2 Hz, 1H), 7.13 (td, J= 7.0, 1.3 Hz, 1H), 4.54 (q, *J* = 7.1 Hz, 2H), 3.74 (q, *J* = 7.4 Hz, 2H), 1.49 (t, *J* = 7.1 Hz, 3H), 1.38 (t, *J* = 7.4 Hz, 3H). LC-MS: Mass found (M+1) =282.1.

### Step-5: Manufacture of 3-ethylsulfonylimidazo[1,2-a] pyridine-2-carboxylic acid

To a stirred composition comprising ethyl 3-ethylsulfanylimidazo[1,2-a] pyridine-2-carboxylate (1g) from step-4 in CH₃CH₂OH (10 mL) was added LiOH·H₂O (0.250g) at 20 to 25 °C. The reaction mixture was stirred for 2 hours upon which the solvent was removed by evaporation. The residue was diluted with an aqueous solution of HCl (1 M) to adjust the pH to approximately 2. The resulting composition was worked-up by extraction to afford 3-ethylsulfonylimidazo[1,2-a] pyridine-2-carboxylic acid (0.7 g, 80 % yield).

¹H NMR (500 MHz, DMSO-*d*₆) δ 8.79 (d, *J* = 6.9 Hz, 1H), 7.80 (d, *J* = 9.0 Hz, 1H), 7.70 (t, *J* = 7.9 Hz, 1H), 7.33 (t, *J* = 6.8 Hz, 1H), 2.93 (t, *J* = 7.3 Hz, 2H), 1.09 (t, *J* = 7.3 Hz, 3H). LC-MS: Mass found (M-1) =253.1.

### Step-6: Manufacture of 3-ethylsulfonyl-N-methoxy-N-methyl-imidazo[1,2-a]pyridine-2-carboxamide

To a stirred composition comprising 3-ethylsulfonylimidazo[1,2-a] pyridine-2-carboxylic acid (0.1 g) in dry DCM (10mL) were added N,O-dimethylhydroxylamine (0.042 g) and diisopropylethylamine (0.203 g) at 0 °C under nitrogen atmosphere The resulting reaction mixture was stirred at 0 °C for 10 min. After that propanephosphonic acid anhydride (0.375 g) was added and the reaction mixture was allowed to warm to 20 to 25 °C upon it was stirred overnight. The reaction mixture was then quenched with water and worked-up by extraction to afford 3-ethylsulfonyl-N-methoxy-N-methyl-imidazo[1,2-a]pyridine-2-carboxamide (0.08 g, 70% yield).

¹H-NMR (500 MHz, Chloroform-*d*) δ 8.89 (d, *J* = 7.0 Hz, 1H), 7.70 (dt, *J* = 9.1, 1.2 Hz, 1H), 7.44 (t, *J* = 7.9 Hz, 1H), 7.03 (t, *J* = 6.9 Hz, 1H), 3.62 (s, 3H), 3.40 (t, *J* = 7.5 Hz, 2H), 3.36 (s, 3H), 1.28 (t, *J* = 7.4 Hz, 3H). LC-MS: Mass found (M-1) =297.1.

### Step-7: Manufacture of 1-(3-ethylsulfonylimidazo[1,2-a] pyridin-2-yl)ethanone

To a stirred composition comprising 3-ethylsulfonyl-N-methoxy-N-methyl-imidazo[1,2-a]pyridine-2-carboxamide (1g) from step-6 in THF (15 mL) was added a solution of CH₃MgBr in diethyl ether (3 M, 0.006 mol) at 0 °C under nitrogen atmosphere. The resulting reaction mixture was stirred at 0°C for 30 min. Work-up of the reaction mixture afforded 1-(3-ethylsulfonylimidazo[1,2-a] pyridin-2-yl)ethanone as white solid. (0.84 g, yield: 75 %). ¹H-NMR (500 MHz, Chloroform-d) δ 9.23 (dt, *J* = 7.2, 1.2 Hz, 1H), 7.72 (dt, *J* = 9.1, 1.2 Hz, 1H), 7.44 (ddd, *J* = 9.1, 6.8, 1.2 Hz, 1H), 7.03 (td, *J* = 7.0, 1.3 Hz, 1H), 3.69 (q, *J* = 7.5 Hz, 2H), 2.71 (s, 3H), 1.27 (t, *J* = 7.4 Hz,3H). LC-MS: Mass found (M+1) = 252.1.

### Step-8: Manufacture of 2-bromo-1-(3-ethylsulfonylimidazor1,2-al pyridin-2-yl)ethanone

To a stirred composition comprising 1-(3-ethylsulfonylimidazo[1,2-a]pyridin-2-yl)ethanone (1g) in glacial CH₃COOH (10 mL) at 0 °C was added 33 wt% HBr in acetic acid solution (10 mL), which was subsequently stirred at 0 °C for 10 min. After that, Br₂ (0.04 mol) in CH2CL2 (10 mL) was added dropwise to the reaction mixture over a period of 5 min. Then, the reaction mixture was heated to 50 °C outer temperature for 2 hours. The reaction mixture was then allowed to cool to 20 to 25 °C and then slowly poured on ice, which composition was diluted with ethyl acetate (30 mL). Work-up of the composition by extraction and purification by column chromatography afforded 2-bromo-1-(3-ethylsulfonylimidazo[1,2-a] pyridin-2-yl)ethanone (1.0 g, 76% yield). ¹H NMR (500 MHz, Chloroform-*d*) δ 9.32 (dd, *J* = 7.1, 1.1 Hz, 1H), 7.82 (d, *J* = 9.2 Hz, 1H), 7.56 (ddd, *J* = 8.9, 6.8, 1.2 Hz, 1H), 7.16 (td, *J* = 7.0, 1.4 Hz, 1H), 4.82 (s, 2H), 3.76 (q, *J* = 7.4 Hz, 2H), 1.37 (s, 3H). LC-MS: Mass found (M+1) = 331.12.

### Step-9: Manufacture of 6-cyclopropyl-2-[3-(ethanesulfonyl)imidazo[1,2-a]pyridin-2-yl]-7-(trifluoromethyl)imidazo[1,2-c]pyrimidin-5-one

To a stirred compositioin comprising 2-bromo-1-[3-(ethanesulfonyl)imidazo[1,2-a]pyridin-2-yl]ethanone (0.113 g) from step-8 in (CH₃)₃COH ( 2 mL) was added 4-amino-1-cyclopropyl-6-(trifluoromethyl)pyrimidin-2-one (0.05 g). Molecular sieves were added to the resulting reaction mixture (0.4 g), which was subsequently heated to 120 °C for 24 hours. Then, the reaction mixture was filtered through a celite bed, upon which the filtrate was collected and concentrated under reduced pressure. The resulting residue was purified by column chromatography to afford 6-cyclopropyl-2-[3-(ethanesulfonyl)imidazo[1,2-a]pyridin-2-yl]-7-(trifluoromethyl)imidazo[1,2-c]pyrimidin-5-one with 91.96% purity as a yellow solid (0.044 g, 45 % yield). ¹H-NMR (500 MHz, DMSO-*d*₆) δ 9.14 - 9.02 (m, 1H), 8.31 (s, 1H), 7.86 (dd, *J* = 9.0, 1.2 Hz, 1H), 7.65(ddd, *J* = 8.8, 6.8, 1.2 Hz, 1H), 7.48 (s, 1H), 7.27 (td, *J* = 7.0, 1.3 Hz, 1H), 3.85 (q, *J* = 7.4 Hz, 2H), 3.17 (s, 1H), 1.22 (t, *J =* 7.4, Hz, 3H), 1.12 (ddt, *J* = 23.1, 7.0, 2.7 Hz, 4H). LC-MS: mass calculated 452, LC-MS: Mass found (M+1) 452.

Table B below lists compounds of formula I.A.1 by the meaning of variables R¹, R³, and D, 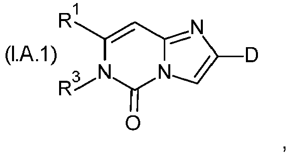 which compounds were synthesized as described in Synthesis Examples A, B, or in analogy thereto.

**Table B: compounds I.1 to !.10 with physical characterization data. & signifies the connection to the remainder of the molecule of formula I.A.1. n.m. means not measured.**

| Compound | R1 | R³ | D | ¹H-NMR data, chemical shif (δ), (500 MHz, DMSO-d6) |
|---|---|---|---|---|
| I.1 | CF₃ | CH₃ | 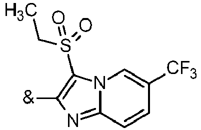 | n.m., identified by LC-MS, see above Synthesis Example A |
| I.2 | CF₃ | cyclopropyl | 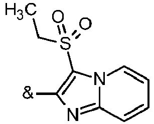 | δ 9.14 - 9.02 (m, 1H), 8.31 (s, 1H), 7.86 (dd, 1H, *J* = 9.0, 1.2 Hz,), 7.65(ddd, 1H, *J* = 8.8, 6.8, 1.2 Hz), 7.48 (s, 1H), 1H, 7.27 (td, *J* = 7.0, 1.3 Hz), 3.85 (q, 2H, *J* = 7.4 Hz), 3.17 (s, 1H), 1.22 (t, 3H, *J* = 7.4, Hz), 1.12 (ddt, 4H, *J* = 23.1, 7.0, 2.7 Hz). |
| I.3 | CF₃ | cyclopropylmethyl | 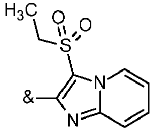 | 9.10 (d, 1H, *J* = 5 Hz), 8.40 (s, 1H), 7.87 (d, 1H, *J* = 5 Hz), 7.66 (dd, 1H, *J* = 7 Hz), 7.56 (s, 1H), 7.28 (d, 1H, *J* = 5 Hz), 4.0 (d, 2H, *J* = 7 Hz), 3.85 (t, 2H, *J* = 7 Hz), 3.18 (s, 1H), 1.22 (t, 3H, *J* = 5 Hz), 0.55 (m, 4H) |
| I.4 | CF₃ | CHCCH₂- | 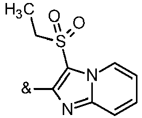 | 9.09 (dd, *J* = 7.0, 1.2 Hz, 1H), 8.43 (s, 1H), 7.87 (dt, *J* = 9.0, 1.2 Hz, 1H), 7.66 - 7.62 (m, 2H), 7.28 (td, *J* = 6.9, 1.3 Hz, 1H), 4.83 (d, *J* = 2.5 Hz, 2H), 3.83 (q, *J* = 7.4 Hz, 2H),3.45(s, 1H), 1.22 (t, *J =* 7.3 Hz, 3H) |
| I.5 | CF₃ | CF₃CH₂ | 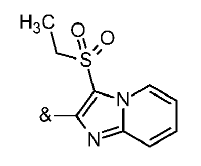 | 9.09 (d, *J*= 7.0 Hz, 1H), 8.45 (s, 1H), 7.87 (d, *J* = 9.0 Hz, 1H), 7.72 (s, 1H), 7.66 (ddd, *J* = 8.7, 6.9, 1.2 Hz, 1H), 7.28 (td, *J* = 7.0, 1.2 Hz, 1H), 4.93 (d, *J* = 8.2 Hz, 2H), 3.84 (q, *J* = 7.3 Hz, 2H), 1.23 (t, *J=* 7.3 Hz, 3H) |
| I.6 | CF₃ | cyclopropyl | 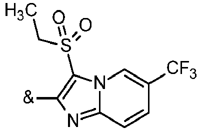 | δ 9.54 - 9.40 (m, 1H), 8.36 (s, 1H), 8.06 (d, 1H, *J* = 9.3 Hz,), 7.91 (dd, 1H, *J=* 9.5, 1.9 Hz,), 7.50 (s, 1H), 3.98 (q, 2H, *J* = 7.3 Hz,), 3.17 (d, 1H, *J* = 4.2 Hz,), 1.26 (t, 3H, *J* = 7.4 Hz,), 1.20-1.01 (m, 4H) |
| I.7 | CF₃ | CH₃ | 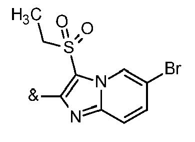 | 9.20 (d, *J* = 1.8 Hz, 1H), 8.38 (s, 1H), 7.97 - 7.70 (m, 2H), 7.53 (s, 1H), 3.91 (q, *J* = 7.3 Hz, 2H), 3.61 (s, 3H), 1.24 (t, *J* = 7.3 Hz, 3H) |
| I.8 | CF₃ | cyclopropyl | 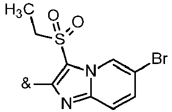 | 9.19 (d, 1H, *J* = 5 Hz), 8.32 (s, 1H), 7.8 (d, 2H, *J* = 5 Hz), 7.47 (s, 1H), 3.90 (t, 2H, *J* = 7 Hz), 3.18 (s, 1H), 1.22 (t, 3H, *J* = 5 Hz), 1.13 (m, 4H) |
| I.9 | CF₃ | cyclopropyl | 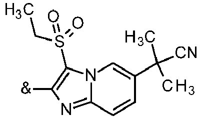 | 9.24 (d, J = 2.0 Hz, 1H), 8.32 (s, 1H), 7.93 (d, J = 9.5 Hz, 1H), 7.86 (dd, J = 9.5, 2.0 Hz, 1H), 7.48 (s, 1H), 3.90 (q, J = 7.3 Hz, 2H), 3.17 (s, 1H), 1.79 (s, 6H), 1.24 (t, J = 7.3 Hz, 3H), 1.17 - 1.04 (m, 4H) |
| I.10 | CF₃ | CH₃ | 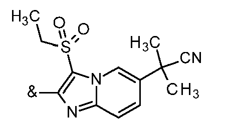 | 9.24 (d, J = 2.0 Hz, 1H), 8.32 (s, 1H), 7.93 (d, J = 9.5 Hz, 1H), 7.86 (dd, J = 9.5, 2.0 Hz, 1H), 7.48 (s, 1H), 3.90 (q, J = 7.3 Hz, 2H), 3.61 (s, 3H), 1.79 (s, 6H), 1.24 (t, J = 7.3 Hz, 3H) |

### Synthesis Example C: 6-cyclopropyl-2-(3-ethylsulfonylpyrazolof1,5-alpyridin-2-vl)-7-(trifluoromethyl)imidazo[1.2-c]pyrimidin-5-one (compound 1.11)

### Step-1: Manufacture of dimethyl pyrazolo[1,5-a]pyridine-2,3-dicarboxylate

To a composition comprising pyridine-1-ium-1-amine iodide (5 g) in DMF (50 mL) was added K₂CO₃ (6.21 g) in portions at 0 °C over a period of 10 min. Subsequently, dimethyl but-2-ynedioate (6.39 g) was added dropwise over a period of 15 minutes at the same temperature. The resulting reaction mixture was stirred at 20 to 25 °C for 12 hours. The reaction was then quenched with water. Work-up by extraction and purification by column chromatography yielded dimethyl pyrazolo[1,5-a]pyridine-2,3-dicarboxylate (2.3 g, 43.39% yield). ¹H-NMR (CDCl₃) 8.52 (d,1H), 8.17-8.19 (d,1H), 7.45-7.49 (dd,1H), 7.04-7.07 (dd.1H), 4.05 (S,3H), 3.95 (S,3H). LC-MS: Mass found (M+1) 235.

### Step-2: Manufacture of pyrazolo[1,5-a]pyridine-2-carboxylic acid

To a composition comprising dimethyl pyrazolo[1,5-a]pyridine-2,3-dicarboxylate (2.3 g) from step-1 in water (6 ml) was added H₂SO₄ (3 mL) at 0 °C. The resulting reaction mixture was heated to 110 °C for 24 hours. Subsequently, the rection mixture was cooled to 20 to 25 °C and quenched in an aqueous solution of NaOH (10 wt%) to adjust the pH to 8-9. Work-up by extraction afforded pyrazolo[1,5-a]pyridine-2-carboxylic acid (1.5 g, 78.6% yield). ¹H-NMR (DMSO-d6) 13.0 (brod S,1H), 8.73-8.74 (d,1H), 7.77-7.79 (d,1H), 7.29-7.31 (dd,1H), 7,28 (S,1H), 7.03-7.06 (dd,1H). LC-MS: Mass found (M+1)=163

### Step-3: Manufacture of methyl pyrazolo[1,5-alpyridine-2-carboxylate

To a composition comprising pyrazolo[1,5-a]pyridine-2-carboxylic acid (9 g) from step-2 in CH₃OH (150 ml) was added H₂SO₄ (10 ml) at 0 °C and the resulting reaction mixture was heated under refluxation to 80 °C for 12 hours. Subsequently, the reaction mixture was concentrated by evaporation of the solvent and the reaction was quenched in cool water. The resulting precipitate was filtered and washed with cold water to afford methyl pyrazolo[1,5-a]pyridine-2-carboxylate (8 g, 81.38% yield). 1HNMR (DMSO-d6) 8.76 (d,1H), 7.79-7.81 (d,1H), 7.31-7.34 (dd,1H), 7.13 (S,1H), 7.07-7.12 (dd,1H), 3.89 (S, 3H). LC-MS: Mass found (M+1) =177

### Step-4: Manufacture of ethyl 3-iodopyrazolo[1,5-a]pyridine-2-carboxylate

To a composition comprising methyl pyrazolo[1,5-a]pyridine-2-carboxylate ( 0.25 g) from step-3 in CH₃CN (5 ml) was added N-lodosuccinimide ( 0.447 g ) at 20 to 25 °C. The resulting reaction mixture was stirred at 40 °C for 12 hours. The reaction was quenched with water. Work-up by extraction and purification by column chromatography afforded ethyl 3-iodopyrazolo[1,5-a]pyridine-2-carboxylate (0.36 g, 84.11% yield). ¹H-NMR (CDCl₃) 8.51(d,1H), 7.61-7.63 (d,1H), 7.28-7.31 (dd,1H), 6.97-7.0 (dd,1H), 4.05 (S,3H). LC-MS: Mass found (M+1) = 303.

### Step-5: Manufacture of methyl 3-ethylsulfanylpyrazolof1,5-alpyridine-2-carboxylate

To a composition comprising ethyl 3-iodopyrazolo[1,5-a]pyridine-2-carboxylate (0.4 g) from step-4 in dioxane (10 ml) was added N,N-diisopropylethylamine (0.523 g) and the resulting reaction mixture was degassed for 20 minutes by purging with nitrogen. Then, tris(dibenzylideneacetone)-dipalladium(0) (0.030 g) and 4,5-Bis(diphenylphosphino)-9,9-dimethylxanthene (0.038 g) were added to the reaction mixture, which was subsequently was again degassed for 10 min by purging with nitrogen. Subsequently, ethanethiol (0.205 g) was added to the reaction mixture, which was subsequently heated to 90 °C for 3 hours. The reaction was then quenched with water. Work-up of the reaction mixture by extraction and column chromatography afforded methyl 3-ethylsulfanyl-pyrazolo[1,5-a]pyridine-2-carboxylate 0.29 g, 92.90 % yield. ¹H-NMR (CDCl₃) 8.50(d,1H), 7.81-7.83 (d,1H), 7.27-7.30 (dd,1H), 6.95-6.98 (dd,1H), 4.05 (S,3H), 2.85-2.90 (q,2H), 1.15-1.18 (t,3H). LC-MS: Mass found (M-1) =237.

### Step-6: Manufacture of of methyl 3-ethylsulfonylpyrazolo[1,5-a]pyridine-2-carboxylate

To a stirred solution of methyl 3-ethylsulfanylpyrazolo[1,5-a]pyridine-2-carboxylate (0.285 g, 1.20 mmol) in DCM (5 mL) was added *meta*-Chloroperoxybenzoic acid (3 equivalents) at 0 °C. The reaction mixture was then allowed to warm to 20 to 25 °C and stirred for 16 hours. The reaction was then quenched with a saturate aqueous solution of NaHCO₃. Work-up of the reaction mixture by extraction and purification by column chromatography afforded methyl 3-ethylsulfonylpyrazolo[1,5-a]pyridine-2-carboxylate (0.295 g, 91.33% yield). ¹H-NMR (CDCl₃) 8.58-8.59(d,1H), 8.34-8.36 (d,1H), 7.50-7.53 (dd,1H), 7.14-7.17 (dd,1H), 4.07 (S,3H), 3.63-3.67 (q,2H), 1.26-1.35 (t,3H). LC-MS: Mass found (M-1) =269.

### Step-7: Manufacture of 3-ethylsulfonylpyrazolo[1,5-alpyridine-2-carboxylic acid

To a composition comprising methyl 3-ethylsulfonylpyrazolo[1,5-a]pyridine-2-carboxylate (8.2 g) in water (100 mL) was added LiOH (3.28 g) at 0 °C and stirred for 10 min. Then, CH₃CH₂OH (50 mL) was added and the resulting reaction mixture was heated to 100 °C for 2 hours. Subsequently, the pH of the reaction mixture was adjusted to pH 2 by addition of HCl (2M). Work-up of the reaction mixture by extraction afforded 3-ethylsulfonylpyrazolo[1,5-a]pyridine-2-carboxylic acid pure product. (6.5 g, 83.6% yield). ¹H-NMR (DMSO-d6) 8.96-8.94 (d,1H), 8.09-8.11 (d,1H), 7.68-7.71 (dd,1H), 7.31-7.34 (dd,1H), 3.54-3.58 (q,2H), 1.06-1.11 (t,3H). LC-MS: Mass found (M+1) = 255.

### Step-8: Manufacture of 3-ethylsulfonyl-N-methoxy-N-methyl-pyrazolo[1,5-a]pyridine-2-carboxamide

To a stirred composition comprising 3-ethylsulfonylpyrazolo[1,5-a]pyridine-2-carboxylic acid (1 g) from step-7 in dry CH₂Cl₂ (20 mL) were added N,O-Dimethylhydroxylamine hydrochloride (0.496 g), diisopropylethylamine (2.03 g) at 0 °C under nitrogen atmosphere The resulting reaction mixture was then stirred at 0°C for 10 min, after which propanephosphonic acid anhydride (3.75 g) was added. The resulting reaction mixture was slowly allowed to warm up to 20 to 25 °C and it was stirred for approximately 16 hours. The reaction was then quenched with water. Work-up of the reaction mixture by extraction afforded 3-ethylsulfonyl-N-methoxy-N-methyl-pyrazolo[1,5-a]pyridine-2-carboxamide (1 g, 85.5 % yield). ¹H-NMR (CDCl₃) 8.48 (d,1H), 8.25-8.26 (d,1H), 7.42-7.45 (dd,1H), 7.01-7.04 (dd,1H), 4.03-4.07 (q,2H), 3.62 (S,3H), 3.49 (S, 3H), 1.17-1.19 (t,3H). LC-MS: Mass found (M-1) = 298.

### Step-9: Manufacture of 1-(3-ethylsulfonylpyrazolo[1,5-a]pyridin-2-yl)ethanone

To a stirred composition comprising 3-ethylsulfonyl-N-methoxy-N-methyl-pyrazolo[1,5-a]pyridine-2-carboxamide (1g) in THF (10 mL) was added a solution of CH₃MgBr in diethyl ether (3M, 6.73 mmol) at 0 °C under nitrogen atmosphere. The resulting reaction mixture was then stirred at 0 °C for 30 min. The reaction mixture was then diluted with a 1N aqueous solution of HCl (10 mL). Work-up of the resulting mixture by extraction afforded off white solid 1-(3-ethylsulfonylpyrazolo[1,5-a]pyridin-2-yl)ethanone (0.6 g, 70.75 % yield). ¹H-NMR (CDCl₃) 8.44 (d,1H), 8.27-8.29 (d,1H), 7.41-7.43 (dd,1H), 7.07-7.08 (dd,1H), 3.58-3.62 (q,2H), 2.68 (S,3H), 1.20-1.24 (t,3H). LC-MS: Mass found (M+1) = 253.

### Step -10: Manufacture of 2-bromo-1-(3-ethylsulfonylpyrazolo[1.5-a]pyridin-2-yl)ethanone

To a composition comprising 1-(3-ethylsulfonylpyrazolo[1 ,5-a]pyridin-2-yl)ethanone (3.8 g) from step-9 in toluene ( 50 ml) was added 33% HBr in CH₃COOH (15 ml) at 25 °C dropwise over a period of 25 min. To this reaction mixture was added Br₂ (2.44 g) dropwise over a period of 20 min, upon which the reaction mixture was stirred at 25 °C for 16 hours. The reaction was then quenched with water. Work-up by extraction and recrystallization in heptane : ethyl acetate (8:2) afforded pure 2-bromo-1-(3-ethylsulfonylpyrazolo[1,5-a]pyridin-2-yl)ethanone (3.5 g, 70.10% yield). ¹H-NMR (CDCl₃) 8.58 (d,1H), 8.39 (d,1H), 7.5 (dd,1H), 7.19 (dd,1H), 4.74 (S,2H) 3.65-3.67 (q,2H), 1.31-1.34 (t,3H). LC-MS: Mass found (M+1) = 331.

### Step - 11: Synthesis of 6-cyclopropyl-2-(3-ethylsulfonylpyrazolo[1,5-a]pyridin-2-yl)-7-(trifluoromethyl)imidazor1,2-clpyrimidin-5-one (compound 1.11)

To a stirred composition comprising 1-cyclopropyl-4-imino-6-(trifluoromethyl)pyrimidin-2-one (2 g) in (CH₃)₃COH (20 mL) was added 2-bromo-1-(3-ethylsulfonylpyrazolo[1,5-a]pyridin-2-yl)ethanone (3.6 g). Molecular sieves was added to the resulting reaction mixture reaction mixture (2 g), which was subsequently heated to 120 °C for 24 hours. The reaction mixture was then filtered through a celite bed and the filtrate was collected and concentrated under reduced pressure. The resulting residue was purified by column chromatography to afford6-cyclopropyl-2-(3-ethylsulfonylpyrazolo[1,5-a]pyridin-2-yl)-7-(trifluoromethyl)imidazo[1,2-c]pyrimidin-5-one as an of white solid (1.5 g, 50% yield). H'-NMR (500 MHz, DMSO-d6): δ 8.95 (d, 1H, *J* = 5 Hz), 8.40 (s, 1H), 8.13 (d, 1H, *J* = 5 Hz ), 7.66 (dd, 1H, *J* = 7 Hz), 7.49 (s, 1H), 7.26 (d, 1H, *J* = 5 Hz), 3.66 (t, 2H, *J* = 7 Hz), 3.18 (s, 1H), 1.22 (t, 3H, *J* = 5 Hz), 1.13 (m, 4H). LC-MS: mass calculated [M+1]⁺ 452.42, LC-MS: Mass found 452.0.

Table C below lists compounds of formula I.A.1 by the meaning of variables R¹, R³, and D, 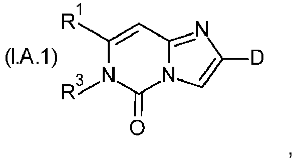 which compounds were synthesized as described in Synthesis Example C, or in analogy thereto.

**Table C: compounds I.11 to I.12 with physical characterization data. & signifies the connection to the remainder of the molecule of formula I.A.1.**

| Compound | R¹ | R³ | D | ¹H-NMR data, δ, (500 MHz), solvent in brackets |
|---|---|---|---|---|
| I.11 | CF₃ | cyclopropyl | 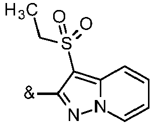 | 8.95 (d, 1H, *J* = 5 Hz), 8.40 (s, 1H), 8.13 (d, 1H, *J* = 5 Hz), 7.66 (dd, 1H, *J* = 7 Hz), 7.49 (s, 1H), 7.26 (d, 1H, *J* = 5 Hz), 3.66 (t, 2H, *J* = 7 Hz), 3.18 (s, 1H), 1.22 (t, 3H, *J* = 5 Hz), 1.13 (m, 4H). (CDCl₃) |
| I.12 | CF₃ | CH₃ | 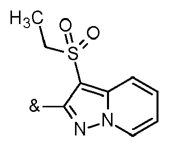 | 8.96 (d, *J* = 6.9 Hz, 1H), 8.46 (s, 1H), 8.14 (d, *J* = 9.0 Hz, 1H), 7.6 (dd, *J* = 9.1, 6.9 Hz, 1H), 7.55 (s, 1H), 7.28 (t, *J* = 6.9 Hz, 1H), 3.65 (q, *J* = 7.4 Hz, 2H), 3.62 (s, 3H), 1.16 (t, *J* = 7.3 Hz, 3H) (DMSO-d6) |

### Synthesis Example D: 2-(3-ethylsulfonyl-5,6,7,8-tetrahydroimidazof1,2-alpyridin-2-yl)-6-methyl-7-(trifluoromethyl)imidazo[1,2-c]pyrimidin-5-one (compound 1.13)

### Step-1: Manufacture of ethyl 5,6,7,8-tetrahydroimidazo[1,2-a]pyridine-2-carboxylate

A composition comprising ethyl imidazo[1,2-a]pyridine-2-carboxylate (5.2 g) as obtained in Synthesis Example B, step-1, in THF (100 mL), CH₃CH₂OH (100mL), CH₃COOH (10 mL) and Pd on carbon (10 %,1.44 g) was prepared. Subsequently, H₂-gas at a pressure of 5 bar pressure was applied to the compositon for 16 hours. The resulting compositon was filtered, and concentrated by evaporation under reduced pressure. Work-up by extraction afforded ethyl 5,6,7,8-tetrahydro-imidazo[1,2-a]pyridine-2-carboxylate.(4.61 g, 86.81% yield). ¹H-NMR (CDCl₃) 7.74(S, 1H),4.18-4.21 (q, 2H), 3.97-3.99 (t, 2H) 2.72-2.74 (t,2H) 1.83-1.89 (m, 4H), 1.23-1.26 (t, 3H). LC-MS: Mass found (M+1) 195.

### Step-2: Manufacture of 1-(3-ethylsulfonyl-5,6,7,8-tetrahydroimidazo[1,2-a]pyridin-2-yl)ethanone

The compound ethyl 5,6,7,8-tetrahydroimidazo[1,2-a]pyridine-2-carboxylate from step-1 was converted in process steps analogous to Synthesis Example C, step-4 to step-9 to compound 1-(3-ethylsulfonyl-5,6,7,8-tetrahydroimidazo[1,2-a]pyridin-2-yl)ethanone. ¹H-NMR (DMSO-d6) 4.22-4.25 (t, 2H), 3.57-3.60 (t,2H), 2.85-2.88 (t,2H), 2.51 (S, 3H) 1.92-1.94 (t, 2H) 1.84-1.86 (t, 2H) 1.19 (t, 3H), LC-MS: Mass found (M+1) 257.

### Step-3: Manufacture of 2-bromo-1-(3-ethylsulfonyl-5,6,7,8-tetrahydroimidazo[1,2-a]pyridin-2-yl)ethanone

To a solution comprising 1-(3-ethylsulfonyl-5,6,7,8-tetrahydroimidazo[1,2-a]pyridin-2-yl)ethanone (2 g) from step-2 in dioxane (30 mL) was added pyridinium tribromide (2.5 g) at 25 °C and the resulting reaction mixture was heated to 105 °C for 2 hours. Quenching with water, extraction of the reaction mixture and purification of the extracted crude product by column chromatography afforded 2-bromo-1-(3-ethylsulfonyl-5,6,7,8-tetrahydroimidazo[1,2-a]pyridin-2-yl)ethanone. (0.9g, 35.24% yield). ¹H-NMR (DMSO-d6) 4.76 (s, 2H), 4.25-4.27 (t, 2H) 3.57-3.61 (q,2H), 2.87-2.89 (t,2H), 1.82-1.96 (m, 4H) 1.25 (t, 3H), LC-MS: Mass found (M+1) 335.

### Step-4: Manufacture of 2-(3-ethylsulfonyl-5,6,7,8-tetrahydroimidazof1,2-alpyridin-2-yl)-6-methyl-7-(trifluoromethyl)imidazo[1,2-c]pyrimidin-5-one (compound 1.13)

To a stirred composition comprising 4-amino-1-methyl-6-(trifluoromethyl)pyrimidin-2-one (0.15 g) in tert-butanol (2 mL) was added 2-bromo-1-(3-ethylsulfonyl-5,6,7,8-tetrahydroimidazo[1,2-a]pyridin-2-yl)ethanone (0.25 g) from step-3. Molecular sieves was added to the above reaction mixture (0.2 g), which was subsequently heated to 120 °C for 24 hours. Then, the reaction mixture was filtered through a celite bed, whereupon the filtrate was collected and concentrated under reduced pressure to afford a crude product. Purification of the crude product by column chromatography afforded compound I.12 as a yellow solid (0.12 g, 33.4 % yield). H¹ NMR (500 MHz, DMSO-d6): 8.12 (s, 1H), 7.45 (s, 1H), 4.22 (t, 2H), 3.63 (q, 2H), 3.33 (s, 3H), 2.74 (t, 2H), 1.20 (q, 2H), 1.19 (q, 2H), 1.18 (t, 3H), LC-MS: mass calculated [M+1] 429, LC-MS: Mass found (M+1) 430.0.

Table D below lists compounds of formula I.A.1 by the meaning of variables R¹, R³, and D, 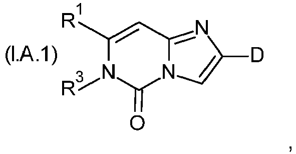 which compounds were synthesized as described in Synthesis Example D, or in analogy thereto.

**Table D: compounds 1.13 to 1.14 with physical characterization data. & signifies the connection to the remainder of the molecule of formula I.A.1.**

| Compound | R¹ | R³ | D | ¹H-NMR data, δ, (500 MHz), solvent in brackets |
|---|---|---|---|---|
| I.13 | CF₃ | CH₃ | 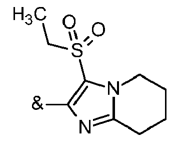 | 8.12 (s, 1H), 7.45 (s, 1H), 4.22 (t, 2H), 3.63 (q, 2H), 3.33 (s, 3H), 2.74 (t, 2H), 1.20 (q, 2H), 1.19 (q, 2H), 1.18 (t, 3H) |
| I.14 | CF₃ | cyclopropyl | 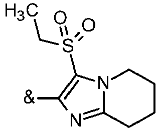 | 8.07 (s, 1H), 7.40 (s, 1H), 4.22 (m, 2H), 3.95 (t, 2H), 3.18 (s, 1H), 2.89 (m, 2H), 1.91 (m, 2H), 1.86 (m, 2H) 1.22 (t, 3H), 1.14 (s, 2H). 1.13 (s, 2H). |

### Synthesis Example E: 6-cyclopropyl-2-(3-ethylsulfonylimidazof 1,2-alpyrimidin-2-yl)-7-(trifluoromethyl)imidazo[1,2-clpyrimidin-5-one (compound 1.15)

### Step-1: Manufacture of 1-imidazo[1,2-a]pyrimidin-2-ylethanone

To a composition comprising 2-aminopyrimidine (1 g) in acetone (10 mL) was added slowly 1-bromobutane-2,3-dione (1.5 g) dropwise over a period a time of 10 min at 20 to 25 °C. Subsequently, the resulting reaction mixture was heatet under refluxation for 2 hours. The solid precipitate was dissolved in a mixture of CH₃CH₂OH : H₂O (weight ratio 10 : 3) and heated to 64 °C. After cooling to 20 to 25 °C and work-up by concentration under reduced pressure, 1-imidazo[1,2-a]pyrimidin-2-ylethanone was afforded (1.0 g, 60% yield). ¹H-NMR (CDCl₃) 9.01-8.99(dd, 1H), 8.69-8.68 (dd,1H), 8.45 (S,1H), 7.17-7.15 (dd, 1H),2.58 (S,3H), LC-MS: Mass found (M+1) 161.1.

### Step-2: Manufacture of 1-(3-chloroimidazo[1,2-a]pyrimidin-2-yl)ethanone

To a stirred composition comprising 1-imidazo[1,2-a]pyrimidin-2-ylethanone (1g) from step-1 in CHCl₃ (10 mL) was added Palau' chlor (0.913 g) at 20 to 25 °C under nitrogen atmosphere. The resulting reaction mixture was stirred at the same temperature for approximately 15 hours. The reaction was subsequently quenched by addition of water and worked-up by extraction to afford 1-(3-chloroimidazo[1,2-a]pyrimidin-2-yl)ethanone as white solid. (1.0 g, 60% yield). ¹H-NMR (d6-CDCl₃) 8.78-8.75 (m,2H), 7.32-7.29 (m,1H), 2.65 (S, 3H), LC-MS: Mass found (M+1) 195.1.

### Step-3: Manufacture of 1-(3-ethylsulfanylimidazo[1,2-a]pyrimidin-2-yl)ethanone

To a stirred composition comprising 1-(3-chloroimidazo[1,2-a]pyrimidin-2-yl)ethanone (4g) from step-2 in THF (40 mL) was added sodium ethanethiolate (2.24 g) at 0 °C. The resulting reaction mixture was stirred at 0 °C for 2 hours. The reaction was then quenched with water. Work-up of the resulting mixture by extraction afforded 1-(3-ethylsulfanylimidazo[1,2-a]pyrimidin-2-yl)ethanone (2.0 g, yield: 60 %). ¹H-NMR (d6-CDCl₃) 9.07-9.06(d,1H), 8.78-8.77 (dd,1H), 7.30-7.27(dd,1H), 3.61(S,3H), 2.93(q,2H), 1.06(t,3H), LC-MS: Mass found (M+1) 221.1.

### Step-4: Manufacture of 1-(3-ethylsulfonylimidazo[1,2-a]pyrimidin-2-yl)ethanone

To a stirred composition comprising ethyl 3-ethylsulfanylimidazo[1,2-a]pyridine-2-carboxylate (5g) in CH₂Cl₂ (100mL) was added *meta*-Chloroperoxybenzoic acid (3eq) at 0 °C. The resulting reaction mixture was then allowed to warm up to 20 to 25 °C and then stirred for 16 hours. The reaction was diluted with water (50 mL) and an aqueous saturated solution of sodium bisulphite was added (50 mL). The reaction mixture was stirred for another 10 minutes, upon which 50 mL of an aqueous solution of NaHCO₃ (10 wt%) was added. Extraction of the reaction mixture afforded 1-(3-ethylsulfonylimidazo[1,2-a]pyrimidin-2-yl)ethanone as yellow colour solid. (3g, yield: 65%). ¹H-NMR(CDCl₃): 9.50-9.48(dd,1H), 8.99-8.98(d,1H), 7.61-7.55(dd,1H), 3.70(s,3H), 3.76-3.72(q,2H), 1.30-1.34(t,3H), LC-MS: Mass found (M+1) 254.2

### Step-5: Manufacture of 2-bromo-1-(3-ethylsulfonylimidazof1,2-alpyrimidin-2-yl)ethanone

A composition comprising 1-(3-ethylsulfonylimidazo[1,2-a]pyrimidin-2-yl)ethanone (0.3g) from step-4 in toluene (3 mL) at 0 °C was prepared. Subsequently, a composition of 33 wt% HBr in CH₃COOH (1 mL) was added to the reaction mixture at 0 °C. The reaction mixture was then stirred for 10 min. After that, Br₂ (0.004 mol) was added dropwise to the reaction mixture, which was then stirred at 20 to 25 °C for approximately 15 hours. Work-up of the reaction mixture by quenching with water, extraction, and column chromatography afforded 2-bromo-1-(3-ethylsulfonylimidazo[1,2-a]pyrimidin-2-yl)ethanone (0.1 g, 60 % yield). ¹H-NMR (DMSO-d6) 9.96(m,1H),8.97 (m,1H),7.50 (m,1H),5.02 (S,2H),3.74 (q,2H), 1.26 (t,3H), LC-MS: Mass found (M+1) 331.12.

### Step-6: Manufacture of 6-cyclopropyl-2-(3-ethylsulfonylimidazo[1,2-a]pyrimidin-2-yl)-7-(trifluoromethyl)imidazo[1,2-c]pyrimidin-5-one (compound 1.15)

To a stirred composition of of 1-cyclopropyl-4-imino-6-(trifluoromethyl)pyrimidin-2-one (0.15 g, 6.8 mmol) in tert-butanol ( 2mL) was added 2-bromo-1-(3-ethylsulfonylimidazo[1,2-a]pyrimidin-2-yl)ethanone (0.23 g, 6.8 mol). Molecular sieves was added to the above reaction mixture (0.2 g) and the resultant reaction mixture was heated to 120 °C for 24 hours. Subsequently, the reaction mixture was filtered through a celite bed, and the filtrate was collected and concentrated under reduced pressure. The resulting residue was subjected to column chromatography to afford 6-cyclopropyl-2-(3-ethylsulfonylimidazo[1,2-a]pyrimidin-2-yl)-7-(trifluoromethyl)imidazo[1,2-c]pyrimidin-5-one as a yellow solid (0.12 g, 30.9 % yield). H¹ NMR (500 MHz, DMSO-d6): δ 9.42 (q, 1H), 8.85 (q, 1H), 8.37 (s, 1H), 7.50 (s, 1H), 7.38 (q, 1H), 3.95 (t, 2H), 3.18 (s, 1H), 1.22 (t, 3H), 1.14 (s, 2H). 1.13 (s, 2H). LC-MS: mass calculated [M+1]⁺ 453.4, LC-MS: Mass found (M+1) 453.0.

### B. Biological Examples

The activity of the compounds of formula (I) of the present invention could be demonstrated and evaluated in biological tests described in the following. If not otherwise specified, the test solutions are prepared as follows: The active compound is dissolved at the desired concentration in a mixture of 1:1 (vol:vol) distilled water: acteone. The test solution is prepared at the day of use. Test solutions are prepared in general at concentrations of 2500 ppm, 1415 ppm, 828 ppm, 800 ppm and 300 ppm (wt/vol).

### Boll weevil (Anthonomus grandis)

For evaluating control of boll weevil (*Anthonomus grandis*) the test unit consisted of 96-well-microtiter plates containing an insect diet and 5-10 *A*. *grandis* eggs. The compounds were formulated using a solution containing 75% v/v water and 25% v/v DMSO. Different concentrations of formulated compounds were sprayed onto the insect diet at 5 µl, using a custom built micro atomizer, at two replications. After application, microtiter plates were incubated at about 25 ± 1°C and about 75 ± 5 % relative humidity for 5 days. Egg and larval mortality was then visually assessed. In this test, compounds I.2, I.3, I.4, I.5, and I.6 at 828 ppm showed over 75% mortality in comparison with untreated controls. In this test, compounds I.1, I.8, I.11 and I.13 at 800 ppm showed over 75% mortality in comparison with untreated controls.

### Tobacco budworm (Heliothis virescens)

For evaluating control of tobacco budworm (*Heliothis virescens*) the test unit consisted of 96-well-microtiter plates containing an insect diet and 15-25 *H. virescens* eggs. The compounds were formulated using a solution containing 75% v/v water and 25% v/v DMSO. Different concentrations of formulated compounds were sprayed onto the insect diet at 10 µl, using a custom built micro atomizer, at two replications. After application, microtiter plates were incubated at about 28 ± 1°C and about 80 ± 5 % relative humidity for 5 days. Egg and larval mortality was then visually assessed. In this test, compound 1.1, I.3, I.4, I.5, and I.6 at 2500 ppm showed over 75% mortality in comparison with untreated controls. In this test, compound I.8, and !.11 at 800 ppm showed over 75% mortality in comparison with untreated controls.

### Vetch aphid (Megoura viciae)

For evaluating control of vetch aphid (*Megoura viciae*) through contact or systemic means the test unit consisted of 24-well-microtiter plates containing broad bean leaf disks.

The compounds were formulated using a solution containing 75% v/v water and 25% v/v DMSO. Different concentrations of formulated compounds were sprayed onto the leaf disks at 2.5 µl, using a custom built micro atomizer, at two replications. After application, the leaf disks were air-dried and 5 - 8 adult aphids placed on the leaf disks inside the microtiter plate wells. The aphids were then allowed to suck on the treated leaf disks and incubated at about 23 ± 1°C and about 50 ± 5 % relative humidity for 5 days. Aphid mortality and fecundity was then visually assessed. In this test, compound 1.1 at 2500 ppm showed over 75% mortality in comparison with untreated controls.

### Green Peach Aphid (Myzus persicae)

For evaluating control of green peach aphid (*Myzus persicae*) through systemic means the test unit consisted of 96-well-microtiter plates containing liquid artificial diet under an artificial membrane. The compounds were formulated using a solution containing 75% v/v water and 25% v/v DMSO. Different concentrations of formulated compounds were pipetted into the aphid diet, using a custom built pipetter, at two replications. After application, 5-8 adult aphids were placed on the artificial membrane inside the microtiter plate wells. The aphids were then allowed to suck on the treated aphid diet and incubated at about 23 ± 1°C and about 50 ± 5 % relative humidity for 3 days. Aphid mortality and fecundity was then visually assessed. In this test, compound I.1, I.2, I.3, I.4, I.5, and I.6 at 2500 ppm showed over 75 % mortality in comparison with untreated controls. In this test, compounds I.7, I.8, I.11, and 1.12, at 800 ppm showed over 75 % mortality in comparison with untreated controls.

### Greenhouse Whitefly (Trialeurodes vaporarirorum)

For evaluating control of Greenhouse Whitefly (*Trialeurodes vaporariorum*) the test unit consisted of 96-well-microtiter plates containing a leaf disk of egg plant leaf disk with white fly eggs. The compounds or mixtures were formulated using a solution containing 75% water and 25% DMSO. Different concentrations of formulated were sprayed onto the insect diet at 2.5µl, using a custom-built micro atomizer, at two replications. After application, microtiter plates were incubated at 23 ± 1°C, 65 ± 5 % RH for 6 days. Mortality of hatched crawlers was then visually assessed. In this test, compounds 1.2 and I.6 at 2500 ppm showed at least 75 % mortality in comparison with untreated controls. In this test, compound I.12 at 800 ppm showed at least 75 % mortality in comparison with untreated controls.

### Yellow Fever Mosquito (Aedes aegypti)

For evaluating control of yellow fever mosquito (*Aedes aegypti)* the test unit consisted of 96- well-microtiter plates containing 200pl of tap water per well and 5-15 freshly hatched *A*. *aegypti* 5 larvae. The active compounds were formulated using a solution containing 75% (v/v) water and 25% (v/v) DMSO. Different concentrations of formulated compounds or mixtures were sprayed onto the insect diet at 2.5µl, using a custom-built micro atomizer, at two replications. After application, microtiter plates were incubated at 28 + 1 °C, 80 + 5 % RH for 2 days. Larval 10 mortality was then visually assessed. In this test, compounds I.1, I.2, I.3, I.4, I.5, and I.6 at 2500 ppm showed at least 75 % mortality in comparison with untreated controls. In this test, compound I.8 and !.11 at 800 ppm showed at least 75 % mortality in comparison with untreated controls.

### Southern Green Stink Bug (Nezara viridula)

The active compound is dissolved at the desired concentration in a mixture of 1:1 (vokvol) distilled water: acetone. Surfactant (KineticR HV) is added at a rate of 0.01 % (vol/vol). The test solution is prepared at the day of use. Soybean pods were placed in 90 x 50 mm glass Petri dishes lined with moist filter paper and inoculated with ten late 3rd instar N. viridula. Using a hand atomizer, an approximately 2 ml solution is sprayed into each Petri dish. Treated cups were kept at about 25-26°C and relative humidity of about 65-70%. Percent mortality was recorded after 5 days. In this test, compound I.6 and I.8 at 300 ppm showed at least 75% mortality in comparison with untreated controls.

## Claims

1. A compound of formula (I) 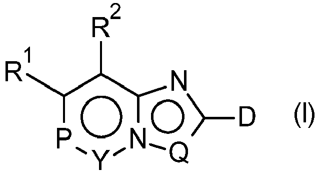 wherein
the circle in ring represents that ring is fully unsaturated;
Y is C=X, wherein X is O or S;
P is N(R³) or C(R⁴);
Q is N(R³) or C(R⁶);
R¹ is H, halogen, C₁-C₆-alkyl, C₂-C₆-alkenyl, C₂-C₆-alkynyl, C₁-C₆-alkoxy, C₁-C₆-alkoxy-C₁-C₆-alkyl, C₃-C₆-cycloalkyl, C₃-C₆-cycloalkoxy, C₁-C₆-sulfenyl, C₁-C₆-sulfinyl, or C₁-C₆-sulfonyl, which groups are unsubstituted or halogenated;
R³, R⁵ are independently C₁-C₆-alkyl, C₁-C₆-alkoxy, C₂-C₆-alkenyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkyl-C₁-C₄-alkyl, or C₃-C₆-cycloalkoxy-C₁-C₄-alkyl, which are unsubstituted or halogenated; C(=O)OR^{A}, NR^{B}R^{C}, C₁-C₆-alkylen-NR^{B}R^{C}, O-C₁-C₆-alkylen-NR^{B}R^{C}, C₁-C₆-alkylen-CN, NH-C₁-C₆-alkylen-NR^{B}R^{C}, C(=O)NR^{B}R^{C}, C(=O)R^{D}, C(=S)R^{D}, SO₂NR^{B}R^{C}, S(=O)ₙR^{E}; phenyl or benzyl, wherein the phenyl ring is unsubstituted or substituted with one or more, same or different substituents R^{F};
R², R⁴, R⁶ are independently H, halogen, N₃, CN, NO₂, SCN, SF₅; C₁-C₆-alkyl, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxyx-C₁-C₄-alkyl, which groups are unsubstituted or substituted with halogen, C(=O)OR^{A}, NR^{B}R^{C}, NOR^{A}, ONR^{B}R^{C}, C₁-C₆-alkylen-NR^{B}R^{C}, O-C₁-C₆-alkylen-NR^{B}R^{C}, C₁-C₆-alkylen-CN, NH-C₁-C₆-alkylen-NR^{B}R^{C}, C(=O)NR^{B}R^{C}, C(=O)R^{D}, C(=S)R^{D}, SO₂NR^{B}R^{C}, S(=O)ₙR^{E}_{;} phenyl or benzyl, wherein the phenyl ring is unsubstituted or substituted with one or more, same or different substituents R^{F};
D is a fused bicyclic ring of the following formula 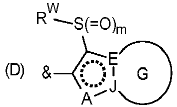
wherein the "&"-symbol signifies the connection to the remainder of formula (I), wherein the dotted circle in the 5-membered ring means that the 5-membered ring may be saturated, partially unsaturated, or fully unsaturated; and wherein
A is N, S, O, CR⁷, or NR⁸;
E, J are independently C or N, wherein at least one of the variables selected from E and J is C;
G is a 5- or 6-membered saturated, partially unsaturated, or fully unsaturated carbo- or heterocycle, which carbo- or heterocycle includes the atoms E and J as ring members and is unsubstituted, or substituted with one or more, same or different substituents R⁹, and wherein said heterocycle comprises no, one or more, same or different heteroatoms O, N, or S in addition to those that may be present as ring members E and J;
R⁷ is H, halogen, OH, CN, NC, NO₂, N₃, SCN, NCS, NCO, SF₅; C₁-C₆-alkyl, C₃-C₆-cycloalkyl, C₂-C₆-alkenyl, C₃-C₆-cycloalkenyl, C₂-C₆-alkynyl, which groups are unsubstituted, or substituted with one or more, same or different substituents R^{G};
a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents R^{H}, and wherein said N- and S-atoms are independently oxidized, or non-oxidized; phenyl, which is unsubstituted, or substituted with one or more, same or different substituents R^{J};
OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T};
R⁸ is H, halogen, CN;
C₁-C₆-alkyl, C₃-C₆-cycloalkyl, C₂-C₆-alkenyl, C₃-C₆-cycloalkenyl, C₂-C₆-alkynyl, which groups are unsubstituted, or substituted with one or more, same or different substituents R^{G};
a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents R^{H}, and wherein said N- and S-atoms are independently oxidized, or non-oxidized; phenyl, which is unsubstituted, or substituted with one or more, same or different substituents R^{J};
OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T};
each R⁹ is independently H, halogen, OH, CN, NC, NO₂, N₃, SCN, NCS, NCO, SF₅; C₁-C₆-alkyl, C₃-C₆-cycloalkyl, C₂-C₆-alkenyl, C₃-C₆-cycloalkenyl, C₂-C₆-alkynyl, C₃-C₆-cycloalkyl-C₁-C₃-alkyl, which groups are unsubstituted, or substituted with one or more, same or different substituents R^{G};
a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents R^{H}, and wherein said N- and S-atoms are independently oxidized, or non-oxidized; phenyl, which is unsubstituted, or substituted with one or more, same or different substituents R^{J};
OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T};
or two substituents R⁹ form, together with the ring members of ring G to which they are bound, a 5- or 6- membered saturated, partially unsaturated, or fully unsaturated carbo- or heterocycle, which carbo- or heterocycle is unsubstituted, or substituted with one or more, same or different substituents R^{J}, and wherein said heterocycle comprises one or more, same or different heteroatoms O, N, or S;
each R^{A} is independently H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; C₁-C₆-alkylen-NR^{b}R^{c}, C₁-C₆-alkylen-CN; or phenyl or benzyl, wherein the phenyl ring is unsubstituted or substituted with one or more, same or different substitutents R^{F};
each R^{B} is independently H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen;
C₁-C₆-alkylen-CN;
phenyl and benzyl, which groups are unsubstituted or substituted with one or more, same or different substituents R^{F};
each R^{C} is independently H; C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; C₁-C₆-alkylen-CN; or phenyl or benzyl, wherein the phenyl ring is unsubstituted or substituted with one or more, same or different substituents R^{F};
each moiety NR^{B}R^{C} may also form an N-bound, saturated 5- to 8-membered heterocycle, which in addition to the nitrogen atom may have 1 or 2 further heteroatoms or heteroatom moieties selected from O, S(=O)ₘ and N-R', wherein R' is H or C₁-C₆-alkyl and wherein the N-bound heterocycle is unsubstituted or substituted with one or more, same or different substituents selected from halogen, C₁-C₄-alkyl, C₁-C₄-haloalkyl, C₁-C₄-alkoxy and C₁-C₄-haloalkoxy;
each R° is independently H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; phenyl or benzyl, wherein the phenyl ring is unsubstituted or substituted with one or more, same or different substituents R^{F};
each R^{E} is independently C₁-C₆-alkyl, C₃-C₆-cycloalkyl, C₃-C₆-cycloalkyl-C₁-C₄-alkyl, which are unsubstituted or substituted with halogen; or phenyl or benzyl, wherein the phenyl ring is unsubstituted or substituted with one or more, same or different substituents R^{F};
each R^{F} is independently halogen, N₃, OH, CN, NO₂, SCN, SF₅;
C₁-C₆ alkyl, C₁-C₆ alkoxy, C₂-C₆ alkenyl, C₂-C₆ alkynyl, C₁-C₆ alkoxy-C₁-C₄ alkyl, C₁-C₆ alkoxy-C₁-C₄ alkoxy, C₃-C₆ cycloalkyl, C₃-C₆ cycloalkoxy, C₃-C₆ cycloalkyl-C₁-C₄ alkyl, C₃-C₆ cycloalkoxy-C₁-C₄ alkyl, which groups are unsubstituted or substituted with halogen;
each R^{G} is independently halogen, OH, CN, NC, NO₂;
C₁-C₆-alkyl, C₃-C₆-cycloalkyl, C₃-C₆-cycloalkenyl, which groups are unsubstituted, or substituted with one or more, same or different substituents selected from halogen, OH, CN, C₁-C₃-alkoxy, C₁-C₃-haloalkoxy, and C₁-C₃-alkyl-carbonyl; a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents selected from halogen, OH, CN, C₁-C₃-alkoxy, C₁-C₃-haloalkoxy, and C₁-C₃-alkyl-carbonyl, and wherein said N- and S-atoms are independently oxidized, or non-oxidized;
phenyl, which is unsubstituted, or substituted with one or more, same or different substituents selected from halogen, OH, CN, NO₂, C₁-C₃-alkyl, C₁-C₃-haloalkyl, C₁-C₃-alkoxy, C₁-C₃-haloalkoxy, and C₁-C₃-alkyl-carbonyl; OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T};
each R^{H} is independently halogen, CN, NC, NO₂, SCN, NCS, NCO;
C₁-C₆-alkyl, C₃-C₆-cycloalkyl, C₃-C₆-cycloalkenyl, which groups are unsubstituted, or substituted with one or more, same or different substituents selected from halogen, OH, CN, C₁-C₁₀-alkoxy, C₁-C₃-haloalkoxy, and C₁-C₃-alkyl-carbonyl; phenyl, which is unsubstituted, or substituted with one or more, same or different substituents selected from halogen, OH, CN, NO₂, C₁-C₃-alkyl, C₁-C₃-haloalkyl, OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T}; or
two geminal substituents R^{H} form together with the atom to which they are bound a group =O, =S, or =NR^{L}.
each R^{J} is independently halogen, CN, NC, NO₂, SCN, NCS, NCO;
C₁-C₆-alkyl, C₃-C₆-cycloalkyl, C₃-C₆-cycloalkenyl, which groups are unsubstituted, or substituted with one or more, same or different substituents selected from halogen, OH, CN, C₁-C₁₀-alkoxy, C₁-C₃-haloalkoxy, and C₁-C₃-alkyl-carbonyl; phenyl, which is unsubstituted, or substituted with one or more, same or different substituents selected from halogen, OH, CN, NO₂, C₁-C₃-alkyl, C₁-C₃-haloalkyl, OR^{K}, SR^{K}, OC(=O)R^{K}, OC(=O)OR^{K}, OC(=O)NR^{L}R^{M}, OC(=O)SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T};
each R^{K} is independently H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with one or more, same or different substituents selected from halogen, CN, NR^{M}R^{N}; C(=O)NR^{M}R^{N}, C(=O)R^{T}; or phenyl or benzyl, wherein the phenyl ring is unsubstituted or substituted with one or more, same or different substitutents R^{X};
each R^{L} is independently H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; C₁-C₆-alkylen-CN;
phenyl and benzyl, which groups are unsubstituted or substituted with one or more, same or different substituents R^{X};
each R^{M}, R^{R} is independently H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; C₁-C₆-alkylen-CN; or phenyl or benzyl, wherein the phenyl ring is unsubstituted or substituted with one or more, same or different substituents R^{X};
each moiety NR^{M}R^{R} or NR^{L}R^{M} may also form an N-bound, saturated 5- to 8-membered heterocycle, which in addition to the nitrogen atom may have 1 or 2 further heteroatoms or heteroatom moieties selected from O, S(=O)ₘ and N-R', wherein R' is H or C₁-C₆-alkyl and wherein the N-bound heterocycle is unsubstituted or substituted with one or more, same or different substituents selected from halogen, C₁-C₄-alkyl, C₁-C₄-haloalkyl, C₁-C₄-alkoxy and C₁-C₄-haloalkoxy;
each R^{N} is independently H, halogen, CN, NO₂, SCN;
C₁-C₁₀-alkyl, C₃-C₈-cycloalkyl, C₂-C₆-alkenyl, C₃-C₆-cycloalkenyl, C₂-C₆-alkynyl, which groups are unsubstituted, or substituted with one or more, same or different substituents selected from halogen, C₁-C₆-alkyl, C₁-C₆-alkoxy, C₁-C₆-haloalkyl, and C₁-C₆-haloalkoxy; a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents selected from halogen, C₁-C₃-alkyl, C₁-C₃-alkoxy, C₁-C₃-haloalkyl, and C₁-C₃-haloalkoxy, and wherein said N- and S-atoms are independently oxidized, or non-oxidized;
phenyl, which is unsubstituted, or substituted with one or more, same or different substituents selected from halogen, C₁-C₃-alkyl, C₁-C₃-alkoxy, C₁-C₃-haloalkyl, and C₁-C₃-haloalkoxy;
each R° is independently H, C₁-C₄-alkyl, C₁-C₆-cycloalkyl, C₁-C₂-alkoxy-C₁-C₂-alkyl, phenyl, or benzyl;
each R^{P} is independently H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen; phenyl or benzyl, wherein the phenyl ring is unsubstituted or substituted with one or more, same or different substituents R^{X};
each R^{S}, R^{T} is independently H, C₁-C₆-alkyl, C₁-C₆-haloalkyl, C₁-C₆-alkoxy, C₁-C₄-alkoxy-C₁-C₄-alkyl, C₃-C₆-cycloalkyl, C₃-C₆-halocycloalkyl, C₁-C₄-haloalkoxy-C₁-C₄-alkyl, or phenyl;
each R^{V} is indepentently C₁-C₆-alkyl, C₃-C₆-cycloalkyl, C₃-C₆-cycloalkyl-C₁-C₄-alkyl, which are unsubstituted or substituted with halogen; or phenyl or benzyl, wherein the phenyl ring is unsubstituted or substituted with R^{X};
each R^{W} is C₁-C₆-alkyl, C₃-C₆-cycloalkyl, C₃-C₆-cycloalkyl-C₁-C₄-alkyl, which are unsubstituted or substituted with halogen; or phenyl or benzyl, wherein the phenyl ring is unsubstituted or substituted with one or more, same or different substituents R^{X};
each R^{X} is independently halogen, N₃, OH, CN, NO₂, SCN, SF₅;
C₁-C₆ alkyl, C₁-C₆ alkoxy, C₂-C₆ alkenyl, C₂-C₆ alkynyl, C₁-C₆ alkoxy-C₁-C₄ alkyl, C₁-C₆ alkoxy-C₁-C₄ alkoxy, C₃-C₆ cycloalkyl, C₃-C₆ cycloalkoxy, C₃-C₆ cycloalkyl-C₁-C₄ alkyl, C₃-C₆ cycloalkoxy-C₁-C₄ alkyl, which groups are unsubstituted or substituted with halogen;
m is 0, 1 or 2;
n is 0, 1, 2, or 3 ;
and the N-oxides, stereoisomers, tautomers and agriculturally or veterinarily acceptable salts thereof.

2. The compound of formula (I) according to claim 1, wherein
R¹ is H;
C₁-C₃-alkyl, C₁-C₃-alkoxy, which groups are unsubstituted or halogenated;
R² is H, halogen;
C₁-C₃-alkyl, C₁-C₃-alkoxy, C₂-C₃-alkenyl, C₂-C₃-alkynyl, which groups are unsubstituted or halogenated,

3. The compound of formula (I) according to claim 1 or 2, wherein the compound of formula (I) is a compound of formula (I.A), (I.B) or (I.C); 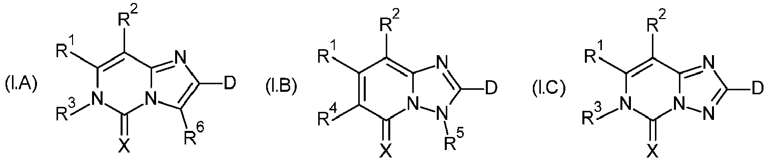 wherein all variables have a meaning as defined for formula (I).

4. The compound of formula (I-A) according to claim 3, wherein
R³ C₁-C₄ -alkyl, C₂-C₄ alkenyl, C₂-C₄ alkynyl, C₁-C₃-alkoxy-C₁-C₃-alkyl, C₃-C₆ cycloalkyl, C₃-C₆ cycloalkyl-C₁-C₂-alkyl, which groups are unsubstituted or halogenated; phenyl or benzyl, in which groups the phenyl ring is unsubstituted or substituted with R^{F};
R⁶ is H; or C₁-C₃-alkyl or C₁-C₃-haloalkyl.

5. The compound of formula (I-B) according to claim 3 or 4, wherein
R⁴ is H; or
C₁-C₃ alkyl, or C₁-C₃-haloalkyl;
R⁵ is C₁-C₃-alkyl, or C₁-C₃-haloalkyl.

6. The compounds of formula (I) according to any of claims 1 to 5, wherein the variable D stands for a fused bicyclic ring of the following formulae (D1), (D3), (D8), or (D51) 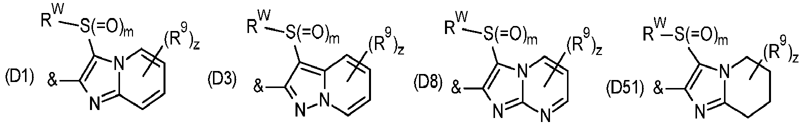 wherein the index z is 0, 1, 2, 3, or 4 for (D1), (D3), (D51), or wherein z is 0, 1, 2, or 3 for (D8), and wherein the other variables are defined as for formula (I), and wherein the "&"-symbol signifies the connection to the remainder of formula (I).

7. The compound of formula (I) according to any of claims 1 to 6, wherein each R⁹ is independently selected from H, halogen; and
C₁-C₃-alkyl, C₂-C₃-alkenyl, and C₂-C₃-alkynyl, which groups are unsubstituted, or halogenated.

8. The compound of formula (I) according to any of claims 1 to 7, wherein
R^{W} is C₁-C₃-alkyl or C₁-C₃-haloalkyl, and
m is 0 or 2

9. A pesticidal mixture comprising the compound of formula (I), as defined in any of claims 1 to 7, an N-oxide or an agriculturally acceptable salt thereof and a further pesticidal ingredient.

10. Use of compounds of formula (I) as defined in any of claims 1 to 8 as an agrochemical pesticide.

11. A non-therapeutic method for combating or controlling invertebrate pests, which method comprises contacting said pest or its food supply, habitat or breeding grounds with a pesticidally effective amount of at least one compound of the formula (I) according to any of claims 1 to 8 or the composition according to claim 9.

12. A method for protecting growing plants from attack or infestation by invertebrate pests, which method comprises contacting a plant, or soil or water in which the plant is growing, with a pesticidally effective amount of at least one compound of the formula (I), according to any of the claims 1 to 8 or the composition according to claim 9.

13. Seed comprising a compound of the formula (I), as defined in any of claims 1 to 8, or the composition, as defined in claim 9, in an amount of from 0.1 g to 10 kg per 100 kg of seed.

14. Use of a compound of the formula (I), as defined in any of the claims 1 to 8, or of the compositions as defined in claim 9, for protecting growing plants from attack or infestation by invertebrate pests.

15. A non-therapeutic method for treating or protecting an animal from infestation or infection by invertebrate pests which comprises bringing the animal in contact with a pesticidally effective amount of a compound of the formula (I) as defined in any of claims 1 to 8, or or a composition as defined in claim 9.

## Patentansprüche

1. Verbindung der Formel (I) 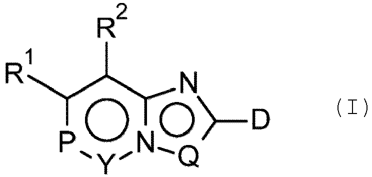 wobei
der Kreis im Ring anzeigt, dass der Ring vollständig ungesättigt ist;
Y für C=X steht, wobei X für 0 oder S steht;
P für N(R³) oder C(R⁴) steht;
Q für N(R⁵) oder C(R⁶) steht;
R¹ für H, Halogen, C₁-C₆-Alkyl, C₂-C₆-Alkenyl, C₂-C₆-Alkinyl, C₁-C₆-Alkoxy, C₁-C₆-Alkoxy-C₁-C₆-alkyl, C₃-C₆-Cycloalkyl, C₁-C₆-Sulfenyl, C₁-C₆-Sulfinyl oder C₁-C₆-Sulfonyl steht, wobei diese Gruppen unsubstituiert oder halogeniert sind;
R³, R⁵ unabhängig für C₁-C₆-Alkyl, C₁-C₆-Alkoxy, C₂-C₆-Alkenyl, C₂-C₆-Alkinyl, C₁-C₆-Alkoxy-C₁-C₄-alkyl, C₁-C₆-Alkoxy-C₁-C₄-alkoxy, C₃-C₆-Cycloalkyl, C₃-C₆-Cycloalkyl-C₁-C₄-alkyl oder C₃-C₆-Cycloalkoxy-C₁-C₄-alkyl, die unsubstituiert oder halogeniert sind;
C (=0) OR^{A}, NR^{B}R^{C}, C₁-C₆-Alkylen-NR^{B}R^{C}, O-C₁-C₆-Alkylen-NR^{B}R^{C}, C₁-C₆-Alkylen-CN, NH-C₁-C₆-Alkylen-NR^{B}R^{C}, C(=O) NR^{B}R^{C}, C (=O) R^{D}, C (=S)R^{D}, SO₂NR^{B}R^{C}, S (=O)ₙR^{E};
Phenyl oder Benzyl stehen, wobei der Phenylring unsubstituiert oder durch einen oder mehrere, gleiche oder verschiedene Substituenten R^{F} substituiert ist;
R², R⁴, R⁶ unabhängig für H, Halogen, N₃, CN, NO₂, SCN, SF₅;
C₁-C₆-Alkyl, C₁-C₆-Alkoxy, C₂-C₆-Alkenyl, Tri-C₁-C₆-alkylsilyl, C₂-C₆-Alkinyl, C₁-C₆-Alkoxy-C₁-C₄-alkyl, C₁-C₆-Alkoxy-C₁-C₄-alkoxy, C₃-C₆-Cycloalkyl, C₃-C₆-Cycloalkoxy, C₃-C₆-Cycloalkyl-C₁-C₄-alkyl, C₃-C₆-Cycloalkoxyx-C₁-C₄-alkyl, wobei diese Gruppen unsubstituiert oder durch Halogen substituiert sind,
C (=O) OR^{A}, NR^{B}R^{C}, NOR^{A}, ONR^{B}R^{C}, C₁-C₆-Alkylen-NR^{B}R^{C}, O-C₁-C₆-Alkylen-NR^{B}R^{C}, C₁-C₆-Alkylen-CN, NH-C₁-C₆-Alkylen-NR^{B}R^{C}, C (=O)NR^{B}R^{C}, C(=O)R^{D}, C(=S)R^{D}, SO₂NR^{B}R^{C}, S(=O)ₙR^{E};
Phenyl oder Benzyl stehen, wobei der Phenylring unsubstituiert oder durch einen oder mehrere, gleiche oder verschiedene Substituenten R^{F} substituiert ist;
D für einen anellierten bicyclischen Ring der folgenden Formel steht: 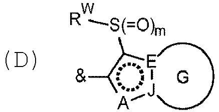
wobei das "&"-Symbol die Verknüpfung mit dem Rest der Formel (I) anzeigt, wobei der gestrichelte Kreis in dem 5-gliedrigen Ring bedeutet, dass der 5-gliedrige Ring gesättigt, teilweise ungesättigten oder vollständig ungesättigt sein kann; und wobei
A für N, S, O, CR⁷ oder NR⁸ steht;
E, J unabhängig für C oder N stehen, wobei mindestens eine der aus E und J ausgewählten Variablen für C steht;
G für einen 5- oder 6-gliedrigen gesättigten, teilweise ungesättigten oder vollständig ungesättigten Carbo- oder Heterocyclus steht, wobei der Carbo- oder Heterocyclus die Atome E und J als Ringglieder enthält und unsubstituiert oder durch einen oder mehrere, gleiche oder verschiedene Substituenten R⁹ substituiert ist und wobei der Heterocyclus kein, ein oder mehrere, gleiche oder verschiedene Heteroatome O, N oder S zusätzlich zu denjenigen, die als Ringglieder E und J vorliegen können, umfasst;
R⁷ für H, Halogen, OH, CN, NC, NO₂, N₃, SCN, NCS, NCO, SF₅;
C₁-C₆-Alkyl, C₃-C₆-Cycloalkyl, C₂-C₆-Alkenyl, C₃-C₆-Cycloalkenyl, C₂-C₆-Alkinyl, wobei diese Gruppen unsubstituiert oder durch einen oder mehrere, gleiche oder verschiedene Substituenten R^{G} substituiert sind;
einen 3- bis 12-gliedrigen gesättigten, teilweise ungesättigten oder vollständig ungesättigten heterocyclischen Ring oder ein 3- bis 12-gliedriges gesättigtes, teilweise ungesättigtes oder vollständig ungesättigtes heterocyclisches Ringsystem, wobei der heterocyclische Ring bzw. das heterocyclische Ringsystem ein oder mehrere, gleiche oder verschiedene Heteroatome O, N oder S umfasst und unsubstituiert oder durch einen oder mehrere, gleiche oder verschiedene Substituenten R^{H} substituiert ist und wobei die N- und S-Atome unabhängig oxidiert oder nicht oxidiert sind;
Phenyl, das unsubstituiert oder durch einen oder mehrere, gleiche oder verschiedene Substituenten R^{J} substituiert ist;
OR^{K}, SR^{K}, OC(=O)R^{K}, OC (=O) OR^{K}, OC (=O) NR^{L}R^{M}, OC(=O)SR^{K}, OC(=S)NR^{L}R^{M}, OC(=S)SR^{K}, OS(=O)ₘR^{K}, OS (=O) NR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L}) C (=O) OR^{K}, S (=O) ₙR^{V}, SC(=O)SR^{K}, SC (=O) NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si (R^{s}) ₂R^{T} steht;
R⁸ für H, Halogen, CN;
C₁-C₆-Alkyl, C₃-C₆-Cycloalkyl, C₂-C₆-Alkenyl, C₃-C₆-Cycloalkenyl, C₂-C₆-Alkinyl, wobei diese Gruppen unsubstituiert oder durch einen oder mehrere, gleiche oder verschiedene Substituenten R^{G} substituiert sind;
einen 3- bis 12-gliedrigen gesättigten, teilweise ungesättigten oder vollständig ungesättigten heterocyclischen Ring oder ein 3- bis 12-gliedriges gesättigtes, teilweise ungesättigtes oder vollständig ungesättigtes heterocyclisches Ringsystem, wobei der heterocyclische Ring bzw. das heterocyclische Ringsystem ein oder mehrere, gleiche oder verschiedene Heteroatome O, N oder S umfasst und unsubstituiert oder durch einen oder mehrere, gleiche oder verschiedene Substituenten R^{H} substituiert ist und wobei die N- und S-Atome unabhängig oxidiert oder nicht oxidiert sind;
Phenyl, das unsubstituiert oder durch einen oder mehrere, gleiche oder verschiedene Substituenten R^{J} substituiert ist;
OR^{K}, SR^{K}, OC (=O) R^{K}, OC(=O)OR^{K}, OC (=O) NR^{L}R^{M}, OC (=O) SR^{K}, OC(=S)NR^{L}R^{M}, OC(=S)SR^{K}, OS(=O)ₘR^{K}, OS (=O)NR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C (=O) OR^{K}, S (=O) ₙR^{V}, SC (=O) SR^{K}, SC (=O) NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{s})₂R^{T} steht;
R⁹ jeweils unabhängig für H, Halogen, OH, CN, NC, NO₂, N₃, SCN, NCS, NCO, SF₅;
C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkyl-C₁-C₃-alkyl, wobei diese Gruppen unsubstituiert oder durch einen oder mehrere, gleiche oder verschiedene Substituenten R^{G} substituiert sind;
einen 3- bis 12-gliedrigen gesättigten, teilweise ungesättigten oder vollständig ungesättigten heterocyclischen Ring oder ein 3- bis 12-gliedriges gesättigtes, teilweise ungesättigtes oder vollständig ungesättigtes heterocyclisches Ringsystem, wobei der heterocyclische Ring bzw. das heterocyclische Ringsystem ein oder mehrere, gleiche oder verschiedene Heteroatome O, N oder S umfasst und unsubstituiert oder durch einen oder mehrere, gleiche oder verschiedene Substituenten R^{H} substituiert ist und wobei die N- und S-Atome unabhängig oxidiert oder nicht oxidiert sind;
Phenyl, das unsubstituiert oder durch einen oder mehrere, gleiche oder verschiedene Substituenten R^{J} substituiert ist;
OR^{K}, SR^{K}, OC (=O) R^{K}, OC (=O)OR^{K}, OC (=O) NR^{L}R^{M}, OC (=O) SR^{K}, OC(=S)NR^{L}R^{M}, OC(=S)SR^{K}, OS (=O)ₘR^{K}, OS (=O) NR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L}) C(=O)OR^{K}, S (=O) ₙR^{V}, SC (=O) SR^{K}, SC (=O) NR^{L}R^{M}, S(=O) NR^{L}R^{M}, C(=O)R^{P}, C(=S)R^{P}, C (=O) NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si (R^{s}) ₂R^{T} steht;
oder zwei Substituenten R⁹ zusammen mit den Ringgliedern von Ring G, an die sie gebunden sind, einen 5- oder 6-gliedrigen gesättigten, teilweise ungesättigten oder vollständig ungesättigten Carbo- oder Heterocyclus bilden, wobei der Carbo- oder Heterocyclus unsubstituiert oder durch einen oder mehrere, gleiche oder verschiedene Substituenten R^{J} substituiert ist und wobei der Heterocyclus ein oder mehrere, gleiche oder verschiedene Heteroatome O, N oder S umfasst;
R^{A} jeweils unabhängig für H;
C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, wobei diese Gruppen unsubstituiert oder durch Halogen substituiert sind;
C₁-C₆-Alkylen-NR^{b}R^{c}, C₁-C₆-Alkylen-CN oder
Phenyl oder Benzyl steht, wobei der Phenylring unsubstituiert oder durch einen oder mehrere, gleiche oder verschiedene Substituenten R^{F} substituiert ist;
R^{B} jeweils unabhängig für H;
C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, wobei diese Gruppen unsubstituiert oder durch Halogen substituiert sind;
C₁-C₆-Alkylen-CN;
Phenyl oder Benzyl steht, wobei diese Gruppen unsubstituiert oder durch einen oder mehrere, gleiche oder verschiedene Substituenten R^{F} substituiert sind;
R^{C} jeweils unabhängig für H;
C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, wobei diese Gruppen unsubstituiert oder durch Halogen substituiert sind;
C₁-C₆-Alkylen-CN oder
Phenyl oder Benzyl steht, wobei der Phenylring unsubstituiert oder durch einen oder mehrere, gleiche oder verschiedene Substituenten R^{F} substituiert ist;
jede Gruppierung NR^{B}R^{C} auch einen N-gebundenen, gesättigten 5- bis 8-gliedrigen Heterocyclus bilden kann, der zusätzlich zu dem Stickstoffatom 1 oder 2 weitere Heteroatome bzw. Heteroatomgruppierungen, die aus O, S(=O)ₘ und N-R' ausgewählt sind, aufweisen kann, wobei R' für H oder C₁-C₆-Alkyl steht, und wobei der N-gebundene Heterocyclus unsubstituiert oder durch einen oder mehrere, gleiche oder verschiedene Substituenten, die aus Halogen, C₁-C₄-Alkyl, C₁-C₄-Halogenalkyl, C₁-C₄-Alkoxy und C₁-C₄-Halogenalkoxy ausgewählt sind, substituiert ist;
R^{D} jeweils unabhängig für H;
C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, wobei diese Gruppen unsubstituiert oder durch Halogen substituiert sind;
Phenyl oder Benzyl steht, wobei der Phenylring unsubstituiert oder durch einen oder mehrere, gleiche oder verschiedene Substituenten R^{F} substituiert ist;
R^{E} jeweils unabhängig für C₁-C₆-Alkyl, C₃-C₆-Cycloalkyl, C₃-C₆-Cycloalkyl-C₁-C₄-alkyl, die unsubstituiert oder durch Halogen substituiert sind; oder
Phenyl oder Benzyl steht, wobei der Phenylring unsubstituiert oder durch einen oder mehrere, gleiche oder verschiedene Substituenten R^{F} substituiert ist;
R^{F} jeweils unabhängig für Halogen, N₃, OH, CN, NO₂, SCN, SF₅;
C₁-C₆-Alkyl, C₁-C₆-Alkoxy, C₂-C₆-Alkenyl, C₂-C₆-Alkinyl, C₁-C₆-Alkoxy-C₁-C₄-alkyl, C₁-C₆-Alkoxy-C₁-C₄-alkoxy, C₃-C₆-Cycloalkyl, C₃-C₆-Cycloalkoxy, C₃-C₆-Cycloalkyl-C₁-C₄ alkyl, C₃-C₆-Cycloalkoxy-C₁-C₄-alkyl, wobei diese Gruppen unsubstituiert oder durch Halogen substituiert sind, steht;
R^{G} jeweils unabhängig für Halogen, OH, CN, NC, NO₂;
C₁-C₆-Alkyl, C₃-C₆-Cycloalkyl, C₃-C₆-Cycloalkenyl, wobei diese Gruppen unsubstituiert oder durch einen oder mehrere, gleiche oder verschiedene Substituenten, die aus Halogen, OH, CN, C₁-C₃-Alkoxy, C₁-C₃-Halogenalkoxy und C₁-C₃-Alkylcarbonyl ausgewählt sind, substituiert sind;
einen 3- bis 12-gliedrigen gesättigten, teilweise ungesättigten oder vollständig ungesättigten heterocyclischen Ring oder ein 3- bis 12-gliedriges gesättigtes, teilweise ungesättigtes oder vollständig ungesättigtes heterocyclisches Ringsystem, wobei der heterocyclische Ring bzw. das heterocyclische Ringsystem ein oder mehrere, gleiche oder verschiedene Heteroatome O, N oder S umfasst und unsubstituiert oder durch einen oder mehrere, gleiche oder verschiedene Substituenten, die aus Halogen, OH, CN, C₁-C₃-Alkoxy, C₁-C₃-Halogenalkoxy und C₁-C₃-Alkylcarbonyl ausgewählt sind, substituiert ist und wobei die N- und S-Atome unabhängig oxidiert oder nicht oxidiert sind;
Phenyl, das unsubstituiert oder durch einen oder mehrere, gleiche oder verschiedene Substituenten, die aus Halogen, OH, CN, NO₂, C₁-C₃-Alkyl, C₁-C₃-Halogenalkyl, C₁-C₃-Alkoxy, C₁-C₃-Halogenalkoxy und C₁-C₃-Alkylcarbonyl ausgewählt sind, substituiert ist;
OR^{K}, SR^{K}, OC(=O)R^{K}, OC (=O) OR^{K}, OC (=O) NR^{L}R^{M}, OC (=O) SR^{K}, OC(=S)NR^{L}R^{M}, OC(=S)SR^{K}, OS (=O)ₘR^{K}, OS (=O) NR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L}) C (=O) OR^{K}, S (=O) ₙR^{V}, SC (=O) SR^{K}, SC (=O) NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T} steht;
R^{H} jeweils unabhängig für Halogen, CN, NC, NO₂, SCN, NCS, NCO;
C₁-C₆-Alkyl, C₃-C₆-Cycloalkyl, C₃-C₆-Cycloalkenyl, wobei diese Gruppen unsubstituiert oder durch einen oder mehrere, gleiche oder verschiedene Substituenten, die aus Halogen, OH, CN, C₁-C₁₀-Alkoxy, C₁-C₃-Halogenalkoxy und C₁-C₃-Alkylcarbonyl ausgewählt sind, substituiert sind;
Phenyl steht, das unsubstituiert oder durch einen oder mehrere, gleiche oder verschiedene Substituenten, die aus Halogen, OH, CN, NO₂, C₁-C₃-Alkyl, C₁-C₃-Halogenalkyl, OR^{K}, SR^{K}, OC (=O) R^{K}, OC (=O) OR^{K}, OC(=O)NR^{L}R^{M}, OC (=O) SR^{K}, OC(=S)NR^{L}R^{M}, OC(=S)SR^{K}, OS (=O)ₘR^{K}, OS (=O) NR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L}) C(=O)OR^{K}, S (=O) ₙR^{V}, SC (=O) SR^{K}, SC (=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T} ausgewählt sind, substituiert ist; oder
zwei geminale Substituenten R^{H} zusammen mit dem Atom, an das sie gebunden sind, eine Gruppe =O, =S oder =NR^{L} bilden;
R^{J} jeweils unabhängig für Halogen, CN, NC, NO₂, SCN, NCS, NCO;
C₁-C₆-Alkyl, C₃-C₆-Cycloalkyl, C₃-C₆-Cycloalkenyl, wobei diese Gruppen unsubstituiert oder durch einen oder mehrere, gleiche oder verschiedene Substituenten, die aus Halogen, OH, CN, C₁-C₁₀-Alkoxy, C₁-C₃-Halogenalkoxy und C₁-C₃-Alkylcarbonyl ausgewählt sind, substituiert sind; Phenyl steht, das unsubstituiert oder durch einen oder mehrere, gleiche oder verschiedene Substituenten, die aus Halogen, OH, CN, NO₂, C₁-C₃-Alkyl, C₁-C₃-Halogenalkyl, OR^{K}, SR^{K}, OC (=O) R^{K}, OC (=O) OR^{K}, OC (=O)NR^{L}R^{M}, OC (=O) SR^{K}, OC(=S)NR^{L}R^{M}, OC(=S)SR^{K}, OS (=O)ₘR^{K}, OS (=O) NR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L}) C(=O) OR^{K}, S(=O)ₙR^{V}, SC (=O) SR^{K}, SC (=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T} ausgewählt sind, substituiert ist;
R^{K} jeweils unabhängig für H;
C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, wobei diese Gruppen unsubstituiert oder durch einen oder mehrere, gleiche oder verschiedene Substituenten, die aus Halogen, CN, NR^{M}R^{N}; C(=O)NR^{M}R^{N}, C(=O)R^{T} ausgewählt sind, substituiert sind; oder
Phenyl oder Benzyl steht, wobei der Phenylring unsubstituiert oder durch einen oder mehrere, gleiche oder verschiedene Substituenten R^{X} substituiert ist;
R^{L} jeweils unabhängig für H;
C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, wobei diese Gruppen unsubstituiert oder durch Halogen substituiert sind;
C₁-C₆-Alkylen-CN;
Phenyl oder Benzyl steht, wobei diese Gruppen unsubstituiert oder durch einen oder mehrere, gleiche oder verschiedene Substituenten R^{X} substituiert ist;
R^{M}, R^{R} jeweils unabhängig für H;
C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, wobei diese Gruppen unsubstituiert oder durch Halogen substituiert sind;
C₁-C₆-Alkylen-CN oder
Phenyl oder Benzyl stehen, wobei der Phenylring unsubstituiert oder durch einen oder mehrere, gleiche oder verschiedene Substituenten R^{X} substituiert ist; jede Gruppierung NR^{M}R^{R} oder NR^{L}R^{M} auch einen N-gebundenen, gesättigten 5- bis 8-gliedrigen Heterocyclus bilden kann, der zusätzlich zu dem Stickstoffatom 1 oder 2 weitere Heteroatome bzw. Heteroatomgruppierungen, die aus O, S(=O)ₘ und N-R' ausgewählt sind, aufweisen kann, wobei R' für H oder C₁-C₆-Alkyl steht, und wobei der N-gebundene Heterocyclus unsubstituiert oder durch einen oder mehrere, gleiche oder verschiedene Substituenten, die aus Halogen, C₁-C₄-Alkyl, C₁-C₄-Halogenalkyl, C₁-C₄-Alkoxy und C₁-C₄-Halogenalkoxy ausgewählt sind, substituiert ist;
R^{N} jeweils unabhängig für H, Halogen, CN, NO₂, SCN;
C₁-C₁₀-Alkyl, C₃-C₈-Cycloalkyl, C₂-C₆-Alkenyl, C₃-C₆-Cycloalkenyl, C₂-C₆-Alkinyl, wobei diese Gruppen unsubstituiert oder durch einen oder mehrere, gleiche oder verschiedene Substituenten, die aus Halogen, C₁-C₆-Alkyl, C₁-C₆-Alkoxy, C₁-C₆-Halogenalkyl und C₁-C₆-Halogenalkoxy ausgewählt sind, substituiert sind;
einen 3- bis 12-gliedrigen gesättigten, teilweise ungesättigten oder vollständig ungesättigten heterocyclischen Ring oder ein 3- bis 12-gliedriges gesättigtes, teilweise ungesättigtes oder vollständig ungesättigtes heterocyclisches Ringsystem, wobei der heterocyclische Ring bzw. das heterocyclische Ringsystem ein oder mehrere, gleiche oder verschiedene Heteroatome O, N oder S umfasst und unsubstituiert oder durch einen oder mehrere, gleiche oder verschiedene Substituenten, die aus Halogen, C₁-C₃-Alkyl, C₁-C₃-Alkoxy, C₁-C₃-Halogenalkyl und C₁-C₃-Halogenalkoxy ausgewählt sind, substituiert ist und wobei die N- und S-Atome unabhängig oxidiert oder nicht oxidiert sind;
Phenyl steht, das unsubstituiert oder durch einen oder mehrere, gleiche oder verschiedene Substituenten, die aus Halogen, C₁-C₃-Alkyl, C₁-C₃-Alkoxy, C₁-C₃-Halogenalkyl und C₁-C₃-Halogenalkoxy ausgewählt sind, substituiert ist;
R° jeweils unabhängig für H, C₁-C₄-Alkyl, C₁-C₆-Cycloalkyl, C₁-C₂-Alkoxy-C₁-C₂-alkyl, Phenyl oder Benzyl steht;
R^{P} jeweils unabhängig für H;
C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, wobei diese Gruppen unsubstituiert oder durch Halogen substituiert sind;
Phenyl oder Benzyl steht, wobei der Phenylring unsubstituiert oder durch einen oder mehrere, gleiche oder verschiedene Substituenten R^{X} substituiert ist;
R^{S}, R^{T} jeweils unabhängig für H, C₁-C₆-Alkyl, C₁-C₆-Halogenalkyl, C₁-C₆-Alkoxy, C₁-C₄-Alkoxy-C₁-C₄-alkyl, C₃-C₆-Cycloalkyl, C₃-C₆-Halogencycloalkyl, C₁-C₄-Halogenalkoxy-C₁-C₄-alkyl oder Phenyl stehen;
R^{V} jeweils unabhängig für C₁-C₆-Alkyl, C₃-C₆-Cycloalkyl, C₃-C₆-Cycloalkyl-C₁-C₄-alkyl, die unsubstituiert oder durch Halogen substituiert sind; oder
Phenyl oder Benzyl steht, wobei der Phenylring unsubstituiert oder durch R^{X} substituiert ist;
R^{W} jeweils für C₁-C₆-Alkyl, C₃-C₆-Cycloalkyl, C₃-C₆-Cycloalkyl-C₁-C₄-alkyl, die unsubstituiert oder durch Halogen substituiert sind; oder
Phenyl oder Benzyl steht, wobei der Phenylring unsubstituiert oder durch einen oder mehrere, gleiche oder verschiedene Substituenten R^{X} substituiert ist;
R^{X} jeweils unabhängig für Halogen, N₃, OH, CN, NO₂, SCN, SF₅;
C₁-C₆-Alkyl, C₁-C₆-Alkoxy, C₂-C₆-Alkenyl, C₂-C₆-Alkinyl, C₁-C₆-Alkoxy-C₁-C₄-alkyl, C₁-C₆-Alkoxy-C₁-C₄-alkoxy, C₃-C₆-Cycloalkyl, C₃-C₆-Cycloalkoxy, C₃-C₆-Cycloalkyl-C₁-C₄ alkyl, C₃-C₆-Cycloalkoxy-C₁-C₄-alkyl, wobei diese Gruppen unsubstituiert oder durch Halogen substituiert sind, steht;
m für 0, 1 oder 2 steht;
n für 0, 1, 2 oder 3 steht;
und die N-Oxide, Stereoisomere, Tautomere und landwirtschaftlich oder veterinärmedizinisch unbedenklichen Salze davon.

2. Verbindung der Formel (I) nach Anspruch 1, wobei R¹ für H;
C₁-C₃-Alkyl, C₁-C₃-Alkoxy, wobei diese Gruppen unsubstituiert oder halogeniert sind, steht;
R² für H, Halogen;
C₁-C₃-Alkyl, C₁-C₃-Alkoxy, C₂-C₃-Alkenyl, C₂-C₃-Alkinyl, wobei diese Gruppen unsubstituiert oder halogeniert sind, steht.

3. Verbindung der Formel (I) nach Anspruch 1 oder 2, wobei es sich bei der Verbindung der Formel (I) um eine Verbindung der Formel (I.A), (I.B) oder (I.C) handelt; 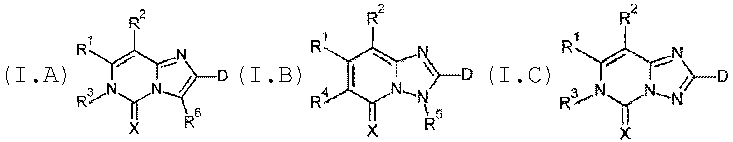 wobei alle Variablen eine wie für Formel (I) definierte Bedeutung haben.

4. Verbindung der Formel (I-A) nach Anspruch 3, wobei R³ für C₁-C₄-Alkyl, C₂-C₄-Alkenyl, C₂-C₄-Alkinyl, C₁-C₃-Alkoxy-C₁-C₃-alkyl, C₃-C₆-Cycloalkyl, C₃-C₆-Cycloalkyl-C₁-C₂-alkyl, wobei diese Gruppen unsubstituiert oder halogeniert sind;
Phenyl oder Benzyl steht, wobei in diesen Gruppen der Phenylring unsubstituiert oder durch R^{F} substituiert ist; R⁶ für H oder
C₁-C₃-Alkyl oder C₁-C₃-Halogenalkyl steht.

5. Verbindung der Formel (I-B) nach Anspruch 3 oder 4, wobei
R⁴ für H oder
C₁-C₃-Alkyl oder C₁-C₃-Halogenalkyl steht;
R⁵ für C₁-C₃-Alkyl oder C₁-C₃-Halogenalkyl steht.

6. Verbindungen der Formel (I) nach einem der Ansprüche 1 bis 5, wobei die Variable D für einen anellierten bicyclischen Ring der folgenden Formeln (D1), (D3), (D8) oder (D51) steht: 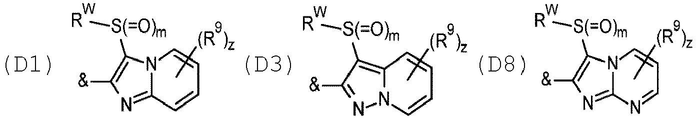 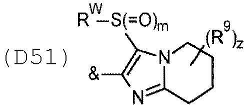 wobei der Index z für (D1), (D3), (D51) für 0, 1, 2, 3 oder 4 steht oder wobei z für (D8) für 0, 1, 2 oder 3 steht und wobei die anderen Variablen wie für Formel (I) definiert sind und wobei das "&"-Symbol die Verknüpfung mit dem Rest der Formel (I) anzeigt.

7. Verbindung der Formel (I) nach einem der Ansprüche 1 bis 6, wobei R⁹ jeweils unabhängig aus H, Halogen und C₁-C₃-Alkyl, C₂-C₃-Alkenyl und C₂-C₃-Alkinyl, wobei diese Gruppen unsubstituiert oder halogeniert sind, ausgewählt ist.

8. Verbindung der Formel (I) nach einem der Ansprüche 1 bis 7, wobei
R^{W} für C₁-C₃-Alkyl oder C₁-C₃-Halogenalkyl steht und
m für 0 oder 2 steht.

9. Pestizide Mischung, umfassend die Verbindung der Formel (I) gemäß einem der Ansprüche 1 bis 7, ein N-Oxid oder ein landwirtschaftlich unbedenkliches Salz davon und einen weiteren pestiziden Bestandteil.

10. Verwendung von Verbindungen der Formel (I) gemäß einem der Ansprüche 1 bis 8 als ein agrochemisches Pestizid.

11. Nichttherapeutisches Verfahren zum Bekämpfen oder Kontrollieren von wirbellosen Schädlingen, bei dem man den Schädling oder seine Nahrungsquelle, seinen Lebensraum oder seine Brutstätten mit einer pestizid wirksamen Menge mindestens einer Verbindung der Formel (I) nach einem der Ansprüche 1 bis 8 oder der Zusammensetzung nach Anspruch 9 in Berührung bringt.

12. Verfahren zum Schützen von wachsenden Pflanzen vor Angriffen oder Befall durch wirbellose Schädlinge, bei dem man eine Pflanze oder den Boden oder das Wasser, in dem die Pflanze wächst, mit einer pestizid wirksamen Menge mindestens einer Verbindung der Formel (I) nach einem der Ansprüche 1 bis 8 oder der Zusammensetzung nach Anspruch 9 in Berührung bringt.

13. Saatgut, umfassend eine Verbindung der Formel (I) gemäß einem der Ansprüche 1 bis 8 oder der Zusammensetzung gemäß Anspruch 9 in einer Menge von 0,1 g bis 10 kg pro 100 kg Saatgut.

14. Verwendung einer Verbindung der Formel (I) gemäß einem der Ansprüche 1 bis 8 oder der Zusammensetzungen gemäß Anspruch 9 zum Schützen von wachsenden Pflanzen vor Angriff oder Befall durch wirbellose Schädlinge.

15. Nichttherapeutisches Verfahren zum Behandeln oder Schützen eines Tiers vor Befall oder Infektion durch wirbellose Schädlinge, bei dem man das Tier mit einer pestizid wirksamen Menge einer Verbindung der Formel (I) gemäß einem der Ansprüche 1 bis 8 oder einer Zusammensetzung gemäß Anspruch 9 in Berührung bringt.

## Revendications

1. Composé de formule (I) 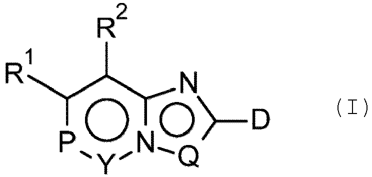
le cercle dans le cycle signifiant que ce cycle est totalement insaturé ;
Y étant C=X, X étant O ou S ;
P étant N(R³) ou C(R⁴) ;
Q étant N(R⁵) ou C(R⁶) ;
R¹ étant H, halogène, C₁-C₆-alkyle, C₂-C₆-alcényle, C₂-C₆-alcynyle, C₁-C₆-alcoxy, C₁-C₆-alcoxy-C₁-C₆-alkyle, C₃-C₆-cycloalkyle, C₃-C₆-cycloalcoxy, C₁-C₆-sulfényle, C₁-C₆-sulfinyle, ou C₁-C₆-sulfonyle, lesquels groupes étant non substitués ou halogénés ;
R³, R⁵ étant indépendamment C₁-C₆-alkyle, C₁-C₆-alcoxy, C₂-C₆-alcényle, C₂-C₆-alcynyle, C₁-C₆-alcoxy-C₁-C₄-alkyle, C₁-C₆-alcoxy-C₁-C₄-alcoxy, C₃-C₆-cycloalkyle, C₃-C₆-cycloalkyl-C₁-C₄-alkyle, ou C₃-C₆-cycloalcoxy-C₁-C₄-alkyle, qui sont non substitués ou halogénés ;
C(=O)OR^{A}, NR^{B}R^{C}, C₁-C₆-alkylène-NR^{B}R^{C}, O-C₁-C₆-alkylène-NR^{B}R^{C}, C₁-C₆-alkylène-CN, NH-C₁-C₆-alkylène-NR^{B}R^{C}, C(=O)NR^{B}R^{C}, C(=O)R^{D}, C(=S)R^{D}, SO₂NR^{B}R^{C}, S(=O)ₙR^{E} ;
phényle ou benzyle, le cycle phényle étant non substitué ou substitué par un ou plusieurs substituants R^{F} identiques ou différents ;
R², R⁴, R⁶ étant indépendamment H, halogène, N₃, CN, NO₂, SCN, SF₅ ;
C₁-C₆-alkyle, C₁-C₆-alcoxy, C₂-C₆-alcényle, tri-C₁-C₆-alkylsilyle, C₂-C₆-alcynyle, C₁-C₆-alcoxy-C₁-C₄-alkyle, C₁-C₆-alcoxy-C₁-C₄-alcoxy, C₃-C₆-cycloalkyle, C₃-C₆-cycloalcoxy, C₃-C₆-cycloalkyl-C₁-C₄-alkyle, C₃-C₆-cycloalcoxy-C₁-C₄-alkyle, lesquels groupes étant non substitués ou substitués par halogène,
C (=O) OR^{A}, NR^{B}R^{C}, NOR^{A}, ONR^{B}R^{C}, C₁-C₆-alkylène-NR^{B}R^{C}, O-C₁-C₆-alkylène-NR^{B}R^{C}, C₁-C₆-alkylène-CN, NH-C₁-C₆-alkylène-NR^{B}R^{C}, C(=O)NR^{B}R^{C}, C(=O)R^{D}, C(=S)R^{D}, SO₂NR^{B}R^{C}, S(=O)ₙR^{E} ;
phényle ou benzyle, le cycle phényle étant non substitué ou substitué par un ou plusieurs substituants R^{F} identiques ou différents ;
D étant un cycle bicyclique condensé de la formule suivante 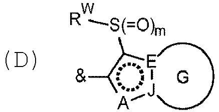
le symbole « & » signifiant la connexion au reste de la formule (I), le cercle en pointillés dans le cycle à 5 chaînons signifiant que le cycle à 5 chaînons peut être saturé, partiellement insaturé ou totalement insaturé ; et
A étant N, S, O, CR⁷, ou NR⁸ ;
E, J étant indépendamment C ou N, au moins l'une des variables choisies parmi E et J étant C ;
G étant un carbocycle ou hétérocycle saturé, partiellement insaturé ou totalement insaturé à 5 ou 6 chaînons, lequel carbocycle ou hétérocycle comprenant les atomes E et J en tant qu'éléments de cycle et étant non substitué, ou substitué par un ou plusieurs substituants R⁹ identiques ou différents, et ledit hétérocycle comprenant aucun, un ou plusieurs hétéroatomes identiques ou différents O, N, ou S en plus de ceux qui peuvent être présents en tant qu'éléments de cycle E et J ;
R⁷ étant H, halogène, OH, CN, NC, NO₂, N₃, SCN, NCS, NCO, SF₅ ;
C₁-C₆-alkyle, C₃-C₆-cycloalkyle, C₂-C₆-alcényle, C₃-C₆-cycloalcényle, C₂-C₆-alcynyle, lesquels groupes étant non substitués, ou substitués par un ou plusieurs substituants R^{G} identiques ou différents ;
un cycle ou système cyclique hétérocyclique saturé, partiellement insaturé ou totalement insaturé à 3 à 12 chaînons, ledit cycle ou système cyclique hétérocyclique comprenant un ou plusieurs hétéroatomes identiques ou différents O, N, ou S, et étant non substitué, ou substitué par un ou plusieurs substituants R^{H} identiques ou différents, et lesdits atomes de N et S étant indépendamment oxydés, ou non oxydés ;
phényle, qui est non substitué, ou substitué par un ou plusieurs substituants R^{J} identiques ou différents ;
OR^{K}, SR^{K}, OC(=O)R^{K}, OC (=O) OR^{K}, OC (=O)NR^{L}R^{M}, OC (=O) SR^{K}, OC(=S)NR^{L}R^{M}, OC(=S)SR^{K}, OS(=O)ₘR^{K}, OS (=O) ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L}) C (=O) OR^{K}, S (=O) ₙR^{V}, SC (=O)SR^{K}, SC (=O)NR^{L}R^{M}, S (=O) ₘNR^{L}R^{M}, C (=O) R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si (R^{s})₂R^{T} ;
R⁸ étant H, halogène, CN ;
C₁-C₆-alkyle, C₃-C₆-cycloalkyle, C₂-C₆-alcényle, C₃-C₆-cycloalcényle, C₂-C₆-alcynyle, lesquels groupes étant non substitués, ou substitués par un ou plusieurs substituants R^{G} identiques ou différents ;
un cycle ou système cyclique hétérocyclique saturé, partiellement insaturé ou totalement insaturé à 3 à 12 chaînons, ledit cycle ou système cyclique hétérocyclique comprenant un ou plusieurs hétéroatomes identiques ou différents O, N, ou S, et étant non substitué, ou substitué par un ou plusieurs substituants R^{H} identiques ou différents, et lesdits atomes de N et S étant indépendamment oxydés, ou non oxydés ;
phényle, qui est non substitué, ou substitué par un ou plusieurs substituants R^{J} identiques ou différents ; OR^{K}, SR^{K}, OC(=O)R^{K}, OC (=O) OR^{K}, OC (=O) NR^{L}R^{M}, OC (=O) SR^{K}, OC(=S)NR^{L}R^{M}, OC(=S)SR^{K}, OS (=O)ₘR^{K}, OS (=O) ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S (=O) ₙR^{V}, SC (=O) SR^{K}, SC (=O) NR^{L}R^{M}, S (=O) ₘNR^{L}R^{M}, C(=O)R^{P}, C(=S)R^{P}, C (=O) NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si (R^{s}) ₂R^{T} ;
chaque R⁹ étant indépendamment H, halogène, OH, CN, NC, NO₂, N₃, SCN, NCS, NCO, SF₅ ;
C₁-C₆-alkyle, C₃-C₆-cycloalkyle, C₂-C₆-alcényle, C₃-C₆-cycloalcényle, C₂-C₆-alcynyle, C₃-C₆-cycloalkyl-C₁-C₃-alkyle, lesquels groupes étant non substitués, ou substitués par un ou plusieurs substituants R^{G} identiques ou différents ;
un cycle ou système cyclique hétérocyclique saturé, partiellement insaturé ou totalement insaturé à 3 à 12 chaînons, ledit cycle ou système cyclique hétérocyclique comprenant un ou plusieurs hétéroatomes identiques ou différents O, N, ou S, et étant non substitué, ou substitué par un ou plusieurs substituants R^{H} identiques ou différents, et lesdits atomes de N et S étant indépendamment oxydés, ou non oxydés ;
phényle, qui est non substitué, ou substitué par un ou plusieurs substituants R^{J} identiques ou différents ;
OR^{K}, SR^{K}, OC(=O)R^{K}, OC (=O) OR^{K}, OC (=O) NR^{L}R^{M}, OC (=O) SR^{K}, OC(=S)NR^{L}R^{M}, OC(=S)SR^{K}, OS (=O)ₘR^{K}, OS (=O) ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L}) C (=O) OR^{K}, S(=O) ₙR^{V}, SC (=O) SR^{K}, SC (=O) NR^{L}R^{M}, S (=O) ₘNR^{L}R^{M}, C (=O) R^{P}, C(=S)R^{P}, C (=O) NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T} ;
ou deux substituants R⁹ formant, conjointement avec les éléments de cycle du cycle G auquel ils sont liés, un carbocycle ou hétérocycle saturé, partiellement insaturé ou totalement insaturé à 5 ou 6 chaînons, lequel carbocycle ou hétérocycle étant non substitué, ou substitué par un ou plusieurs substituants R^{J} identiques ou différents, et ledit hétérocycle comprenant un ou plusieurs hétéroatomes identiques ou différents O, N, ou S ;
chaque R^{A} étant indépendamment H ;
C₁-C₆-alkyle, C₂-C₆-alcényle, C₂-C₆-alcynyle, C₁-C₆-alcoxy-C₁-C₄-alkyle, C₃-C₆-cycloalkyle, C₃-C₆-cycloalkyl-C₁-C₄-alkyle, C₃-C₆-cycloalcoxy-C₁-C₄-alkyle, lesquels groupes étant non substitués ou substitués par halogène ;
C₁-C₆-alkylène-NR^{b}R^{c}, C₁-C₆-alkylène-CN ; ou
phényle ou benzyle, le cycle phényle étant non substitué ou substitué par un ou plusieurs substituants R^{F} identiques ou différents ;
chaque R^{B} étant indépendamment H ;
C₁-C₆-alkyle, C₂-C₆-alcényle, C₂-C₆-alcynyle, C₁-C₆-alcoxy-C₁-C₄-alkyle, C₃-C₆-cycloalkyle, C₃-C₆-cycloalkyl-C₁-C₄-alkyle, C₃-C₆-cycloalcoxy-C₁-C₄-alkyle, lesquels groupes étant non substitués ou substitués par halogène ;
C₁-C₆-alkylène-CN ;
phényle et benzyle, lesquels groupes étant non substitués ou substitués par un ou plusieurs substituants R^{F} identiques ou différents ;
chaque R^{C} étant indépendamment H ;
C₁-C₆-alkyle, C₂-C₆-alcényle, C₂-C₆-alcynyle, C₁-C₆-alcoxy-C₁-C₄-alkyle, C₃-C₆-cycloalkyle, C₃-C₆-cycloalkyl-C₁-C₄-alkyle, C₃-C₆-cycloalcoxy-C₁-C₄-alkyle, lesquels groupes étant non substitués ou substitués par halogène ;
C₁-C₆-alkylène-CN ; ou
phényle ou benzyle, le cycle phényle étant non substitué ou substitué par un ou plusieurs substituants R^{F} identiques ou différents ;
chaque groupement NR^{B}R^{C} pouvant également former un hétérocycle à 5 à 8 chaînons saturé, lié à N, qui en plus de l'atome d'azote peut avoir 1 ou 2 hétéroatomes ou groupements d'hétéroatomes supplémentaires choisis parmi O, S(=O)ₘ et N-R', R' étant H ou C₁-C₆-alkyle et l'hétérocycle lié à N étant non substitué ou substitué par un ou plusieurs substituants identiques ou différents choisis parmi halogène, C₁-C₄-alkyle, C₁-C₄-halogénoalkyle, C₁-C₄-alcoxy et C₁-C₄-halogénoalcoxy ; chaque R^{D} étant indépendamment H ;
C₁-C₆-alkyle, C₂-C₆-alcényle, C₂-C₆-alcynyle, C₁-C₆-alcoxy-C₁-C₄-alkyle, C₃-C₆-cycloalkyle, C₃-C₆-cycloalkyl-C₁-C₄-alkyle, C₃-C₆-cycloalcoxy-C₁-C₄-alkyle, lesquels groupes étant non substitués ou substitués par halogène ;
phényle ou benzyle, le cycle phényle étant non substitué ou substitué par un ou plusieurs substituants R^{F} identiques ou différents ;
chaque R^{E} étant indépendamment C₁-C₆-alkyle, C₃-C₆-cycloalkyle, C₃-C₆-cycloalkyl-C₁-C₄-alkyle, qui sont non substitués ou substitués par halogène ; ou
phényle ou benzyle, le cycle phényle étant non substitué ou substitué par un ou plusieurs substituants R^{F} identiques ou différents ;
chaque R^{F} étant indépendamment halogène, N₃, OH, CN, NO₂, SCN, SF₅ ;
C₁-C₆ alkyle, C₁-C₆ alcoxy, C₂-C₆ alcényle, C₂-C₆ alcynyle, C₁-C₆ alcoxy-C₁-C₄ alkyle, C₁-C₆ alcoxy-C₁-C₄ alcoxy, C₃-C₆ cycloalkyle, C₃-C₆ cycloalcoxy, C₃-C₆ cycloalkyl-C₁-C₄ alkyle, C₃-C₆ cycloalcoxy-C₁-C₄ alkyle, lesquels groupes étant non substitués ou substitués par halogène ;
chaque R^{G} étant indépendamment halogène, OH, CN, NC, NO₂ ; C₁-C₆-alkyle, C₃-C₆-cycloalkyle, C₃-C₆-cycloalcényle, lesquels groupes étant non substitués, ou substitués par un ou plusieurs substituants identiques ou différents choisis parmi halogène, OH, CN, C₁-C₃-alcoxy, C₁-C₃-halogénoalcoxy, et C₁-C₃-alkyl-carbonyle ;
un cycle ou système cyclique hétérocyclique saturé, partiellement insaturé ou totalement insaturé à 3 à 12 chaînons, ledit cycle ou système cyclique hétérocyclique comprenant un ou plusieurs hétéroatomes identiques ou
différents O, N, ou S, et étant non substitué, ou substitué par un ou plusieurs substituants identiques ou différents choisis parmi halogène, OH, CN, C₁-C₃-alcoxy, C₁-C₃-halogénoalcoxy, et C₁-C₃-alkyl-carbonyle, et lesdits atomes de N et S étant indépendamment oxydés, ou non oxydés ;
phényle, qui est non substitué, ou substitué par un ou plusieurs substituants identiques ou différents choisis parmi halogène, OH, CN, NO₂, C₁-C₃-alkyle, C₁-C₃-halogénoalkyle, C₁-C₃-alcoxy, C₁-C₃-halogénoalcoxy, et C₁-C₃-alkyl-carbonyle ;
OR^{K}, SR^{K}, OC(=O)R^{K}, OC (=O) OR^{K}, OC (=O)NR^{L}R^{M}, OC (=O) SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{V}, SC(=O) SR^{K}, SC(=O)NR^{L}R^{M}, S(=O)ₘNR^{L}R^{M}, C(=O)R^{P}, C(=S)R^{P}, C(=O)NR^{L}R^{M}, C(=O)OR^{K}, C(=S)NR^{L}R^{M}, C(=S)OR^{K}, C(=S)SR^{K}, C(=NR^{L})R^{M}, C(=NR^{L})NR^{M}R^{R}, Si(R^{S})₂R^{T} ;
chaque R^{H} étant indépendamment halogène, CN, NC, NO₂, SCN, NCS, NCO ;
C₁-C₆-alkyle, C₃-C₆-cycloalkyle, C₃-C₆-cycloalcényle, lesquels groupes étant non substitués, ou substitués par un ou plusieurs substituants identiques ou différents choisis parmi halogène, OH, CN, C₁-C₁₀-alcoxy, C₁-C₃-halogénoalcoxy, et C₁-C₃-alkyl-carbonyle ;
phényle, qui est non substitué, ou substitué par un ou plusieurs substituants identiques ou différents choisis parmi halogène, OH, CN, NO₂, C₁-C₃-alkyle, C₁-C₃-halogénoalkyle, OR^{K}, SR^{K}, OC(=O)R^{K}, OC (=O) OR^{K}, OC(=O)NR^{L}R^{M}, OC (=O) SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{v}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O) OR^{K}, C(=S)NR^{L}R^{M}, C(=S) OR^{K}, C(=S) SR^{K}, C(=NR^{L})R^{M}, C(=NR^{L})NR^{M}R^{R}, Si(R^{S})₂R^{T} ; ou
deux substituants R^{H} géminaux formant conjointement avec l'atome auquel ils sont liés un groupe =O, =S, ou =NR^{L}.
chaque R^{J} étant indépendamment halogène, CN, NC, NO₂, SCN, NCS, NCO ;
C₁-C₆-alkyle, C₃-C₆-cycloalkyle, C₃-C₆-cycloalcényle, lesquels groupes étant non substitués, ou substitués par un ou plusieurs substituants identiques ou différents choisis parmi halogène, OH, CN, C₁-C₁₀-alcoxy, C₁-C₃-halogénoalcoxy, et C₁-C₃-alkyl-carbonyle ;
phényle, qui est non substitué, ou substitué par un ou plusieurs substituants identiques ou différents choisis parmi halogène, OH, CN, NO₂, C₁-C₃-alkyle, C₁-C₃-halogénoalkyle, OR^{K}, SR^{K}, OC(=O)R^{K}, OC(=O)OR^{K}, OC(=O)NR^{L}R^{M}, OC (=O) SR^{K}, OC(=S)NR^{L}R^{M}, OC(=S)SR^{K}, OS(=O)ₘR^{K}, OS(=O)ₘNR^{L}R^{M}, ONR^{L}R^{M}, ON=CR^{N}R^{O}, NR^{L}R^{M}, NOR^{K}, ONR^{L}R^{M}, N=CR^{N}R^{O}, NNR^{L}, N(R^{L})C(=O)R^{K}, N(R^{L})C(=O)OR^{K}, S(=O)ₙR^{v}, SC(=O)SR^{K}, SC(=O)NR^{L}R^{M}, S(=O)ₘNR^{L}R^{M}, C(=O)R^{P}, C(=S)R^{P}, C(=O)NR^{L}R^{M}, C(=O) OR^{K}, C(=S)NR^{L}R^{M}, C(=S)OR^{K}, C(=S) SR^{K}, C(=NR^{L})R^{M}, C(=NR^{L})NR^{M}R^{R}, Si(R^{S})₂R^{T} ;
chaque R^{K} étant indépendamment H ;
C₁-C₆-alkyle, C₂-C₆-alcényle, C₂-C₆-alcynyle, C₁-C₆-alcoxy-C₁-C₄-alkyle, C₃-C₆-cycloalkyle, C₃-C₆-cycloalkyl-C₁-C₄-alkyle, C₃-C₆-cycloalcoxy-C₁-C₄-alkyle, lesquels groupes étant non substitués ou substitués par un ou plusieurs substituants identiques ou différents choisis parmi halogène, CN, NR^{M}R^{N}, C(=O)NR^{M}R^{N}, C(=O)R^{T} ; ou
phényle ou benzyle, le cycle phényle étant non substitué ou substitué par un ou plusieurs substituants R^{x} identiques ou différents ;
chaque R^{L} étant indépendamment H ;
C₁-C₆-alkyle, C₂-C₆-alcényle, C₂-C₆-alcynyle, C₁-C₆-alcoxy-C₁-C₄-alkyle, C₃-C₆-cycloalkyle, C₃-C₆-cycloalkyl-C₁-C₄-alkyle, C₃-C₆-cycloalcoxy-C₁-C₄-alkyle, lesquels groupes étant non substitués ou substitués par halogène ;
C₁-C₆-alkylène-CN ;
phényle et benzyle, lesquels groupes étant non substitués ou substitués par un ou plusieurs substituants R^{x} identiques ou différents ;
chaque R^{M}, R^{R} étant indépendamment H ;
C₁-C₆-alkyle, C₂-C₆-alcényle, C₂-C₆-alcynyle, C₁-C₆-alcoxy-C₁-C₄-alkyle, C₃-C₆-cycloalkyle, C₃-C₆-cycloalkyl-C₁-C₄-alkyle, C₃-C₆-cycloalcoxy-C₁-C₄-alkyle, lesquels groupes étant non substitués ou substitués par halogène ;
C₁-C₆-alkylène-CN ; ou
phényle ou benzyle, le cycle phényle étant non substitué ou substitué par un ou plusieurs substituants R^{x} identiques ou différents ;
chaque groupement NR^{M}R^{R} ou NR^{L}R^{M} pouvant également former un hétérocycle à 5 à 8 chaînons saturé, lié à N, qui en plus de l'atome d'azote peut avoir 1 ou 2 hétéroatomes ou groupements d'hétéroatomes supplémentaires choisis parmi O, S(=O)ₘ et N-R', R' étant H ou C₁-C₆-alkyle et l'hétérocycle lié à N étant non substitué ou substitué par un ou plusieurs substituants identiques ou différents choisis parmi halogène, C₁-C₄-alkyle, C₁-C₄-halogénoalkyle, C₁-C₄-alcoxy et C₁-C₄-halogénoalcoxy ;
chaque R^{N} étant indépendamment H, halogène, CN, NO₂, SCN ; C₁-C₁₀-alkyle, C₃-C₈-cycloalkyle, C₂-C₆-alcényle, C₃-C₆-cycloalcényle, C₂-C₆-alcynyle, lesquels groupes étant non substitués, ou substitués par un ou plusieurs substituants identiques ou différents choisis parmi halogène, C₁-C₆-alkyle, C₁-C₆-alcoxy, C₁-C₆-halogénoalkyle, et C₁-C₆-halogénoalcoxy ;
un cycle ou système cyclique hétérocyclique saturé, partiellement insaturé ou totalement insaturé à 3 à 12 chaînons, ledit cycle ou système cyclique hétérocyclique comprenant un ou plusieurs hétéroatomes identiques ou différents O, N, ou S, et étant non substitué, ou substitué par un ou plusieurs substituants identiques ou différents choisis parmi halogène, C₁-C₃-alkyle, C₁-C₃-alcoxy, C₁-C₃-halogénoalkyle, et C₁-C₃-halogénoalcoxy, et lesdits atomes de N et S étant indépendamment oxydés, ou non oxydés ;
phényle, qui est non substitué, ou substitué par un ou plusieurs substituants identiques ou différents choisis parmi halogène, C₁-C₃-alkyle, C₁-C₃-alcoxy, C₁-C₃-halogénoalkyle, et C₁-C₃-halogénoalcoxy ;
chaque R^{O} étant indépendamment H, C₁-C₄-alkyle, C₁-C₆-cycloalkyle, C₁-C₂-alcoxy-C₁-C₂-alkyle, phényle, ou benzyle ;
chaque R^{P} étant indépendamment H ;
C₁-C₆-alkyle, C₂-C₆-alcényle, C₂-C₆-alcynyle, C₁-C₆-alcoxy-C₁-C₄-alkyle, C₃-C₆-cycloalkyle, C₃-C₆-cycloalkyl-C₁-C₄-alkyle, C₃-C₆-cycloalcoxy-C₁-C₄-alkyle, lesquels groupes étant non substitués ou substitués par halogène ;
phényle ou benzyle, le cycle phényle étant non substitué ou substitué par un ou plusieurs substituants Rx identiques ou différents ;
chaque R^{S}, R^{T} étant indépendamment H, C₁-C₆-alkyle, C₁-C₆-halogénoalkyle, C₁-C₆-alcoxy, C₁-C₄-alcoxy-C₁-C₄-alkyle, C₃-C₆-cycloalkyle, C₃-C₆-halogénocycloalkyle, C₁-C₄-halogénoalcoxy-C₁-C₄-alkyle, ou phényle ;
chaque R^{V} étant indépendamment C₁-C₆-alkyle, C₃-C₆-cycloalkyle, C₃-C₆-cycloalkyl-C₁-C₄-alkyle, qui sont non substitués ou substitués par halogène ; ou
phényle ou benzyle, le cycle phényle étant non substitué ou substitué par R^{x} ;
chaque R^{W} étant C₁-C₆-alkyle, C₃-C₆-cycloalkyle, C₃-C₆-cycloalkyl-C₁-C₄-alkyle, qui sont non substitués ou substitués par halogène ; ou
phényle ou benzyle, le cycle phényle étant non substitué ou substitué par un ou plusieurs substituants Rx identiques ou différents ;
chaque R^{X} étant indépendamment halogène, N₃, OH, CN, NO₂, SCN, SF₅ ;
C₁-C₆ alkyle, C₁-C₆ alcoxy, C₂-C₆ alcényle, C₂-C₆ alcynyle, C₁-C₆ alcoxy-C₁-C₄ alkyle, C₁-C₆ alcoxy-C₁-C₄ alcoxy, C₃-C₆ cycloalkyle, C₃-C₆ cycloalcoxy, C₃-C₆ cycloalkyl-C₁-C₄ alkyle, C₃-C₆ cycloalcoxy-C₁-C₄ alkyle, lesquels groupes étant non substitués ou substitués par halogène ;
m étant 0, 1 ou 2 ;
n étant 0, 1, 2, ou 3 ;
et sels acceptables sur le plan agricole ou sur le plan vétérinaire, N-oxydes, stéréoisomères et formes tautomères correspondant(e)s.

2. Composé de formule (I) selon la revendication 1,
R¹ étant H ;
C₁-C₃-alkyle, C₁-C₃-alcoxy, lesquels groupes étant non substitués ou halogénés ;
R² étant H, halogène ;
C₁-C₃-alkyle, C₁-C₃-alcoxy, C₂-C₃-alcényle, C₂-C₃-alcynyle, lesquels groupes étant non substitués ou halogénés.

3. Composé de formule (I) selon la revendication 1 ou 2, le composé de formule (I) étant un composé de formule (I.A), (I.B) ou (I.C) ; 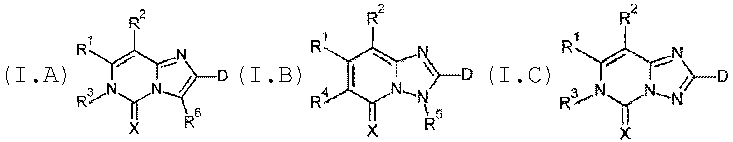 toutes les variables ayant une signification telle que définie pour la formule (I).

4. Composé de formule (I-A) selon la revendication 3, R³ étant C₁-C₄ -alkyle, C₂-C₄ alcényle, C₂-C₄ alcynyle, C₁-C₃-alcoxy-C₁-C₃-alkyle, C₃-C₆ cycloalkyle, C₃-C₆ cycloalkyl-C₁-C₂-alkyle, lesquels groupes étant non substitués ou halogénés ;
phényle ou benzyle, dans lesquels groupes le cycle phényle étant non substitué ou substitué par R^{F} ;
R⁶ étant H ; ou
C₁-C₃-alkyle ou C₁-C₃-halogénoalkyle.

5. Composé de formule (I-B) selon la revendication 3 ou 4,
R⁴ étant H ; ou
C₁-C₃ alkyle, ou C₁-C₃-halogénoalkyle ;
R⁵ étant C₁-C₃-alkyle, ou C₁-C₃-halogénoalkyle.

6. Composés de formule (I) selon l'une quelconque des revendications 1 à 5, la variable D représentant un cycle bicyclique condensé des formules suivantes (D1), (D3), (D8), ou (D51) 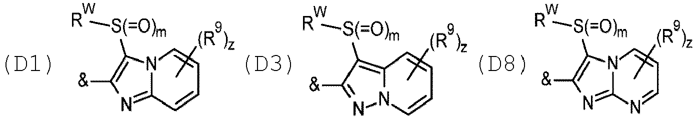 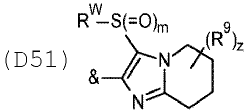 l'indice z étant 0, 1, 2, 3, ou 4 pour (D1), (D3), (D51), ou z étant 0, 1, 2, ou 3 pour (D8), et les autres variables étant définies comme pour la formule (I), et le symbole « & » signifiant la connexion au reste de la formule (I).

7. Composé de formule (I) selon l'une quelconque des revendications 1 à 6, chaque R⁹ étant indépendamment choisi parmi H, halogène ; et
C₁-C₃-alkyle, C₂-C₃-alcényle, et C₂-C₃-alcynyle, lesquels groupes étant non substitués ou halogénés.

8. Composé de formule (I) selon l'une quelconque des revendications 1 à 7,
R^{W} étant C₁-C₃-alkyle ou C₁-C₃-halogénoalkyle, et
m étant 0 ou 2.

9. Mélange pesticide comprenant le composé de formule (I), tel que défini dans l'une des revendications 1 à 7, un N-oxyde, ou un sel acceptable sur le plan agricole correspondant et un ingrédient pesticide supplémentaire.

10. Utilisation de composés de formule (I) tels que définis dans l'une quelconque des revendications 1 à 8 comme pesticide agrochimique.

11. Procédé non thérapeutique pour la lutte contre ou la régulation d'organismes nuisibles invertébrés, lequel procédé comprenant la mise en contact dudit organisme nuisible ou de son approvisionnement en nourriture, son habitat ou ses lieux de reproduction avec une quantité efficace sur le plan pesticide d'au moins un composé de la formule (I) selon l'une quelconque des revendications 1 à 8 ou de la composition selon la revendication 9.

12. Procédé de protection de végétaux en croissance contre une attaque ou une infestation par des organismes nuisibles invertébrés, lequel procédé comprenant la mise en contact d'un végétal, ou du sol ou de l'eau dans lequel/laquelle le végétal pousse, avec une quantité efficace sur le plan pesticide d'au moins un composé de la formule (I) selon l'une quelconque des revendications 1 à 8 ou de la composition selon la revendication 9.

13. Graine comprenant un composé de la formule (I), tel que défini dans l'une quelconque des revendications 1 à 8, ou la composition, telle que définie dans la revendication 9, en une quantité allant de 0,1 g à 10 kg pour 100 kg de graines.

14. Utilisation d'un composé de la formule (I), tel que défini dans l'une quelconque des revendications 1 à 8, ou des compositions telles que définies dans la revendication 9, pour protéger des végétaux en croissance contre une attaque ou une infestation par des organismes nuisibles invertébrés.

15. Procédé non thérapeutique pour le traitement ou la protection d'un animal contre une infestation ou une infection par des organismes nuisibles invertébrés qui comprend la mise en contact de l'animal avec une quantité efficace sur le plan pesticide d'un composé de la formule (I) tel que défini dans l'une quelconque des revendications 1 à 8, ou d'une composition telle que définie dans la revendication 9.
